# EUROPEAN PATENT SPECIFICATION

(11) **EP 1 049 699 B1**
(45) Date of publication and mention of the grant of the patent: **21.04.2004**
(21) Application number: 99904382.1
(22) Date of filing: 28.01.1999
(51) Int. Cl.: C07D 487/04, A61K 31/495

(54) **PYRAZOLOTRIAZINES AS CRF ANTAGONISTS**
PYRAZOLOTRIAZINE ALS CRF ANTAGONISTEN
PYRAZOLOTRIAZINES COMME ANTAGONISTES DU CRF

(30) Priority: 28.01.1998 US 15002; 28.01.1998 US 14734; 28.01.1998 US 15001
(43) Date of publication of application: 08.11.2000
(60) Divisional application: 03075887.4
(73) Proprietor: Bristol-Myers Squibb Pharma Company, Princeton, New Jersey 08443-4000 (US)
(72) Inventor: HE, Liqi, West Chester, PA 19380 (US); GILLIGAN, Paul, Wilmington, DE 19810 (US); CHORVAT, Robert, West Chester, PA 19382 (US); ARVANITIS, Argyrios, Georgios, Kennett Square, PA 19348 (US)
(74) Representative: Carpmaels & Ransford
(86) International application number: PCT/US1999/001824
(87) International publication number: WO 1999/038868

(56) References cited:
- WO-A-95/33750
- WO-A-96/35689
- WO-A-97/29109
- WO-A-98/03510

## Description

### FIELD OF THE INVENTION

This invention relates a treatment of psychiatric disorders and neurological diseases including major depression, anxiety-related disorders, post-traumatic stress disorder, supranuclear palsy and feeding disorders as well as treatment of immunological, cardiovascular or heart-related diseases and colonic hypersensitivity associated with psychopathological disturbance and stress, by administration of certain [1,5-a]-pyrazolo-1,3,5-triazines.

### BACKGROUND OF THE INVENTION

Corticotropin releasing factor (herein referred to as CRF), a 41 amino acid peptide, is the primary physiological regulator of propiomelanocortin (POMC) - derived peptide secretion from the anterior pituitary gland [J. Rivier et al., *Proc*. *Nat*. *Acad*. *Sci*. *(USA)* 80:4851 (**1983**); W. Vale et al., *Science* 213:1394 (**1981**)]. In addition to its endocrine role at the pituitary gland, immunohistochemical localization of CRF has demonstrated that the hormone has a broad extrahypothalamic distribution in the central nervous system and produces a wide spectrum of autonomic, electrophysiological and behavioral effects consistent with a neurotransmitter or neuromodulator role in brain [W. Vale et al., *Rec. Prog. Horm. Res*. 39:245 (1983); G.F. Koob, *Persp. Behav. Med.* 2:39 (**1985**); E.B. De Souza et al., *J. Neurosci.* 5:3189 (**1985**)]. There is also evidence that CRF plays a significant role in integrating the response of the immune system to physiological, psychological, and immunological stressors [J.E. Blalock, *Physiological Reviews* 69:1 (**1989**); [J.E. Morley, *Life Sci.* 41:527 (**1987**)].

Clinical data provide evidence that CRF has a role in psychiatric disorders and neurological diseases including depression, anxiety-related disorders and feeding disorders. A role for CRF has also been postulated in the etiology and pathophysiology of Alzheimer's disease, Parkinson's disease, Huntington's disease, progressive supranuclear palsy and amyotrophic lateral sclerosis as they relate to the dysfunction of CRF neurons in the central nervous system [for review see E.B. De Souza, *Hosp*. *Practice* 23:59 (**1988**)].

In affective disorder, or major depression, the concentration of CRF is significantly increased in the cerebral spinal fluid (CSF) of drug-free individuals [C.B. Nemeroff et al., *Science* 226:1342 (**1984**); C.M. Banki et al., *Am. J. Psychiatry* 144:873 (**1987**); R.D. France et al., *Biol. Psychiatry* 28:86 (**1988**); M. Arato et al., *Biol Psychiatry* 25:355 (**1989**)]. Furthermore, the density of CRF receptors is significantly decreased in the frontal cortex of suicide victims, consistent with a hypersecretion of CRF [C.B. Nemeroff et al., *Arch. Gen. Psychiatry* 45:577 (**1988**)]. In addition, there is a blunted adrenocorticotropin (ACTH) response to CRF (i.v. administered) observed in depressed patients [P.W. Gold et al., *Am J. Psychiatry* 141:619 (**1984**); F. Holsboer et al., *Psychoneuroendocrinology* 9:147 (**1984**); P.W. Gold et al., *New Eng. J. Med.* 314:1129 (**1986**)]. Preclinical studies in rats and non-human primates provide additional support for the hypothesis that hypersecretion of CRF may be involved in the symptoms seen in human depression [R.M. Sapolsky, *Arch. Gen. Psychiatry* 46:1047 (**1989**)]. There is preliminary evidence that tricyclic antidepressants can alter CRF levels and thus modulate the numbers of CRF receptors in brain [Grigoriadis et al., *Neuropsychopharmacology* 2:53 (**1989**)].

There has also been a role postulated for CRF in the etiology of anxiety-related disorders. CRF produces anxiogenic effects in animals and interactions between benzodiazepine / non-benzodiazepine anxiolytics and CRF have been demonstrated in a variety of behavioral anxiety models [D.R. Britton et al., *Life Sci.* 31:363 (**1982**); C.W. Berridge and A.J. Dunn *Regul*. *Peptides* 16:83 (**1986**)]. Preliminary studies using the putative CRF receptor antagonist a-helical ovine CRF (9-41) in a variety of behavioral paradigms demonstrate that the antagonist produces "anxiolytic-like" effects that are qualitatively similar to the benzodiazepines [C.W. Berridge and A.J. Dunn *Horm. Behav.* 21:393 (**1987**), *Brain Research Reviews* 15:71 (**1990**)]. Neurochemical, endocrine and receptor binding studies have all demonstrated interactions between CRF and benzodiazepine anxiolytics providing further evidence for the involvement of CRF in these disorders. Chlordiazepoxide attenuates the "anxiogenic" effects of CRF in both the conflict test [K.T. Britton et al., *Psychopharmacology* 86:170 (**1985**); K.T. Britton et al., *Psychopharmacology* 94:306 (**1988**)] and in the acoustic startle test [N.R. Swerdlow et al., *Psychopharmacology* 88:147 (**1986**)] in rats. The benzodiazepine receptor antagonist (Ro15-1788), which was without behavioral activity alone in the operant conflict test, reversed the effects of CRF in a dose-dependent manner while the benzodiazepine inverse agonist (FG7142) enhanced the actions of CRF [K.T. Britton et al., *Psychopharmacology* 94:306 (**1988**)].

The mechanisms and sites of action through which the standard anxiolytics and antidepressants produce their therapeutic effects remain to be elucidated. It has been hypothesized however, that they are involved in the suppression of the CRF hypersecretion that is observed in these disorders. Of particular interest is that preliminary studies examining the effects of a CRF receptor antagonist (α-helical CRF₉₋₄₁) in a variety of behavioral paradigms have demonstrated that the CRF antagonist produces "anxiolytic-like" effects qualitatively similar to the benzodiazepines (for review see G.F. Koob and K.T. Britton, In: *Corticotropin-Releasing Factor: Basic and Clinical Studies* of a *Neuropeptide*, E.B. De Souza and C.B. Nemeroff eds., CRC Press p221 (1990)].

Several publications describe corticotropin releasing factor antagonist compounds and their use to treat psychiatric disorders and neurological diseases. Examples of such publications include DuPont Merck PCT application US94/11050 , Pfizer WO 95/33750, Pfizer WO 95/34563, Pfizer WO 95/33727 and Pfizer EP 0778 277 A1.

Insofar as is known, [1,5-a]-pyrazolo-1,3,5-triazines have not been previously reported as corticotropin releasing factor antagonist compounds useful in the treatment of psychiatric disorders and neurological diseases. However, there have been publications which teach some of these compounds for other uses.

For instance, EP 0 269 859 (Ostuka, 1988) discloses pyrazolotriazine compounds of the formula 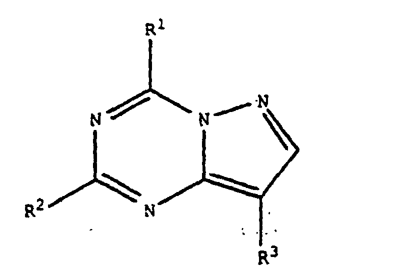 where R¹ is OH or alkanoyl, R² is H, OH, or SH, and R³ is an unsaturated heterocyclic group, naphthyl or substituted phenyl, and states that the compounds have xanthine oxidase inhibitory activity and are useful for treatment of gout.

EP 0 594 149 (Ostuka, 1994) discloses pyrazolotriazine and pyrazolopyrimidine compounds of the formula 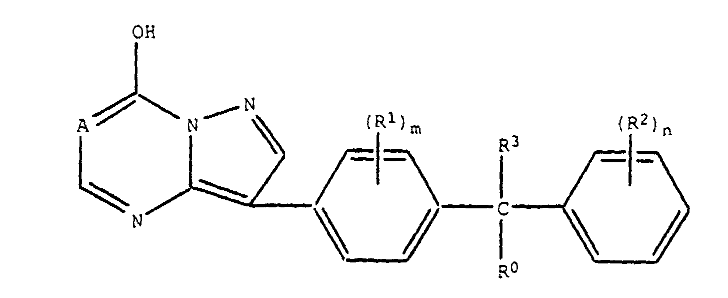 where A is CH or N, R⁰ and R³ are H or alkyl, and R¹ and R² are H, alkyl, alkoxyl, alkylthio, nitro, etc., and states that the compounds inhibit androgen and are useful in treatment of benign prostatic hypertrophy and prostatic carcinoma.

US 3,910,907 (ICI, 1975) discloses pyrazolotriazines of the formula: 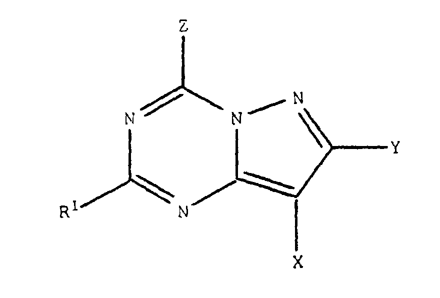 where R1 is CH₃, C₂H₅ or C₆H₅, X is H, C₆H₅, m-CH₃C₆H₄, CN, COOEt, Cl, I or Br, Y is H, C₆H₅, o-CH₃C₆H₄, or p-CH₃C₆H₄, and Z is OH, H, CH₃, C₂H₅, C₆H₅, n-C₃H₇, i-C₃H₇, SH, SCH₃, NHC₄H₉, or N(C₂H₅)₂, and states that the compounds are c-AMP phosphodiesterase inhibitors useful as bronchodilators.

US 3,995,039 discloses pyrazolotriazines of the formula: 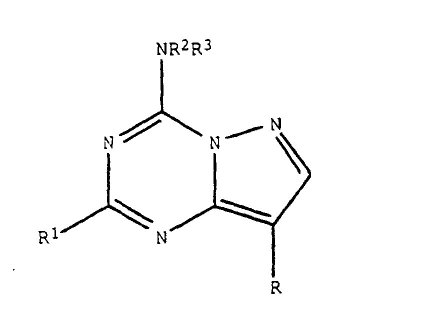 where R¹ is H or alkyl, R² is H or alkyl, R³ is H, alkyl, alkanoyl, carbamoyl, or lower alkylcarbamoyl, and R is pyridyl, pyrimidinyl, or pyrazinyl, and states that the compounds are useful as bronchodilators.

US 5,137,887 discloses pyrazolotriazines of the formula 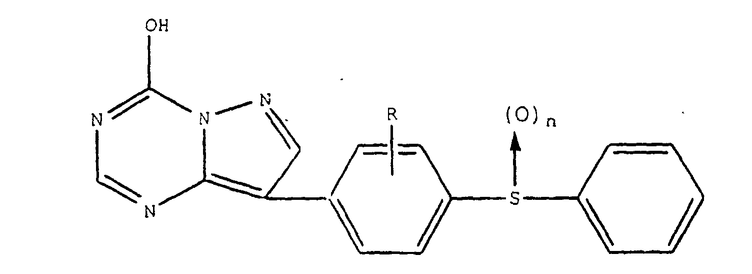 where R is lower alkoxy, and teaches that the compounds are xanthine oxidase inhibitors and are useful for treatment of gout.

US 4,892,576 discloses pyrazolotriazines of the formula 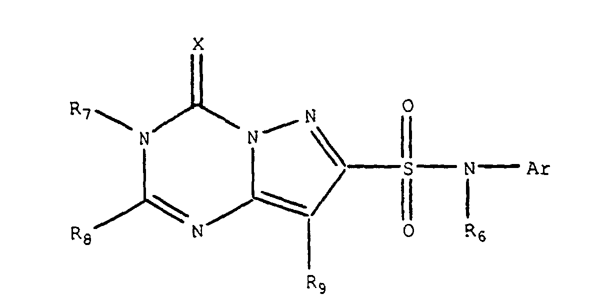 where X is O or S, Ar is a phenyl, naphthyl, pyridyl or thienyl group, R₆-R₈ are H, alkyl, etc., and R₉ is H, alkyl, phenyl, etc. The patent states that the compounds are useful as herbicides and plant growth regulants.

US 5,484,760 and WO 92/10098 discloses herbicidal compositions containing, among other things, a herbicidal compound of the formula 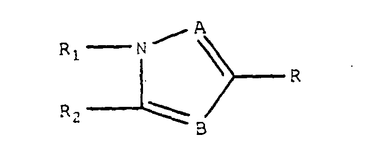 where A can be N, B can be CR₃, R₃ can be phenyl or substituted phenyl, etc., R is -N(R₄)SO₂R₅ or-SO₂N(R₆)R₇ and R₁ and R₂ can be taken together to form 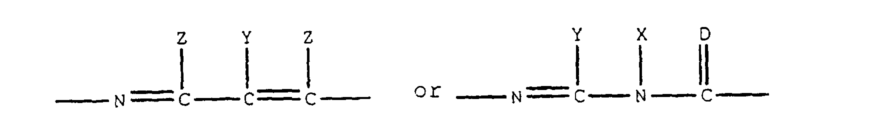 where X, Y and Z are H, alkyl, acyl, etc. and D is O or S.

US 3,910,907 and Senga et al., J. Med. Chem. , 1982, 25, 243-249, disclose triazolotriazines cAMP phosphodiesterase inhibitors of the formula 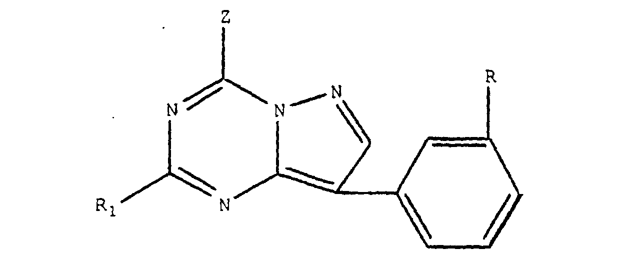 where Z is H, OH, CH₃, C₂H₅, C₆H₅, n-C₃H₇, iso-C₃H₇, SH, SCH₃, NH(n-C₄H₉), or N(C₂H₅)₂, R is H or CH₃, and R₁ is CH₃ or C₂H₅. The reference lists eight therapeutic areas where inhibitors of cAMP phosphodiesterase could have utility: asthma, diabetes mellitus, female fertility control, male infertility, psoriasis, thrombosis, anxiety, and hypertension.

WO95/35298 (Otsuka, 1995) discloses pyrazolopyrimidines and states that they are useful as analgesics. The compounds are represented by the formula 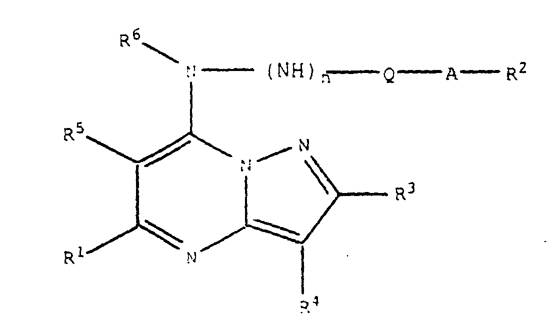 where Q is carbonyl or sulfonyl, n is 0 or 1, A is a single bond, alkylene or alkenylene, R¹ is H, alkyl, etc., R² is naphthyl, cycloalkyl, heteroaryl, substituted phenyl or phenoxy, R³ is H, alkyl or phenyl, R⁴ is H, alkyl, alkoxycarbonyl, phenylalkyl, optionally phenylthio-substituted phenyl, or halogen, R⁵ and R⁶ are H or alkyl.

EP 0 591 528 (Otsuka,1991) discloses antiinflammatory use of pyrazolopyrimidines represented by the formula 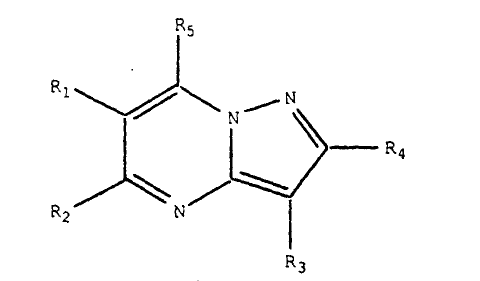 where R₁, R₂, R₃ and R₄ are H, carboxyl, alkoxycarbonyl, optionally substituted alkyl, cycloalkyl, or phenyl, R₅ is SR₆ or NR₇R₈, R₆ is pyridyl or optionally substituted phenyl, and R₇ and R₈ are H or optionally substituted phenyl.

Springer et al, J. Med. Chem., 1976, vol. 19, no. 2, 291-296 and Springer U.S. patents 4021,556 and 3,920,652 disclose pyrazolopyrimidines of the formula 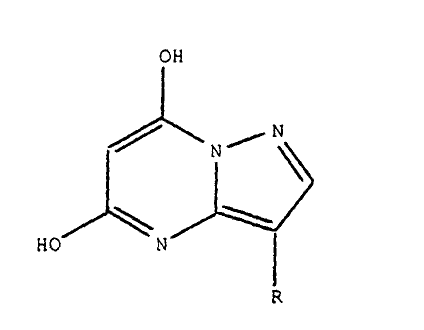 where R can be phenyl, substituted phenyl or pyridyl, and their use to treat gout, based on their ability to inhibit xanthine oxidase.

Joshi et al., J. Prakt. Chemie, 321, 2, 1979, 341-344, discloses compounds of the formula 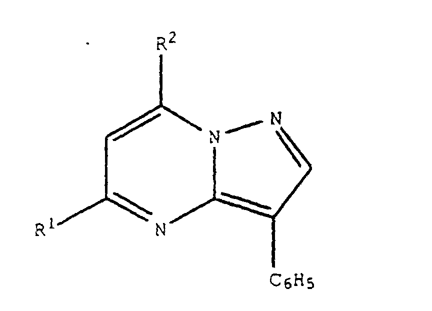 where R¹ is CF₃, C₂F₅, or C₆H₄F, and R² is CH₃, C₂H₅, CF₃, or C₆H₄F.

Maquestiau et al., Bull. Soc. Belg., vol.101, no. 2, 1992, pages 131-136 discloses a pyrazolo[1,5-a]pyrimidine of the formula

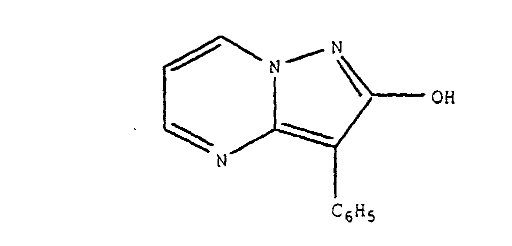

Ibrahim et al., Arch. Pharm. (weinheim) 320, 487.-491 (1987) discloses pyrazolo[1,5-a]pyrimidines of the formula 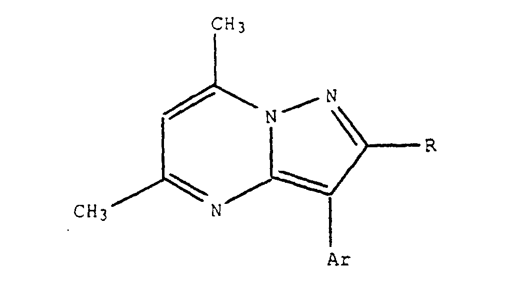 where R is NH2 or OH and Ar is 4-phenyl-3-cyano-2-aminopyrid-2-yl.

Other references which disclose azolopyrimidines inclued EP 0 511 528 (Otsuka, 1992), US 4,997,940 (Dow, 1991), EP 0 374 448 (Nissan, 1990), US 4,621,556 (ICN, 1997), EP 0 531 901 (Fujisawa, 1993), US 4,567,263 (BASF, 1986), EP 0 662 477 (Isagro, 1995), DE 4 243 279 (Bayer, 1994), US 5,397,774 (Upjohn, 1995), EP 0 521 622 (Upjohn, 1993), WO 94/109017 (Upjohn, 1994), J. Med. Chem., 24, 610-613 (1981), and J. Het. Chem., 22, 601 (1985).

### SUMMARY OF THE INVENTION

In accordance with one aspect, the present invention provides novel compounds, pharmaceutical compositions and methods which may be used in the treatment of affective disorder, anxiety, depression, irritable bowel syndrome, post-traumatic stress disorder, supranuclear palsy, immune suppression, Alzheimer's disease, gastrointestinal disease, anorexia nervosa or other feeding disorder, drug or alcohol withdrawal symptoms, drug addiction, inflammatory disorder, fertility problems, disorders, the treatment of which can be effected or facilitated by antagonizing CRF, including but not limited to disorders induced or facilitated by CRF, or a disorder selected from inflammatory disorders such as rheumatoid arthritis and osteoarthritis, pain, asthma, psoriasis and allergies; generalized anxiety disorder; panic, phobias, obsessive-compulsive disorder; post-traumatic stress disorder; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer, human immunodeficiency virus (HIV) infections; neurodegenerative diseases such as Alzheimer's disease, Parkinson's disease and Huntington's disease; gastrointestinal diseases such as ulcers, irritable bowel syndrome, Crohn's disease, spastic colon, diarrhea, and post operative ilius and colonic hypersensitivity associated by psychopathological disturbances or stress; eating disorders such as anorexia and bulimia nervosa; hemorrhagic stress; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; cardiovascular and hear related disorders including hypertension, tachycardia and congestive heart failure; stroke; immune dysfunctions including stress induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependencies on alcohol, cocaine, heroin, benzodiazepines, or other drugs); drug and alcohol withdrawal symptoms; osteoporosis; psychosocial dwarfism and hypoglycemia in a mammal.

The present invention provides novel compounds which bind to corticotropin releasing factor receptors, thereby altering the anxiogenic effects of CRF secretion. The compounds of the present invention are useful for the treatment of psychiatric disorders and neurological diseases, anxiety-related disorders, post-traumatic stress disorder, supranuclear palsy and feeding disorders as well as treatment of immunological, cardiovascular or heart-related diseases and colonic hypersensitivity associated with psychopathological disturbance and stress in a mammal.

According to another aspect, the present invention provides novel compounds described below which are useful as antagonists of the corticotropin releasing factor. The compounds of the present invention exhibit activity as corticotropin releasing factor antagonists and appear to suppress CRF hypersecretion. The present invention also includes pharmaceutical compositions containing such compounds within Formula (1), and methods of using such compounds for the suppression of CRF hypersecretion, and/or for the treatment of anxiogenic disorders.

According to yet another aspect of the invention, the compounds provided by this invention (and especially labelled compounds of this invention) are also useful as standards and reagents in determining the ability of a potential pharmaceutical to bind to the CRF receptor.

### DETAILED DESCRIPTION OF INVENTION

[1] The present invention provides compounds of Formula (50) 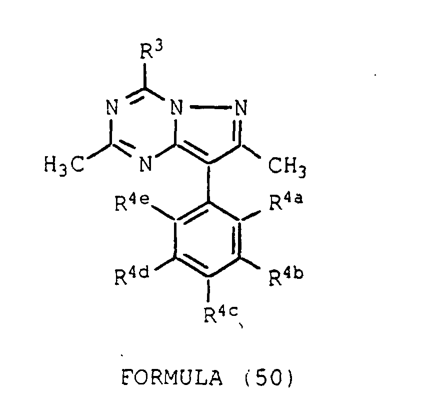 and geometric isomers thereof, stereoisomeric forms thereof, or mixtures of stereoisomeric forms thereof, and pharmaceutically acceptable salt forms thereof, selected from the group:
   a compound of Formula (50) wherein R³ is -NHCH(CH₂CH₂OMe)(CH₂OMe), R^{4a} is Me, R^{4b} is H, R^{4c} is Me, R^{4d} is H and R^{4e} is Me;
   a compound of Formula (50) wherein R³ is 2-ethylpiperid-1-yl, R^{4a} is Me, R^{4b} is H, R^{4c} is OMe, R^{4d} is H and R^{4e} is H;
   a compound of Formula (50) wherein R³ is cyclobutylamino, R^{4a} is Me, R^{4b} is H, R^{4c} is OMe, R^{4d} is H and R^{4e} is H;
   a compound of Formula (50) wherein R³ is N(Me)CH₂CH=CH₂, R^{4a} is Me, R^{4b} is H, R^{4c} is OMe, R^{4d} is H and R^{4e} is H;
   a compound of Formula (50) wherein R³ is N(Et)CH₂CH=CH₂, R^{4a} is Me, R^{4b} is H, R^{4c} is OMe, R^{4d} is H and R^{4e} is H;
   a compound of Formula (50) wherein R³ is N(Me)CH₂cPr, R^{4a} is Me, R^{4b} is H, R^{4c} is OMe, R^{4d} is H and R^{4e} is H;
   a compound of Formula (50) wherein R³ is N(Et)CH₂cPr, R^{4a} is Me, R^{4b} is H, R^{4c} is OMe, R^{4d} is H and R^{4e} is H;
   a compound of Formula (50) wherein R³ is N(Pr)CH₂cPr, R^{4a} is Me, R^{4b} is H, R^{4c} is OMe, R^{4d} is H and R^{4e} is H;
   a compound of Formula (50) wherein R³ is N(Me)Pr, R^{4a} is Me, R^{4b} is H, R^{4c} is OMe, R^{4d} is H and R^{4e} is H;
   a compound of Formula (50) wherein R³ is N(Me)Et, R^{4a} is Me, R^{4b} is H, R^{4c} is OMe, R^{4d} is H and R^{4e} is H;
   a compound of Formula (50) wherein R³ is N(Me)Bu, R^{4a} is Me, R^{4b} is H, R^{4c} is OMe, R^{4d} is H and R^{4e} is H;
   a compound of Formula (50) wherein R³ is N(Me)propargyl, R^{4a} is Me, R^{4b} is H, R^{4c} is OMe, R^{4d} is H and R^{4e} is H;
   a compound of Formula (50) wherein R³ is N(Et)propargyl, R^{4a} is Me, R^{4b} is H, R^{4c} is OMe, R^{4d} is H and R^{4e} is H;
   a compound of Formula (50) wherein R³ is NHCH(CH₃)CH(CH₃)CH₃, R^{4a} is Me, R^{4b} is H, R^{4c} is OMe, R^{4d} is H and R^{4e} is H;
   a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is H;
   a compound of Formula (50) wherein R³ is N(CH₂CH₂OMe)Me, R^{4a} is Me, R^{4b} is H, R^{4c} is OMe, R^{4d} is H and R^{4e} is H;
   a compound of Formula (50) wherein R³ is N(CH₂CH₂OMe)Et, R^{4a} is Me, R^{4b} is H, R^{4c} is OMe, R^{4d} is H and R^{4e} is H;
   a compound of Formula (50) wherein R³ is N(CH₂CH₂OMe)Pr, R^{4a} is Me, R^{4b} is H, R^{4c} is OMe, R^{4d} is H and R^{4e} is H;
   a compound of Formula (50) wherein R³ is N(CH₂CH₂OMe)-CH₂cPr, R^{4a} is Me, R^{4b} is H, R^{4c} is OMe, R^{4d} is H and R^{4e} is H;
   a compound of Formula (50) wherein R³ is NHCH(CH₃)CH₂CH₃, R^{4a} is Me, R^{4b} is H, R^{4c} is OMe, R^{4d} is H and R^{4e} is H;
   a compound of Formula (50) wherein R³ is NHCH(cPr)₂, R^{4a} is Me, R^{4b} is H, R^{4c} is OMe, R^{4d} is H and R^{4e} is H;
   a compound of Formula (50) wherein R³ is 2-ethylpiperid-1-yl, R^{4a} is Me, R^{4b} is H, R^{4c} is OMe, R^{4d} is Me and R^{4e} is H;
   a compound of Formula (50) wherein R³ is cyclobutylamino, R^{4a} is Me, R^{4b} is H, R^{4c} is OMe, R^{4d} is Me and R^{4e} is H;
   a compound of Formula (50) wherein R³ is N(Me)CH₂CH=CH₂, R^{4a} is Me, R^{4b} is H, R^{4c} is OMe, R^{4d} is Me and R^{4e} is H;
   a compound of Formula (50) wherein R³ is N(Et)CH₂CH=CH₂, R^{4a} is Me, R^{4b} is H, R^{4c} is OMe, R^{4d} is Me and R^{4e} is H;
   a compound of Formula (50) wherein R³ is N(Me)CH₂cPr, R^{4a} is Me, R^{4b} is H, R^{4c} is OMe, R^{4d} is Me and R^{4e} is H;
   a compound of Formula (50) wherein R³ is N(Et)CH₂cPr, R^{4a} is Me, R^{4b} is H, R^{4c} is OMe, R^{4d} is Me and R^{4e} is H;
   a compound of Formula (50) wherein R³ is N(Pr)CH₂cPr, R^{4a} is Me, R^{4b} is H, R^{4c} is OMe, R^{4d} is Me and R^{4e} is H;
   a compound of Formula (50) wherein R³ is N(Me)Pr, R^{4a} is Me, R^{4b} is H, R^{4c} is OMe, R^{4d} is Me and R^{4e} is H;
   a compound of Formula (50) wherein R³ is N(Me)Et, R^{4a} is Me, R^{4b} is H, R^{4c} is OMe, R^{4d} is Me and R^{4e} is H;
   a compound of Formula (50) wherein R³ is N(Me)Bu, R^{4a} is Me, R^{4b} is H, R^{4c} is OMe, R^{4d} is Me and R^{4e} is H;
   a compound of Formula (50) wherein R³ is N(Me)propargyl, R^{4a} is Me, R^{4b} is H, R^{4c} is OMe, R^{4d} is Me and R^{4e} is H;
   a compound of Formula (50) wherein R³ is N(Et)propargyl, R^{4a} is Me, R^{4b} is H, R^{4c} is OMe, R^{4d} is Me and R^{4e} is H;
   a compound of Formula (50) wherein R³ is NHCH(CH₃)CH(CH₃)CH₃, R^{4a} is Me, R^{4b} is H, R^{4c} is OMe, R^{4d} is Me and R^{4e} is H;
   a compound of Formula (50) wherein R³ is N(CH₂CH₂OMe)-CH₂CH=CH₂, R^{4a} is Me, R^{4b} is H, R^{4c} is OMe, R^{4d} is Me and R^{4e} is H;
   a compound of Formula (50) wherein R³ is N(CH₂CH₂OMe)Me, R^{4a} is Me, R^{4b} is H, R^{4c} is OMe, R^{4d} is Me and R^{4e} is H;
   a compound of Formula (50) wherein R³ is N(CH₂CH₂OMe)Et, R^{4a} is Me, R^{4b} is H, R^{4c} is OMe, R^{4d} is Me and R^{4e} is H;.
   a compound of Formula (50) wherein R³ is N(CH₂CH₂OMe)Pr, R^{4a} is Me, R^{4b} is H, R^{4c} is OMe, R^{4d} is Me and R^{4e} is H;
   a compound of Formula (50) wherein R³ is N(CH₂CH₂OMe)-CH₂cPr, R^{4a} is Me, R^{4b} is H, R^{4c} is OMe, R^{4d} is Me and R^{4e} is H;
   a compound of Formula (50) wherein R³ is NHCH(CH₃)CH₂CH₃, R^{4a} is Me, R^{4b} is H, R^{4c} is OMe, R^{4d} is Me and R^{4e} is H;
   a compound of Formula (50) wherein R³ is NHCH(cPr)₂, R^{4a} is Me, R^{4b} is H, R^{4c} is OMe, R^{4d} is Me and R^{4e} is H;
   a compound of Formula (50) wherein R³ is -NHCH(Et)₂, R^{4a} is OMe, R^{4b} is H, R^{4c} is OMe, R^{4d} is H and R^{4e} is H;
   a compound of Formula (50) wherein R³ is 2-ethylpiperid-1-yl, R^{4a} is OMe, R^{4b} is H, R^{4c} is OMe, R^{4d} is H and R^{4e} is H;
   a compound of Formula (50) wherein R³ is cyclobutylamino, R^{4a} is OMe, R^{4b} is H, R^{4c} is OMe, R^{4d} is H and R^{4e} is H;
   a compound of Formula (50) wherein R³ is N(Me)CH₂CH=CH₂, R^{4a} is OMe, R^{4b} is H, R^{4c} is OMe, R^{4d} is H and R^{4e} is H;
   a compound of Formula (50) wherein R³ is N(Et)CH₂CH=CH₂, R^{4a} is OMe, R^{4b} is H, R^{4c} is OMe, R^{4d} is H and R^{4e} is H;
   a compound of Formula (50) wherein R³ is N(Me)CH₂cPr, R^{4a} is OMe, R^{4b} is H, R^{4c} is OMe, R^{4d} is H and R^{4e} is H;
   a compound of Formula (50) wherein R³ is N(Et)CH₂cPr, R^{4a} is OMe, R^{4b} is H, R^{4c} is OMe, R^{4d} is H and R^{4e} is H;
   a compound of Formula (50) wherein R³ is N(Pr)CH₂cPr, R^{4a} is OMe, R^{4b} is H, R^{4c} is OMe, R^{4d} is H and R^{4e} is H;
   a compound of Formula (50) wherein R³ is N(Me)Pr, R^{4a} is OMe, R^{4b} is H, R^{4c} is OMe, R^{4d} is H and R^{4e} is H;
   a compound of Formula (50) wherein R³ is N(Me)Et, R^{4a} is OMe, R^{4b} is H, R^{4c} is OMe, R^{4d} is H and R^{4e} is H;
   a compound of Formula (50) wherein R³ is N(Me)Bu R^{4a} is OMe, R^{4b} is H, R^{4c} is OMe, R^{4d} is H and R^{4e} is H;
   a compound of Formula (50) wherein R³ is N(Me)propargyl, R^{4a} is OMe, R^{4b} is H, R^{4c} is OMe, R^{4d} is H and R^{4e} is H;
   a compound of Formula (50) wherein R³ is N(Et)propargyl, R^{4a} is OMe, R^{4b} is H, R^{4c} is OMe, R^{4d} is H and R^{4e} is H;
   a compound of Formula (50) wherein R³ is NHCH(CH₃)CH(CH₃)CH₃, R^{4a} is OMe, R^{4b} is H, R^{4c} is OMe, R^{4d} is H and R^{4e} is H;
   a compound of Formula (50) wherein R³ is N(CH₂CH₂OMe)-CH₂CH=CH₂, R^{4a} is OMe, R^{4b} is H, R^{4c} is OMe, R^{4d} is H and R^{4e} is H;
   a compound of Formula (50) wherein R³ is N(CH₂CH₂OMe)Me, R^{4a} is OMe, R^{4b} is H; R^{4c} is OMe, R^{4d} is H and R^{4e} is H;
   a compound of Formula (50) wherein R³ is N(CH₂CH₂OMe)Et, R^{4a} is OMe, R^{4b} is H, R^{4c} is OMe, R^{4d} is H and R^{4e} is H;
   a compound of Formula (50) wherein R³ is N(CH₂CH₂OMe)Pr, R^{4a} is OMe, R^{4b} is H, R^{4c} is OMe, R^{4d} is H and R^{4e} is H;
   a compound of Formula (50) wherein R³ is N(CH₂CH₂OMe)-CH₂cPr, R^{4a} is OMe, R^{4b} is H, R^{4c} is OMe, R^{4d} is H and R^{4e} is H;
   a compound of Formula (50) wherein R³ is NHCH(CH₃)CH₂CH₃, R^{4a} is OMe, R^{4b} is H, R^{4c} is OMe, R^{4d} is H and R^{4e} is H;
   a compound of Formula (50) wherein R³ is NHCH(cPr)₂ R^{4a} is OMe, R^{4b} is H, R^{4c} is OMe, R^{4d} is H and R^{4e} is H;
   a compound of Formula (50) wherein R³ is N(CH₂CH₂OMe)₂, R^{4a} is OMe, R^{4b} is H, R^{4c} is OMe, R^{4d} is H and R^{4e} is H;
   a compound of Formula (50) wherein R³ is NHCH(Et)₂, R^{4a} is OMe, R^{4b} is H, R^{4c} is OMe, R^{4d} is Me and R^{4e} is H;
   a compound of Formula (50) wherein R³ is N(Et)₂, R^{4a} is OMe, R^{4b} is H, R^{4c} is OMe, R^{4d} is Me and R^{4e} is H;
   a compound of Formula (50) wherein R³ is 2-ethylpiperid-1-yl, R^{4a} is OMe, R^{4b} is H, R^{4c} is OMe, R^{4d} is Me and R^{4e} is H;
   a compound of Formula (50) wherein R³ is cyclobutylamino, R^{4a} is OMe, R^{4b} is H, R^{4c} is OMe, R^{4d} is Me and R^{4e} is H;
   a compound of Formula (50) wherein R³ is N(Me)CH₂CH=CH₂, R^{4a} is OMe, R^{4b} is H, R^{4c} is OMe, R^{4d} is Me and R^{4e} is H;
   a compound of Formula (50) wherein R³ is N(Et)CH₂CH=CH₂, R^{4a} is OMe, R^{4b} is H, R^{4c} is OMe, R^{4d} is Me and R^{4e} is H;
   a compound of Formula (50) wherein R³ is N(Me)CH₂cPr, R^{4a} is OMe, R^{4b} is H, R^{4c} is OMe, R^{4d} is Me and R^{4e} is H;
   a compound of Formula (50) wherein R³ is N(Et)CH₂cPr, R^{4a} is OMe, R^{4b} is H, R^{4c} is OMe, R^{4d} is Me and R^{4e} is H;
   a compound of Formula (50) wherein R³ is N(Pr)CH₂cPr, R^{4a} is OMe, R^{4b} is H, R^{4c} is OMe, R^{4d} is Me and R^{4e} is H;
   a compound of Formula (50) wherein R³ is N(Me)Pr, R^{4a} is OMe, R^{4b} is H, R^{4c} is OMe, R^{4d} is Me and R^{4e} is H;
   a compound of Formula (50) wherein R³ is N(Me)Et, R^{4a} is OMe, R^{4b} is H, R^{4c} is OMe, R^{4d} is Me and R^{4e} is H;
   a compound of Formula (50) wherein R³ is N(Me)Bu, R^{4a} is OMe, R^{4b} is H, R^{4c} is OMe, R^{4d} is Me and R^{4e} is H;
   a compound of Formula (50) wherein R³ is N(Me)propargyl, R^{4a} is OMe, R^{4b} is H, R^{4c} is OMe, R^{4d} is Me and R^{4e} is H;
   a compound of Formula (50) wherein R³ is N(Et)propargyl, R^{4a} is OMe, R^{4b} is H, R^{4c} is OMe, R^{4d} is Me and R^{4e} is H;
   a compound of Formula (50) wherein R³ is NHCH(CH₃)CH(CH₃)CH₃, R^{4a} is OMe, R^{4b} is H, R^{4c} is OMe, R^{4d} is Me and R^{4e} is H;
   a compound of Formula (50) wherein R³ is N(CH₂CH₂OMe)-CH₂CH=CH₂, R^{4a} is OMe, R^{4b} is H, R^{4c} is OMe, R^{4d} is Me and R^{4e} is H;
   a compound of Formula (50) wherein R³ is N(CH₂CH₂OMe)Me, R^{4a} is OMe, R^{4b} is H, R^{4c} is OMe, R^{4d} is Me and R^{4e} is H;
   a compound of Formula (50) wherein R³ is N(CH₂CH₂OMe)Et, R^{4a} is OMe, R^{4b} is H, R^{4c} is OMe, R^{4d} is Me and R^{4e} is H;
   a compound of Formula (50) wherein R³ is N(CH₂CH₂OMe)Pr, R^{4a} is OMe, R^{4b} is H, R^{4c} is OMe, R^{4d} is Me and R^{4e} is H;
   a compound of Formula (50) wherein R³ is N(CH₂CH₂OMe)-CH₂cPr, R^{4a} is OMe, R^{4b} is H, R^{4c} is OMe, R^{4d} is Me and R^{4e} is H;
   a compound of Formula (50) wherein R³ is NHCH(CH₃)CH₂CH₃, R^{4a} is OMe, R^{4b} is H, R^{4c} is OMe, R^{4d} is Me and R^{4e} is H;
   a compound of Formula (50) wherein R³ is NHCH(cPr)₂, R^{4a} is OMe, R^{4b} is H, R^{4c} is OMe, R^{4d} is Me and R^{4e} is H;
   a compound of Formula (50) wherein R³ is N(CH₂CH₂OMe)₂, R^{4a} is OMe, R^{4b} is H, R^{4c} is OMe, R^{4d} is Me and R^{4e} is H;
   a compound of Formula (50) wherein R³ is NHCH(Et)₂, R^{4a} is OMe, R^{4b} is H, R^{4c} is OMe, R^{4d} is Me and R^{4e} is H;
   a compound of Formula (50) wherein R³ is N(Et)₂, R^{4a} is OMe, R^{4b} is H, R^{4c} is OMe, R^{4d} is Me and R^{4e} is H;
   a compound of Formula (50) wherein R³ is 2-ethylpiperid-1-yl, R^{4a} is Me, R^{4b} is H, R^{4c} is OMe, R^{4d} is H and R^{4e} is Me;
   a compound of Formula (50) wherein R³ is cyclobutylamino, R^{4a} is Me, R^{4b} is H, R^{4c} is OMe, R^{4d} is H and R^{4e} is Me;
   a compound of Formula (50) wherein R³ is N(Me)CH₂CH=CH₂, R^{4a} is Me, R^{4b} is H, R^{4c} is OMe, R^{4d} is H and R^{4e} is Me;
   a compound of Formula (50) wherein R³ is N(Et)CH₂CH=CH₂, R^{4a} is Me, R^{4b} is H, R^{4c} is OMe, R^{4d} is H and R^{4e} is Me;
   a compound of Formula (50) wherein R³ is N(Me)CH₂cPr, R^{4a} is Me, R^{4b} is H, R^{4c} is OMe, R^{4d} is H and R^{4e} is Me;
   a compound of Formula (50) wherein R³ is N(Et)CH₂cPr, R^{4a} is Me, R^{4b} is H, R^{4c} is OMe, R^{4d} is H and R^{4e} is Me;
   a compound of Formula (50) wherein R³ is N(Pr)CH₂cPr, R^{4a} is Me, R^{4b} is H, R^{4c} is OMe, R^{4d} is H and R^{4e} is Me;
   a compound of Formula (50) wherein R³ is N(Me)Pr, R^{4a} is Me, R^{4b} is H, R^{4c} is OMe, R^{4d} is H and R^{4e} is Me;
   a compound of Formula (50) wherein R³ is N(Me)Et, R^{4a} is Me, R^{4b} is H, R^{4c} is OMe, R^{4d} is H and R^{4e} is Me;
   a compound of Formula (50) wherein R³ is N(Me)Bu, R^{4a} is Me, R^{4b} is H, R^{4c} is OMe, R^{4d} is H and R^{4e} is Me;
   a compound of Formula (50) wherein R³ is N(Me)propargyl, R^{4a} is Me, R^{4b} is H, R^{4c} is OMe, R^{4d} is H and R^{4e} is Me;
   a compound of Formula (50) wherein R³ is N(Et)propargyl, R^{4a} is Me, R^{4b} is H, R^{4c} is OMe, R^{4d} is H and R^{4e} is Me;
   a compound of Formula (50) wherein R³ is NHCH(CH₃)CH(CH₃)CH₃, R^{4a} is Me, R^{4b} is H, R^{4c} is OMe, R^{4d} is H and R^{4e} is Me;
   a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is Me;
   a compound of Formula (50) wherein R³ is N(CH₂CH₂OMe)Me, R^{4a} is Me, R^{4b} is H, R^{4c} is OMe, R^{4d} is H and R^{4e} is Me;
   a compound of Formula (50) wherein R³ is N(CH₂CH₂OMe)Et, R^{4a} is Me, R^{4b} is H, R^{4c} is OMe, R^{4d} is H and R^{4e} is Me;
   a compound of Formula (50) wherein R³ is N(CH₂CH₂OMe)Pr, R^{4a} is Me, R^{4b} is H, R^{4c} is OMe, R^{4d} is H and R^{4e} is Me;
   a compound of Formula (50) wherein R³ is N(CH₂CH₂OMe)-CH₂cPr, R^{4a} is Me, R^{4b} is H, R^{4c} is OMe, R^{4d} is H and R^{4e} is Me;
   a compound of Formula (50) wherein R³ is NHCH(CH₃)CH₂CH₃, R^{4a} is Me, R^{4b} is H, R^{4c} is OMe, R^{4d} is H and R^{4e} is Me;
   a compound of Formula (50) wherein R³ is NHCH(Et)₂, R^{4a} is Me, R^{4b} is H, R^{4c} is OMe, R^{4d} is H and R^{4e} is Me;
   a compound of Formula (50) wherein R³ is NHCH(cPr)₂, R^{4a} is Me, R^{4b} is H, R^{4c} is OMe, R^{4d} is H and R^{4e} is Me;
   a compound of Formula (50) wherein R³ is NHCH(Et)₂, R^{4a} is Me, R^{4b} is H, R^{4c} is OMe, R^{4d} is H and R^{4e} is OMe;
   a compound of Formula (50) wherein R³ is 2-ethylpiperid-1-yl, R^{4a} is Me, R^{4b} is H, R^{4c} is OMe, R^{4d} is H and R^{4e} is OMe;
   a compound of Formula (50) wherein R³ is cyclobutylamino, R^{4a} is Me, R^{4b} is H, R^{4c} is OMe, R^{4d} is H and R^{4e} is OMe;
   a compound of Formula (50) wherein R³ is N(Me)CH₂CH=CH₂, R^{4a} is Me, R^{4b} is H, R^{4c} is OMe, R^{4d} is H and R^{4e} is OMe;
   a compound of Formula (50) wherein R³ is N(Et)CH₂CH=CH₂, R^{4a} is Me, R^{4b} is H, R^{4c} is OMe, R^{4d} is H and R^{4e} is OMe;
   a compound of Formula (50) wherein R³ is N(Me)CH₂cPr, R^{4a} is Me, R^{4b} is H, R^{4c} is OMe, R^{4d} is H and R^{4e} is OMe;
   a compound of Formula (50) wherein R³ is N(Et)CH₂cPr, R^{4a} is Me, R^{4b} is H, R^{4c} is OMe, R^{4d} is H and R^{4e} is OMe;
   a compound of Formula (50) wherein R³ is N(Pr)CH₂cPr, R^{4a} is Me, R^{4b} is H, R^{4c} is OMe, R^{4d} is H and R^{4e} is OMe;
   a compound of Formula (50) wherein R³ is N(Me)Pr, R^{4a} is Me, R^{4b} is H, R^{4c} is OMe, R^{4d} is H and R^{4e} is OMe;
   a compound of Formula (50) wherein R³ is N(Me)Et, R^{4a} is Me, R^{4b} is H, R^{4c} is OMe, R^{4d} is H and R^{4e} is OMe;
   a compound of Formula (50) wherein R³ is N(Me)Bu, R^{4a} is Me, R^{4b} is H, R^{4c} is OMe, R^{4d} is H and R^{4e} is OMe;
   a compound of Formula (50) wherein R³ is N(Me)propargyl, R^{4a} is Me, R^{4b} is H, R^{4c} is OMe, R^{4d} is Hand R^{4e} is OMe;
   a compound of Formula (50) wherein R³ is N(Et)propargyl, R^{4a} is Me, R^{4b} is H, R^{4c} is OMe, R^{4d} is H and R^{4e} is OMe;
   a compound of Formula (50) wherein R³ is NHCH(CH₃)CH(CH₃)CH₃, R^{4a} is Me, R^{4b} is H, R^{4c} is OMe, R^{4d} is H and R^{4e} is OMe;
   a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is OMe;
   a compound of Formula (50) wherein R³ is N(CH₂CH₂OMe)Me, R^{4a} is Me, R^{4b} is H, R^{4c} is OMe, R^{4d} is H and R^{4e} is OMe;
   a compound of Formula (50) wherein R³ is N(CH₂CH₂OMe)Et, R^{4a} is Me, R^{4b} is H, R^{4c} is OMe, R^{4d} is H and R^{4e} is OMe;
   a compound of Formula (50) wherein R³ is N(CH₂CH₂OMe)Pr, R^{4a} is Me, R^{4b} is H, R^{4c} is OMe, R^{4d} is H and R^{4e} is OMe;
   a compound of Formula (50) wherein R³ is N(CH₂CH₂OMe) CH₂cPr, R^{4a} is Me, R^{4b} is H, R^{4c} is OMe, R^{4d} is H and R^{4e} is OMe;
   a compound of Formula (50) wherein R³ is NHCH(CH₃)CH₂CH₃, R^{4a} is Me, R^{4b} is H, R^{4c} is OMe, R^{4d} is H and R^{4e} is OMe;
   a compound of Formula (50) wherein R³ is NHCH(cPr)₂, R^{4a} is Me, R^{4b} is H, R^{4c} is OMe, R^{4d} is H and R^{4e} is OMe;
   a compound of Formula (50) wherein R³ is NHCH(Et)₂, R^{4a} is Cl, R^{4b} is H, R^{4c} is OMe, R^{4d} is H and R^{4e} is OMe;
   a compound of Formula (50) wherein R³ is 2-ethylpiperid-1-yl, R^{4a} is Cl, R^{4b} is H, R^{4c} is OMe, R^{4d} is H and R^{4e} is OMe;
   a compound of Formula (50) wherein R³ is cyclobutylamino, R^{4a} is Cl, R^{4b} is H, R^{4c} is OMe, R^{4d} is H and R^{4e} is OMe;
   a compound of Formula (50) wherein R³ is N(Me)CH₂CH=CH₂, R^{4a} is Cl, R^{4b} is H, R^{4c} is OMe, R^{4d} is H and R^{4e} is OMe;
   a compound of Formula (50) wherein R³ is N(Et)CH₂CH=CH₂, R^{4a} is Cl, R^{4b} is H, R^{4c} is OMe, R^{4d} is H and R^{4e} is OMe;
   a compound of Formula (50) wherein R³ is N(Me)CH₂cPr, R^{4a} is Cl, R^{4b} is H, R^{4c} is OMe, R^{4d} is H and R^{4e} is OMe;
   a compound of Formula (50) wherein R³ is N(Et)CH₂cPr, R^{4a} is Cl, R^{4b} is H, R^{4c} is OMe, R^{4d} is H and R^{4e} is OMe;
   a compound of Formula (50) wherein R³ is N(Pr)CH₂cPr, R^{4a} is Cl, R^{4b} is H, R^{4c} is OMe, R^{4d} is H and R^{4e} is OMe;
   a compound of Formula (50) wherein R³ is N(Me)Pr, R^{4a} is Cl, R^{4b} is H, R^{4c} is OMe, R^{4d} is H and R^{4e} is OMe;
   a compound of Formula (50) wherein R³ is N(Me)Et, R^{4a} is Cl, R^{4b} is H, R^{4c} is OMe, R^{4d} is H and R^{4e} is OMe;
   a compound of Formula (50) wherein R³ is N(Me)Bu, R^{4a} is Cl, R^{4b} is H, R^{4c} is OMe, R^{4d} is H and R^{4e} is OMe;
   a compound of Formula (50) wherein R³ is N(Me)propargyl, R^{4a} is Cl, R^{4b} is H, R^{4c} is OMe, R^{4d} is H and R^{4e} is OMe;
   a compound of Formula (50) wherein R³ is N(Et)propargyl, R^{4a} is Cl, R^{4b} is H, R^{4c} is OMe, R^{4d} is H and R^{4e} is OMe;
   a compound of Formula (50) wherein R³ is NHCH(CH₃)CH(CH₃)CH₃, R^{4a} is Cl, R^{4b} is H, R^{4c} is OMe, R^{4d} is H and R^{4e} is OMe;
   a compound of Formula (50) wherein R³ is N(CH₂CH₂OMe)-CH₂CH=CH₂, R^{4a} is Cl, R^{4b} is H, R^{4c} is OMe, R^{4d} is H and R^{4e} is OMe;
   a compound of Formula (50) wherein R³ is N(CH₂CH₂OMe)Me, R^{4a} is Cl, R^{4b} is H, R^{4c} is OMe, R^{4d} is H and R^{4e} is OMe;
   a compound of Formula (50) wherein R³ is N(CH₂CH₂OMe)Et, R^{4a} is Cl, R^{4b} is H, R^{4c} is OMe, R^{4d} is H and R^{4e} is OMe;
   a compound of Formula (50) wherein R³ is N(CH₂CH₂OMe)Pr, R^{4a} is Cl, R^{4b} is H, R^{4c} is OMe, R^{4d} is H and R^{4e} is OMe;
   a compound of Formula (50) wherein R³ is N(CH₂CH₂OMe)-CH₂cPr, R^{4a} is Cl, R^{4b} is H, R^{4c} is OMe, R^{4d} is H and R^{4e} is OMe;
   a compound of Formula (50) wherein R³ is NHCH(CH₃)CH₂CH₃, R^{4a} is Cl, R^{4b} is H, R^{4c} is OMe, R^{4d} is H and R^{4e} is OMe;
   a compound of Formula (50) wherein R³ is NHCH(cPr)₂, R^{4a} is Cl, R^{4b} is H, R^{4c} is OMe, R^{4d} is H and R^{4e} is OMe;
   a compound of Formula (50) wherein R³ is N(CH₂CH₂OMe)₂, R^{4a} is Cl, R^{4b} is H, R^{4c} is OMe, R^{4d} is H and R^{4e} is OMe;
   a compound of Formula (50) wherein R³ is NHCH(Et)₂, R^{4a} is Cl, R^{4b} is H, R^{4c} is OMe, R^{4d} is H and R^{4e} is OMe;
   a compound of Formula (50) wherein R³ is N(Et)₂, R^{4a} is Cl, R^{4b} is H, R^{4c} is OMe, R^{4d} is H and R^{4e} is OMe;
   a compound of Formula (50) wherein R³ is NHCH(Et)₂, R^{4a} is Cl, R^{4b} is H, R^{4c} is OMe, R^{4d} is H and R^{4e} is H;
   a compound of Formula (50) wherein R³ is 2-ethylpiperid-1-yl, R^{4a} is Cl, R^{4b} is H, R^{4c} is OMe, R^{4d} is H and R^{4e} is H;
   a compound of Formula (50) wherein R³ is cyclobutylamino, R^{4a} is Cl, R^{4b} is H, R^{4c} is OMe, R^{4d} is H and R^{4e} is H;
   a compound of Formula (50) wherein R³ is N(Me)CH₂CH=CH₂, R^{4a} is Cl, R^{4b} is H, R^{4c} is OMe, R^{4d} is H and R^{4e} is H;
   a compound of Formula (50) wherein R³ is N(Et)CH₂CH=CH₂, R^{4a} is Cl, R^{4b} is H, R^{4c} is OMe, R^{4d} is H and R^{4e} is H;
   a compound of Formula (50) wherein R³ is N(Me)CH₂cPr, R^{4a} is Cl, R^{4b} is H, R^{4c} is OMe, R^{4d} is H and R^{4e} is H;
   a compound of Formula (50) wherein R³ is N(Et)CH₂cPr, R^{4a} is Cl, R^{4b} is H, R^{4c} is OMe, R^{4d} is H and R^{4e} is H;
   a compound of Formula (50) wherein R³ is N(Pr)CH₂cPr, R^{4a} is Cl, R^{4b} is H, R^{4c} is OMe, R^{4d} is H and R^{4e} is H;
   a compound of Formula (50) wherein R³ is N(Me)Pr, R^{4a} is Cl, R^{4b} is H, R^{4c} is OMe, R^{4d} is H and R^{4e} is H;
   a compound of Formula (50) wherein R³ is N(Me)Et, R^{4a} is Cl, R^{4b} is H, R^{4c} is OMe, R^{4d} is H and R^{4e} is H;
   a compound of Formula (50) wherein R³ is N(Me)Bu, R^{4a} is Cl, R^{4b} is H, R^{4c} is OMe, R^{4d} is H and R^{4e} is H;
   a compound of Formula (50) wherein R³ is N(Me)propargyl, R^{4a} is Cl, R^{4b} is H, R^{4c} is OMe, R^{4d} is H and R^{4e} is H;
   a compound of Formula (50) wherein R³ is N(Et)propargyl, R^{4a} is Cl, R^{4b} is H, R^{4c} is OMe, R^{4d} is H and R^{4e} is H;
   a compound of Formula (50) wherein R³ is NHCH(CH₃)CH(CH₃)CH₃, R^{4a} is Cl, R^{4b} is H, R^{4c} is OMe, R^{4d} is H and R^{4e} is H;
   a compound of Formula (50) wherein R³ is N(CH₂CH₂OMe)-CH₂CH=CH₂, R^{4a} is Cl, R^{4b} is H, R^{4c} is OMe, R^{4d} is H and R^{4e} is H;
   a compound of Formula (50) wherein R³ is N(CH₂CH₂OMe)Me, R^{4a} is Cl, R^{4b} is H, R^{4c} is OMe, R^{4d} is H and R^{4e} is H;
   a compound of Formula (50) wherein R³ is N(CH₂CH₂OMe)Et, R^{4a} is Cl, R^{4b} is H, R^{4c} is OMe, R^{4d} is H and R^{4e} is H;
   a compound of Formula (50) wherein R³ is N(CH₂CH₂OMe)Pr, R^{4a} is Cl, R^{4b} is H, R^{4c} is OMe, R^{4d} is H and R^{4e} is H;
   a compound of Formula (50) wherein R³ is N(CH₂CH₂OMe)-CH₂cPr, R^{4a} is Cl, R^{4b} is H, R^{4c} is OMe, R^{4d} is H and R^{4e} is H;
   a compound of Formula (50) wherein R³ is NHCH(CH₃)CH₂CH₃, R^{4a} is Cl, R^{4b} is H, R^{4c} is OMe, R^{4d} is H and R^{4e} is H;
   a compound of Formula (50) wherein R³ is NHCH(cPr)₂, R^{4a} is Cl, R^{4b} is H, R^{4c} is OMe, R^{4d} is H and R^{4e} is H;
   a compound of Formula (50) wherein R³ is N(CH₂CH₂OMe)₂ R^{4a} is Cl, R^{4b} is H, R^{4c} is OMe, R^{4d} is H and R^{4e} is H;
   a compound of Formula (50) wherein R³ is NHCH(Et)₂, R^{4a} is Cl, R^{4b} is H, R^{4c} is OMe, R^{4d} is H and R^{4e} is H;
   a compound of Formula (50) wherein R³ is NHCH(Et)₂, R^{4a} is Cl, R^{4b} is H, R^{4c} is OMe, R^{4d} is F and R^{4e} is H;
   a compound of Formula (50) wherein R³ is 2-ethylpiperid-1-yl, R^{4a} is Cl, R^{4b} is H, R^{4c} is OMe, R^{4d} is F and R^{4e} is H;
   a compound of Formula (50) wherein R³ is cyclobutylamino, R^{4a} is Cl, R^{4b} is H, R^{4c} is OMe, R^{4d} is F and R^{4e} is H;
   a compound of Formula (50) wherein R³ is N(Me)CH₂CH=CH₂, R^{4a} is Cl, R^{4b} is H, R^{4c} is OMe, R^{4d} is F and R^{4e} is H;
   a compound of Formula (50) wherein R³ is N(Et)CH₂CH=CH₂, R^{4a} is Cl, R^{4b} is H, R^{4c} is OMe, R^{4d} is F and R^{4e} is H;
   a compound of Formula (50) wherein R³ is N(Me)CH₂cPr, R^{4a} is Cl, R^{4b} is H, R^{4c} is OMe, R^{4d} is F and R^{4e} is H;
   a compound of Formula (50) wherein R³ is N(Et)CH₂cPr, R^{4a} is Cl, R^{4b} is H, R^{4c} is OMe, R^{4d} is F and R^{4e} is H;
   a compound of Formula (50) wherein R³ is N(Pr)CH₂cPr, R^{4a} is Cl, R^{4b} is H, R^{4c} is OMe, R^{4d} is F and R^{4e} is H;
   a compound of Formula (50) wherein R³ is N(Me)Pr, R^{4a} is Cl, R^{4b} is H, R^{4c} is OMe, R^{4d} is F and R^{4e} is H;
   a compound of Formula (50) wherein R³ is N(Me)Et, R^{4a} is Cl, R^{4b} is H, R^{4c} is OMe, R^{4d} is F and R^{4e} is H;
   a compound of Formula (50) wherein R³ is N(Me)Bu, R^{4a} is Cl, R^{4b} is H, R^{4c} is OMe, R^{4d} is F and R^{4e} is H;
   a compound of Formula (50) wherein R³ is N(Me)propargyl, R^{4a} is Cl, R^{4b} is H, R^{4c} is OMe, R^{4d} is F and R^{4e} is H;
   a compound of Formula (50) wherein R³ is NH(CH(CH₃)CH(CH₃)CH₃, R^{4a} is Cl, R^{4b} is H, R^{4c} is OMe, R^{4d} is F and R^{4e} is H;
   a compound of Formula (50) wherein R³ is N(CH₂CH₂OMe)-CH₂CH=CH₂, R^{4a} is Cl, R^{4b} is H, R^{4c} is OMe, R^{4d} is F and R^{4e} is H;
   a compound of Formula (50) wherein R³ is N(CH₂CH₂OMe)Me, R^{4a} is Cl, R^{4b} is H, R^{4c} is OMe, R^{4d} is F and R^{4e} is H;
   a compound of Formula (50) wherein R³ is N(CH₂CH₂OMe)Et, R^{4a} is Cl, R^{4b} is H, R^{4c} is OMe, R^{4d} is F and R^{4e} is H;
   a compound of Formula (50) wherein R³ is N(CH₂CH₂OMe)Pr, R^{4a} is Cl, R^{4b} is H, R^{4c} is OMe, R^{4d} is F and R^{4e} is H;
   a compound of Formula (50) wherein R³ is N(CH₂CH₂OMe)-CH₂cPr, R^{4a} is Cl, R^{4b} is H, R^{4c} is OMe, R^{4d} is F and R^{4e} is H;
   a compound of Formula (50) wherein R³ is NHCH(CH₃)CH₂CH₃, R^{4a} is Cl, R^{4b} is F, R^{4c} is OMe, R^{4d} is H and R^{4e} is H;
   a compound of Formula (50) wherein R³ is NHCH(cPr)₂, R^{4a} is Cl, R^{4b} is H, R^{4c} is OMe, R^{4d} is F and R^{4e} is H;
   a compound of Formula (50) wherein R³ is N(CH₂CH₂OMe)₂, R^{4a} is Cl, R^{4b} is H, R^{4c} is OMe, R^{4d} is F and R^{4e} is H;
   a compound of Formula (50) wherein R³ is NHCH(Et)₂, R^{4a} is Cl, R^{4b} is H, R^{4c} is OMe, R^{4d} is F and R^{4e} is H;
   a compound of Formula (50) wherein R³ is N(Et)₂, R^{4a} is Cl, R^{4b} is H, R^{4c} is OMe, R^{4d} is F and R^{4e} is H.
   a compound of Formula (50) wherein R³ is NHCH(Et)₂, R^{4a} is Cl, R^{4b} is H, R^{4c} is OMe, R^{4d} is OMe and R^{4e} is H;
   a compound of Formula (50) wherein R³ is 2-ethylpiperid-1-yl, R^{4a} is Cl, R^{4b} is H, R^{4c} is OMe, R^{4d} is OMe and R^{4e} is H;
   a compound of Formula (50) wherein R³ is cyclobutylamino, R^{4a} is Cl, R^{4b} is H, R^{4c} is OMe, R^{4d} is OMe and R^{4e} is H;
   a compound of Formula (50) wherein R³ is N(Me)CH₂CH=CH₂, R^{4a} is Cl, R^{4b} is H, R^{4c} is OMe, R^{4d} is OMe and R^{4e} is H;
   a compound of Formula (50) wherein R³ is N(Et)CH₂CH=CH₂, R^{4a} is Cl, R^{4b} is H, R^{4c} is OMe, R^{4d} is OMe and R^{4e} is H;
   a compound of Formula (50) wherein R³ is N(Me)CH₂cPr, R^{4a} is Cl, R^{4b} is H, R^{4c} is OMe, R^{4d} is F and R^{4e} is H;
   a compound of Formula (50) wherein R³ is N(Et)CH₂cPr, R^{4a} is Cl, R^{4b} is H, R^{4c} is OMe, R^{4d} is OMe and R^{4e} is H;
   a compound of Formula (50) wherein R³ is N(Pr)CH₂cPr, R^{4a} is Cl, R^{4b} is H, R^{4c} is OMe, R^{4d} is OMe and R^{4e} is H;
   a compound of Formula (50) wherein R³ is N(Me)Pr, R^{4a} is Cl, R^{4b} is H, R^{4c} is OMe, R^{4d} is OMe and R^{4e} is H;
   a compound of Formula (50) wherein R³ is N(Me)Et, R^{4a} is Cl, R^{4b} is H, R^{4c} is OMe, R^{4d} is OMe and R^{4e} is H;
   a compound of Formula (50) wherein R³ is N(Me)Bu, R^{4a} is Cl, R^{4b} is H, R^{4c} is OMe, R^{4d} is OMe and R^{4e} is H;
   a compound of Formula (50) wherein R³ is N(Me)propargyl, R^{4a} is Cl, R^{4b} is H, R^{4c} is OMe, R^{4d} is OMe and R^{4e} is H;
   a compound of Formula (50) wherein R³ is NHCH(CH₃)CH(CH₃)CH₃, R^{4a} is Cl, R^{4b} is H, R^{4c} is OMe, R^{4d} is OMe and R^{4e} is H;
   a compound of Formula (50) wherein R³ is N(CH₂CH₂OMe) - CH₂CH=CH₂, R^{4a} is Cl, R^{4b} is H, R^{4c} is OMe, R^{4d} is F and R^{4e} is H;
   a compound of Formula (50) wherein R³ is N(CH₂CH₂OMe)Me, R^{4a} is Cl, R^{4b} is H, R^{4c} is OMe, R^{4d} is OMe and R^{4e} is H;
   a compound of Formula (50) wherein R³ is N(CH₂CH₂OMe)Et, R^{4a} is Cl, R^{4b} is H, R^{4c} is OMe, R^{4d} is OMe and R^{4e} is H;
   a compound of Formula (50) wherein R³ is N(CH₂CH₂OMe)Pr, R^{4a} is Cl, R^{4b} is H, R^{4c} is OMe, R^{4d} is OMe and R^{4e} is H;
   a compound of Formula (50) wherein R³ is N(CH₂CH₂OMe) - CH₂cPr, R^{4a} is Cl, R^{4b} is H, R^{4c} is OMe, R^{4d} is OMe and R^{4e} is H;
   a compound of Formula (50) wherein R³ is NHCH(CH₃)CH₂CH₃, R^{4a} is Cl, R^{4b} is H, R^{4c} is OMe, R^{4d} is OMe and R^{4e} is H;
   a compound of Formula (50) wherein R³ is NHCH(cPr)₂, R^{4a} is Cl, R^{4b} is H, R^{4c} is OMe, R^{4d} is OMe and R^{4e} is H;
   a compound of Formula (50) wherein R³ is N(CH₂CH₂OMe)₂, R^{4a} is Cl, R^{4b} is H, R^{4c} is OMe, R^{4d} is OMe and R^{4e} is H;
   a compound of Formula (50) wherein R³ is NHCH(Et)₂, R^{4a} is Cl, R^{4b} is H, R^{4c} is OMe, R^{4d} is OMe and R^{4e} is H;
   a compound of Formula (50) wherein R³ is N(Et)₂, R^{4a} is Cl, R^{4b} is H, R^{4c} is OMe, R^{4d} is OMe and R^{4e} is H;
   a compound of Formula (50) wherein R³ is NHCH(Et)₂ R^{4a} is Br, R^{4b} is H, R^{4c} is OMe, R^{4d} is OMe and R^{4e} is H;
   a compound of Formula (50) wherein R³ is 2-ethylpiperid-1-yl, R^{4a} is Br, R^{4b} is H, R^{4c} is OMe, R^{4d} is OMe and R^{4e} is H;
   a compound of Formula (50) wherein R³ is cyclobutylamino, R^{4a} is Br, R^{4b} is H, R^{4c} is OMe, R^{4d} is OMe and R^{4e} is H;
   a compound of Formula (50) wherein R³ is N(Me)CH₂CH=CH₂, R^{4a} is Br, R^{4b} is H, R^{4c} is OMe, R^{4d} is OMe and R^{4e} is H;
   a compound of Formula (50) wherein R³ is N(Et)CH₂CH=CH₂, R^{4a} is Br, R^{4b} is H, R^{4c} is OMe, R^{4d} is OMe and R^{4e} is H;
   a compound of Formula (50) wherein R³ is N(Me)CH₂cPr, R^{4a} is Br, R^{4b} is H, R^{4c} is OMe, R^{4d} is F and R^{4e} is H;
   a compound of Formula (50) wherein R³ is N(Et)CH₂cPr, R^{4a} is Br, R^{4b} is H, R^{4c} is OMe, R^{4d} is OMe and R^{4e} is H;
   a compound of Formula (50) wherein R³ is N(Pr)CH₂cPr, R^{4a} is Br, R^{4b} is H, R^{4c} is OMe, R^{4d} is OMe and R^{4e} is H;
   a compound of Formula (50) wherein R³ is N(Me)Pr, R^{4a} is Br, R^{4b} is H, R^{4c} is OMe, R^{4d} is OMe and R^{4e} is H;
   a compound of Formula (50) wherein R³ is N(Me)Et, R^{4a} is Br, R^{4b} is H, R^{4c} is OMe, R^{4d} is OMe and R^{4e} is H;
   a compound of Formula (50) wherein R³ is N(Me)Bu, R^{4a} is Br, R^{4b} is H, R^{4c} is OMe, R^{4d} is OMe and R^{4e} is H;
   a compound of Formula (50) wherein R³ is N(Me)propargyl, R^{4a} is Br, R^{4b} is H, R^{4c} is OMe, R^{4d} is OMe and R^{4e} is H;
   a compound of Formula (50) wherein R³ is NHCH(CH₃)CH(CH₃)CH₃, R^{4a} is Br, R^{4b} is H, R^{4c} is OMe, R^{4d} is OMe and R^{4e} is H;
   a compound of Formula (50) wherein R³ is N(CH₂CH₂OMe)-CH₂CH=CH₂, R^{4a} is Br, R^{4b} is H, R^{4c} is OMe, R^{4d} is F and R^{4e} is H;
   a compound of Formula (50) wherein R³ is N(CH₂CH₂OMe)Me, R^{4a} is Br, R^{4b} is H, R^{4c} is OMe, R^{4d} is OMe and R^{4e} is H;
   a compound of Formula (50) wherein R³ is N(CH₂CH₂OMe)Et, R^{4a} is Br, R^{4b} is H, R^{4c} is OMe, R^{4d} is OMe and R^{4e} is H;
   a compound of Formula (50) wherein R³ is N(CH₂CH₂OMe)Pr, R^{4a} is Br, R^{4b} is H, R^{4c} is OMe, R^{4d} is OMe and R^{4e} is H;
   a compound of Formula (50) wherein R³ is N(CH₂CH₂OMe)-CH₂cPr, R^{4a} is Br, R^{4b} is H, R^{4c} is OMe, R^{4d} is OMe and R^{4e} is H;
   a compound of Formula (50) wherein R³ is NH(CH(CH₃)CH₂CH₃, R^{4a} is Br, R^{4b} is H, R^{4c} is OMe, R^{4d} is OMe and R^{4e} is H;
   a compound of Formula (50) wherein R³ is NHCH(cPr)₂, R^{4a} is Br, R^{4b} is H, R^{4c} is OMe, R^{4d} is OMe and R^{4e} is H;
   a compound of Formula (50) wherein R³ is N(CH₂CH₂OMe)₂, R^{4a} is Br, R^{4b} is H, R^{4c} is OMe, R^{4d} is OMe and R^{4e} is H;
   a compound of Formula (50) wherein R³ is NHCH(Et)₂, R^{4a} is Br, R^{4b} is H, R^{4c} is OMe, R^{4d} is OMe and R^{4e} is H;
   a compound of Formula (50) wherein R³ is NHCH(Et)₂, R^{4a} is Me, R^{4b} is H, R^{4c} is OMe, R^{4d} is OMe and R^{4e} is H;
   a compound of Formula (50) wherein R³ is 2-ethylpiperid-1-yl, R^{4a} is Me, R^{4b} is H, R^{4c} is OMe, R^{4d} is OMe and R^{4e} is H;
   a compound of Formula (50) wherein R³ is cyclobutylamino, R^{4a} is Me, R^{4b} is H, R^{4c} is OMe, R^{4d} is OMe and R^{4e} is H;
   a compound of Formula (50) wherein R³ is N(Me)CH₂CH=CH₂, R^{4a} is Me, R^{4b} is H, R^{4c} is OMe, R^{4d} is OMe and R^{4e} is H;
   a compound of Formula (50) wherein R³ is N(Et)CH₂CH=CH₂, R^{4a} is Me, R^{4b} is H, R^{4c} is OMe, R^{4d} is OMe and R^{4e} is H;
   a compound of Formula (50) wherein R³ is N(Me)CH₂cPr, R^{4a} is Me, R^{4b} is H, R^{4c} is OMe, R^{4d} is F and R^{4e} is H;
   a compound of Formula (50) wherein R³ is N(Et)CH₂cPr, R^{4a} is Me, R^{4b} is H, R^{4c} is OMe, R^{4d} is OMe and R^{4e} is H;
   a compound of Formula (50) wherein R³ is N(Pr)CH₂cPr, R^{4a} is Me, R^{4b} is H, R^{4c} is OMe, R^{4d} is OMe and R^{4e} is H;
   a compound of Formula (50) wherein R³ is N(Me)Pr, R^{4a} is Me, R^{4b} is H, R^{4c} is OMe, R^{4d} is OMe and R^{4e} is H;
   a compound of Formula (50) wherein R³ is N(Me)Et, R^{4a} is Me, R^{4b} is H, R^{4c} is OMe, R^{4d} is OMe and R^{4e} is H;
   a compound of Formula (50) wherein R³ is N(Me)Bu, R^{4a} is Me, R^{4b} is H, R^{4c} is OMe, R^{4d} is OMe and R^{4e} is H;
   a compound of Formula (50) wherein R³ is N(Me)propargyl, R^{4a} is Me, R^{4b} is H, R^{4c} is OMe, R^{4d} is OMe and R^{4e} is H;
   a compound of Formula (50) wherein R³ is NHCH(CH₃)CH(CH₃)CH₃, R^{4a} is Br, R^{4b} is H, R^{4c} is OMe, R^{4d} is OMe and R^{4e} is H;
   a compound of Formula (50) wherein R³ is N(CH₂CH₂OMe)-CH₂CH=CH₂, R^{4a} is Me, R^{4b} is H, R^{4c} is OMe, R^{4d} is F and R^{4e} is H;
   a compound of Formula (50) wherein R³ is N(CH₂CH₂OMe)Me, R^{4a} is Me, R^{4b} is H, R^{4c} is OMe, R^{4d} is OMe and R^{4e} is H;
   a compound of Formula (50) wherein R³ is N(CH₂CH₂OMe)Et, R^{4a} is Me, R^{4b} is H, R^{4c} is OMe, R^{4d} is OMe and R^{4e} is H;
   a compound of Formula (50) wherein R³ is N(CH₂CH₂OMe)Pr, R^{4a} is Br, R^{4b} is H, R^{4c} is OMe, R^{4d} is OMe and R^{4e} is H;
   a compound of Formula (50) wherein R³ is N(CH₂CH₂OMe)-CH₂cPr, R^{4a} is Me, R^{4b} is H, R^{4c} is OMe, R^{4d} is OMe and R^{4e} is H;
   a compound of Formula (50) wherein R³ is NHCH(CH₃)CH₂CH₃, R^{4a} is Me, R^{4b} is H, R^{4c} is OMe, R^{4d} is OMe and R^{4e} is H;
   a compound of Formula (50) wherein R³ is NHCH(cPr)₂, R^{4a} is Me, R^{4b} is H, R^{4c} is OMe, R^{4d} is OMe and R^{4e} is H;
   a compound of Formula (50) wherein R³ is N(CH₂CH₂OMe)₂, R^{4a} is Me, R^{4b} is H, R^{4c} is OMe, R^{4d} is OMe and R^{4e} is H;
   a compound of Formula (50) wherein R³ is NHCH(Et)₂ R^{4a} is Me, R^{4b} is H, R^{4c} is OMe, R^{4d} is OMe and R^{4e} is H; and
   a compound of Formula (50) wherein R³ is N(Et)₂, R^{4a} is Me, R^{4b} is H, R^{4c} is OMe, R^{4d} is OMe and R^{4e} is H.
[2] The present invention also provides compounds of Formula (60) 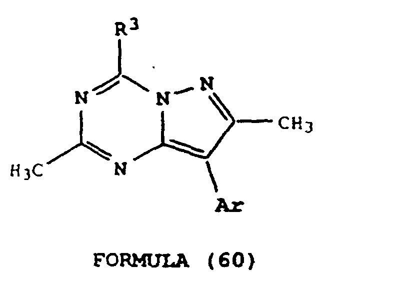 and geometric isomers thereof, stereoisomeric forms thereof, or mixtures of stereoisomeric forms thereof, and pharmaceutically acceptable salt forms thereof, selected from the group:
   a compound of Formula (60) wherein R³ is NHCH(Et)₂, Ar is 6-dimethylamino-4-methylpyrid-3-yl;
   a compound of Formula (60) wherein R³ is 2-ethylpiperid-1-yl, Ar is 6-dimethylamino-4-methylpyrid-3-yl;
   a compound of Formula (60) wherein R³ is cyclobutylamino, Ar is 6-dimethylamino-4-methylpyrid-3-yl;
   a compound of Formula (60) wherein R³ is N(Me)CH₂CH=CH₂, Ar is 6-dimethylamino-4-methylpyrid-3-yl;
   a compound of Formula (60) wherein R³ is N(Et)CH₂cPr, Ar is 6-dimethylamino-4-methylpyrid-3-yl;
   a compound of Formula (60) wherein R³ is N(Pr)CH₂cPr, Ar is 6-dimethylamino-4-methylpyrid-3-yl;
   a compound of Formula (60) wherein R³ is N(Me)Pr, Ar is 6-dimethylamino-4-methylpyrid-3-yl;
   a compound of Formula (60) wherein R³ is N(Me)Et, Ar is 6-dimethylamino-4-methylpyrid-3-yl;
   a compound of Formula (60) wherein R³ is N(Me)Bu, Ar is 6-dimethylamino-4-methylpyrid-3-yl;
   a compound of Formula (60) wherein R³ is N(Me)propargyl, Ar is 6-dimethylamino-4-methylpyrid-3-yl;
   a compound of Formula (60) wherein R³ is N(Et)propargyl, Ar is 6-dimethylamino-4-methylpyrid-3-yl;
   a compound of Formula (60) wherein R³ is NHCH(CH₃)CH(CH₃)CH₃, Ar is 6-dimethylamino-4-methylpyrid-3-yl;
   a compound of Formula (60) wherein R³ is N(CH₂CH₂OMe)-CH₂CH=CH₂, Ar is 6-dimethylamino-4-methylpyrid-3-yl;
   a compound of Formula (60) wherein R³ is N(CH₂CH₂OMe)Me, Ar is 6-dimethylamino-4-methylpyrid-3-yl;
   a compound of Formula (60) wherein R³ is N(CH₂CH₂OMe)Et, Ar is 6-dimethylamino-4-methylpyrid-3-yl;
   a compound of Formula (60) wherein R³ is N(CH₂CH₂OMe)Pr, Ar is 6-dimethylamino-4-methylpyrid-3-yl;
   a compound of Formula (60) wherein R³ is N(CH₂CH₂OMe)-CH₂cPr, Ar is 6-dimethylamino-4-methylpyrid-3-yl;
   a compound of Formula (60) wherein R³ is NHCH(CH₃)CH₂CH₃, Ar is 6-dimethylamino-4-methylpyrid-3-yl;
   a compound of Formula (60) wherein R³ is NHCH(cPr)₂, Ar is 6-dimethylamino-4-methylpyrid-3-yl;
   a compound of Formula (60) wherein R³ is N(CH₂CH₂OMe)₂, Ar is 6-dimethylamino-4-methylpyrid-3-yl;
   a compound of Formula (60) wherein R³ is NHCH(Et)₂, Ar is 6-dimethylamino-4-methylpyrid-3-yl;
   a compound of Formula (60) wherein R³ is N(Et)₂, Ar is 6-dimethylamino-4-methylpyrid-3-yl;
   a compound of Formula (60) wherein R³ is 2-ethylpiperid-1-yl, Ar is 6-dimethylamino-4-methylpyrid-3-yl;
   a compound of Formula (60) wherein R³ is cyclobutylamino, Ar is 6-dimethylamino-4-methylpyrid-3-yl;
   a compound of Formula (60) wherein R³ is N(Me)CH₂CH=CH₂, Ar is 6-dimethylamino-4-methylpyrid-3-yl;
   a compound of Formula (60) wherein R³ is N(Et)CH₂cPr, Ar is 6-dimethylamino-4-methylpyrid-3-yl;
   a compound of Formula (60) wherein R³ is N(Pr)CH₂cPr, Ar is 6-dimethylamino-4-methylpyrid-3-yl;
   a compound of Formula (60) wherein R³ is N(Me)Pr, Ar is 6-dimethylamino-4-methylpyrid-3-yl;
   a compound of Formula (60) wherein R³ is N(Me)Et, Ar is 6-dimethylamino-4-methylpyrid-3-yl;
   a compound of Formula (60) wherein R³ is N(Me)Bu, Ar is 6-dimethylamino-4-methylpyrid-3-yl;
   a compound of Formula (60) wherein R³ is N(Me)propargyl, Ar is 6-dimethylamino-4-methylpyrid-3-yl;
   a compound of Formula (60) wherein R³ is NHCH(CH₃)CH(CH₃)CH₃, Ar is 6-dimethylamino-4-methylpyrid-3-yl;
   a compound of Formula (60) wherein R³ is N(CH₂CH₂OMe)-CH₂CH=CH₂, Ar is 6-dimethylamino-4-methylpyrid-3-yl;
   a compound of Formula (60) wherein R³ is N(CH₂CH₂OMe)Me, Ar is 6-dimethylamino-4-methylpyrid-3-yl;
   a compound of Formula (60) wherein R³ is N(CH₂CH₂OMe)Et, Ar is 6-dimethylamino-4-methylpyrid-3-yl;
   a compound of Formula (60) wherein R³ is N(CH₂CH₂OMe)Pr, Ar is 6-dimethylamino-4-methylpyrid-3-yl;
   a compound of Formula (60) wherein R³ is N(CH₂CH₂OMe)-CH₂cPr, Ar is 6-dimethylamino-4-methylpyrid-3-yl;
   a compound of Formula (60) wherein R³ is NHCH(CH₃)CH₂CH₃, Ar is 6-dimethylamino-4-methylpyrid-3-yl;
   a compound of Formula (60) wherein R³ is NHCH(cPr)₂, Ar is 6-dimethylamino-4-methylpyrid-3-yl;
   a compound of Formula (60) wherein R³ is N(CH₂CH₂OMe)₂, Ar is 6-dimethylamino-4-methylpyrid-3-yl;
   a compound of Formula (60) wherein R³ is NHCH(Et)₂, Ar is 6-dimethylamino-4-methylpyrid-3-yl;
   a compound of Formula (60) wherein R³ is N(Et)₂, Ar is 6-dimethylamino-4-methylpyrid-3-yl.
   a compound of Formula (60) wherein R³ is 2-ethylpiperid-1-yl, Ar is 6- methoxy -4-methylpyrid-3-yl;
   a compound of Formula (60) wherein R³ is cyclobutylamino, Ar is 6- methoxy -4-methylpyrid-3-yl;
   a compound of Formula (60) wherein R³ is N(Me)CH₂CH=CH₂, Ar is 6- methoxy -4-methylpyrid-3-yl;
   a compound of Formula (60) wherein R³ is N(Et)CH₂cPr, Ar is 6- methoxy -4-methylpyrid-3-yl;
   a compound of Formula (60) wherein R³ is N(Pr)CH₂cPr, Ar is 6- methoxy -4-methylpyrid-3-yl;
   a compound of Formula (60) wherein R³ is N(Me)Pr, Ar is 6- methoxy -4-methylpyrid-3-yl;
   a compound of Formula (60) wherein R³ is N(Me)Et, Ar is 6- methoxy -4-methylpyrid-3-yl;
   a compound of Formula (60) wherein R³ is N(Me)Bu, Ar is 6- methoxy -4-methylpyrid-3-yl;
   a compound of Formula (60) wherein R³ is N(Me)propargyl, Ar is 6- methoxy -4-methylpyrid-3-yl;
   a compound of Formula (60) wherein R³ is N(Et)propargyl, Ar is 6- methoxy -4-methylpyrid-3-yl;
   a compound of Formula (60) wherein R³ is NHCH(CH₃)CH(CH₃)CH₃, Ar is 6- methoxy -4-methylpyrid-3-yl;
   a compound of Formula (60) wherein R³ is N(CH₂CH₂OMe) - CH₂CH=CH₂, Ar is 6- methoxy -4-methylpyrid-3-yl;
   a compound of Formula (60) wherein R³ is N(CH₂CH₂OMe)Me, Ar is 6- methoxy -4-methylpyrid-3-yl;
   a compound of Formula (60) wherein R³ is N(CH₂CH₂OMe)Et, Ar is 6- methoxy -4-methylpyrid-3-yl;
   a compound of Formula (60) wherein R³ is N(CH₂CH₂OMe)Pr, Ar is 6- methoxy -4-methylpyrid-3-yl;
   a compound of Formula (60) wherein R³ is N(CH₂CH₂OMe)-CH₂cPr, Ar is 6- methoxy -4-methylpyrid-3-yl;
   a compound of Formula (60) wherein R³ is NHCH(CH₃)CH₂CH₃, Ar is 6- methoxy -4-methylpyrid-3-yl;
   a compound of Formula (60) wherein R³ is NHCH(cPr)₂, Ar is 6- methoxy -4-methylpyrid-3-yl;
   a compound of Formula (60) wherein R³ is N(CH₂CH₂OMe)₂, Ar is 6- methoxy -4-methylpyrid-3-yl;
   a compound of Formula (60) wherein R³ is NHCH(Et)₂, Ar is 6- methoxy -4-methylpyrid-3-yl;
   a compound of Formula (60) wherein R³ is N(Et)₂, Ar is 6- methoxy -4-methylpyrid-3-yl;
   a compound of Formula (60) wherein R³ is 2-ethylpiperid-1-yl, Ar is 4-methoxy-6-methylpyrid-3-yl;
   a compound of Formula (60) wherein R³ is cyclobutylamino, Ar is 4-methoxy-6-methylpyrid-3-yl;
   a compound of Formula (60) wherein R³ is N(Me)CH₂CH=CH₂, Ar is 4-methoxy-6-methylpyrid-3-yl;
   a compound of Formula (60) wherein R³ is N(Et)CH₂cPr, Ar is 4-methoxy-6-methylpyrid-3-yl;
   a compound of Formula (60) wherein R³ is N(Pr)CH₂cPr, Ar is 4-methoxy-6-methylpyrid-3-yl;
   a compound of Formula (60) wherein R³ is N(Me)Pr, Ar is 4-methoxy-6-methylpyrid-3-yl;
   a compound of Formula (60) wherein R³ is N(Me)Et, Ar is 4-methoxy-6-methylpyrid-3-yl;
   a compound of Formula (60) wherein R³ is N(Me)Bu, Ar is 4-methoxy-6-methylpyrid-3-yl;
   a compound of Formula (60) wherein R³ is N(Me)propargyl, Ar is 4-methoxy-6-methylpyrid-3-yl;
   a compound of Formula (60) wherein R³ is NHCH(CH₃)CH(CH₃)CH₃, Ar is 4-methoxy-6-methylpyrid-3-yl;
   a compound of Formula (60) wherein R³ is N(CH₂CH₂OMe)-CH₂CH=CH₂, Ar is 4-methoxy-6-methylpyrid-3-yl;
   a compound of Formula (60) wherein R³ is N(CH₂CH₂OMe)Me, Ar is 4-methoxy-6-methylpyrid-3-yl;
   a compound of Formula (60) wherein R³ is N(CH₂CH₂OMe)Et, Ar is 4-methoxy-6-methylpyrid-3-yl;
   a compound of Formula (60) wherein R³ is N(CH₂CH₂OMe)Pr, Ar is 4-methoxy-6-methylpyrid-3-yl;
   a compound of Formula (60) wherein R³ is N(CH₂CH₂OMe)-CH₂cPr, Ar is 4-methoxy-6-methylpyrid-3-yl;
   a compound of Formula (60) wherein R³ is NHCH(CH₃)CH₂CH₃, Ar is 4-methoxy-6-methylpyrid-3-yl;
   a compound of Formula (60) wherein R³ is NHCH(cPr)₂, Ar is 4-methoxy-6-methylpyrid-3-yl;
   a compound of Formula (60) wherein R³ is N(CH₂CH₂OMe)₂, Ar is 4-methoxy-6-methylpyrid-3-yl;
   a compound of Formula (60) wherein R³ is NHCH(Et)₂, Ar is 6- methoxy -4-methylpyrid-3-yl;
   a compound of Formula (60) wherein R³ is N(Et)₂, Ar is 4-methoxy-6-methylpyrid-3-yl;
   a compound of Formula (60) wherein R³ is 2-ethylpiperid-1-yl, Ar is 4,6-dimethylpyrid-3-yl;
   a compound of Formula (60) wherein R³ is cyclobutylamino, Ar is 4,6-dimethylpyrid-3-yl;
   a compound of Formula (60) wherein R³ is N(Me)CH₂CH=CH₂, Ar is 4,6-dimethylpyrid-3-yl;
   a compound of Formula (60) wherein R³ is N(Et)CH₂cPr, Ar is 4,6-dimethylpyrid-3-yl;
   a compound of Formula (60) wherein R³ is N(Pr)CH₂cPr, Ar is 4,6-dimethylpyrid-3-yl;
   a compound of Formula (60) wherein R³ is N(Me)Pr, Ar is 4,6-dimethylpyrid-3-yl;
   a compound of Formula (60) wherein R³ is N(Me)Et Ar is 4,6-dimethylpyrid-3-yl;
   a compound of Formula (60) wherein R³ is N(Me)Bu, Ar is 4,6-dimethylpyrid-3-yl;
   a compound of Formula (60) wherein R³ is N(Me)propargyl, Ar is 4,6-dimethylpyrid-3-yl;
   a compound of Formula (60) wherein R³ is N(Et)propargyl, Ar is 4,6-dimethylpyrid-3-yl;
   a compound of Formula (60) wherein R³ is NHCH(CH₃)CH(CH₃)CH₃, Ar is 4,6-dimethylpyrid-3-yl;
   a compound of Formula (60) wherein R³ is N(CH₂CH₂OMe)-CH₂CH=CH₂, Ar is 4,6-dimethylpyrid-3-yl;
   a compound of Formula (60) wherein R³ is N(CH₂CH₂OMe)Me, Ar is 4,6-dimethylpyrid-3-yl;
   a compound of Formula (60) wherein R³ is N(CH₂CH₂OMe)Et, Ar is 4,6-dimethylpyrid-3-yl;
   a compound of Formula (60) wherein R³ is N(CH₂CH₂OMe)Pr, Ar is 4,6-dimethylpyrid-3-yl;
   a compound of Formula (60) wherein R³ is N(CH₂CH₂O_{M}e)-CH₂cPr, Ar is 4,6-dimethylpyrid-3-yl;
   a compound of Formula (60) wherein R³ is NHCH(CH₃)CH₂CH₃, Ar is 4,6-dimethylpyrid-3-yl;
   a compound of Formula (60) wherein R³ is NHCH(cPr)₂, Ar is 4,6-dimethylpyrid-3-yl;
   a compound of Formula (60) wherein R³ is N(CH₂CH₂OMe)₂, Ar is 4,6-dimethylpyrid-3-yl;
   a compound of Formula (60) wherein R³ is NHCH(Et)₂, Ar is 4,6-dimethylpyrid-3-yl;
   a compound of Formula (60) wherein R³ is N(Et)₂, Ar is 4,6-dimethylpyrid-3-yl;
   a compound of Formula (60) wherein R³ is 2-ethylpiperid-1-yl, Ar is 2,6-dimethylpyrid-3-yl;
   a compound of Formula (60) wherein R³ is cyclobutylamino, Ar is 2,6-dimethylpyrid-3-yl;
   a compound of Formula (60) wherein R³ is N(Me)CH₂CH=CH₂, Ar is 2,6-dimethylpyrid-3-yl;
   a compound of Formula (60) wherein R³ is N(Et)CH₂cPr, Ar is 2,6-dimethylpyrid-3-yl;
   a compound of Formula (60) wherein R³ is N(Pr)CH₂cPr, Ar is 2,6-dimethylpyrid-3-yl;
   a compound of Formula (60) wherein R³ is N(Me)Pr, Ar is 2,6-dimethylpyrid-3-yl;
   a compound of Formula (60) wherein R³ is N(Me)Et, Ar is 2,6-dimethylpyrid-3-yl;
   a compound of Formula (60) wherein R³ is N(Me)Bu, Ar is 2,6-dimethylpyrid-3-yl;
   a compound of Formula (60) wherein R³ is N(Me)propargyl, Ar is 2,6-dimethylpyrid-3-yl;
   a compound of Formula (60) wherein R³ is NHCH(CH₃)CH(CH₃)CH₃, Ar is 2,6-dimethylpyrid-3-yl;
   a compound of Formula (60) wherein R³ is N(CH₂CH₂OMe)-CH₂CH=CH₂, Ar is 2,6-dimethylpyrid-3-yl;
   a compound of Formula (60) wherein R³ is N(CH₂CH₂OMe)Me, Ar is 2,6-dimethylpyrid-3-yl;
   a compound of Formula (60) wherein R³ is N(CH₂CH₂OMe)Et, Ar is 2,6-dimethylpyrid-3-yl;
   a compound of Formula (60) wherein R³ is N(CH₂CH₂OMe)Pr, Ar is 2,6-dimethylpyrid-3-yl;
   a compound of Formula (60) wherein R³ is N(CH₂CH₂OMe)-CH₂cPr, Ar is 2,6-dimethylpyrid-3-yl;
   a compound of Formula (60) wherein R³ is NH(CH(CH₃)CH₂CH₃, Ar is 2,6-dimethylpyrid-3-yl; and
   a compound of Formula (60) wherein R³ is NHCH(cPr)₂, Ar is 2,6-dimethylpyrid-3-yl.
[3] Specifically preferred compounds of the present invention include compounds and geometric isomers thereof, stereoisomeric forms thereof, or mixtures of stereoisomeric forms thereof, and pharmaceutically acceptable salt forms thereof, wherein said compound is selected from the group:
   4-((2-butyl)amino)-2,7-dimethyl-8-(2-methyl-4-methoxyphenyl)-[1,5-a]-pyrazolo-1,3,5-triazine;
   4-((2-butyl)amino)-2,7-dimethyl-8-(2,5-dimethyl-4-methoxyphenyl)-[1,5-a]-pyrazolo-1,3,5-triazine;
   4-((3-pentyl)amino)-2,7-dimethyl-8-(2-methyl-4-methoxyphenyl)-[1,5-a]-pyrazolo-1,3,5-triazine;
   4-(N-cyclopropylmethyl-N-propylamino)-2,7-dimethyl-8- (2-methyl-4-methoxyphenyl)-[1,5-a]-pyrazolo-1,3,5-triazine;
   4-(N-cyclopropylmethyl-N-propylamino)-2,7-dimethyl-8-(2,5-dimethyl-4-methoxyphenyl)-[1,5-a]-pyrazolo-1,3,5-triazine;
   4-(N-allyl-N-(2-methoxyethyl)amino)-2,7-dimethyl-8-(2-methyl-4-methoxyphenyl)-[1,5-a]-pyrazolo-1,3,5-triazine;
   4-( N-allyl-N-(2-methoxyethyl) amino) -2, 7-dimethyl-8-(2,5-dimethyl-4-methoxyphenyl)-[1,5-a]-pyrazolo-1,3,5-triazine;
   4-(N-ethyl-N-(2-methoxyethyl)amino)-2,7-dimethyl-8-(2-methyl-4-methoxyphenyl)-[1,5-a]-pyrazolo-1,3,5-triazine; and
   4-( N-ethyl-N-(2-methoxyethyl)amino)-2,7-dimethyl-8-(2,5-dimethyl-4-methoxyphenyl)-[1,5-a]-pyrazolo-1,3,5-triazine.
[6] The present invention also provides pharmaceutical compositions comprising a therapeutically effective amount of the above-described compounds and a pharmaceutically acceptable carrier.
[7] The present invention still further provides methods of treating affective disorder, anxiety, depression, headache, irritable bowel syndrome, post-traumatic stress disorder, supranuclear palsy, immune suppression, Alzheimer's disease, gastrointestinal diseases, anorexia nervosa or other feeding disorder, drug addiction, drug or alcohol withdrawal symptoms, inflammatory diseases, cardiovascular or heart-related diseases, fertility problems, human immunodeficiency virus infections, hemorrhagic stress, obesity, infertility, head and spinal cord traumas, epilepsy, stroke, ulcers, amyotrophic lateral sclerosis, hypoglycemia or a disorder the treatment of which can be effected or facilitated by antagonizing CRF, including but not limited to disorders induced or facilitated by CRF, in mammals comprising administering to the mammal a therapeutically effective amount of the above-described compounds.

Many compounds of this invention have one or more asymmetric centers or planes. Unless otherwise indicated, all chiral (enantiomeric and diastereomeric) and racemic forms are included in the present invention. Many geometric isomers of olefins, C=N double bonds, and the like can also be present in the compounds, and all such stable isomers are contemplated in the present invention. The compounds may be isolated in optically active or racemic forms. It is well known in the art how to prepare optically active forms, such as by resolution of racemic forms or by synthesis from optically active starting materials. All chiral, (enantiomeric and diastereomeric) and racemic forms and all geometric isomeric forms of a structure are intended, unless the specific stereochemistry or isomer form is specifically indicated.

The term "alkyl" includes both branched and straight-chain alkyl having the specified number of carbon atoms. Commonly used abbreviations have the following meanings: Me is methyl, Et is ethyl, Pr is propyl, Bu is butyl. The prefix "n" means a straight chain alkyl. The prefix "c" means a cycloalkyl. The prefix "(*S*)" means the S enantiomer and the prefix "(*R*)" means the R enantiomer. Alkenyl" includes hydrocarbon chains of either a straight or branched configuration and one or more unsaturated carbon-carbon bonds which may occur in any stable point along the chain, such as ethenyl, propenyl, and the like. "Alkynyl" includes hydrocarbon chains of either a straight or branched configuration and one or more triple carbon-carbon bonds which may occur in any stable point along the chain, such as ethynyl, propynyl and the like. "Haloalkyl" is intended to include both branched and straight-chain alkyl having the specified number of carbon atoms, substituted with 1 or more halogen; "alkoxy" represents an alkyl group of indicated number of carbon atoms attached through an oxygen bridge; "cycloalkyl" is intended to include saturated ring groups, including mono-,bi- or polycyclic ring systems, such as cyclopropyl, cyclobutyl, cyclopentyl, cyclohexyl, and so forth. "Halo" or "halogen" includes fluoro, chloro, bromo, and iodo.

The term "substituted", as used herein, means that one or more hydrogen on the designated atom is replaced with a selection from the indicated group, provided that the designated atom's normal valency is not exceeded, and that the substitution results in a stable compound. when a substitent is keto (i.e., =O), then 2 hydrogens on the atom are replaced.

Combinations of substituents and/or variables are permissible only if such combinations result in stable compounds. By "stable compound" or "stable structure" is meant a compound that is sufficiently robust to survive isolation to a useful degree of purity from a reaction mixture, and formulation into an efficacious therapeutic agent.

The term "appropriate amino acid protecting group" means any group known in the art of organic synthesis for the protection of amine or carboxylic acid groups. Such amine protecting groups include those listed in Greene and Wuts, "Protective Groups in Organic Synthesis" John Wiley & Sons, New York (1991) and "The Peptides: Analysis, Synthesis, Biology, Vol. 3, Academic Press, New York (1981), the disclosure of which is hereby incorporated by reference. Any amine protecting group known in the art can be used. Examples of amine protecting groups include, but are not limited to, the following: 1) acyl types such as formyl, trifluoroacetyl, phthalyl, and p-toluenesulfonyl; 2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); 3) aliphatic carbamate types such as tert-butyloxycarbonyl (Boc), ethoxycarbonyl, diisopropylmethoxycarbonyl, and allyloxycarbonyl; 4) cyclic alkyl carbamate types such as cyclopentyloxycarbonyl and adamantyloxycarbonyl; 5) alkyl types such as triphenylmethyl and benzyl; 6) trialkylsilane such as trimethylsilane; and 7) thiol containing types such as phenylthiocarbonyl and dithiasuccinoyl.

The term "pharmaceutically acceptable salts" includes acid or base salts of the compounds within Formula (1). Examples of pharmaceutically acceptable salts include, but are not limited to, mineral or organic acid salts of basic residues such as amines; alkali or organic salts of acidic residues such as carboxylic acids; and the like.

Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical sciences, 17th ed., Mack Publishing Company, Easton, PA, 1985, p. 1418, the disclosure of which is hereby incorporated by reference. "Prodrugs" are considered to be any covalently bonded carriers which release the active parent drug within formula (I) *in vivo* when such prodrug is administered to a mammalian subject. Prodrugs of the compounds within formula (I) are prepared by modifying functional groups present in the compounds in such a way that the modifications are cleaved, either in routine manipulation or *in vivo*, to the parent compounds. Prodrugs include compounds wherein hydroxy, amine, or sulfhydryl groups are bonded to any group that, when administered to a mammalian subject, cleaves to form a free hydroxyl, amino, or sulfhydryl group, respectively. Examples of prodrugs include, but are not limited to, acetate, formate and benzoate derivatives of alcohol and amine functional groups in the compounds within formula (I) ; and the like.

The term "therapeutically effective amount" of a compound of this invention means an amount effective to antagonize abnormal level of CRF or treat the symptoms of affective disorder, anxiety or depression in a host.

### Syntheses

Some compounds of Formula (1) may be prepared from intermediate compounds of Formula (7), using the procedures outlined in Scheme 1: 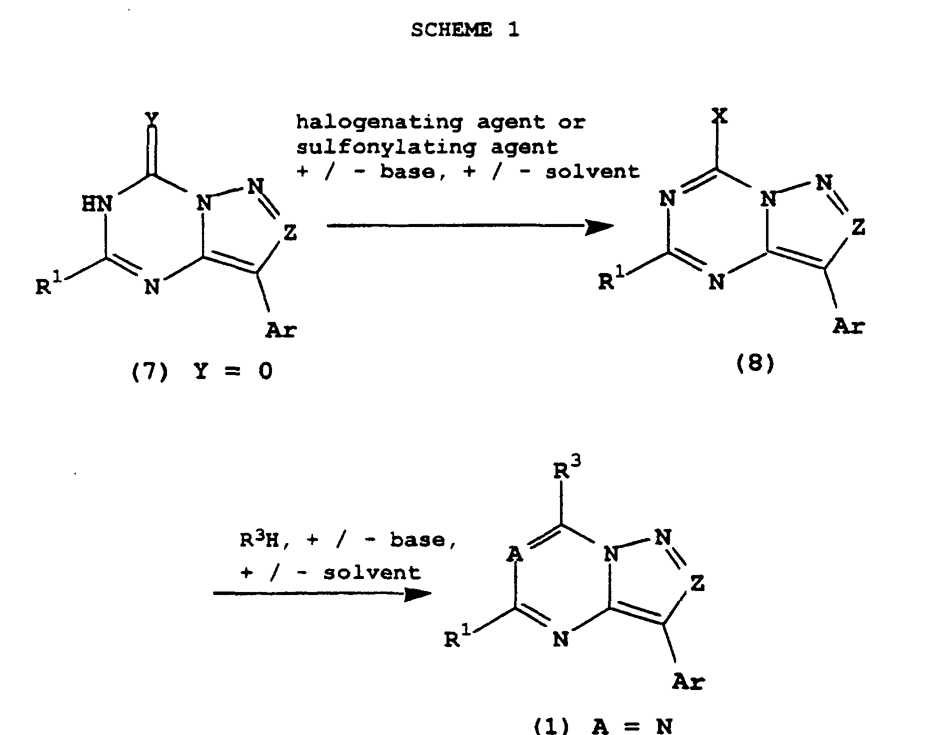 Compounds of Formula (7) (where Y is O) may be treated with a halogenating agent or sulfonylating agent in the presence or absence of a base in the presence or absence of an inert solvent at reaction temperatures ranging from -80°C to 250°C to give products of Formula (8) (where X is halogen, alkanesulfonyloxy, arylsulfonyloxy or haloalkane-sulfonyloxy). Halogenating agents include, but are not limited to, SOCl₂, POCl₃, PCl₃, PCl₅, POBr₃, PBr₃ or PBr₅. Sulfonylating agents include, but are not limited to, alkanesulfonyl halides or anhydrides (such as methanesulfonyl chloride or methanesulfonic acid anhydride), arylsulfonyl halides or anhydrides (such as p-toluenesulfonyl chloride or anhydride) or haloalkylsulfonyl halides or anhydrides (preferably trifluoromethanesulfonic anhydride). Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal bis(trialkylsilyl)amides (preferably sodium bis(trimethylsilyl)amide), trialkyl amines (preferably N,N-di-isopropyl-N-ethyl amine or triethylamine) or aromatic amines (preferably pyridine). Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from -20°C to 100°C.

Compounds of Formula (8) may be reacted with compounds of Formula R³H (where R³ is defined as above except R³ is not SH, COR⁷, CO₂R⁷, aryl or heteroaryl) in the presence or absence of a base in the presence or absence of an inert solvent at reaction temperatures ranging from -80 to 250°C to generate compounds of Formula (1). Bases may include, but are not limited to, alkali metal hydrides (preferably sodium hydride) , alkali metal alkoxides (1 to 6 carbons) (preferably sodium methoxide or sodium ethoxide), alkaline earth metal hydrides, alkali metal dialkylamides (preferably lithium di-isopropylamide), alkali metal carbonates, alkali metal bicarbonates, alkali metal bis(trialkylsilyl)amides (preferably sodium bis(trimethylsilyl)amide), trialkyl amines (preferably N,N-di-isopropyl-N-ethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 0°C to 140°C.

Scheme 2 delineates the procedures for converting intermediate compounds of Formula (7) (where Y is S) to some compounds of Formula (1). 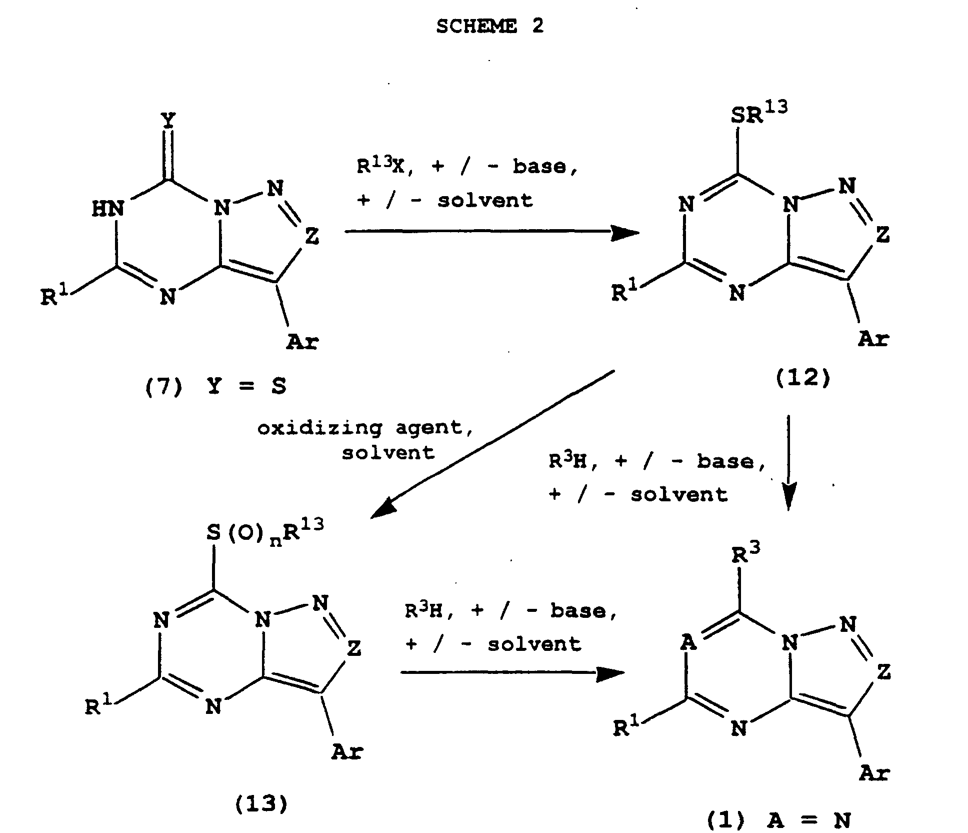 Compounds of Formula (7) (where Y is S) may be treated with an alkylating agent R¹³X (where R¹³ is defined as above, except R¹³ is not aryl or heteroaryl) in the presence or absence of a base in the presence or absence of an inert solvent at reaction temperatures ranging from -80°C to 250°C. Bases may include, but are not limited to, alkali metal hydrides (preferably sodium hydride), alkali metal alkoxides (1 to 6 carbons) (preferably sodium methoxide or sodium ethoxide), alkaline earth metal hydrides, alkali metal dialkylamides (preferably lithium di-isopropylamide), alkali metal carbonates, alkali metal hydroxides, alkali metal bis(trialkylsilyl)amides (preferably sodium bis(trimethylsilyl)amide), trialkyl amines (prefereably N,N-di-isopropyl-N-ethyl amine or triethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from -80°C to 100°C.

Compounds of Formula (12) (Formula (1) where R³ is SR¹³) may then be reacted with compounds of Formula R³H to give compounds of Formula (1), using the same conditions and reagents as were used for the conversion of compounds of Formula (8) to compounds of Formula (1) as outlined for Scheme 1 above. Alternatively, compounds of Formula (12) (Formula (1) where R³ is SR¹³) may be oxidized to compounds of Formula (13) (Formula (1) where R³ is S(O)ₙR¹²' n is 1,2) by treatment with an oxidizing agent in the presence of an inert solvent at temperatures ranging from -80°C to 250°C. Oxidizing agents include, but are not limited to, hydrogen peroxide, alkane or aryl peracids (preferably peracetic acid or m-chloro-perbenzoic acid), dioxirane, oxone, or sodium periodate. Inert solvents may include, but are not limited to, alkanones (3 to 10 carbons, preferably acetone), water, alkyl alcohols (1 to 6 carbons), aromatic hydrocarbons (preferably benzene or toluene) or haloalkanes of 1 to 10 carbons and 1 to 10 halogens (preferably dichloromethane) or combinations thereof. The choices of oxidant and solvent are known to those skilled in the art (cf. Uemura, S.; Oxidation of Sulfur, Selenium and Tellurium, in Comprehensive Organic Synthesis, Trost, B.M. ed., (Elmsford, NY: Pergamon Press, 1991), 7, 762-769). Preferred reaction temperatures range from -20°C to 100°C. Compounds of Formula (13) (Formula (1) where R³ is S(O)ₙR¹³' n is 1,2) may then be reacted with compounds of Formula R³H to give compounds of Formula (1), using the same conditions and reagents as were used for the conversion of compounds of Formula (8) to compounds of Formula (1) as outlined for Scheme (1) above.

Compounds of Formula (1), where R³ may be -NR⁸COR⁷, -N(COR⁷)₂, -NR⁸CONR⁶R⁷, -NR⁸CO₂R¹³, -NR⁶R⁷' -NR⁸SO₂R⁷, may be prepared from compounds of Formula (7), where Y is NH, by the procedures depicted in Scheme 3. 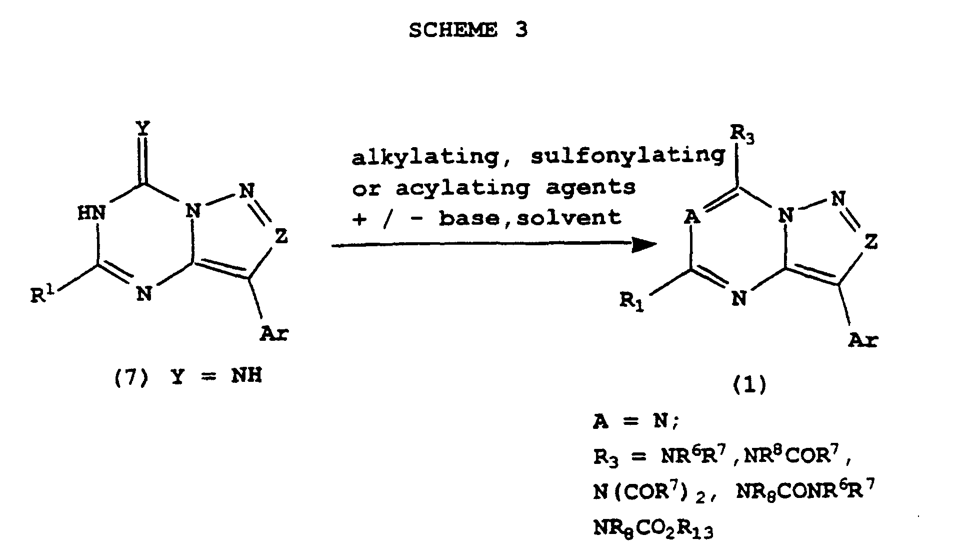 Reaction of compounds of Formula (7), where Y is NH, with alkylating agents, sulfonylating agents or acylating agents or sequential reactions with combinations thereof, in the presence or absence of a base in an inert solvent at reaction temperatures ranging from -80°C to 250°C may afford compounds of Formula (1), where R³ may be -NR⁸COR⁷, -N(COR⁷)₂, -NR⁸CONR⁶R⁷, -NR⁸CO₂R¹³, -NR⁶R⁷, -NR⁸SO₂R⁷. Alkylating agents may include, but are not limited to, C₁-C₁₀ alkyl -halides, -tosylates, -mesylates or -triflates; C₁-C₁₀ haloalkyl(1 - 10 halogens)-halides, -tosylates, - mesylates or -triflates; C₂-C₈ alkoxyalkyl-halides,-tosylates, -mesylates or -triflates; C₃-C₆ cycloalkyl-halides, -tosylates, -mesylates or -triflates; C₄-C₁₂ cycloalkylalkyl-halides, -tosylates, -mesylates or - triflates; aryl(C₁-C₄ alkyl)-halides, -tosylates,-mesylates or -triflates; heteroaryl(C₁-C₄ alkyl)-halides, -tosylates, -mesylates or -triflates; or heterocyclyl(C₁-C₄ alkyl)-halides, -tosylates,-mesylates or -triflates. Acylating agents may include, but are not limited to, C₁-C₁₀ alkanoyl halides or anhydrides, C₁-C₁₀ haloalkanoyl halides or anhydrides with 1 - 10 halogens, C₂-C₈ alkoxyalkanoyl halides or anhydrides, C₃-C₆ cycloalkanoyl halides or anhydrides, C₄-C₁₂ cycloalkylalkanoyl halides or anhydrides, aroyl halides or anhydrides, aryl(C₁-C₄) alkanoyl halides or anhydrides, heteroaroyl halides or anhydrides, heteroaryl(C₁-C₄) alkanoyl halides or anhydrides, heterocyclylcarboxylic acid halides or anhydrides or heterocyclyl(C₁-C₄) alkanoyl halides or anhydrides. Sulfonylating agents include, but are not limited to, C₁-C₁₀ alkylsulfonyl halides or anhydrides, C₁-C₁₀ haloalkylsulfonyl halides or anhydrides with 1 - 10 halogens, C₂-C₈ alkoxyalkylsulfonyl halides or anhydrides, C₃-C₆ cycloalkylsulfonyl halides or anhydrides, C₄-C₁₂ cycloalkylalkylsulfonyl halides or anhydrides, arylsulfonyl halides or anhydrides, aryl(C₁-C₄ alkyl)-, heteroarylsulfonyl halides or anhydrides, heteroaryl(C₁-C₄ alkyl)sulfonyl halides or anhydrides, heterocyclylsulfonyl halides or anhydrides or heterocyclyl(C₁-C₄ alkyl)sulfonyl halides or anhydrides. Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal bis(trialkylsilyl)amides (preferably sodium bis(trimethylsilyl)amide), trialkyl amines (prefereably di-isopropylethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred reaction temperatures range from 0°C to 100°C.

Scheme 4 delineates procedures, which may be employed to prepare intermediate compounds of Formula (7), where Y is O, S and Z is CR². 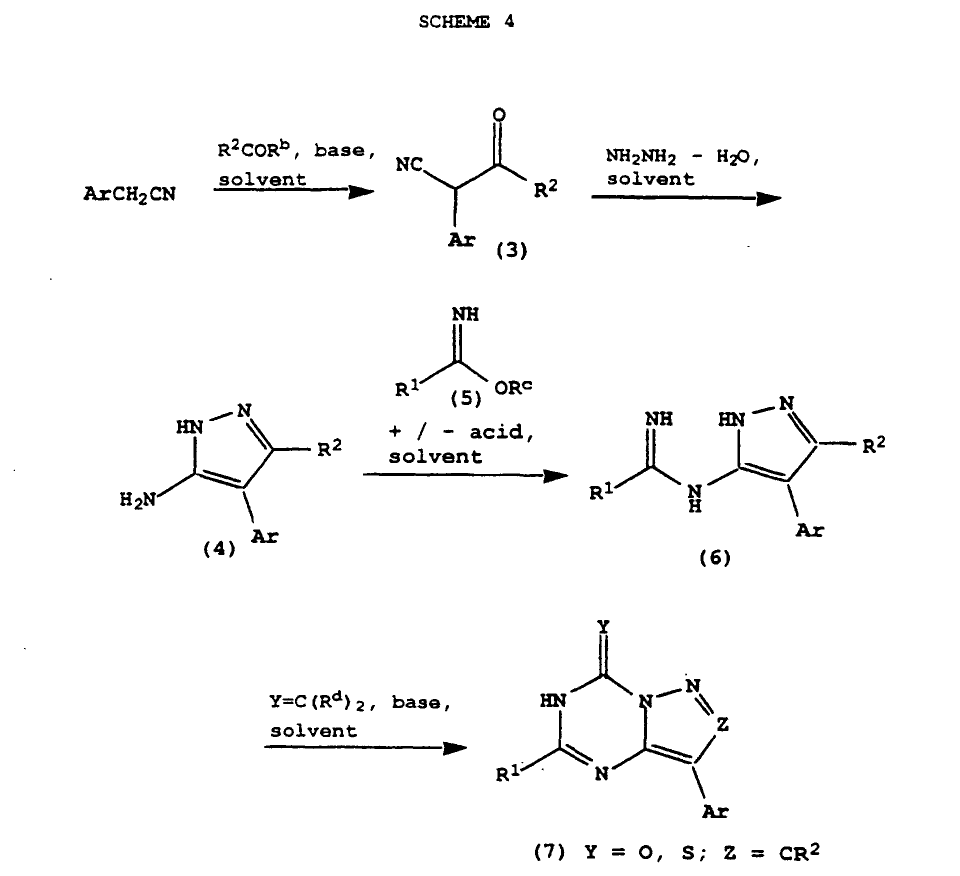 Compounds of the formula ArCH₂CN are reacted with compounds of the formula R²COR^{b}, where R² is defined above and R^{b} is halogen, cyano, lower alkoxy (1 to 6 carbons) or lower alkanoyloxy (1 to 6 carbons), in the presence of a base in an inert solvent at reaction temperatures ranging from -78°C to 200°C to afford compounds of Formula (3). Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal hydroxides, alkali metal bis(trialkylsilyl)amides (preferably sodium bis(trimethylsilyl)amide), trialkyl amines (preferably N,N-di-isopropyl-N-ethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), water, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred reaction temperatures range from 0°C to 100°C.

Compounds of Formula (3) may be treated with hydrazine-hydrate in the presence of an inert solvent at temperatures ranging from 0°C to 200°C, preferably 70°C to 150°C, to produce compounds of Formula (4). Inert solvents may include, but are not limited to, water, alkyl alcohols (1 to 8 carbons, preferably methanol or ethanol), lower alkanenitriles (1 to 6 carbons, preferably acetonitrile), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Compounds of Formula (4) may be reacted with compounds of Formula (5) (where R^{c} is alkyl (1-6 carbons)) in the presence or absence of an acid in the presence of an inert solvent at temperatures ranging from 0°C to 200°C to produce compounds of Formula (6). Acids may include, but are not limited to alkanoic acids of 2 to 10 carbons (preferably acetic acid), haloalkanoic acids (2 - 10 carbons, 1-10 halogens, such as trifluoroacetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, water, alkanenitriles (1 to 6 carbons, preferably acetonitrile), halocarbons of 1 to 6 carbons and 1 to 6 halogens (preferably dichloromethane or chloroform), alkyl alcohols of 1 to 10 carbons (preferably ethanol), dialkyl ethers (4 to 12 carbons, preferably diethyl ether or di-isopropylether) or cyclic ethers such as dioxan or tetrahydrofuran. Preferred temperatures range from ambient temprature to 100°C.

Compounds of Formula (6) may be converted to intermediate compounds of Formula (7) by treatment with compounds C=Y(R^{d})₂ (where Y is O or S and R^{d} is halogen (preferably chlorine), alkoxy (1 to 4 carbons) or alkylthio (1 to 4 carbons)) in the presence or absence of a base in an inert solvent at reaction temperatures from -50°C to 200°C. Bases may include, but are not limited to, alkali metal hydrides (preferably sodium hydride), alkali metal alkoxides (1 to 6 carbons)(preferably sodium methoxide or sodium ethoxide), alkali metal carbonates, alkali metal hydroxides, trialkyl amines (preferably N,N-di-isopropyl-N-ethyl amine or triethylamine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred temperatures are 0°C to 150°C.

Compounds of Formula (1) may also be prepared from compounds of Formula (7) (where Y is O, S and Z is defined above) as outlined in Scheme 6: 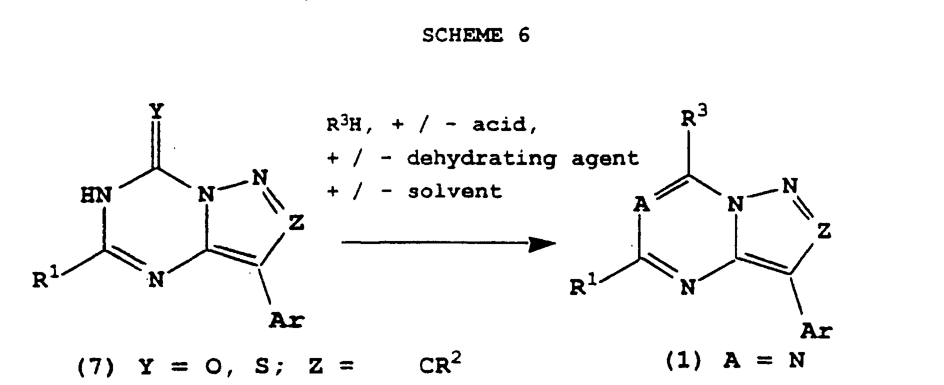 Compounds of Formula (7) may be reacted with compounds of Formula R³H in the presence of a dehydrating agent in an inert solvent at reaction temperatures ranging from 0°C to 250°C. Dehydrating agents include, but are not limited to, P₂O₅, molecular sieves or inorganic or organic acids. Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Inert solvents may include, but are not limited to, alkyl alcohols (1 to 8 carbons, preferably methanol or ethanol), lower alkanenitriles (1 to 6 carbons, preferably acetonitrile), dialkyl ethers (preferably glyme or diglyme), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or halocarbons of 1 to 10 carbons and 1 to 10 halogens (preferably chloroform). Preferred reaction temperatures range from ambient temperature to 150°C.

Some compounds of Formula (1) (where A is N) may also be prepared by the methods shown in Scheme 7: 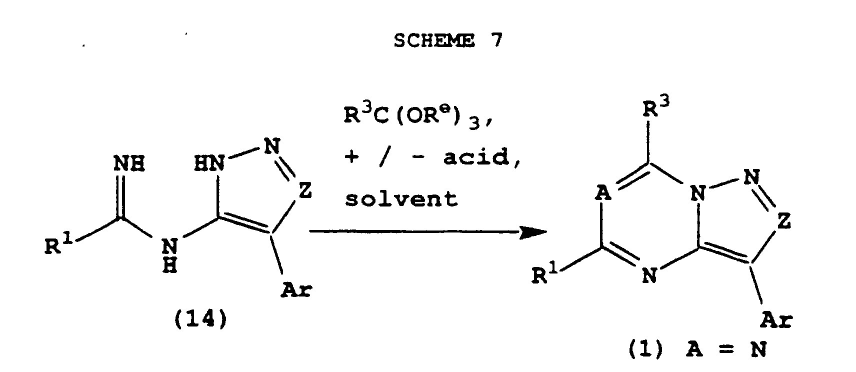 Intermediate compounds of Formula (14), where Z is defined above, may be reacted with compounds of Formula R³C(OR^{e})3, where R^{e} may be alkyl (1 to 6 carbons) in the presence or absence of an acid in an inert solvent at temperatures ranging from 0°C to 250°C. Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 50°C to 150°C.

Intermediate compounds of Formula (7) may also be synthesized by the reactions displayed in Scheme 8. 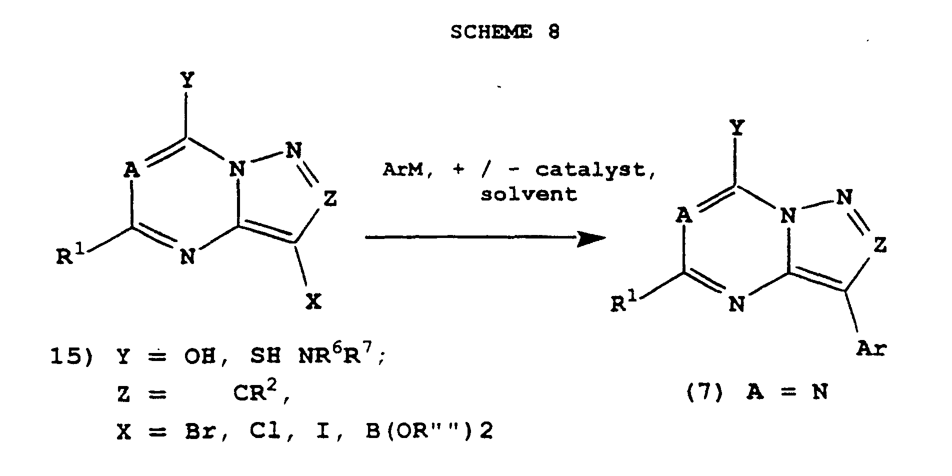 Compounds of Formula (15), (where Y is OH, SH, NR⁶R⁷; Z is defined above, X is Br, Cl, I, O₃SCF₃ or B(OR"")₂ and R"" is H or alkyl (1 to 6 carbons)) may be reacted with a compound of Formula ArM (where M is halogen, alkali metal, ZnCl, ZnBr, ZnI, MgBr, MgCl, MgI, CeCl₂, CeBr₂ or copper halides) in the presence or absence of an organometallic catalyst in the presence or absence of a base in an inert solvents at temperatures ranging from -100°C to 200°C. Those skilled in the art will recognize that the reagents ArM may be generated in situ. Organometallic catalysts include, but are not limited to, palladium phosphine complexes (such as Pd(PPh₃)₄), palladium halides or alkanoates (such as PdCl₂(PPh₃)₂ or Pd(OAc)₂) or nickel complexes (such as NiCl₂(PPh₃)₂). Bases may include, but are not limited to, alkali metal carbonates or trialkyl amines (preferably N,N-di-isopropyl-N-ethyl amine or triethylamine). Inert solvents may include, but are not limited to, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or water. Preferred reaction temperatures range from -80°C to 100°C. The choices of M and X are known to those skilled in the art (cf. Imamoto, T., Organocerium Reagents in Comprehensive Organic Synthesis, Trost, B.M. ed., (Elmsford, NY: Pergamon Press, 1991), 1, 231-250; Knochel, P., Organozinc, Organocadmium and Organomercury Reagents in Comprehensive Organic Synthesis, Trost, B.M. ed., (Elmsford, NY: Pergamon Press, 1991), 1, 211-230; Knight, D.W., Coupling Reactions between sp² Carbon Centers, in Comprehensive Organic Synthesis, Trost, B.M. ed., (Elmsford, NY: Pergamon Press, 1991), 3, 481-520).

Compounds of Formula (1) may also be prepared using the methods shown in Scheme 9. 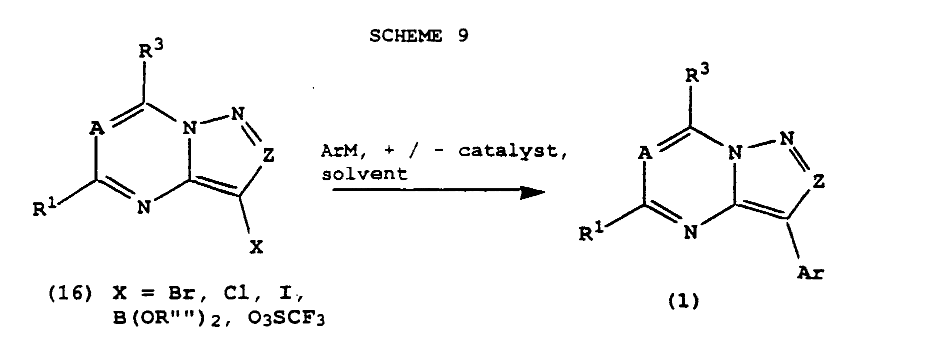 Compounds of Formula (16), where A, Z, R¹ and R³ are defined above and X is Br, Cl, I, O₃SCF₃ or B(OR"")₂ and R"" is H or alkyl (1 to 6 carbons)) may be reacted with a compound of Formula ArM (where M is halogen, alkali metal, ZnCl, ZnBr, ZnI, MgBr, MgCl, MgI, CeCl₂, CeBr₂ or copper halides) in the presence or absence of an organometallic catalyst in the presence or absence of a base in an inert solvents at temperatures ranging from -100°C to 200°C. Those skilled in the art will recognize that the reagents ArM may be generated in situ (see the above references in Comprehensive Organic Synthesis). Organometallic catalysts include, but are not limited to, palladium phosphine complexes (such as Pd(PPh₃)₄), palladium halides or alkanoates (such as PdCl₂(PPh₃)₂ or Pd(OAc)₂) or nickel complexes (such as NiCl₂(PPh₃)₂). Bases may include, but are not limited to, alkali metal carbonates or trialkyl amines (preferably N,N-di-isopropyl-N-ethyl amine or triethylamine). Inert solvents may include, but are not limited to, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or water. Preferred reaction temperatures range from -80°C to 100°C.

Intermediate compounds of Formula (7) (where Y is O, S, NH, Z is CR² and R¹, R² and Ar are defined as above) may be prepared as illustrated in Scheme 10. 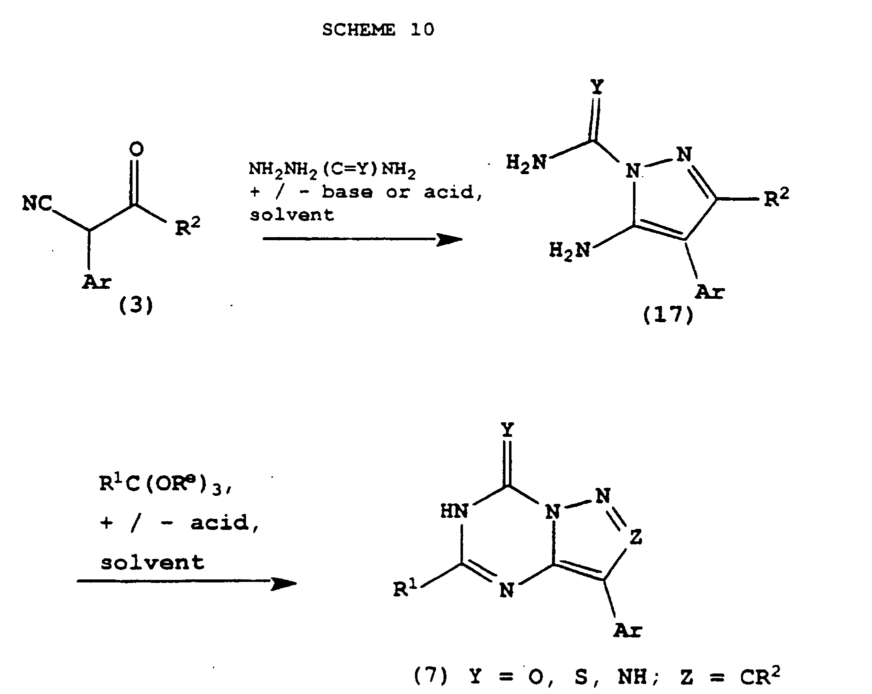 Compounds of Formula (3) may be reacted with compounds of Formula H₂NNH(C=Y)NH₂, where Y is O, S or NH, in the presence or absence of a base or acid in an inert solvent at temperatures from 0°C to 250°C to produce compounds of Formula (17). Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal bis(trialkylsilyl)amides (preferably sodium bis(trimethylsilyl)amide), trialkyl amines (preferably N,N-di-isopropyl-N-ethyl amine or triethylamine) or aromatic amines (preferably pyridine). Inert solvents may include, but are not limited to, alkyl alcohols (1 to 6 carbons), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane).

Preferred reaction temperatures range from 0°C to 150°C. Compounds of Formula (17) may then be reacted with compounds of Formula R³C(OR^{e})3, where R^{e} may be alkyl (1 to 6 carbons) in the presence or absence of an acid in an inert solvent at temperatures ranging from 0°C to 250°C. Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 50°C to 150°C.

In Scheme 11, the procedures which may be used to convert compounds of Formula (1), where R³ is COR⁷, CO₂R⁷, NR⁸COR⁷ and CONR⁶R⁷, to other compounds of Formula (1), where R³ is CH(OH)R⁷, CH₂OH, NR⁸CH₂R⁷ and CH₂NR⁶R⁷ by treatment with a reducing agent in an inert solvent at temperatures ranging from -80°C to 250°C. 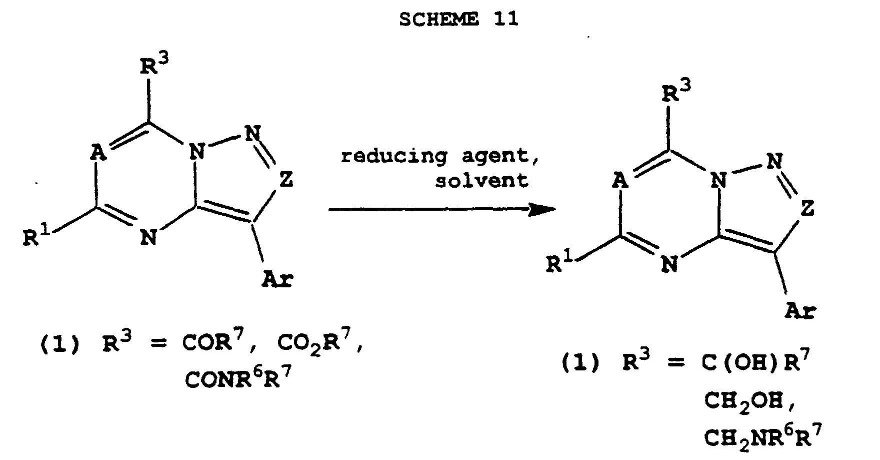 Reducing agents include, but are not limited to, alkali metal or alkaline earth metal borohydrides (preferably lithium or sodium borohydride), borane, dialkylboranes (such as di-isoamylborane), alkali metal aluminum hydrides (preferably lithium aluminum hydride), alkali metal (trialkoxy)aluminum hydrides, or dialkyl aluminum hydrides (such as di-isobutylaluminum hydride). Inert solvents may include, but are not limited to, alkyl alcohols (1 to 6 carbons), dialkyl ethers (preferably diethyl ether), cyclic ethers (preferably tetrahydrofuran or 1,4-dioxane), aromatic hydrocarbons (preferably benzene or toluene). Preferred reaction temperatures range from -80°C to 100°C.

In Scheme 12, the procedures are shown which may be used to convert compounds of Formula (1), where R³ is COR⁷ or CO₂R⁷, to other compounds of Formula (1), where R³ is C(OH)(R⁷)₂ by treatment with a reagent of Formula R⁷M in an inert solvent at temperatures ranging from -80°C to 250°C. 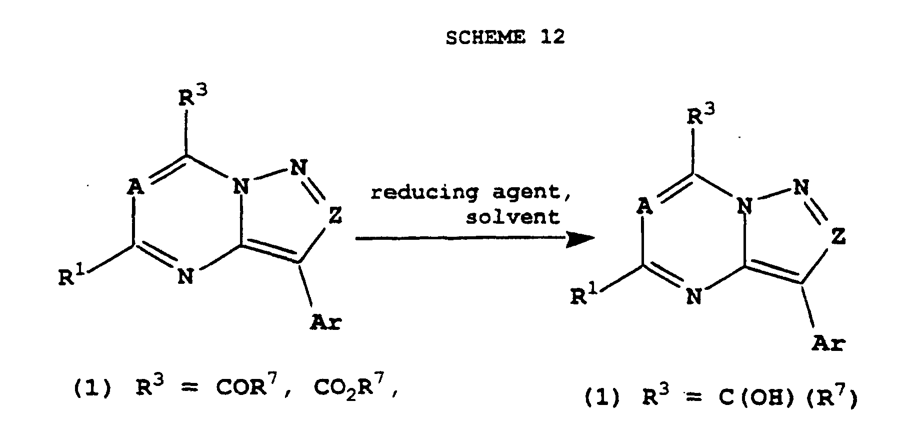 M is halogen, alkali metal, ZnCl, ZnBr, ZnI, MgBr, MgCl, MgI, CeCl₂, CeBr₂ or copper halides. Inert solvents may include, but are not limited to, dialkyl ethers (preferably diethyl ether), cyclic ethers (preferably tetrahydrofuran) or aromatic hydrocarbons (preferably benzene or toluene). Preferred reaction temperatures range from -80°C to 100°C.

Some compounds of Formula (1) may also be prepared using the methods shown in Scheme 15. 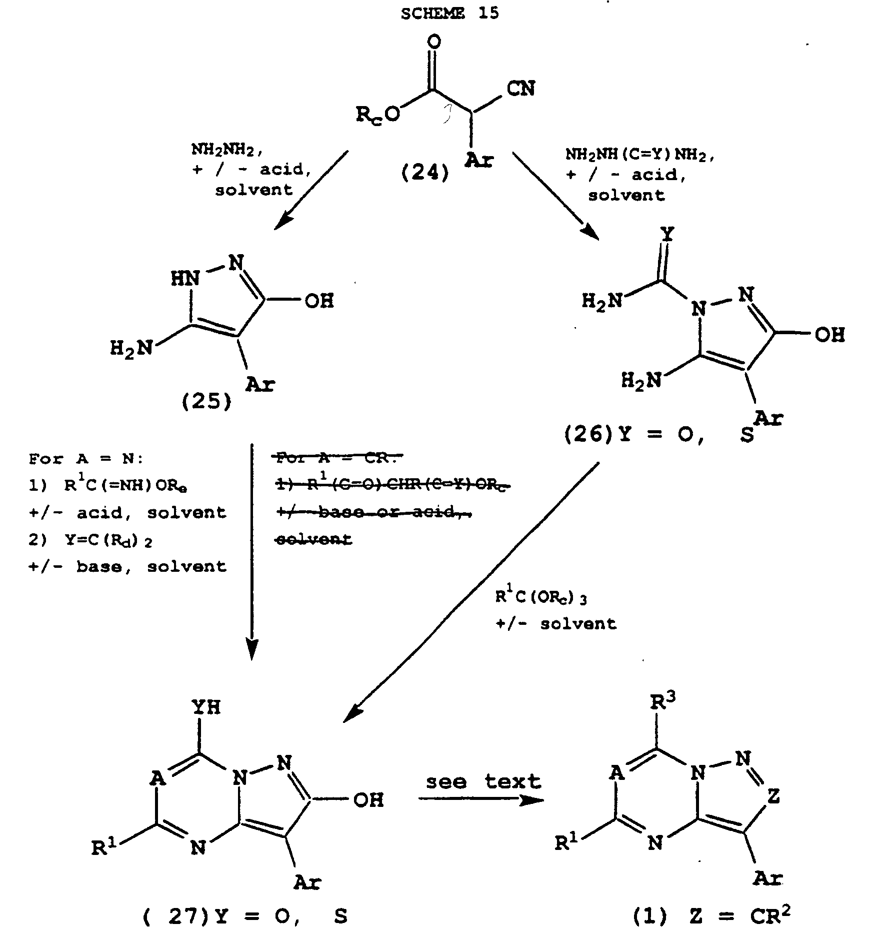 A compound of Formula (24) (R_{c} is a lower alkyl group and Ar is defined as above) may be reacted with hydrazine in the presence or absence of an inert solvent to afford an intermediate of Formula (25), where Ar is defined as above. The conditions employed are similar to those used for the preparation of intermediate of Formula (4) from compound of Formula (3) in Scheme 4. Compounds of Formula (25), where A is N, may be reacted with reagents of the formula R¹C(=NH)ORₑ, where R¹ is defined above and Rₑ is a lower alkyl group) in the presence or absence of an acid in an inert solvent, followed by reaction with a compound of formula YisC(R_{d})2 (where Y is O or S and R^{d} is halogen (preferably chlorine), alkoxy (1 to 4 carbons) or alkylthio (1 to 4 carbons)) in the presence or absence of a base in an inert solvent to give compounds of Formula (27) (where A is N and Y is 0, S). The conditions for these transformations are the same as those employed for the conversions of compound of Formula (4) to compound of Formula (7) in Scheme 4. Intermediates of Formula (27) (where Y is O) may be treated with halogenating agents or sulfonylating agents in the presence or absence of a base in an inert solvent, followed by reaction with R³H or R²H in the presence or absence of a base in an inert solvent to give compounds of Formula (1) (where Z is CR²).

It will be recognized by chose skilled in the art that various combinations of halogenating agents, sulfonylating agents, R³H or R²H may be used in different orders of reaction sequences in Scheme 15 to afford compounds of Formula (1). For example, in some cases, it may be desirable to react compounds with stoichiometric amounts of halogenating agents or sulfonylating agents, react with R²H (or R³H), then repeat the reaction with halogenating agents or sulfonylating agents and react with R³H (or R²H) to give compounds of Formula (1). The reaction conditions and reagents used for these conversions are similar to the ones employed for the conversion of intermediate compounds of Formulae (7) to (8) to (1) in Scheme 1 (where A is N).

Alternatively, compounds of Formula (27) (where Y is S) may be converted to compounds of Formula (1) in Scheme 15. Intermediate compounds of Formula (27) may be alkylated with a compound R^{f}X (where R^{f} is lower alkyl and X is halogen, alkanesulfonyloxy or haloalkanesulfonyloxy) in an inert solvent, (then optionally oxidized with an oxidizing agent in an inert solvent) and then reacted with R³H in the presence or absence of a base in an inert solvent to give a compound of Formula (1). The conditions and reagents employed are similar to those used in the conversion of intermediate compounds of Formulae (7) to (12) (or to (13)) to compounds of Formula (1) in Scheme 2.

Compounds of Formula (1) may be prepared from compounds of Formula (24), using an alternate route as depicted in Scheme 15. Compounds of Formula (24) may be converted to compounds of Formula (27) via reaction with compounds of formula NH₂NH(C=NH)NH₂ in the presence or absence of an acid in an inert solvent, followed by reaction with compounds R¹C(OR_{c})₃ (where R_{c} is lower alkyl and R¹ is defined as above), using the conditions employed for the conversion of compounds of Formulae (3) to (17) to (7) in Scheme 10.

### EXAMPLES

Analytical data were recorded for the compounds described below using the following general procedures. Proton NMR spectra were recorded on an IBM-Bruker FT-NMR (300 MHz); chemical shifts were recorded in ppm (δ) from an internal tetramethysilane standard in deuterochloroform or deuterodimethylsulfoxide as specified below. Mass spectra (MS) or high resolution mass spectra (HRMS) were recorded on a Finnegan MAT 8230 spectrometer (using chemi-ionization (CI) with NH₃ as the carrier gas or gas chromatography (GC) as specified below) or a Hewlett Packard 5988A model spectrometer. Melting points were recorded on a Buchi Model 510 melting point apparatus and are uncorrected. Boiling points are uncorrected. All pH determinations during workup were made with indicator paper.

Reagents were purchased from commercial sources and, where necessary, purified prior to use according to the general procedures outlined by D. Perrin and W.L.F. Armarego, *Purification of Laboratory Chemicals*, 3rd ed., (New York: Pergamon Press, 1988). Chromatography was performed on silica gel using the solvent systems indicated below. For mixed solvent systems, the volume ratios are given. Otherwise, parts and percentages are by weight.

The following examples are provided to describe the invention in further detail. These examples, which set forth the best mode presently contemplated for carrying out the invention, are intended to illustrate and not to limit the invention.

### EXAMPLE 1

### Preparation of 2,7-dimethyl-8-(2,4-dimethylphenyl)[1,5-a] - pyrazolo-[1,3,5]-triazin-4(3H)-one

### (Formula 7, where Y is O, R₁ is CH₃, Z is C-CH₃, Ar is 2,4-dimethylphenyl)

### A. 1-Cyano-1-(2,4-dimethylphenyl)propan-2-one

Sodium pellets (9.8g, 0.43 mol) were added portionwise to a solution of 2,4-dimethylphenylacetonitrile (48 g, 0.33 mol) in ethyl acetate (150 mL) at ambient temperature. The reaction mixture was heated to reflux temperature and stirred for 16 hours. The resulting suspension was cooled to room temperature and filtered. The collected precipitate was washed with copious amounts of ether and then air-dried. The solid was dissolved in water and a 1N HCl solution was added until the pH = 5-6. The mixture was extracted with ethyl acetate (3 X 200 mL); the combined organic layers were dried over MgSO₄ and filtered. Solvent was removed *in vacuo* to afford a white solid (45.7g, 74% yield): NMR (CDCl₃,300 MHz):; CI-MS: 188 (M + H).

### B. 5-Amino-4-(2,4-dimethylphenyl)-3-methylpyrazole

A mixture of 1-cyano-1-(2,4-dimethylphenyl)propan-2-one (43.8g, 0.23 mol), hydrazine-hydrate (22 mL, 0.46 mol), glacial acetic acid (45 mL, 0.78 mol) and toluene (500 mL) were stirred at reflux temperature for 18 hours in an apparatus fitted with a Dean-Stark trap. The reaction mixture was cooled to ambient temperature and solvent was removed in vacuo. The residue was dissolved in 6N HCl and the resulting solution was extracted with ether three times. A concentrated ammonium hydroxide solution was added to the aqueous layer until pH = 11. The resulting semi-solution was extracted three times with ethyl acetate. The combined organic layers were dried over MgSO₄ and filtered. Solvent was removed *in vacuo* to give a pale brown viscous oil (34.6g, 75% yield): NMR (CDCl₃,300 MHz): 7.10 (s, 1H), 7.05 (d, 2H, J=1), 2.37 (s, 3H), 2.10 (s, 3H) ; CI-MS:. 202 (M + H).

### C. 5-Acetamidino-4-(2,4-dimethylphenyl)-3-methylpyrazole, acetic acid salt

Ethyl acetamidate hydrochloride (60g, 0.48 mol) was added quickly to a rapidly stirred mixture of potassium carbonate (69.5g, 0.50 mol), dichloromethane (120 mL) and water (350 mL). The layers were separated and the aqueous layer was extracted with dichloromethane (2 X 120 mL). The combined organic layers were dried over MgSO₄ and filtered. Solvent was removed by simple distillation and the pot residue, a clear pale yellow liquid, (35.0 g) was used without further purification.

Glacial aetic acid (9.7 mL, 0.17 mol) was added to a stirred mixture of 5-amino-4-(2,4-dimethylphenyl)-3-methylpyrazole ( 34g, 0.17 mol), ethyl acetamidate (22g, 0.25 mol) and acetonitrile (500 mL). The resulting reaction mixture was stirred at room temperature for 3 days; at the end of which time, it was concentrated in vacuo to about one-third of its original volume. The resulting suspension was filtered and the collected solid was washed with copious amounts of ether. The white solid was dried *in vacuo* (31.4g, 61% yield): NMR (DMSO-d₆, 300 MHz): 7.00 (s, 1H), 6.90 (dd, 2H, J=7, 1), 2.28 (s, 3H), 2.08 (s, 3H), 2.00 (s, 3H) , 1.90 (s, 3H) , 1.81 (s, 3H) ; CI-MS: 243 (M + H).

### D. 2,7-dimethyl-8-(2,4-dimethylphenyl)[1,5-a]-pyrazolo-[1,3,5]-triazin-4(3H)-one

Sodium pellets (23g, 1 mol) were added portionwise to ethanol (500 mL) with vigorous stirring. After all the sodium reacted, 5-acetamidino-4-(2,4-dimethylphenyl)-3-methylpyrazole, acetic acid salt (31.2g, 0.1 mol) and diethyl carbonate ( 97 mL, 0.8 mol) were added. The resulting reaction mixture was heated to reflux temperature and stirred for 18 hours. The mix was cooled to room temperature and solvent was removed in vacuo. The residue was dissolved in water and a 1N HCl solution was added slowly until pH = 5-6. The aqueous layer was extracted with ethyl acetate three times; the combined organic layers were dried over MgSO₄ and filtered. Solvent was removed *in vacuo* to give a pale tan solid (26g, 98% yield): NMR (CDCl₃, 300 MHz) : 7.15(s, 1H), 7.09 (s, 2H), 2.45 (s, 3H), 2.39 (s, 3H), 2.30 (s, 3H); CI-MS: 269 (M + H).

### EXAMPLE 3

### Preparation of 4-(di(carbomethoxy)methyl)-2,7-dimethyl-8-(2,4-dimethylphenyl) [1,5-a]-pyrazolo-1,3,5-triazine

### (Formula 1, where R³ is CH(CHCO₂CH₃)₂, R₁ is CH₃, Z is C-CH₃, Ar is 2,4-dimethylphenyl)

### A. 4-chloro-2,7-dimethyl-8-(2,4-dichlorophenyl) [1,5-a]- pyrazolotriazine

A mixture of 2,7-dimethyl-8-(2,4-dimethylphenyl) [1,5-a] -pyrazolo-1,3,5-triazin-4-one (Example 1, 1.38g, 4.5 mmol), N,N-dimethylaniline (1 mL, 8 mmol) and phosphorus oxychloride (10 mL) was stirred at reflux temperature for 48 hours. The excess phosphorus oxychloride was removed *in vacuo*. The residue was poured onto ice-water, stirred briefly and extracted quickly with ethyl acetate three times. The combined organic layers were washed with ice water, then dried over MgSO₄ and filtered. Solvent was removed *in vacuo* to give a brown oil. Flash column chromatography (ethyl acetate:hexanes::1:4) gave one fraction (Rf = 0.5) Solvent was removed in vacuo to afford a yellow oil (1.0g, 68% yield): NMR (CDCl₃, 300 MHz): 7.55 (d, 1H, J = 1), 7.38 (dd, 1H, J = 7,1 ), 7.30 (d, 1H, J = 7), 2.68 (s, 3H), 2.45 (s, 3H); CI-MS: 327 (M + H).

### B. 4-(di(carbomethoxy)methyl)-2,7-dimethyl-8-(2,4-dimethylphenyl)[1,5-a]-pyrazolo-1,3,5-triazine

Sodium hydride (60% in oil, 80 mg, 2 mmol) was washed with hexanes twice, decanted after each washing and taken up in anhydrous tetrahydrofuran (THF, 1 mL). A solution of diethyl malonate (0.32g, 2 mmol) in THF (2 mL) was added dropwise over 5 min, during which time vigorous gas evolution ensued. A solution of 4-chloro-2,7-dimethyl-8-(2,4-dichlorophenyl)[1,5-a]-pyrazolotriazine (0.5g, 1.75 mmol) in THF (2 mL) was added and the reaction mixture was then stirred under a nitrogen atmosphere for 48 hours. The resulting suspension was poured onto water and extracted three times with ethyl acetate. The combined organic layers were washed once with brine, dried over MgSO₄ and filtered. Solvent was removed *in vacuo* to give a brown oil. Column chromatography (ethyl acetate:hexanes::1:9) afforded, after removal of solvent *in vacuo*, a pale yellow solid (Rf = 0.2, 250 mg, 35% yield): mp 50-52°C; NMR (CDCl₃, 300 MHz): 12.35 (br.s, 1H, 7.15-7.00 (m, 3H), 4.40 (q, 2H, J = 7), 4.30 (q, 2H, J = 7), 2.4, 2.35, 2.3, 2.2, 2.1 (5 s, 12H), 1.4 (t, 3H, J = 7), 1.35-1.25 (m, 3H); CI-HRMS: Calcd: 411.2032, Found: 411.2023.

### EXAMPLE 6

### Preparation of 4-(1,3-dimethoxy-2-propylamino)-2,7-dimethyl-8-(2,4-dichlorophenyl)[1,5-a]-pyrazolo-1,3,5-triazine

### (Formula 1, where R³ is NHCH(CH₂OCH₃)₂, R₁ is CH₃, Z is C-CH₃, Ar is 2,4-dichlorophenyl)

### A. 4-chloro-2,7-dimethyl-8-(2,4-dichlorophenyl)[1,5-a]- pyrazolotriazine

A mixture of 2,7-dimethyl-8-(2,4 dimethylphenyl)[1,5-a]-pyrazolo-1,3,5-triazin-4-one (Example 1, 1.38g, 4.5 mmol), N,N-dimethylaniline (1 mL, 8 mmol) and phosphorus oxychloride (10 mL) was stirred at reflux temperature for 48 hours. The excess phosphorus oxychloride was removed in vacuo. The residue was poured onto ice-water, stirred briefly and extracted quickly with ethyl acetate three times. The combined organic layers were washed with ice water, then dried over MgSO₄ and filtered. Solvent was removed *in vacuo* to give a brown oil. Flash column chromatography (ethyl acetate:hexanes::1:4) gave one fraction (Rf = 0.5) Solvent was removed *in vacuo* to afford a yellow oil (1.0g, 68% yield): NMR (CDCl₃,300 MHz): 7.55 (d, 1H, J = 1), 7.38 (dd, 1H, J = 7,1 ), 7.30 (d, 1H, J = 7), 2.68 (s, 3H), 2.45 (s, 3H); CI-MS: 327 (M + H).

### B. 4-(1,3-dimethoxy-2-propylamino)-2,7-dimethyl-8-(2,4- dichlorophenyl)[1,5-a]-pyrazolo-1,3,5-triazine

A mixture of 4-chloro-2,7-dimethyl-8-(2,4-dichlorophenyl)[1,5-a]-pyrazolo-1,3,5-triazine (Part A, 570 mg, 1.74 mmol), 1,3-dimethoxypropyl-2-aminopropane (25mg, 2.08 mmol) and ethanol (10 mL) was stirred at ambient temperature for 18 hours. The reaction mixture was poured onto water (25 mL) and extracted three times with ethyl acetate. The combined organic layers were dried over MgSO₄ and filtered. Solvent was removed *in vacuo*. Column chromatography (CH₂Cl₂:CH₃OH::50:1) afforded one fraction. Removal of solvent *in vacuo* gave a solid (250 mg, 35% yield): mp 118-120°C; NMR (CDCl₃, 300 MHz): 7.50 (s, 1H), 7.28 (dd, 2H, J = 8,1), 6.75 (d, 1H, J = 8), 4.70-4.58 (m, 1H), 3.70-3.55 (m, 4H), 3.43 (s, 6H), 2.50 (s, 3H), 2.35 (s, 3H); CI-HRMS: Calcd: 409.1072, Found: 409.1085; Analysis Calcd. for C₁₈H₂₁Cl₂N₅O₂: C, 52.69, H, 5.17, N, 17.07, Cl, 17.28; Found: C, 52.82, H, 5.06, N, 16.77, Cl, 17.50.

The examples delineated in Table 7 may be prepared by the methods outlined in Examples 1, 3 or 6.
Commonly used abbreviations are: Ph is phenyl, Pr is propyl, Me is methyl, Et is ethyl, Bu is butyl, Ex is Example.

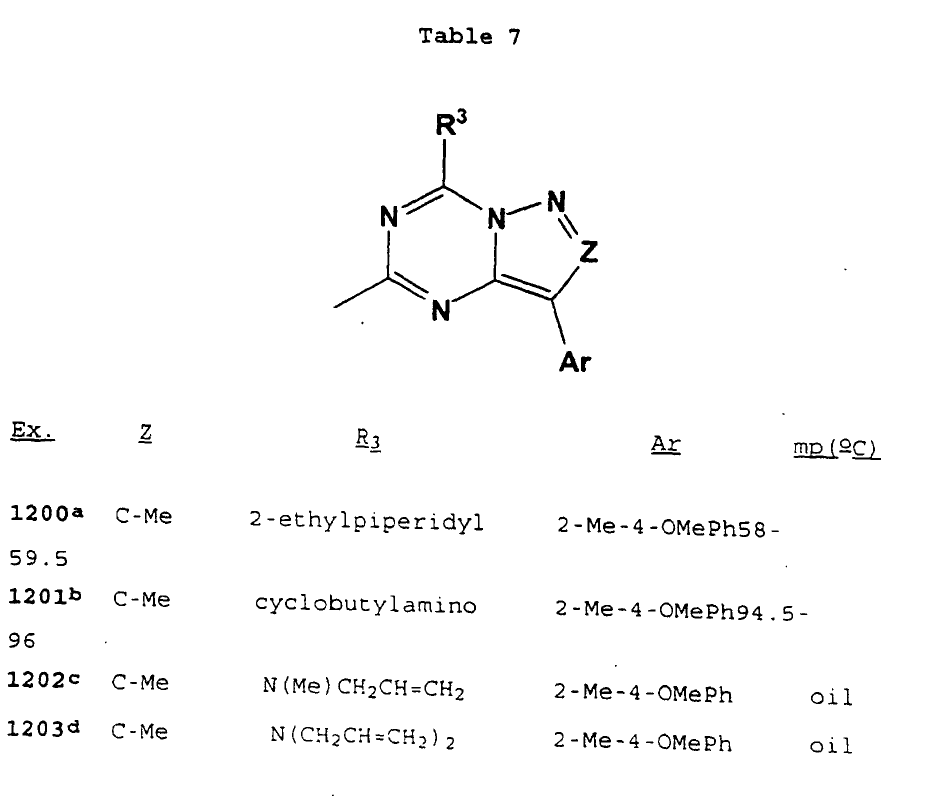

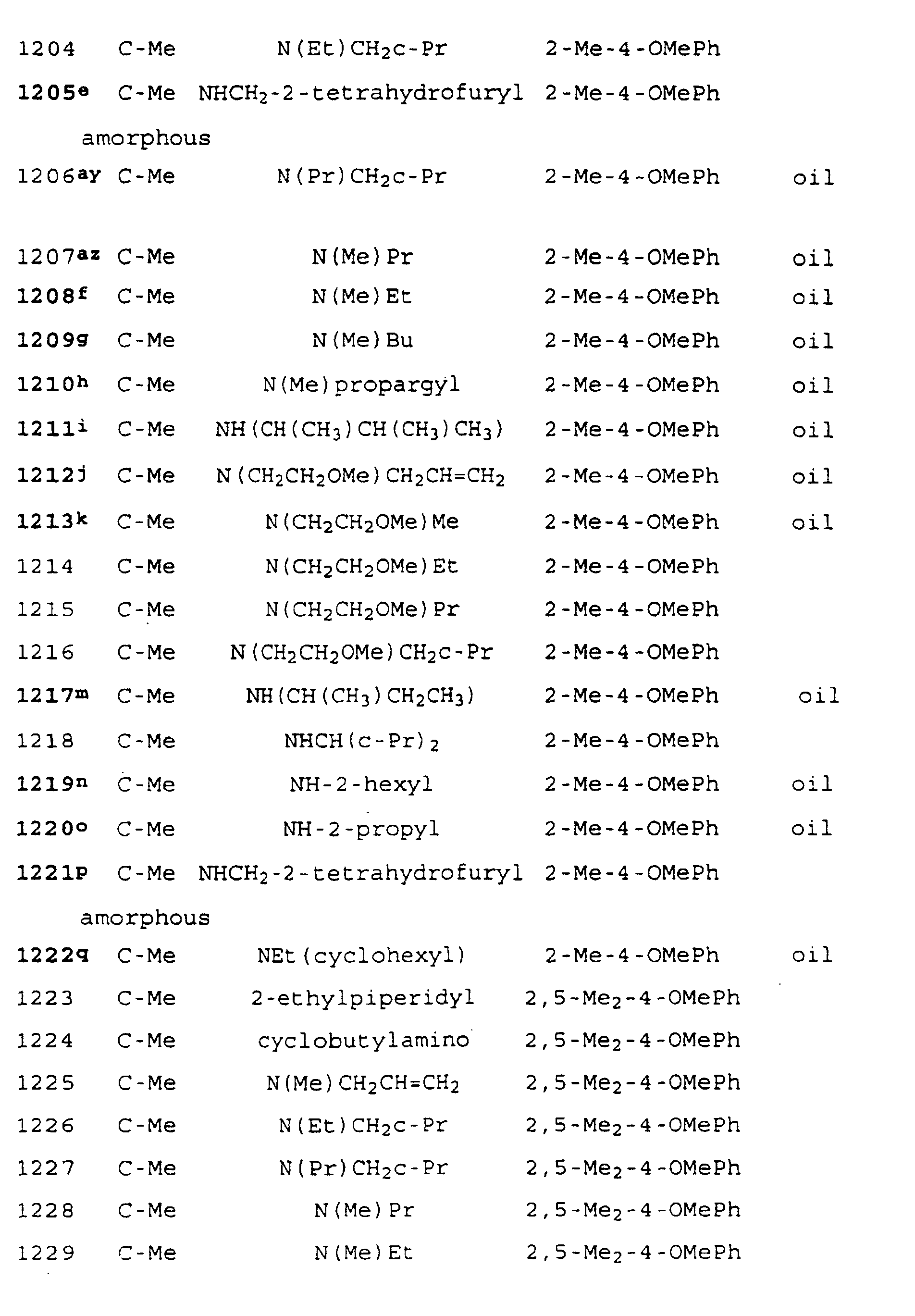

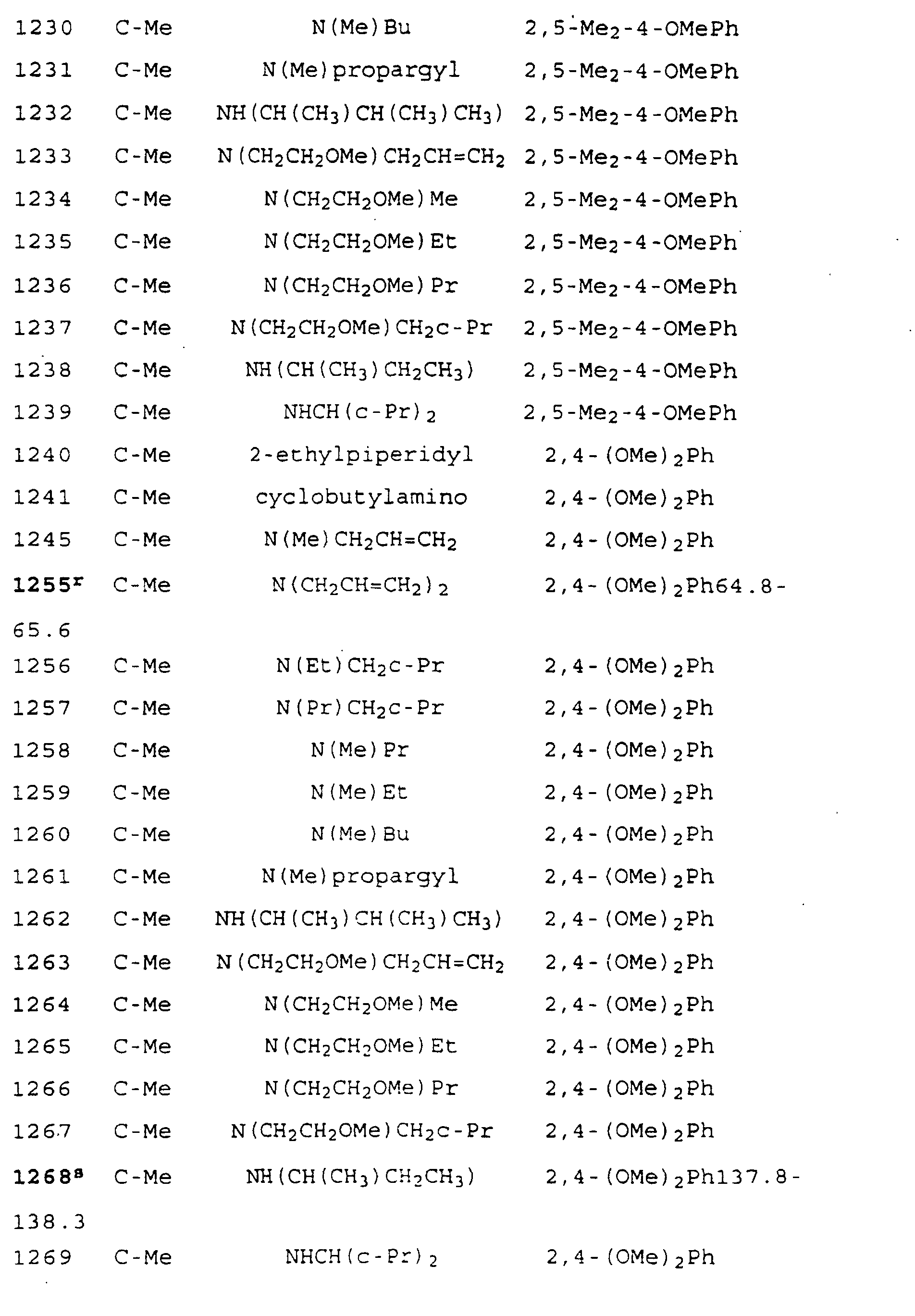

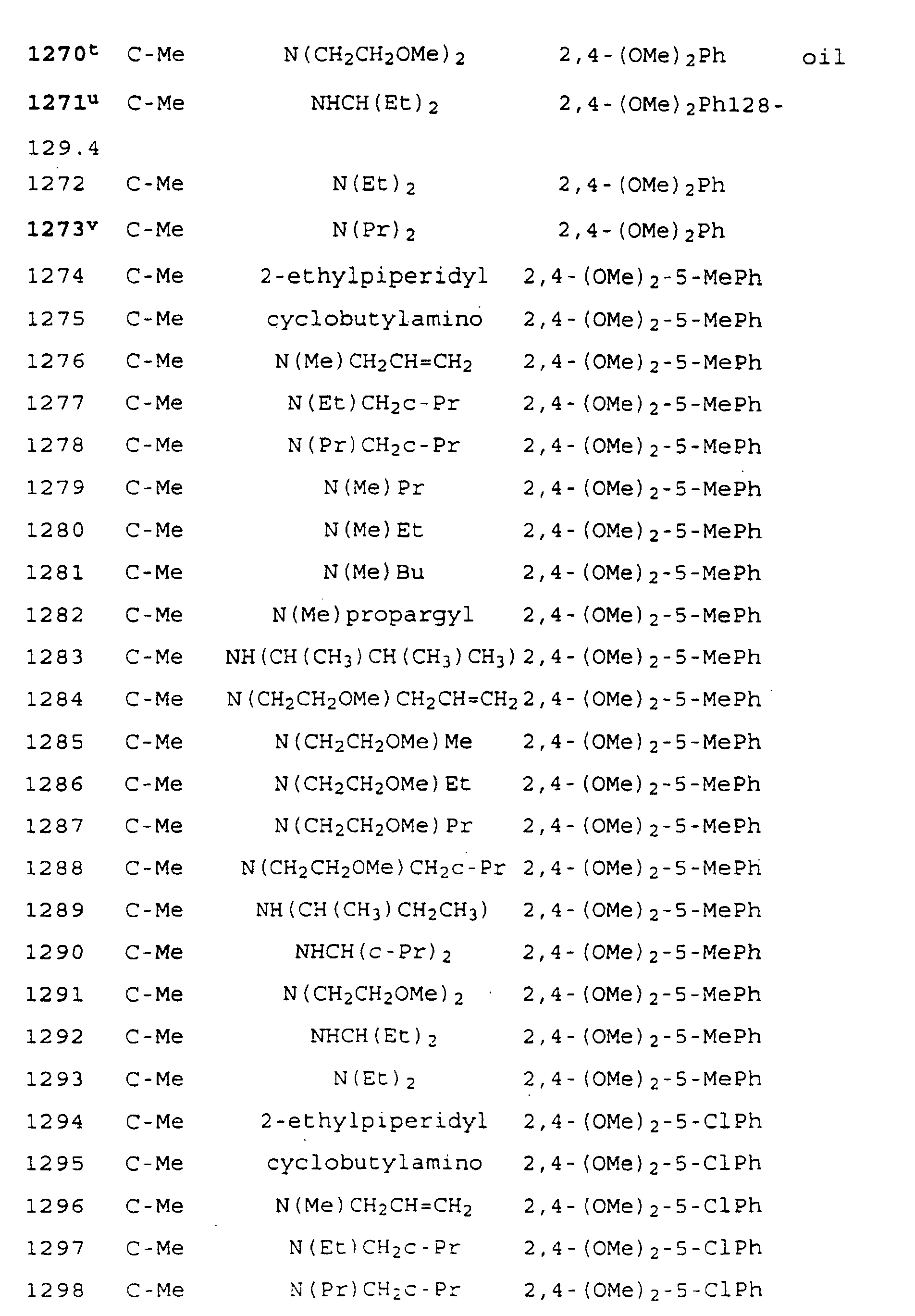

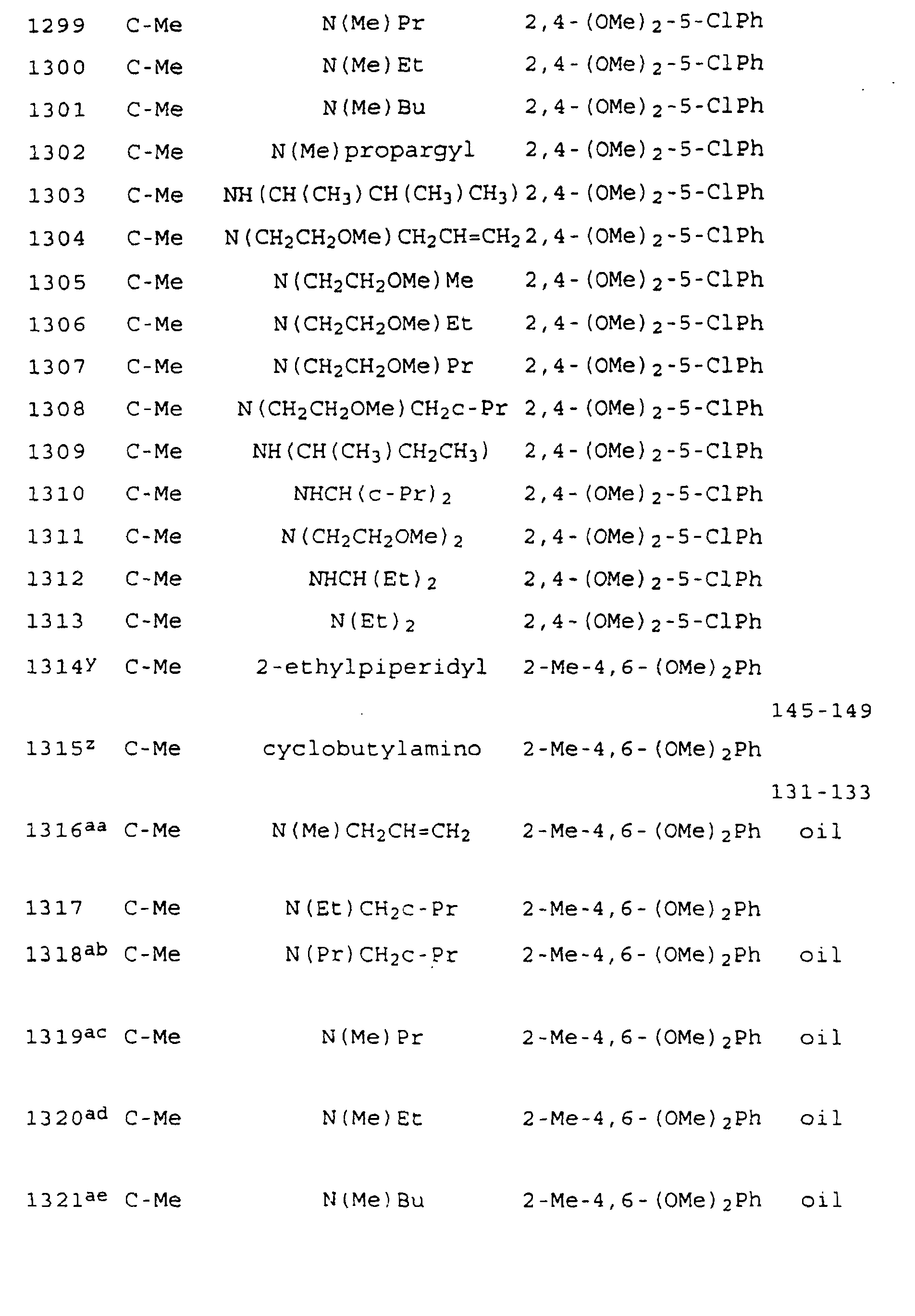

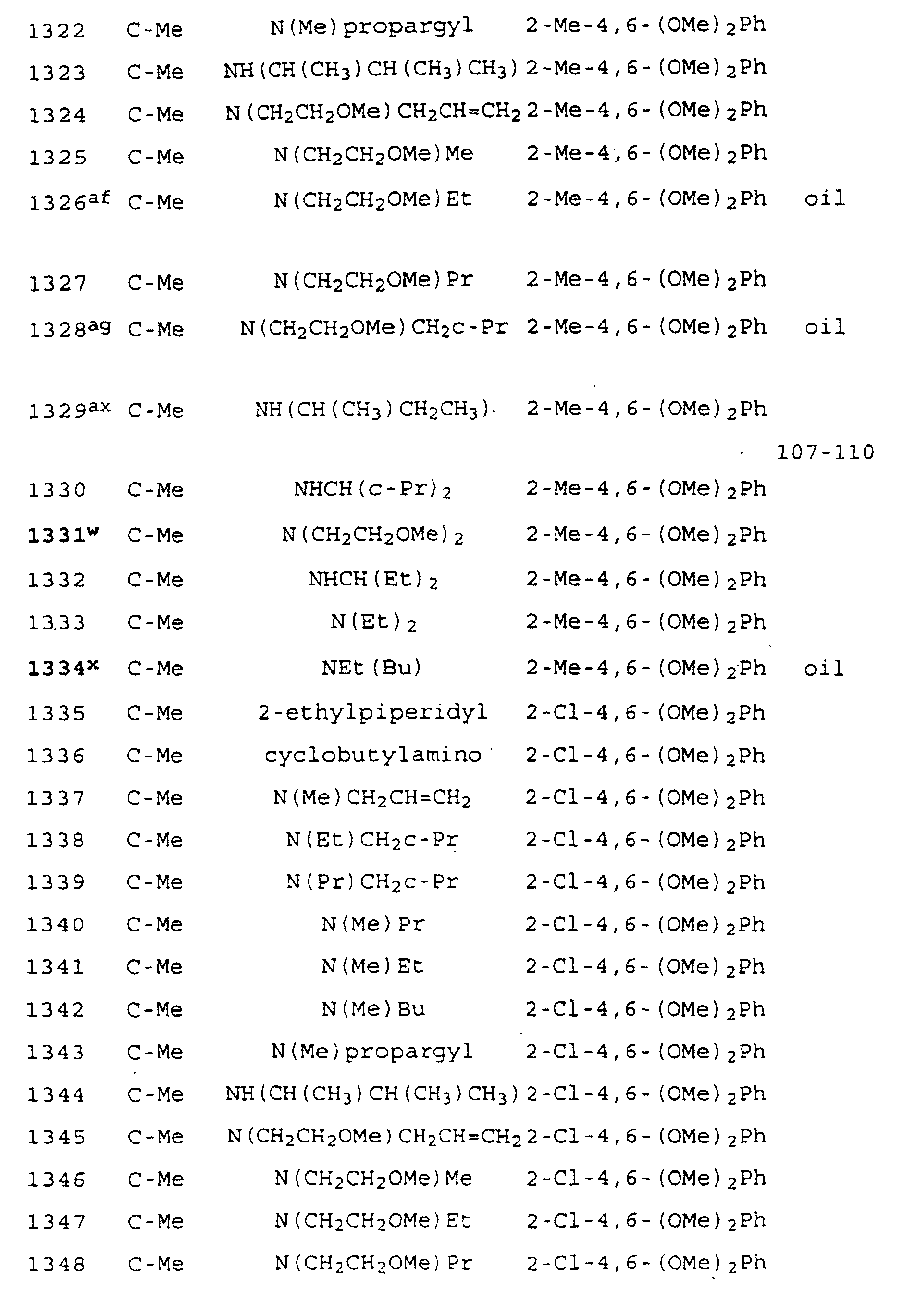

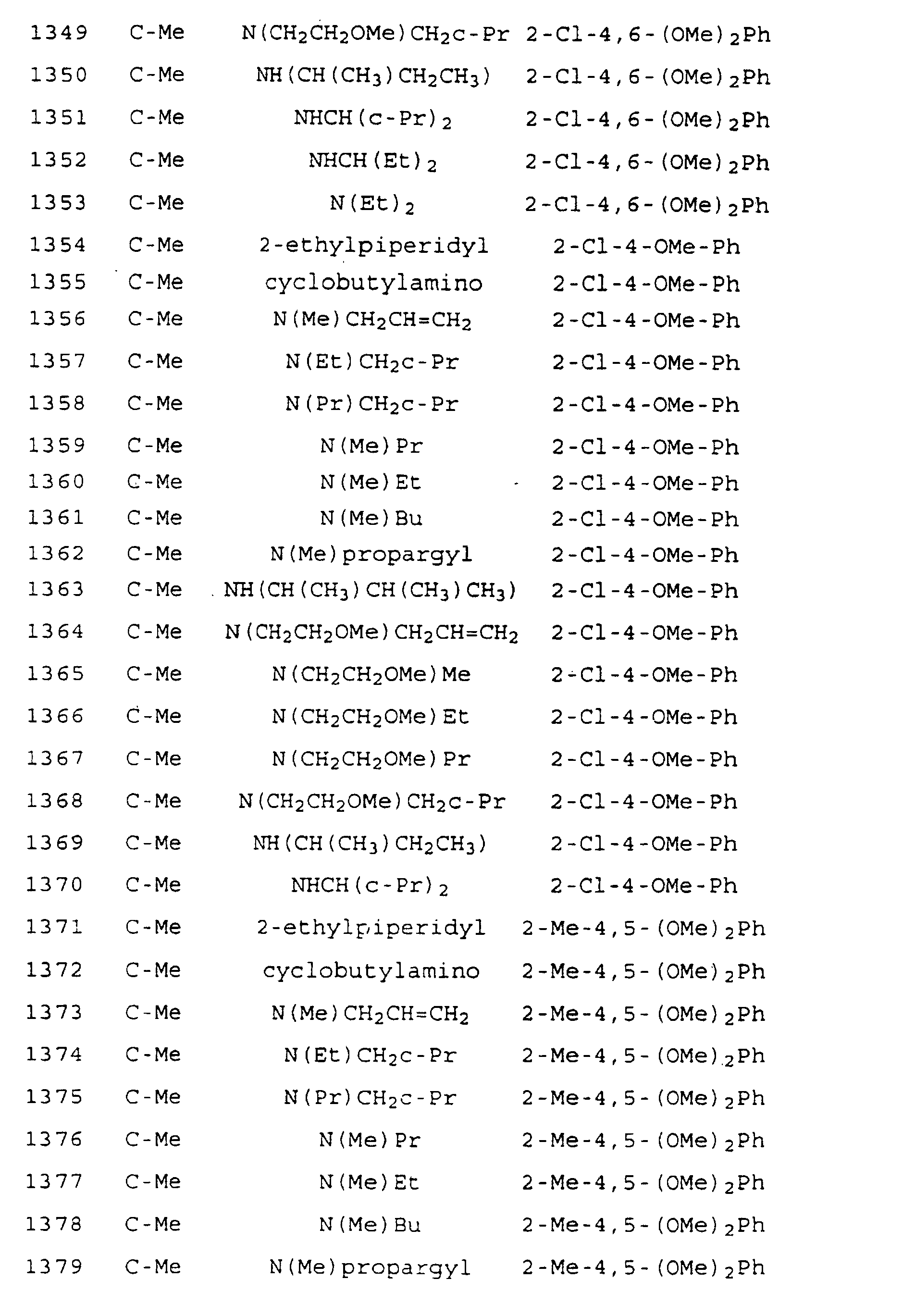

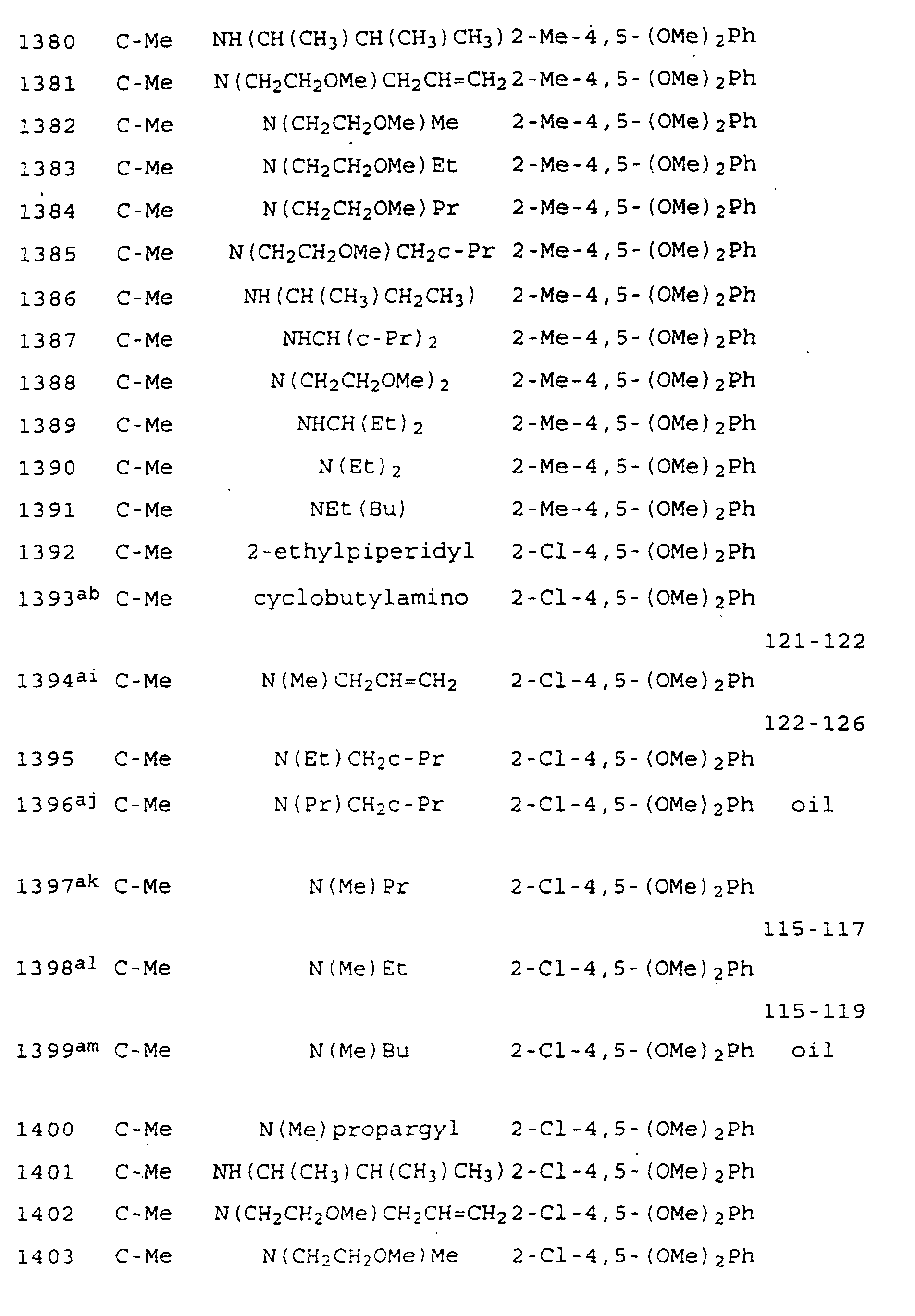

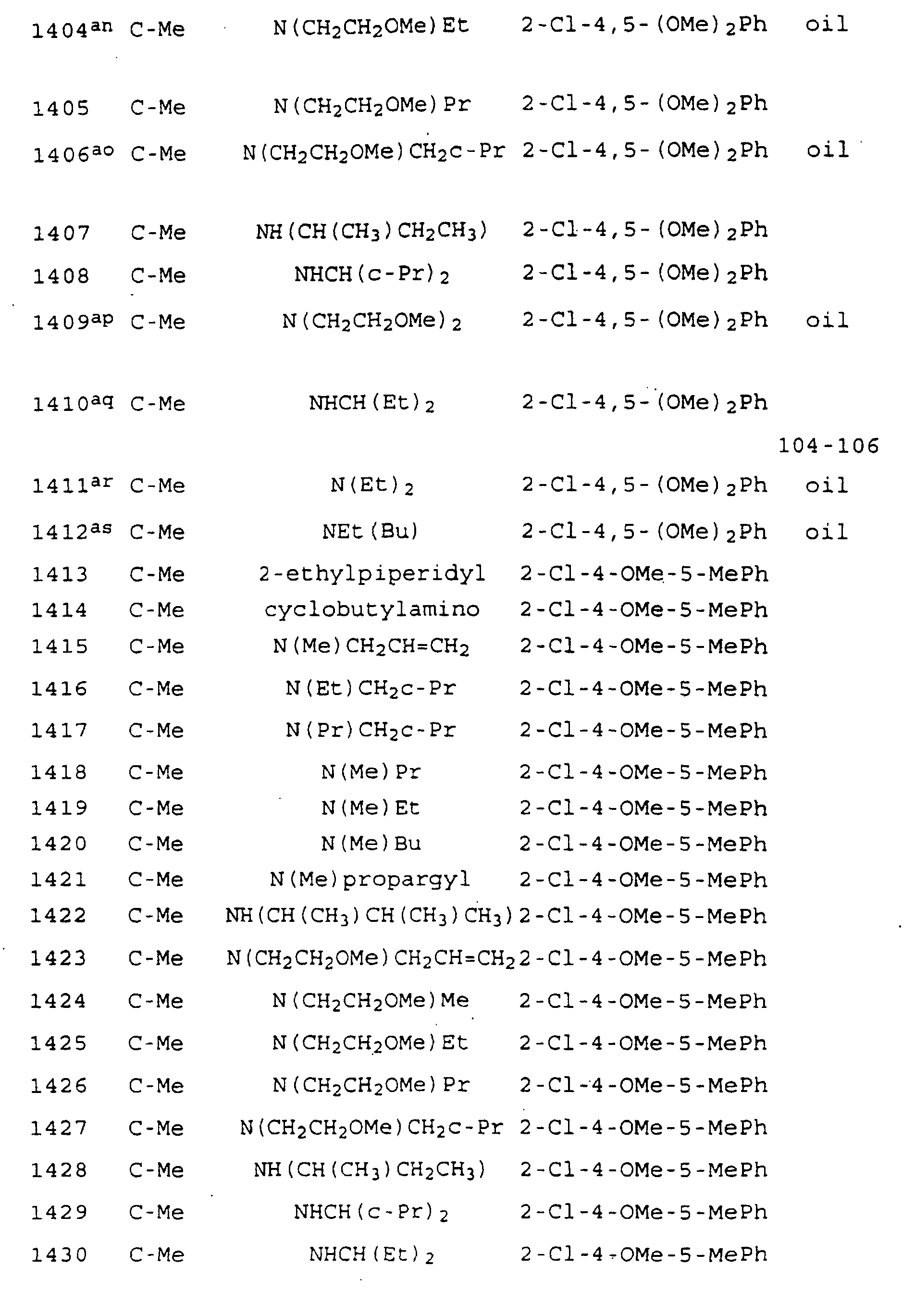

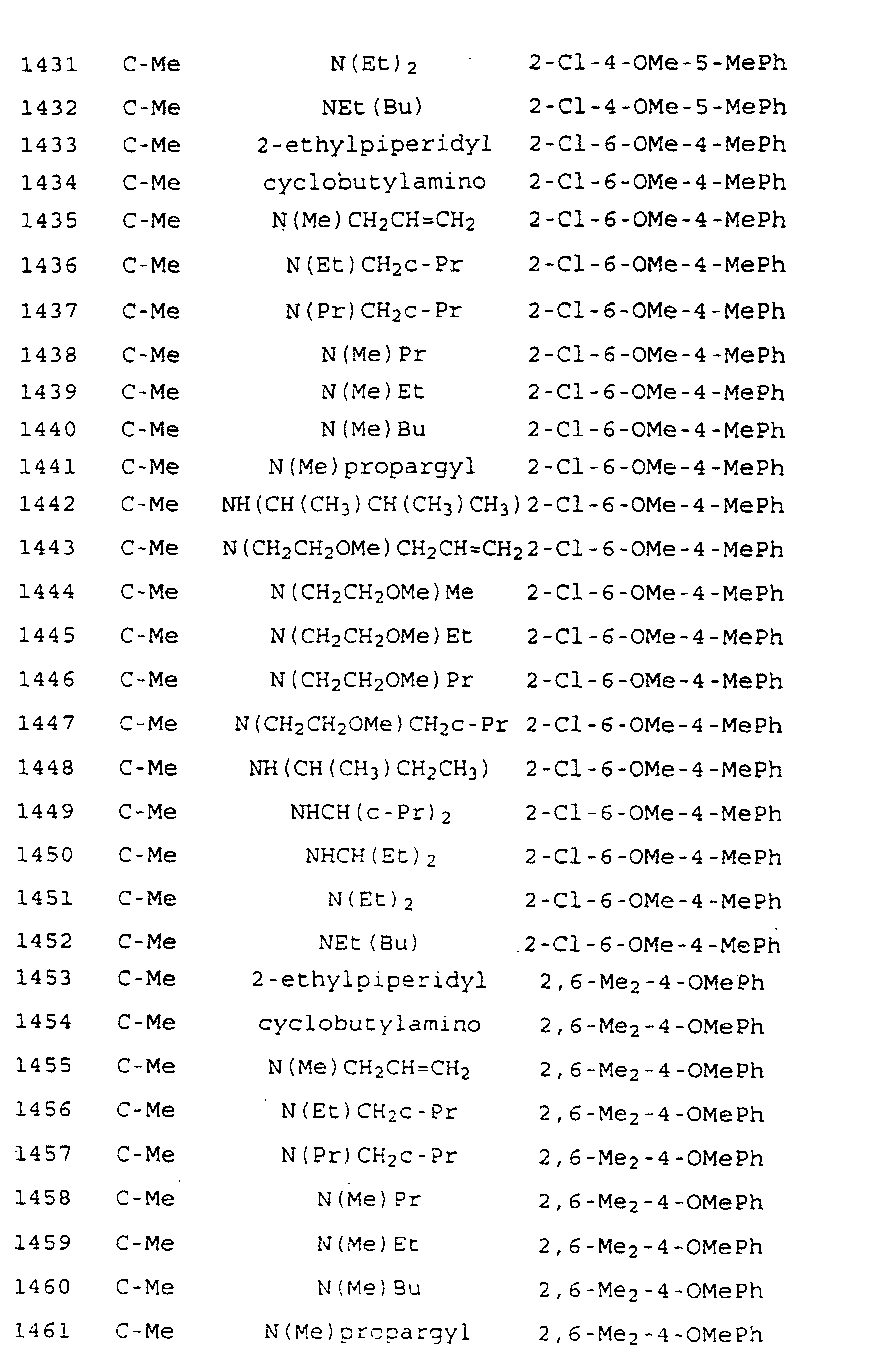

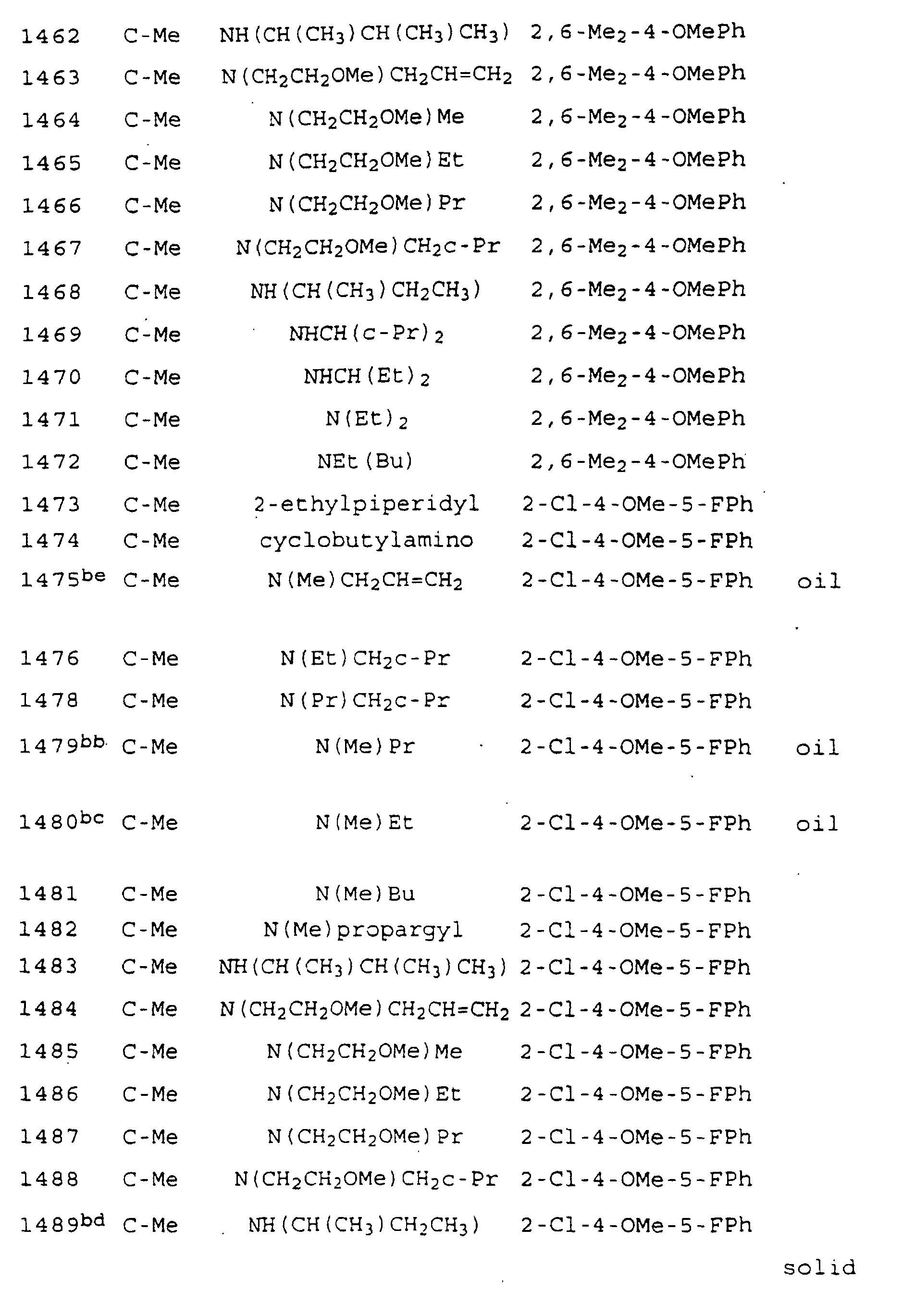

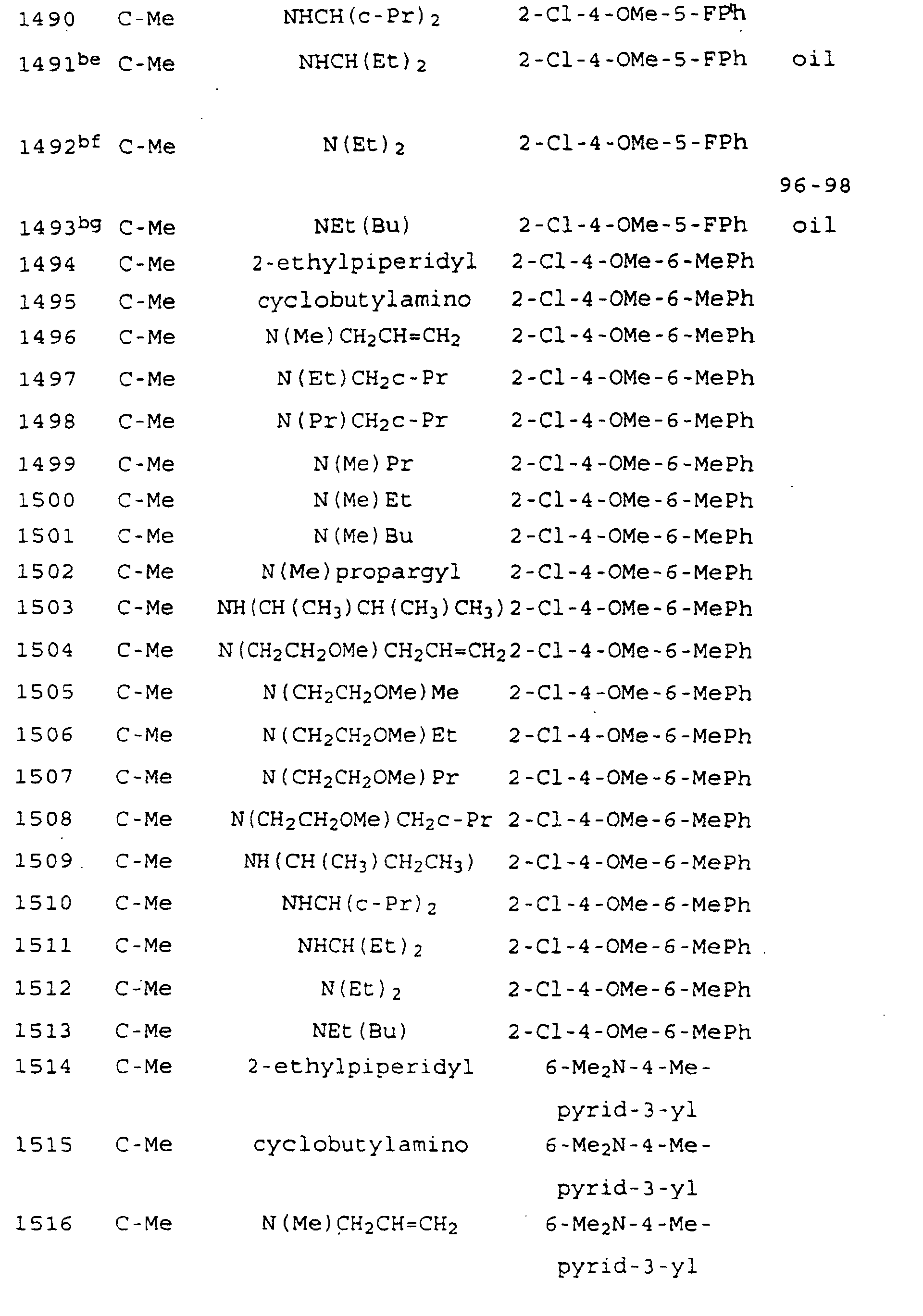

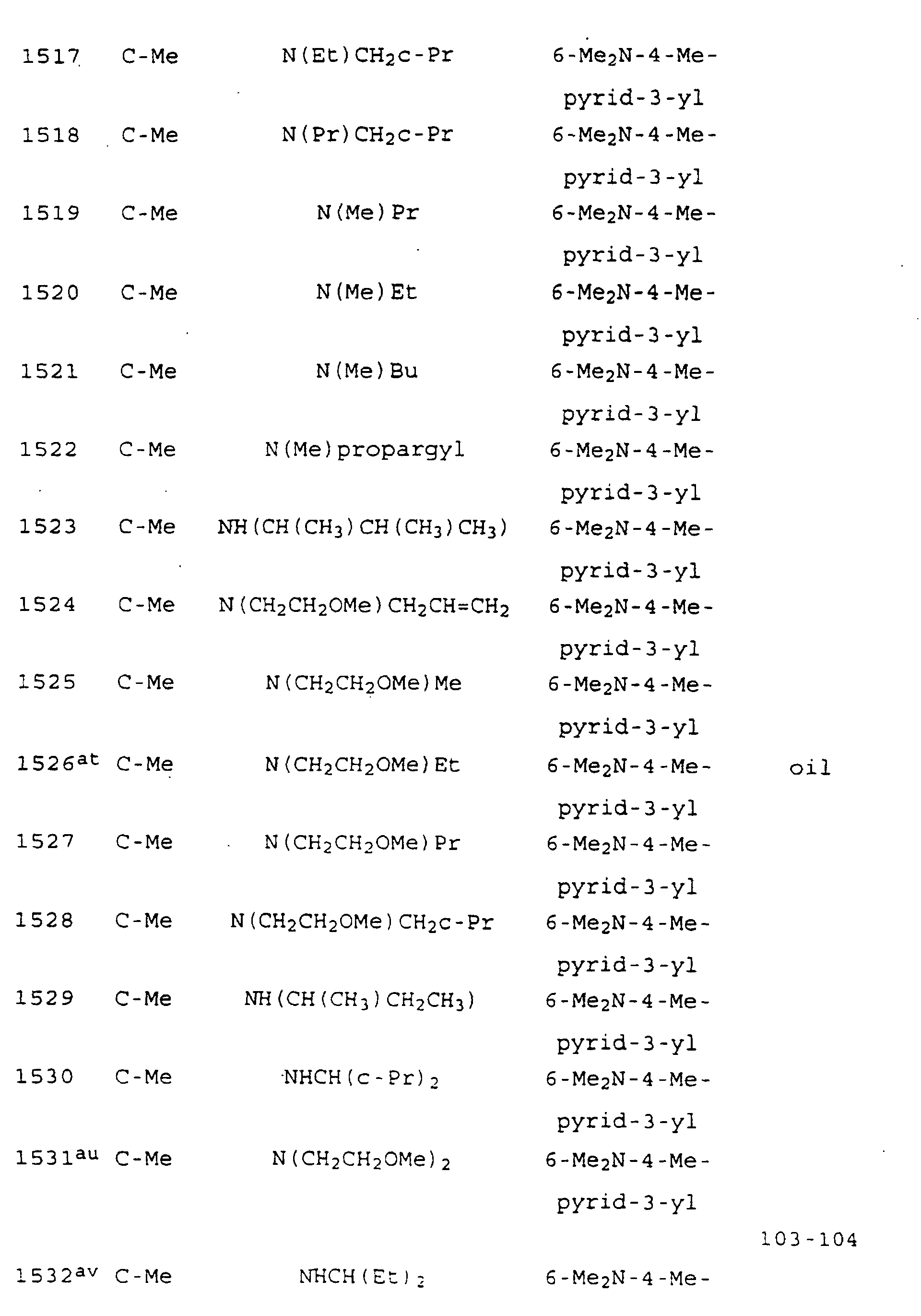

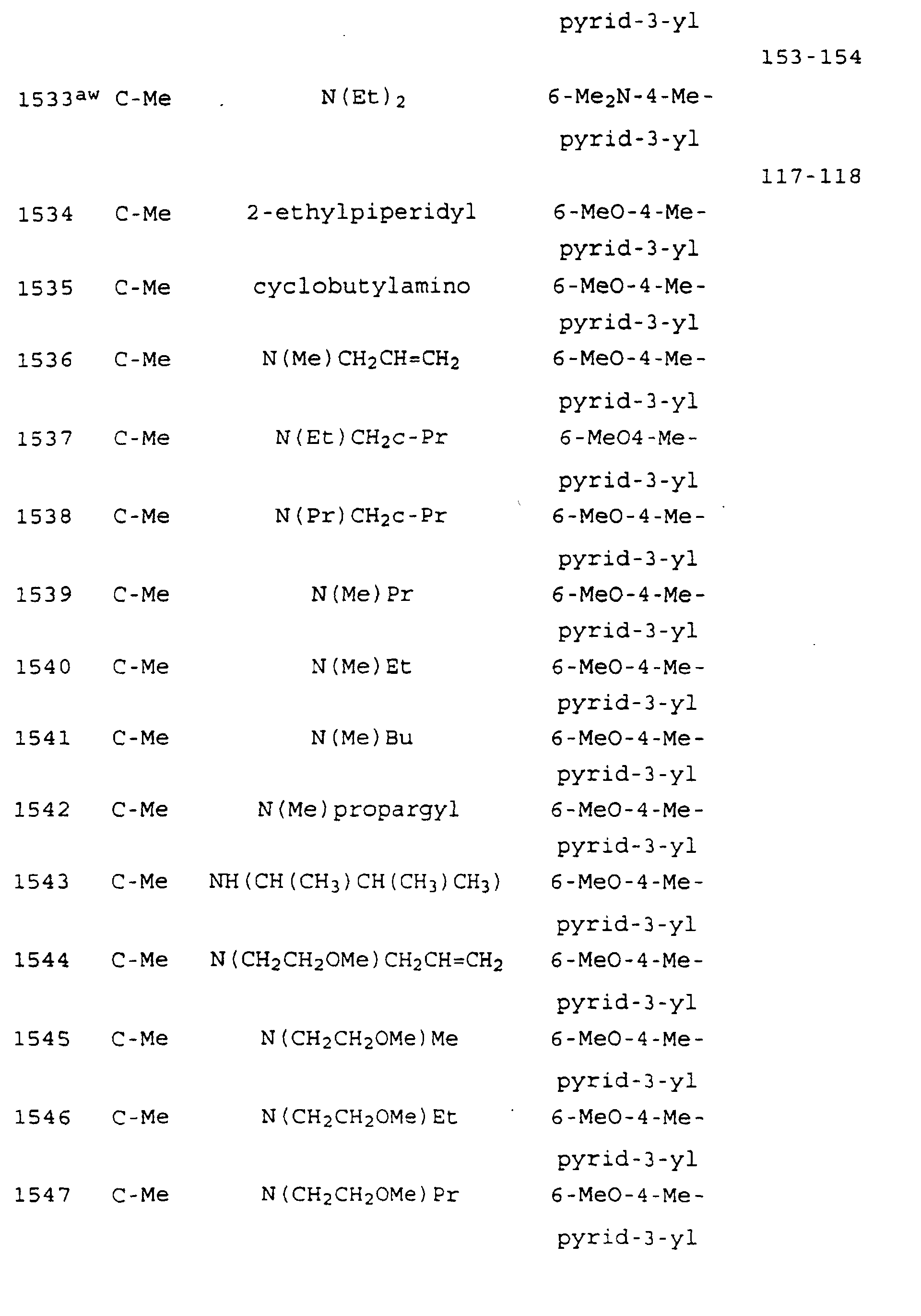

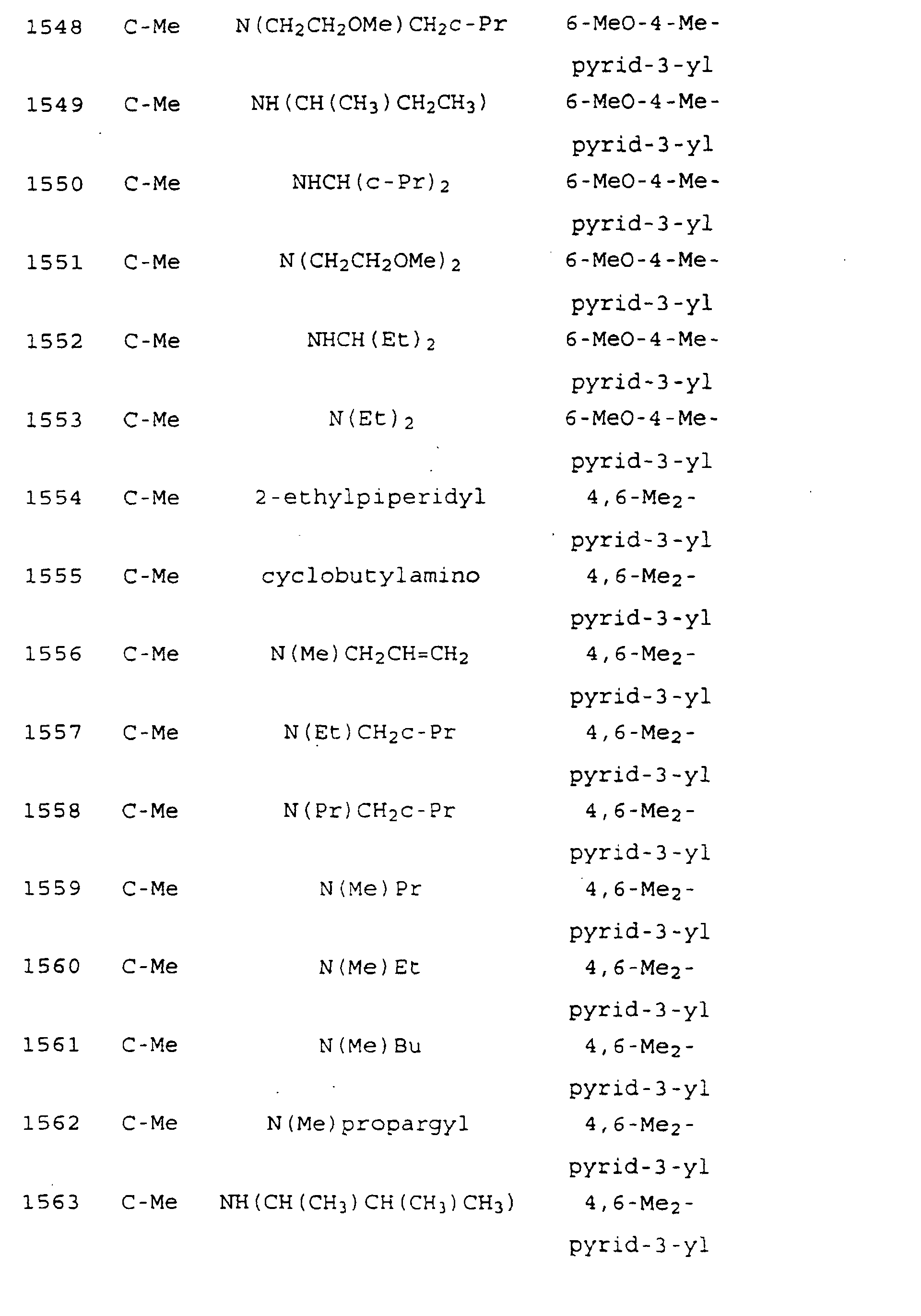

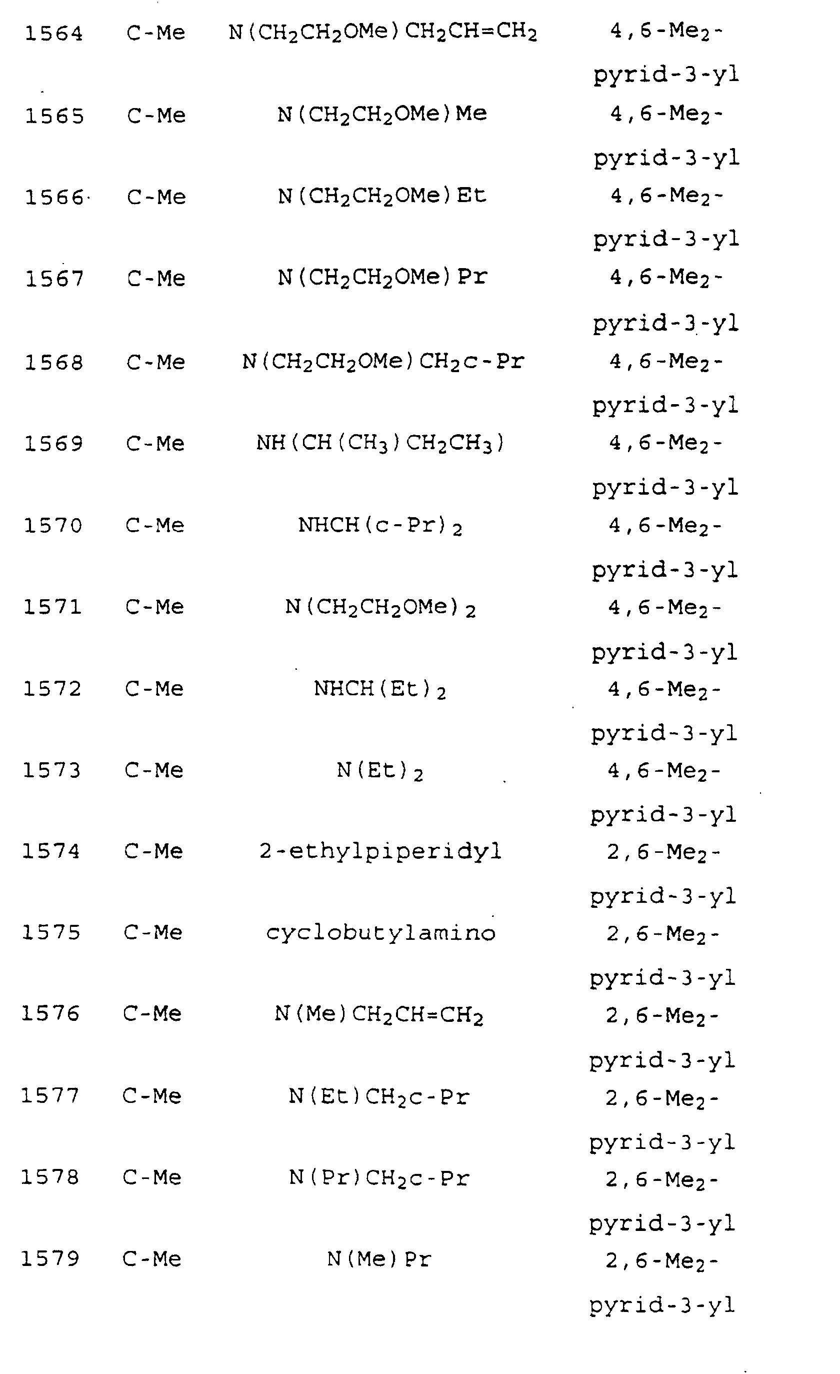

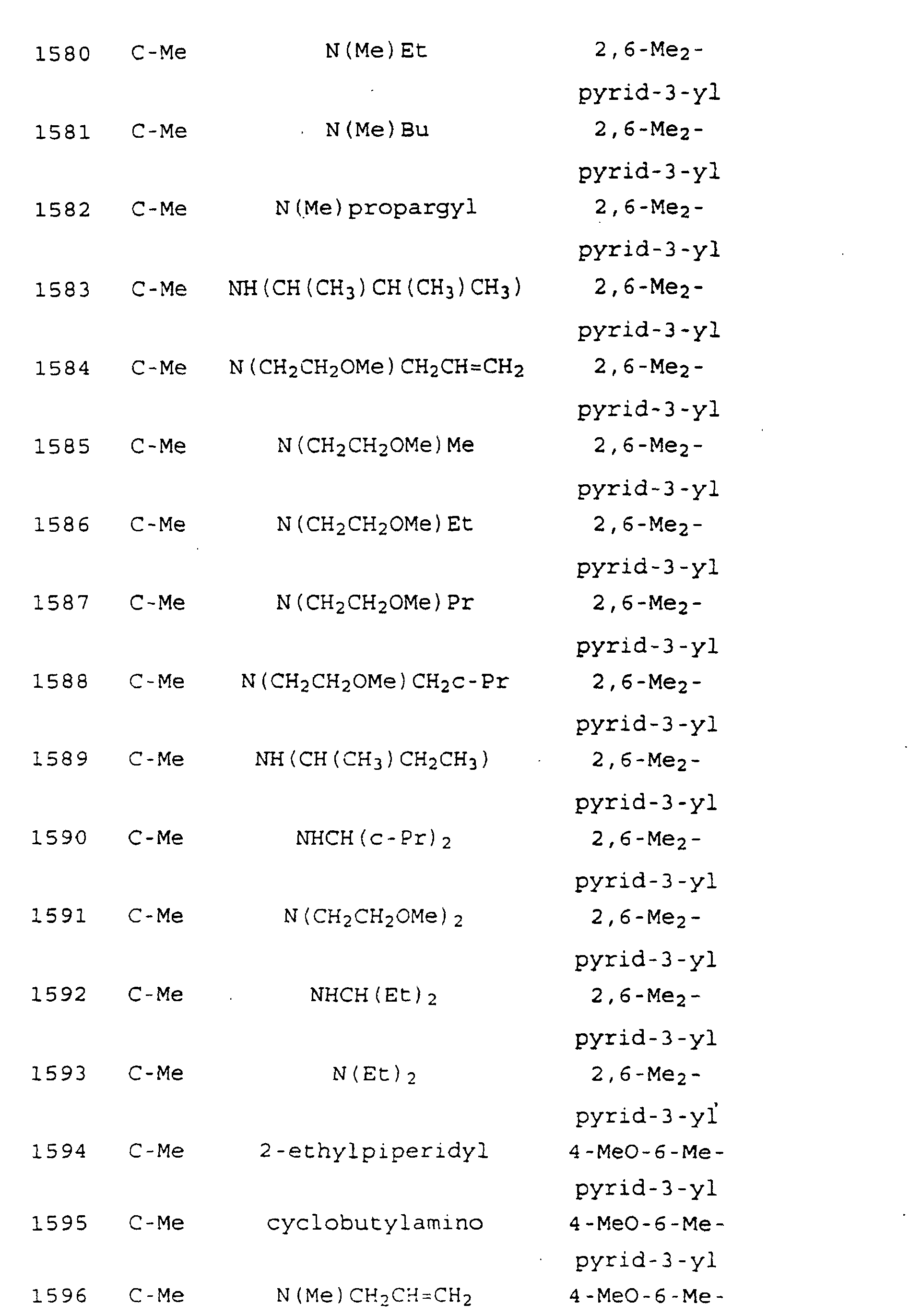

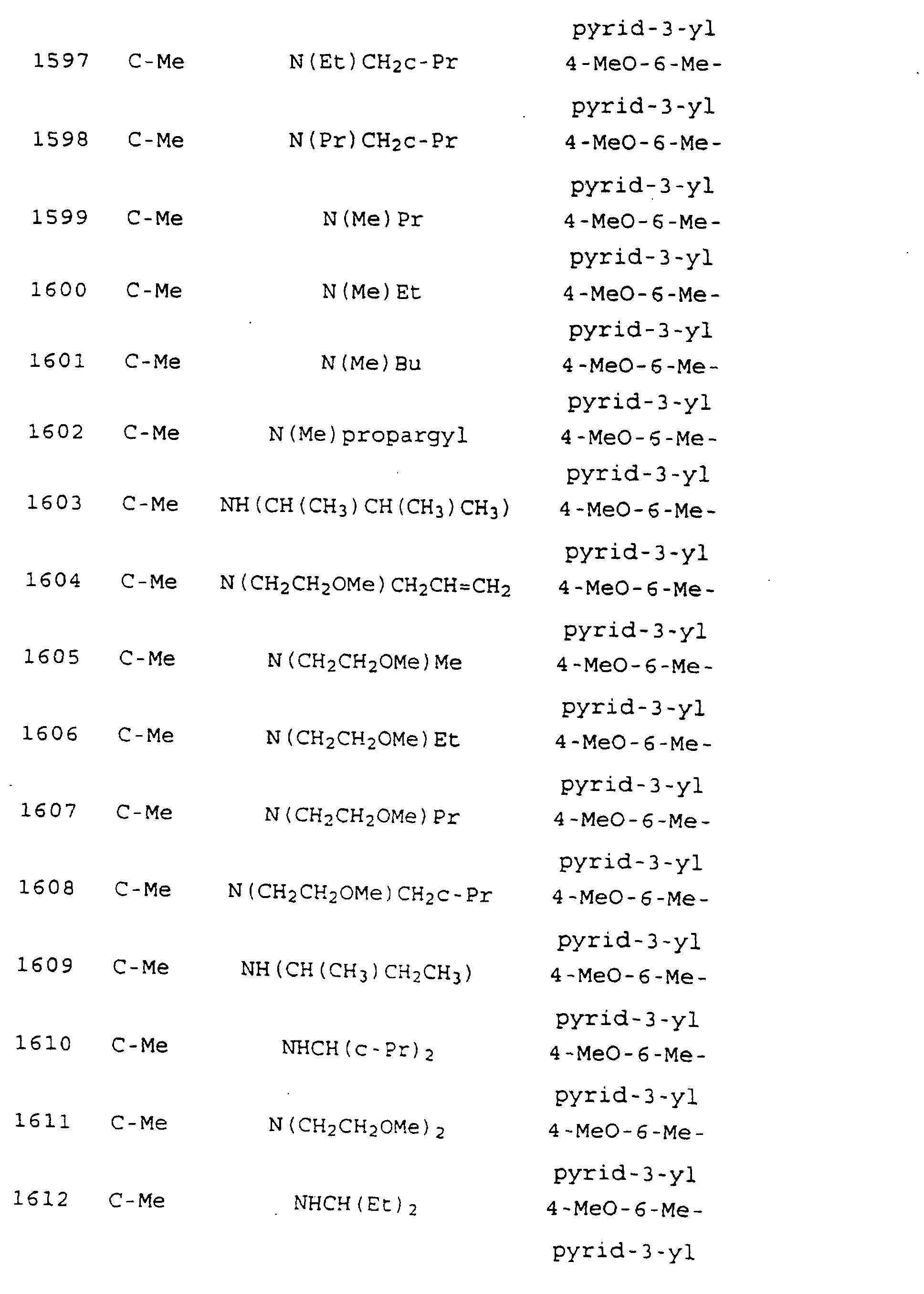

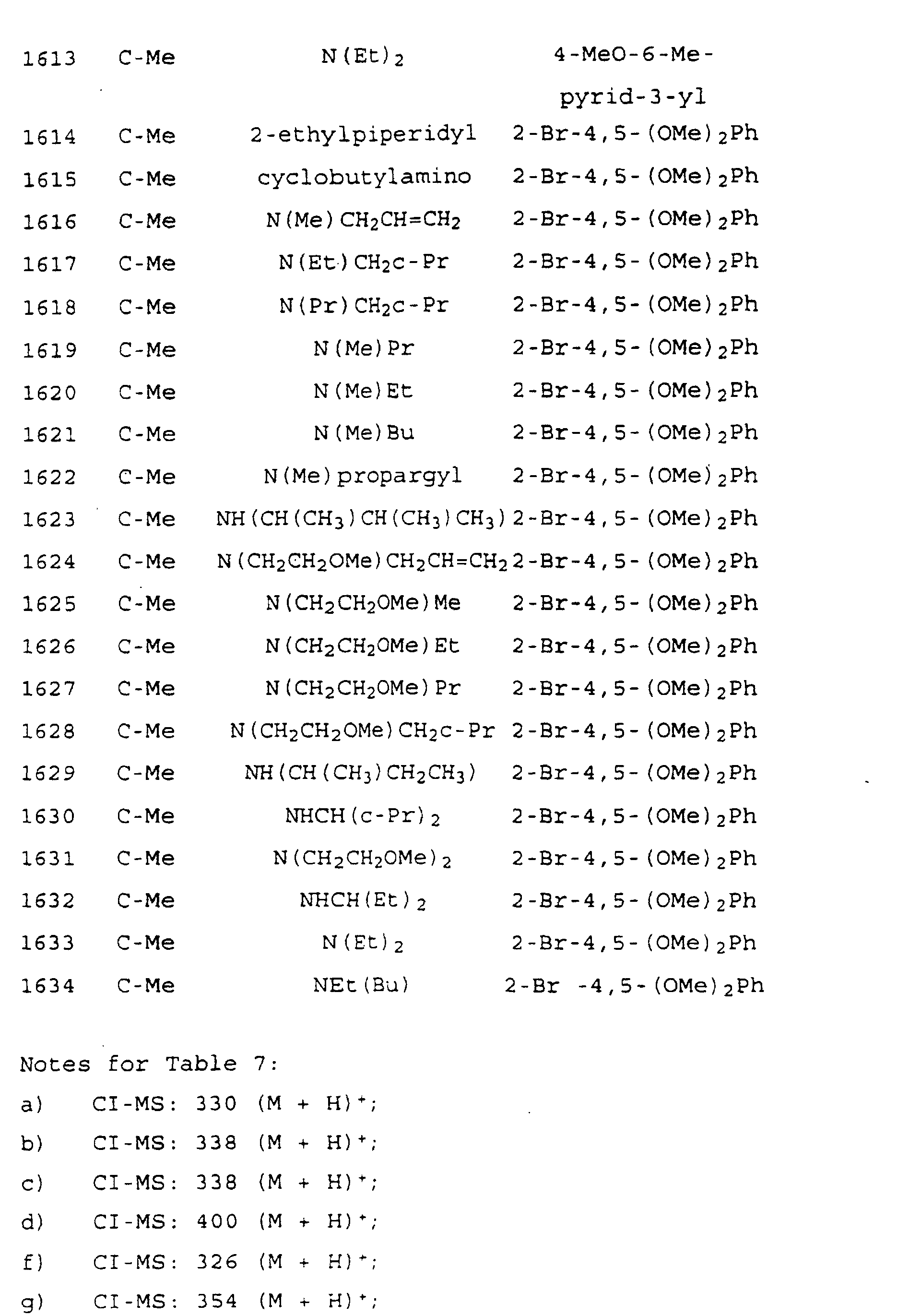

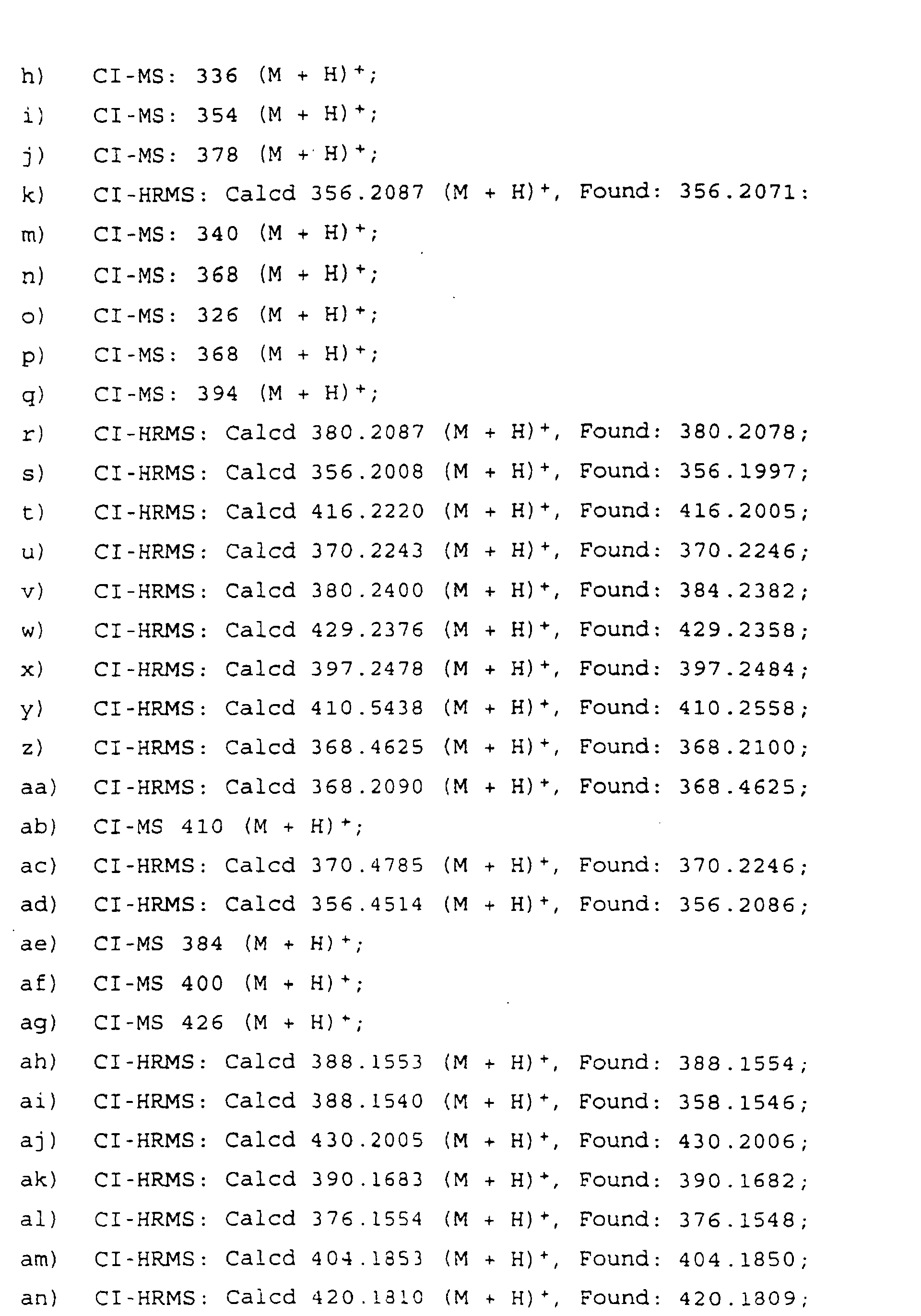

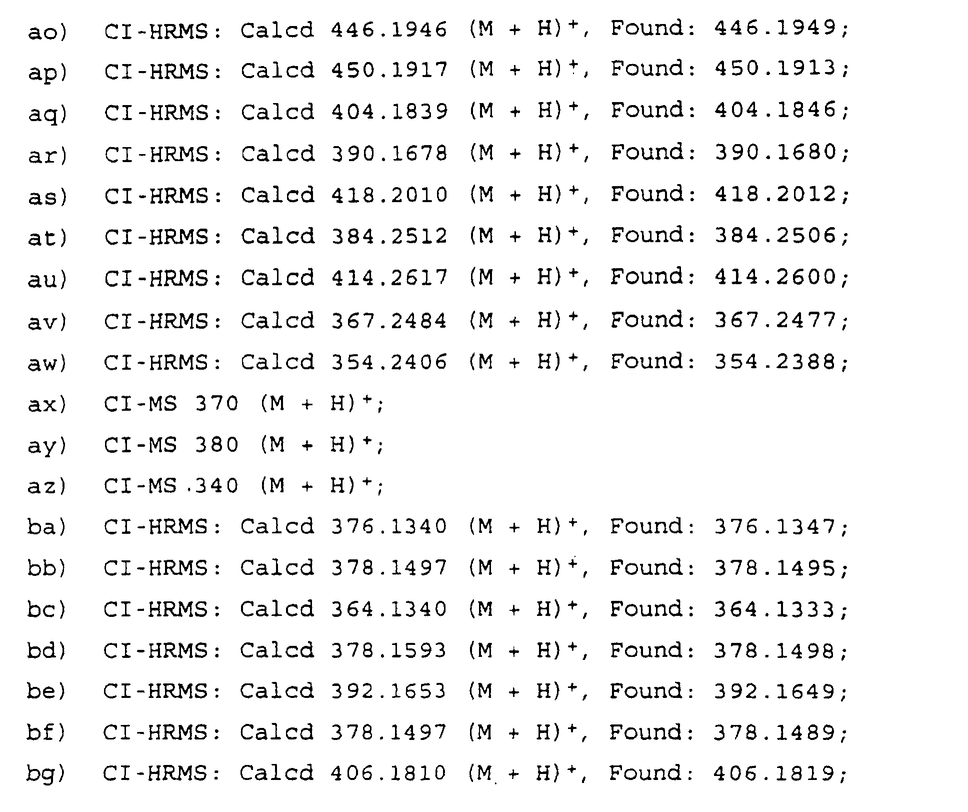

### Utility

### CRF-R1 Receptor Binding Assay for the Evaluation of Biological Activity

The following is a description of the isolation of cell membranes containing cloned human CRF-R1 receptors for use in the standard binding assay as well as a description of the assay itself.

Messenger RNA was isolated from human hippocampus. The mRNA was reverse transcribed using oligo (dt) 12-18 and the coding region was amplified by PCR from start to stop codons The resulting PCR fragment was cloned into the EcoRV site of pGEMV, from whence the insert was reclaimed using XhoI + XbaI and cloned into the XhoI + XbaI sites of vector pm3ar ( which contains a CMV promoter, the SV40 't' splice and early poly A signals, an Epstein-Barr viral origin of replication, and a hygromycin selectable marker). The resulting expression vector, called phchCRFR was transfected in 293EBNA cells and cells retaining the episome were selected in the presence of 400 µM hygromycin. Cells surviving 4 weeks of selection in hygromycin were pooled, adapted to growth in suspension and used to generate membranes for the binding assay described below. Individual aliquots containing approximately 1 x 10⁸ of the suspended cells were then centrifuged to form a pellet and frozen.

For the binding assay a frozen pellet described above containing 293EBNA cells transfected with hCRFR1 receptors is homogenized in 10 ml of ice cold tissue buffer ( 50 mM HEPES buffer pH 7.0, containing 10 mM MgCl₂, 2 mM EGTA, 1 µg/l aprotinin, 1 µg/ml leupeptin and 1 µg/ml pepstatin). The homogenate is centrifuged at 40,000 x g for 12 min and the resulting pellet rehomogenized in 10 ml of tissue buffer. After another centrifugation at 40,000 x g for 12 min, the pellet is resuspended to a protein concentration of 360 µg/ml to be used in the assay.

Binding assays are performed in 96 well plates; each well having a 300 µl capacity. To each well is added 50 µl of test drug dilutions (final concentration of drugs range from 10⁻¹⁰ - 10⁻⁵ M), 100 µl of ¹²⁵I-ovine-CRF (¹²⁵I-o-CRF) (final concentration 150 pM) and 150 µl of the cell homogenate described above. Plates are then allowed to incubate at room temperature for 2 hours before filtering the incubate over GF/F filters (presoaked with 0.3% polyethyleneimine) using an appropriate cell harvester. Filters are rinsed 2 times with ice cold assay buffer before removing individual filters and assessing them for radioactivity on a gamma counter.

Curves of the inhibition of ¹²⁵I-o-CRF binding to cell membranes at various dilutions of test drug are analyzed by the iterative curve fitting program LIGAND [P.J. Munson and D. Rodbard, *Anal. Biochem.* 107:220 (**1980**), which provides Ki values for inhibition which are then used to assess biological activity.

A compound is considered to be active if it has a Ki value of less than about 10000 nM for the inhibition of CRF.

### Inhibition of CRF-Stimulated Adenylate Cyclase Activity

Inhibition of CRF-stimulated adenylate cyclase activity can be performed as described by G. Battaglia et al. *Synapse* 1:572 (**1987**). Briefly, assays are carried out at 37° C for 10 min in 200 ml of buffer containing 100 mM Tris-HCl (pH 7.4 at 37° C), 10 mM MgCl₂, 0.4 mM EGTA, 0.1% BSA, 1 mM isobutylmethylxanthine (IBMX), 250 units/ml phosphocreatine kinase, 5 mM creatine phosphate, 100 mM guanosine 5'-triphosphate, 100 nM oCRF, antagonist peptides (concentration range 10⁻⁹ to 10^{-6m}) and 0.8 mg original wet weight tissue (approximately 40-60 mg protein). Reactions are initiated by the addition of 1 mM ATP/³²P]ATP (approximately 2-4 mCi/tube) and terminated by the addition of 100 ml of 50 mM Tris-HCL, 45 mM ATP and 2% sodium dodecyl sulfate. In order to monitor the recovery of CAMP, 1 µl of [³H]cAMP (approximately 40,000 dpm) is added to each tube prior to separation. The separation of [³²P]cAMP from [³²P]ATP is performed by sequential elution over Dowex and alumina columns.

### In vivo Biological Assay

The in vivo activity of the compounds of the present invention can be assessed using any one of the biological assays available and accepted within the art. Illustrative of these tests include the Acoustic Startle Assay, the Stair Climbing Test, and the Chronic Administration Assay. These and other models useful for the testing of compounds of the present invention have been outlined in C.W. Berridge and A.J. Dunn *Brain Research Reviews* 15:71 (**1990**).
Compounds may be tested in any species of rodent or small mammal.

Compounds of this invention have utility in the treatment of inbalances associated with abnormal levels of corticotropin releasing factor in patients suffering from depression, affective disorders, and/or anxiety.

Compounds of this invention can be administered to treat these abnormalities by means that produce contact of the active agent with the agent's site of action in the body of a mammal. The compounds can be administered by any conventional means available for use in conjunction with pharmaceuticals either as individual therapeutic agent or in combination of therapeutic agents. They can be administered alone, but will generally be administered with a pharmaceutical carrier selected on the basis of the chosen route of administration and standard pharmaceutical practice.

The dosage administered will vary depending on the use and known factors such as pharmacodynamic character of the particular agent, and its mode and route of administration; the recipient's age, weight, and health; nature and extent of symptoms; kind of concurrent treatment; frequency of treatment; and desired effect. For use in the treatment of said diseases or conditions, the compounds of this invention can be orally administered daily at a dosage of the active ingredient of 0.002 to 200 mg/kg of body weight. Ordinarily, a dose of 0.01 to 10 mg/kg in divided doses one to four times a day, or in sustained release formulation will be effective in obtaining the desired pharmacological effect.

Dosage forms (compositions) suitable for administration contain from about 1 mg to about 100 mg of active ingredient per unit. In these pharmaceutical compositions, the active ingredient will ordinarily be present in an amount of about 0.5 to 95% by weight based on the total weight of the composition.

The active ingredient can be administered orally is solid dosage forms, such as capsules, tablets and powders; or in liquid forms such as elixirs, syrups, and/or suspensions. The compounds of this invention can also be administered parenterally in sterile liquid dose formulations.

Gelatin capsules can be used to contain the active ingredient and a suitable carrier such as but not limited to lactose, starch, magnesium stearate, steric acid, or cellulose derivatives. Similar diluents can be used to make compressed tablets. Both tablets and capsules can be manufactured as sustained release products to provide for continuous release of medication over a period of time. Compressed tablets can be sugar-coated or film-coated to mask any unpleasant taste, or used to protect the active ingredients from the atmosphere, or to allow selective disintegration of the tablet in the gastrointestinal tract.

Liquid dose forms for oral administration can contain coloring or flavoring agents to increase patient acceptance.

In general, water, pharmaceutically acceptable oils, saline, aqueous dextrose (glucose), and related sugar solutions and glycols, such as propylene glycol or polyethylene glycol, are suitable carriers for parenteral solutions. Solutions for parenteral administration preferably contain a water soluble salt of the active ingredient, suitable stabilizing agents, and if necessary, butter substances. Antioxidizing agents, such as sodium bisulfite, sodium sulfite, or ascorbic acid, either alone or in combination, are suitable stabilizing agents. Also used are citric acid and its salts, and EDTA. In addition, parenteral solutions can contain preservatives such as benzalkonium chloride, methyl- or propyl-paraben, and chlorobutanol.

Suitable pharmaceutical carriers are described in "Remington's Pharmaceutical Sciences", A. Osol, a standard reference in the field.

Useful pharmaceutical dosage-forms for administration of the compounds of this invention can be illustrated as follows:

### Capsules

A large number of units capsules are prepared by filling standard two-piece hard gelatin capsules each with 100 mg of powdered active ingredient, 150 mg lactose, 50 mg cellulose, and 6 mg magnesium stearate.

### Soft Gelatin Capsules

A mixture of active ingredient in a digestible oil such as soybean, cottonseed oil, or olive oil is prepared and injected by means of a positive displacement was pumped into gelatin to form soft gelatin capsules containing 100 mg of the active ingredient. The capsules were washed and dried.

### Tablets

A large number of tablets are prepared by conventional procedures so that the dosage unit was 100 mg active ingredient, 0.2 mg of colloidal silicon dioxide, 5 mg of magnesium stearate, 275 mg of microcrystalline cellulose, 11 mg of starch, and 98.8 mg lactose. Appropriate coatings may be applied to increase palatability or delayed adsorption.

The compounds of this invention may also be used as reagents or standards in the biochemical study of neurological function, dysfunction, and disease.

Although the present invention has been described and exemplified in terms of certain preferred embodiments, other embodiments will be apparent to those skilled in the art. The invention is, therefore, not limited to the particular embodiments described and exemplified, but is capable of modification or variation without departing from the invention, the full scope of which is delineated by the appended claims.

## Claims

1. A compound of Formula (50) 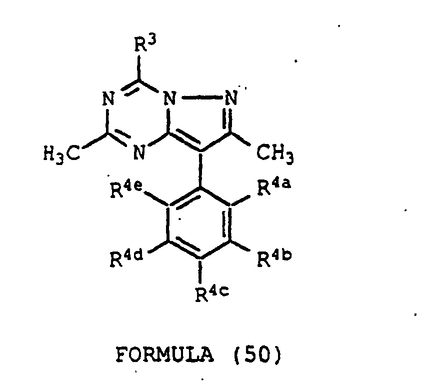 and geometric isomers thereof, stereoisomeric forms thereof, or mixtures of stereoisomeric forms thereof, and pharmaceutically acceptable salt forms thereof, selected from the group:
a compound of Formula (50) wherein R³ is-NHCH(CH₂CH₂OMe) (CH₂OMe), R^{4a} is Me, R^{4b} is H, R^{4c} is Me, R^{4d} is H and R^{4e} is Me;
a compound of Formula (50) wherein R³ is 2-ethylpiperid-l-yl, R^{4a} is Me, R^{4b} is H, R^{4c} is OMe, R^{4d} is H and R^{4e} is H;
a compound of Formula (50) wherein R³ is cyclobutylamino, R^{4a} is Me, R^{4b} is H, R^{4c} is OMe, R^{4d} is H and R^{4e} is H;
a compound of Formula (50) wherein R³ is N(Me)CH₂CH=CH₂, R^{4a} is Me, R^{4b} is H, R^{4c} is OMe, R^{4d} is H and R^{4e} is H;
a compound of Formula (50) wherein R³ is N(Et)CH₂CH=CH₂, R^{4a} is Me, R^{4b} is H, R^{4c} is OMe, R^{4d} is H and R^{4e} is H;
a compound of Formula (50) wherein R³ is N(Me)CH₂cPr, R^{4a} is Me, R^{4b} is H, R^{4c} is OMe, R^{4d} is H and R^{4e} is H;
a compound of Formula (50) wherein R³ is N(Et)CH₂cPr, R^{4a} is Me, R^{4b} is H, R^{4c} is OMe, R^{4d} is H and R^{4e} is H;
a compound of Formula (50) wherein R³ is N(Pr)CH₂cPr, R^{4a} is Me, R^{4b} is H, R^{4c} is OMe, R^{4d} is H and R^{4e} is H;
a compound of Formula (50) wherein R³ is N(Me)Pr, R^{4a} is Me, R^{4b} is H, R^{4c} is OMe, R^{4d} is H and R^{4e} is H;
a compound of Formula (50) wherein R³ is N(Me)Et, R^{4a} is Me, R^{4b} is H, R^{4c} is OMe, R^{4d} is H and R^{4e} is H;
a compound of Formula (50) wherein R³ is N(Me)Bu, R^{4a} is Me, R^{4b} is H, R^{4c} is OMe, R^{4d} is H and R^{4e} is H;
a compound of Formula (50) wherein R³ is N(Me)propargyl, R^{4a} is Me, R^{4b} is H, R^{4c} is OMe, R^{4d} is H and R^{4e} is H;
a compound of Formula (50) wherein R³ is N(Et)propargyl, R^{4a} is Me, R^{4b} is H, R^{4c} is OMe, R^{4d} is H and R^{4e} is H;
a compound of Formula (50) wherein R³ is NHCH(CH₃)CH(CH₃)CH₃, R^{4a} is Me, R^{4b} is H, R^{4c} is OMe, R^{4d} is H and R^{4e} is H;
a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is H;
a compound of Formula (50) wherein R³ is N(CH₂CH₂OMe)Me, R^{4a} is Me, R^{4b} is H, R^{4c} is OMe, R^{4d} is H and R^{4e} is H;
a compound of Formula (50) wherein R³ is N(CH₂CH₂OMe)Et, R^{4a} is Me, R^{4b} is H, R^{4c} is OMe, R^{4d} is H and R^{4e} is H;
a compound of Formula (50) wherein R³ is N(CH₂CH₂OMe)Pr, R^{4a} is Me, R^{4b} is H, R^{4c} is OMe, R^{4d} is H and R^{4e} is H;
a compound of Formula (50) wherein R³ is N(CH₂CH₂OMe)-CH₂cPr, R^{4a} is Me, R^{4b} is H, R^{4c} is OMe, R^{4d} is H and R^{4e} is H;
a compound of Formula (50) wherein R³ is NHCH(CH₃)CH₂CH₃, R^{4a} is Me, R^{4b} is H, R^{4c} is OMe, R^{4d} is H and R^{4e} is H;
a compound of Formula (50) wherein R³ is NHCH(cPr)₂, R^{4a} is Me, R^{4b} is H, R^{4c} is OMe, R^{4d} is H and R^{4e} is H;
a compound of Formula (50) wherein R³ is 2-ethylpiperid-1-yl, R^{4a} is Me, R^{4b} is H, R^{4c} is OMe, R^{4d} is Me and R^{4e} is H;
a compound of Formula (50) wherein R³ is cyclobutylamino, R^{4a} is Me, R^{4b} is H, R^{4c} is OMe, R^{4d} is Me and R^{4e} is H;
a compound of Formula (50) wherein R³ is N(Me)CH₂CH=CH₂, R^{4a} is Me, R^{4b} is H, R^{4c} is OMe, R^{4d} is Me and R^{4e} is H;
a compound of Formula (50) wherein R³ is N(Et)CH₂CH=CH₂, R^{4a} is Me, R^{4b} is H, R^{4c} is OMe, R^{4d} is Me and R^{4e} is H;
a compound of Formula (50) wherein R³ is N(Me)CH₂cPr, R^{4a} is Me, R^{4b} is H, R^{4c} is OMe, R^{4d} is Me and R^{4e} is H;
a compound of Formula (50) wherein R³ is N(Et)CH₂cPr, R^{4a} is Me, R^{4b} is H, R^{4c} is OMe, R^{4d} is Me and R^{4e} is H;
a compound of Formula (50) wherein R³ is N(Pr)CH₂cPr, R^{4a} is Me, R^{4b} is H, R^{4c} is OMe, R^{4d} is Me and R^{4e} is H;
a compound of Formula (50) wherein R³ is N(Me)Pr, R^{4a} is Me, R^{4b} is H, R^{4c} is OMe, R^{4d} is Me and R^{4e} is H;
a compound of Formula (50) wherein R³ is N(Me)Et, R^{4a} is Me, R^{4b} is H, R^{4c} is OMe, R^{4d} is Me and R^{4e} is H;
a compound of Formula (50) wherein R³ is N(Me)Bu, R^{4a} is Me, R^{4b} is H, R^{4c} is OMe, R^{4d} is Me and R^{4e} is H;
a compound of Formula (50) wherein R³ is N(Me)propargyl, R^{4a} is Me, R^{4b} is H, R^{4c} is OMe, R^{4d} is Me and R^{4e} is H;
a compound of Formula (50) wherein R³ is N(Et)propargyl, R^{4a} is Me, R^{4b} is H, R^{4c} is OMe, R^{4d} is Me and R^{4e} is H;
a compound of Formula (50) wherein R³ is NHCH(CH₃)CH(CH₃)CH₃, R^{4a} is Me, R^{4b} is H, R^{4c} is OMe, R^{4d} is Me and R^{4e} is H;
a compound of Formula (50) wherein R³ is N(CH₂CH₂OMe)-CH₂CH=CH₂, R^{4a} is Me, R^{4b} is H, R^{4c} is OMe, R^{4d} is Me and R^{4e} is H;
a compound of Formula (50) wherein R³ is N(CH₂CH₂OMe)Me, R^{4a} is Me, R^{4b} is H, R^{4c} is OMe, R^{4d} is Me and R^{4e} is H;
a compound of Formula (50) wherein R³ is N(CH₂CH₂OMe)Et, R^{4a} is Me, R^{4b} is H, R^{4c} is OMe, R^{4d} is Me and R^{4e} is H;
a compound of Formula (50) wherein R³ is N(CH₂CH₂OMe)Pr, R^{4a} is Me, R^{4b} is H, R^{4c} is OMe, R^{4d} is Me and R^{4e} is H;
a compound of Formula (50) wherein R³ is N(CH₂CH₂OMe)-CH₂cPr, R^{4a} is Me, R^{4b} is H, R^{4c} is OMe, R^{4d} is Me and R^{4e} is H;
a compound of Formula (50) wherein R³ is NHCH(CH₃)CH₂CH₃, R^{4a} is Me, R^{4b} is H, R^{4c} is OMe, R^{4d} is Me and R^{4e} is H;
a compound of Formula (50) wherein R³ is NHCH(cPr)₂, R^{4a} is Me, R^{4b} is H, R^{4c} is OMe, R^{4d} is Me and R^{4e} is H;
a compound of Formula (50) wherein R³ is -NHCH(Et)₂, R^{4a} is OMe, R^{4b} is H, R^{4c} is OMe, R^{4d} is H and R^{4e} is H;
a compound of Formula (50) wherein R³ is 2-ethylpiperid-1-yl, R^{4a} is OMe, R^{4b} is H, R^{4c} is OMe, R^{4d} is H and R^{4e} is H;
a compound of Formula (50) wherein R³ is cyclobutylamino, R^{4a} is OMe, R^{4b} is H, R^{4c} is OMe, R^{4d} is H and R^{4e} is H;
a compound of Formula (50) wherein R³ is N(Me)CH₂CH=CH₂, R^{4a} is OMe, R^{4b} is H, R^{4c} is OMe, R^{4d} is H and R^{4e} is H;
a compound of Formula (50) wherein R³ is N(Et)CH₂CH=CH₂, R^{4a} is OMe, R^{4b} is H, R^{4c} is OMe, R^{4d} is H and R^{4e} is H;
a compound of Formula (50) wherein R³ is N(Me)CH₂cPr, R^{4a} is OMe, R^{4b} is H, R^{4c} is OMe, R^{4d} is H and R^{4e} is H;
a compound of Formula (50) wherein R³ is N(Et)CH₂cPr, R^{4a} is OMe, R^{4b} is H, R^{4c} is OMe, R^{4d} is H and R^{4e} is H;
a compound of Formula (50) wherein R³ is N(Pr)CH₂cPr, R^{4a} is OMe, R^{4b} is H, R^{4c} is OMe, R^{4d} is H and R^{4e} is H;
a compound of Formula (50) wherein R³ is N(Me)Pr, R^{4a} is OMe, R^{4b} is H, R^{4c} is OMe, R^{4d} is H and R^{4e} is H;
a compound of Formula (50) wherein R³ is N(Me)Et, R^{4a} is OMe, R^{4b} is H, R^{4c} is OMe, R^{4d} is H and R^{4e} is H;
a compound of Formula (50) wherein R³ is N(Me)Bu, R^{4a} is OMe, R^{4b} is H, R^{4c} is OMe, R^{4d} is H and R^{4e} is H;
a compound of Formula (50) wherein R³ is N(Me)propargyl, R^{4a} is OMe, R^{4b} is H, R^{4c} is OMe, R^{4d} is H and R^{4e} is H;
a compound of Formula (50) wherein R³ is N(Et)propargyl, R^{4a} is OMe, R^{4b} is H, R^{4c} is OMe, R^{4d} is H and R^{4e} is H;
a compound of Formula (50) wherein R³ is NHCH(CH₃)CH(CH₃)CH₃, R^{4a} is OMe, R^{4b} is H, R^{4c} is OMe, R^{4d} is H and R^{4e} is H;
a compound of Formula (50) wherein R³ is N(CH₂CH₂OMe)-CH₂CH=CH₂, R^{4a} is OMe, R^{4b} is H, R^{4c} is OMe, R^{4d} is H and R^{4e} is H;
a compound of Formula (50) wherein R³ is N(CH₂CH₂OMe)Me, R^{4a} is OMe, R^{4b} is H, R^{4c} is OMe, R^{4d} is H and R^{4e} is H;
a compound of Formula (50) wherein R³ is N(CH₂CH₂OMe)Et, R^{4a} is OMe, R^{4b} is H, R^{4c} is OMe, R^{4d} is H and R^{4e} is H;
a compound of Formula (50) wherein R³ is N(CH₂CH₂OMe)Pr, R^{4a} is OMe, R^{4b} is H, R^{4c} is OMe, R^{4d} is H and R^{4e} is H;
a compound of Formula (50) wherein R³ is N(CH₂CH₂OMe)-CH_{2C}Pr, R^{4a} is OMe, R^{4b} is H, R^{4c} is OMe, R^{4d} is H and R^{4e} is H;
a compound of Formula (50) wherein R³ is NHCH(CH₃)CH₂CH₃, R^{4a} is OMe, R^{4b} is H, R^{4c} is OMe, R^{4d} is H and R^{4e} is H;
a compound of Formula (50) wherein R³ is NHCH(cPr)₂, R^{4a} is OMe, R^{4b} is H, R^{4c} is OMe, R^{4d} is H and R^{4e} is H;
a compound of Formula (50) wherein R³ is N(CH₂CH₂OMe)₂, R^{4a} is OMe, R^{4b} is H, R^{4c} is OMe, R^{4d} is H and R^{4e} is H;
a compound of Formula (50) wherein R³ is NHCH(Et)₂, R^{4a} is OMe, R^{4b} is H, R^{4c} is OMe, R^{4d} is Me and R^{4e} is H;
a compound of Formula (50) wherein R³ is N(Et)₂, R^{4a} is OMe, R^{4b} is H, R^{4c} is OMe, R^{4d} is Me and R^{4e} is H;
a compound of Formula (50) wherein R³ is 2-ethylpiperid-1-yl, R^{4a} is OMe, R^{4b} is H, R^{4c} is OMe, R^{4d} is Me and R^{4e} is H;
a compound of Formula (50) wherein R³ is cyclobutylamino, R^{4a} is OMe, R^{4b} is H, R^{4c} is OMe, R^{4d} is Me and R^{4e} is H;
a compound of Formula (50) wherein R³ is N(Me)CH₂CH=CH₂, R^{4a} is OMe, R^{4b} is H, R^{4c} is OMe, R^{4d} is Me and R^{4e} is H;
a compound of Formula (50) wherein R³ is N(Et)CH₂CH=CH₂, R^{4a} is OMe, R^{4b} is H, R^{4c} is OMe, R^{4d} is Me and R^{4e} is H;
a compound of Formula (50) wherein R³ is N(Me)CH₂cPr, R^{4a} is OMe, R^{4b} is H, R^{4c} is OMe, R^{4d} is Me and R^{4e} is H;
a compound of Formula (50) wherein R³ is N(Et)CH₂cPr, R^{4a} is OMe, R^{4b} is H, R^{4c} is OMe, R^{4d} is Me and R^{4e} is H;
a compound of Formula (50) wherein R³ is N(Pr)CH₂cPr, R^{4a} is OMe, R^{4b} is H, R^{4c} is OMe, R^{4d} is Me and R^{4e} is H;
a compound of Formula (50) wherein R³ is N(Me)Pr, R^{4a} is OMe, R^{4b} is H, R^{4c} is OMe, R^{4d} is Me and R^{4e} is H;
a compound of Formula (50) wherein R³ is N(Me)Et, R^{4a} is OMe, R^{4b} is H, R^{4c} is OMe, R^{4d} is Me and R^{4e} is H;
a compound of Formula (50) wherein R³ is N(Me)Bu, R^{4a} is OMe, R^{4b} is H, R^{4c} is OMe, R^{4d} is Me and R^{4e} is H;
a compound of Formula (50) wherein R³ is N(Me)propargyl, R^{4a} is OMe, R^{4b} is H, R^{4c} is OMe, R^{4d} is Me and R^{4e} is H;
a compound of Formula (50) wherein R³ is N(Et)propargyl, R^{4a} is OMe, R^{4b} is H, R^{4c} is OMe, R^{4d} is Me and R^{4e} is H;
a compound of Formula (50) wherein R³ is NHCH(CH₃)CH(CH₃)CH₃, R^{4a} is OMe, R^{4b} is H, R^{4c} is OMe, R^{4d} is Me and R^{4e} is H;
a compound of Formula (50) wherein R³ is N(CH₂CH₂OMe) -CH₂CH=_{C}H₂, R^{4a} is OMe, R^{4b} is H, R^{4c} is OMe, R^{4d} is Me and R^{4e} is H;
a compound of Formula (50) wherein R³ is N(CH₂CH₂OMe)Me, R^{4a} is OMe, R^{4b} is H, R^{4c} is OMe, R^{4d} is Me and R^{4e} is H;
a compound of Formula (50) wherein R³ is N(CH₂CH₂OMe)Et, R^{4a} is OMe, R^{4b} is H, R^{4c} is OMe, R^{4d} is Me and R^{4e} is H;
a compound of Formula (50) wherein R³ is N(CH₂CH₂OMe)Pr, R^{4a} is OMe, R^{4b} is H, R^{4c} is OMe, R^{4d} is Me and R^{4e} is H;
a compound of Formula (50) wherein R³ is N(CH₂CH₂OMe) -CH₂cPr, R^{4a} is OMe, R^{4b} is H, R^{4c} is OMe, R^{4d} is Me and R^{4e} is H;
a compound of Formula (50) wherein R³ is NHCH(CH₃)CH₂CH₃, R^{4a} is OMe, R^{4b} is H, R^{4c} is OMe, R^{4d} is Me and R^{4e} is H;
a compound of Formula (50) wherein R³ is NHCH(cPr)₂, R^{4a} is OMe, R^{4b} is H, R^{4c} is OMe, R^{4d} is Me and R^{4e} is H;
a compound of Formula (50) wherein R³ is N(CH₂CH₂OMe)₂, R^{4a} is OMe, R^{4b} is H, R^{4c} is OMe, R^{4d} is Me and R^{4e} is H;
a compound of Formula (50) wherein R³ is NHCH(Et)₂, R^{4a} is OMe, R^{4b} is H, R^{4c} is OMe, R^{4d} is Me and R^{4e} is H;
a compound of Formula (50) wherein R³ is N(Et)₂, R^{4a} is OMe, R^{4b} is H, R^{4c} is OMe, R^{4d} is Me and R^{4e} is H;
a compound of Formula (50) wherein R³ is 2-ethylpiperid-1-yl, R^{4a} is Me, R^{4b} is H, R^{4c} is OMe, R^{4d} is H and R^{4e} is Me;
a compound of Formula (50) wherein R³ is cyclobutylamino, R^{4a} is Me, R^{4b} is H, R^{4c} is OMe, R^{4d} is H and R^{4e} is Me;
a compound of Formula (50) wherein R³ is N(Me)CH₂CH=CH₂, R^{4a} is Me, R^{4b} is H, R^{4c} is OMe, R^{4d} is H and R^{4e} is Me;
a compound of Formula (50) wherein R³ is N(Et)CH₂CH=CH₂, R^{4a} is Me, R^{4b} is H, R^{4c} is OMe, R^{4d} is H and R^{4e} is Me;
a compound of Formula (50) wherein R³ is N(Me)CH₂cPr, R^{4a} is Me, R^{4b} is H, R^{4c} is OMe, R^{4d} is H and R^{4e} is Me;
a compound of Formula (50) wherein R³ is N(Et)CH₂cPr, R^{4a} is Me, R^{4b} is H, R^{4c} is OMe, R^{4d} is H and R^{4e} is Me;
a compound of Formula (50) wherein R³ is N(Pr)CH₂cPr, R^{4a} is Me, R^{4b} is H, R^{4c} is OMe, R^{4d} is H and R^{4e} is Me;
a compound of Formula (50) wherein R³ is N(Me)Pr, R^{4a} is Me, R^{4b} is H, R^{4c} is OMe, R^{4d} is H and R^{4e} is Me;
a compound of Formula (50) wherein R³ is N(Me)Et, R^{4a} is Me, R^{4b} is H, R^{4c} is OMe, R^{4d} is H and R^{4e} is Me;
a compound of Formula (50) wherein R³ is N(Me)Bu, R^{4a} is Me, R^{4b} is H, R^{4c} is OMe, R^{4d} is H and R^{4e} is Me;
a compound of Formula (50) wherein R³ is N(Me)propargyl, R^{4a} is Me, R^{4b} is H, R^{4c} is OMe, R^{4d} is H and R^{4e} is Me;
a compound of Formula (50) wherein R³ is N(Et)propargyl, R^{4a} is Me, R^{4b} is H, R^{4c} is OMe, R^{4d} is H and R^{4e} is Me;
a compound of Formula (50) wherein R³ is NHCH(CH₃)CH(CH₃)CH₃, R^{4a} is Me, R^{4b} is H, R^{4c} is OMe, R^{4d} is H and R^{4e} is Me;
a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is Me;
a compound of Formula (50) wherein R³ is N(CH₂CH₂OMe)Me, R^{4a} is Me, R^{4b} is H, R^{4c} is OMe, R^{4d} is H and R^{4e} is Me;
a compound of Formula (50) wherein R³ is N(CH₂CH₂OMe)Et, R^{4a} is Me, R^{4b} is H, R^{4c} is OMe, R^{4d} is H and R^{4e} is Me;
a compound of Formula (50) wherein R³ is N(CH₂CH₂OMe)Pr, R^{4a} is Me, R^{4b} is H, R^{4c} is OMe, R^{4d} is H and R^{4e} is Me;
a compound of Formula (50) wherein R³ is N(CH₂CH₂OMe) - CH₂cPr, R^{4a} is Me, R^{4b} is H, R^{4c} is OMe, R^{4d} is H and R^{4e} is Me;
a compound of Formula (50) wherein R³ is NHCH(CH₃)CH₂CH₃, R^{4a} is Me, R^{4b} is H, R^{4c} is OMe, R^{4d} is H and R^{4e} is Me;
a compound of Formula (50) wherein R³ is NHCH(Et)₂, R^{4a} is Me, R^{4b} is H, R^{4c} is OMe, R^{4d} is H and R^{4e} is Me;
a compound of Formula (50) wherein R³ is NHCH(cPr)₂, R^{4a} is Me, R^{4b} is H, R^{4c} is OMe, R^{4d} is H and R^{4e} is Me;
a compound of Formula (50) wherein R³ is NHCH(Et)₂ R^{4a} is Me, R^{4b} is H, R^{4c} is OMe, R^{4d} is H and R^{4e} is OMe;
a compound of Formula (50) wherein R³ is 2-ethylpiperid-1-yl, R^{4a} is Me, R^{4b} is H, R^{4c} is OMe, R^{4d} is H and R^{4e} is OMe;
a compound of Formula (50) wherein R³ is cyclobutylamino, R^{4a} is Me, R^{4b} is H, R^{4c} is OMe, R^{4d} is H and R^{4e} is OMe;
a compound of Formula (50) wherein R³ is N(Me)CH₂CH=CH₂, R^{4a} is Me, R^{4b} is H, R^{4c} is OMe, R^{4d} is H and R^{4e} is OMe;
a compound of Formula (50) wherein R³ is N(Et)CH₂CH=CH₂, R^{4a} is Me, R^{4b} is H, R^{4c} is OMe, R^{4d} is H and R^{4e} is OMe;
a compound of Formula (50) wherein R³ is N(Me)CH₂cPr, R^{4a} is Me, R^{4b} is H, R^{4c} is OMe, R^{4d} is H and R^{4e} is OMe;
a compound of Formula (50) wherein R³ is N(Et)CH₂cPr, R^{4a} is Me, R^{4b} is H, R^{4c} is OMe, R^{4d} is H and R^{4e} is OMe;
a compound of Formula (50) wherein R³ is N(Pr)CH₂cPr, R^{4a} is Me, R^{4b} is H, R^{4c} is OMe, R^{4d} is H and R^{4e} is OMe;
a compound of Formula (50) wherein R³ is N(Me)Pr, R^{4a} is Me, R^{4b} is H, R^{4c} is OMe, R^{4d} is H and R^{4e} is OMe;
a compound of Formula (50) wherein R³ is N(Me)Et, R^{4a} is Me, R^{4b} is H, R^{4c} is OMe, R^{4d} is H and R^{4e} is OMe;
a compound of Formula (50) wherein R³ is N(Me)Bu, R^{4a} is Me, R^{4b} is H, R^{4c} is OMe, R^{4d} is H and R^{4e} is OMe;
a compound of Formula (50) wherein R³ is N(Me)propargyl, R^{4a} is Me, R^{4b} is H, R^{4c} is OMe, R^{4d} is H and R^{4e} is OMe;
a compound of Formula (50) wherein R³ is N(Et)propargyl, R^{4a} is Me, R^{4b} is H, R^{4c} is OMe, R^{4d} is H and R^{4e} is OMe;
a compound of Formula (50) wherein R³ is NHCH(CH₃)CH(CH₃)CH₃, R^{4a} is Me, R^{4b} is H, R^{4c} is OMe, R^{4d} is H and R^{4e} is OMe;
a compound of Formula (50) wherein R³ is N(CH₂CH₂OMe)-CH₂CH=CH₂, R^{4a} is Me, R^{4b} is H, R^{4c} is OMe, R^{4d} is H and R^{4e} is OMe;
a compound of Formula (50) wherein R³ is N(CH₂CH₂OMe)Me, R^{4a} is Me, R^{4b} is H, R^{4c} is OMe, R^{4d} is H and R^{4e} is OMe;
a compound of Formula (50) wherein R³ is N(CH₂CH₂OMe)Et, R^{4a} is Me, R^{4b} is H, R^{4c} is OMe, R^{4d} is H and R^{4e} is OMe;
a compound of Formula (50) wherein R³ is N(CH₂CH₂OMe)Pr, R^{4a} is Me, R^{4b} is H, R^{4c} is OMe, R^{4d} is H and R^{4e} is OMe;
a compound of Formula (50) wherein R³ is N(CH₂CH₂OMe)-CH₂cPr, R^{4a} is Me, R^{4b} is H, R^{4c} is OMe, R^{4d} is H and R^{4e} is OMe;
a compound of Formula (50) wherein R³ is NHCH(CH₃)CH₂CH₃, R^{4a} is Me, R^{4b} is H, R^{4c} is OMe, R^{4d} is H and R^{4e} is OMe;
a compound of Formula (50) wherein R³ is NHCH(cPr)₂, R^{4a} is Me, R^{4b} is H, R^{4c} is OMe, R^{4d} is H and R^{4e} is OMe;
a compound of Formula (50) wherein R³ is NHCH(Et)₂, R^{4a} is Cl, R^{4b} is H, R^{4c} is OMe, R^{4d} is H and R^{4e} is OMe;
a compound of Formula (50) wherein R³ is 2-ethylpiperid-1-yl, R^{4a} is Cl, R^{4b} is H, R^{4c} is OMe, R^{4d} is H and R^{4e} is OMe;
a compound of Formula (50) wherein R³ is cyclobutylamino, R^{4a} is Cl, R^{4b} is H, R^{4c} is OMe, R^{4d} is H and R^{4e} is OMe;
a compound of Formula (50) wherein R³ is N(Me)CH₂CH=CH₂, R^{4a} is Cl, R^{4b} is H, R^{4c} is OMe, R^{4d} is H and R^{4e} is OMe;
a compound of Formula (50) wherein R³ is N(Et)CH₂CH=CH₂, R^{4a} is Cl, R^{4b} is H, R^{4c} is OMe, R^{4d} is H and R^{4e} is OMe;
a compound of Formula (50) wherein R³ is N(Me)CH₂cPr, R^{4a} is Cl, R^{4b} is H, R^{4c} is OMe, R^{4d} is H and R^{4e} is OMe;
a compound of Formula (50) wherein R³ is N(Et)CH₂cPr, R^{4a} is Cl, R^{4b} is H, R^{4c} is OMe, R^{4d} is H and R^{4e} is OMe;
a compound of Formula (50) wherein R³ is N(Pr)CH₂cPr, R^{4a} is Cl, R^{4b} is H, R^{4c} is OMe, R^{4d} is H and R^{4e} is OMe;
a compound of Formula (50) wherein R³ is N(Me)Pr, R^{4a} is Cl, R^{4b} is H, R^{4c} is OMe, R^{4d} is H and R^{4e} is OMe;
a compound of Formula (50) wherein R³ is N(Me)Et, R^{4a} is Cl, R^{4b} is H, R^{4c} is OMe, R^{4d} is H and R^{4e} is OMe;
a compound of Formula (50) wherein R³ is N(Me)Bu, R^{4a} is Cl, R^{4b} is H, R^{4c} is OMe, R^{4d} is H and R^{4e} is OMe;
a compound of Formula (50) wherein R³ is N(Me)propargyl, R^{4a} is Cl, R^{4b} is H, R^{4c} is OMe, R^{4d} is H and R^{4e} is OMe;
a compound of Formula (50) wherein R³ is N(Et)propargyl, R^{4a} is Cl, R^{4b} is H, R^{4c} is OMe, R^{4d} is H and R^{4e} is OMe;
a compound of Formula (50) wherein R³ is NHCH(CH₃)CH(CH₃)CH₃, R^{4a} is Cl, R^{4b} is H, R^{4c} is OMe, R^{4d} is H and R^{4e} is OMe;
a compound of Formula (50) wherein R³ is N(CH₂CH₂OMe) - CH₂CH=CH₂, R^{4a} is Cl, R^{4b} is H, R^{4c} is OMe, R^{4d} is H and R^{4e} is OMe;
a compound of Formula (50) wherein R³ is N(CH₂CH₂OMe)Me, R^{4a} is Cl, R^{4b} is H, R^{4c} is OMe, R^{4d} is H and R^{4e} is OMe;
a compound of Formula (50) wherein R³ is N(CH₂CH₂OMe)Et, R^{4a} is Cl, R^{4b} is H, R^{4c} is OMe, R^{4d} is H and R^{4e} is OMe;
a compound of Formula (50) wherein R³ is N(CH₂CH₂OMe)Pr, R^{4a} is Cl, R^{4b} is H, R^{4c} is OMe, R^{4d} is H and R^{4e} is OMe;
a compound of Formula (50) wherein R³ is N(CH₂CH₂OMe)-CH₂cPr, R^{4a} is Cl, R^{4b} is H, R^{4c} is OMe, R^{4d} is H and R^{4e} is OMe;
a compound of Formula (50) wherein R³ is NHCH(CH₃)CH₂CH₃, R^{4a} is Cl, R^{4b} is H, R^{4c} is OMe, R^{4d} is H and R^{4e} is OMe;
a compound of Formula (50) wherein R³ is NHCH(cPr)₂ R^{4a} is Cl, R^{4b} is H, R^{4c} is OMe, R^{4d} is H and R^{4e} is OMe;
a compound of Formula (50) wherein R³ is N(CH₂CH₂OMe)₂, R^{4a} is Cl, R^{4b} is H, R^{4c} is OMe, R^{4d} is H and R^{4e} is OMe;
a compound of Formula (50) wherein R³ is NHCH(Et)₂, R^{4a} is Cl, R^{4b} is H, R^{4c} is OMe, R^{4d} is H and R^{4e} is OMe;
a compound of Formula (50) wherein R³ is N(Et)₂, R^{4a} is Cl, R^{4b} is H, R^{4c} is OMe, R^{4d} is H and R^{4e} is OMe;
a compound of Formula (50) wherein R³ is NHCH(Et)₂, R^{4a} is Cl, R^{4b} is H, R^{4c} is OMe, R^{4d} is H and R^{4e} is H;
a compound of Formula (50) wherein R³ is 2-ethylpiperid-1-yl, R^{4a} is Cl, R^{4b} is H, R^{4c} is OMe, R^{4d} is H and R^{4e} is H;
a compound of Formula (50) wherein R³ is cyclobutylamino, R^{4a} is Cl, R^{4b} is H, R^{4c} is OMe, R^{4d} is H and R^{4e} is H;
a compound of Formula (50) wherein R³ is N(Me)CH₂CH=CH₂, R^{4a} is Cl, R^{4b} is H, R^{4c} is OMe, R^{4d} is H and R^{4e} is H;
a compound of Formula (50) wherein R³ is N(Et)CH₂CH=CH₂, R^{4a} is Cl, R^{4b} is H, R^{4c} is OMe, R^{4d} is H and R^{4e} is H;
a compound of Formula (50) wherein R³ is N(Me)CH₂cPr, R^{4a} is Cl, R^{4b} is H, R^{4c} is OMe, R^{4d} is H and R^{4e} is H;
a compound of Formula (50) wherein R³ is N(Et)CH₂cPr, R^{4a} is Cl, R^{4b} is H, R^{4c} is OMe, R^{4d} is H and R^{4e} is H;
a compound of Formula (50) wherein R³ is N(Pr)CH₂cPr, R^{4a} is Cl, R^{4b} is H, R^{4c} is OMe, R^{4d} is H and R^{4e} is H;
a compound of Formula (50) wherein R³ is N(Me)Pr, R^{4a} is Cl, R^{4b} is H, R^{4c} is OMe, R^{4d} is H and R^{4e} is H;
a compound of Formula (50) wherein R³ is N(Me)Et, R^{4a} is Cl, R^{4b} is H, R^{4c} is OMe, R^{4d} is H and R^{4e} is H;
a compound of Formula (50) wherein R³ is N(Me)Bu, R^{4a} is Cl, R^{4b} is H, R^{4c} is OMe, R^{4d} is H and R^{4e} is H;
a compound of Formula (50) wherein R³ is N(Me)propargyl, R^{4a} is Cl, R^{4b} is H, R^{4c} is OMe, R^{4d} is H and R^{4e} is H;
a compound of Formula (50) wherein R³ is N(Et)propargyl, R^{4a} is Cl, R^{4b} is H, R^{4c} is OMe, R^{4d} is H and R^{4e} is H;
a compound of Formula (50) wherein R³ is NHCH(CH₃)CH(CH₃)CH₃, R^{4a} is Cl, R^{4b} is H, R^{4c} is OMe, R^{4d} is H and R^{4e} is H;
a compound of Formula (50) wherein R³ is N(CH₂CH₂OMe)-CH₂CH=CH₂, R^{4a} is Cl, R^{4b} is H, R^{4c} is OMe, R^{4d} is H and R^{4e} is H;
a compound of Formula (50) wherein R³ is N(CH₂CH₂OMe)Me, R^{4a} is Cl, R^{4b} is H, R^{4c} is OMe, R^{4d} is H and R^{4e} is H;
a compound of Formula (50) wherein R³ is N(CH₂CH₂OMe)Et, R^{4a} is Cl, R^{4b} is H, R^{4c} is OMe, R^{4d} is H and R^{4e} is H;
a compound of Formula (50) wherein R³ is N(CH₂CH₂OMe)Pr, R^{4a} is Cl, R^{4b} is H, R^{4c} is OMe, R^{4d} is H and R^{4e} is H;
a compound of Formula (50) wherein R³ is N(CH₂CH₂OMe)-CH₂cPr, R^{4a} is Cl, R^{4b} is H, R^{4c} is OMe, R^{4d} is H and R^{4e} is H;
a compound of Formula (50) wherein R³ is NHCH(CH₃)CH₂CH₃, R^{4a} is Cl, R^{4b} is H, R^{4c} is OMe, R^{4d} is H and R^{4e} is H;
a compound of Formula (50) wherein R³ is NHCH(cPr)₂, R^{4a} is Cl, R^{4b} is H, R^{4c} is OMe, R^{4d} is H and R^{4e} is H;
a compound of Formula (50) wherein R³ is N(CH₂CH₂OMe)₂, R^{4a} is Cl, R^{4b} is H, R^{4c} is OMe, R^{4d} is H and R^{4e} is H;
a compound of Formula (50) wherein R³ is NHCH(Et)₂, R^{4a} is Cl, R^{4b} is H, R^{4c} is OMe, R^{4d} is H and R^{4e} is H;
a compound of Formula (50) wherein R³ is NHCH(Et)₂, R^{4a} is Cl, R^{4b} is H, R^{4c} is OMe, R^{4d} is F and R^{4e} is H;
a compound of Formula (50) wherein R³ is 2-ethylpiperid-1-yl, R^{4a} is Cl, R^{4b} is H, R^{4c} is OMe, R^{4d} is F and R^{4e} is H;
a compound of Formula (50) wherein R³ is cyclobutylamino, R^{4a} is Cl, R^{4b} is H, R^{4c} is OMe, R^{4d} is F and R^{4e} is H;
a compound of Formula (50) wherein R³ is N(Me)CH₂CH=CH₂, R^{4a} is Cl, R^{4b} is H, R^{4c} is OMe, R^{4d} is F and R^{4e} is H;
a compound of Formula (50) wherein R³ is N(Et)CH₂CH=CH₂, R^{4a} is Cl, R^{4b} is H, R^{4c} is OMe, R^{4d} is F and R^{4e} is H;
a compound of Formula (50) wherein R³ is N(Me)CH₂cPr, R^{4a} is Cl, R^{4b} is H, R^{4c} is OMe, R^{4d} is F and R^{4e} is H;
a compound of Formula (50) wherein R³ is N(Et)CH₂cPr, R^{4a} is Cl, R^{4b} is H, R^{4c} is OMe, R^{4d} is F and R^{4e} is H;
a compound of Formula (50) wherein R³ is N(Pr)CH₂cPr, R^{4a} is Cl, R^{4b} is H, R^{4c} is OMe, R^{4d} is F and R^{4e} is H;
a compound of Formula (50) wherein R³ is N(Me)Pr, R^{4a} is Cl, R^{4b} is H, R^{4c} is OMe, R^{4d} is F and R^{4e} is H;
a compound of Formula (50) wherein R³ is N(Me)Et, R^{4a} is Cl, R^{4b} is H, R^{4c} is OMe, R^{4d} is F and R^{4e} is H;
a compound of Formula (50) wherein R³ is N(Me)Bu, R^{4a} is Cl, R^{4b} is H, R^{4c} is OMe, R^{4d} is F and R^{4e} is H;
a compound of Formula (50) wherein R³ is N(Me)propargyl, R^{4a} is Cl, R^{4b} is H, R^{4c} is OMe, R^{4d} is F and R^{4e} is H;
a compound of Formula (50) wherein R³ is NHCH(CH₃)CH(CH₃)CH₃, R^{4a} is Cl, R^{4b} is H, R^{4c} is OMe, R^{4d} is F and R^{4e} is H;
a compound of Formula (50) wherein R³ is N(CH₂CH₂OMe) - CH₂CH=CH₂, R^{4a} is Cl, R^{4b} is H, R^{4c} is OMe, R^{4d} is F and R^{4e} is H;
a compound of Formula (50) wherein R³ is N(CH₂CH₂OMe)Me, R^{4a} is Cl, R^{4b} is H, R^{4c} is OMe, R^{4d} is F and R^{4e} is H;
a compound of Formula (50) wherein R³ is N(CH₂CH₂OMe)Et, R^{4a} is Cl, R^{4b} is H, R^{4c} is OMe, R^{4d} is F and R^{4e} is H;
a compound of Formula (50) wherein R³ is N(CH₂CH₂OMe)Pr, R^{4a} is Cl, R^{4b} is H, R^{4c} is OMe, R^{4d} is F and R^{4e} is H;
a compound of Formula (50) wherein R³ is N(CH₂CH₂OMe)-CH₂cPr, R^{4a} is Cl, R^{4b} is H, R^{4c} is OMe, R^{4d} is F and R^{4e} is H;
a compound of Formula (50) wherein R³ is NHCH(CH₃)CH₂CH₃, R^{4a} is Cl, R^{4b} is F, R^{4c} is OMe, R^{4d} is H and R^{4e} is H;
a compound of Formula (50) wherein R³ is NHCH(cPr)₂, R^{4a} is Cl, R^{4b} is H, R^{4c} is OMe, R^{4d} is F and R^{4e} is H;
a compound of Formula (50) wherein R³ is N(CH₂CH₂OMe)₂, R^{4a} is Cl, R^{4b} is H, R^{4c} is OMe, R^{4d} is F and R^{4e} is H;
a compound of Formula (50) wherein R³ is NHCH(Et)₂, R^{4a} is Cl, R^{4b} is H, R^{4c} is OMe, R^{4d} is F and R^{4e} is H;
a compound of Formula (50) wherein R³ is N(Et)₂, R^{4a} is Cl, R^{4b} is H, R^{4c} is OMe, R^{4d} is F and R^{4e} is H;
a compound of Formula (50) wherein R³ is NHCH(Et)₂, R^{4a} is Cl, R^{4b} is H, R^{4c} is OMe, R^{4d} is OMe and R^{4e} is H;
a compound of Formula (50) wherein R³ is 2-ethylpiperid-1-yl, R^{4a} is Cl, R^{4b} is H, R^{4c} is OMe, R^{4d} is OMe and R^{4e} is H;
a compound of Formula (50) wherein R³ is cyclobutylamino, R^{4a} is Cl, R^{4b} is H, R^{4c} is OMe, R^{4d} is OMe and R^{4e} is H;
a compound of Formula (50) wherein R³ is N(Me)CH₂CH=CH₂, R^{4a} is Cl, R^{4b} is H, R^{4c} is OMe, R^{4d} is OMe and R^{4e} is H;
a compound of Formula (50) wherein R³ is N(Et)CH₂CH=CH₂, R^{4a} is Cl, R^{4b} is H, R^{4c} is OMe, R^{4d} is OMe and R^{4e} is H;
a compound of Formula (50) wherein R³ is N(Me)CH₂cPr, R^{4a} is Cl, R^{4b} is H, R^{4c} is OMe, R^{4d} is F and R^{4e} is H;
a compound of Formula (50) wherein R³ is N(Et)CH₂cPr, R^{4a} is Cl, R^{4b} is H, R^{4c} is OMe, R^{4d} is OMe and R^{4e} is H;
a compound of Formula (50) wherein R³ is N(Pr)CH₂cPr, R^{4a} is Cl, R^{4b} is H, R^{4c} is OMe, R^{4d} is OMe and R^{4e} is H;
a compound of Formula (50) wherein R³ is N(Me)Pr, R^{4a} is Cl, R^{4b} is H, R^{4c} is OMe, R^{4d} is OMe and R^{4e} is H;
a compound of Formula (50) wherein R³ is N(Me)Et, R^{4a} is Cl, R^{4b} is H, R^{4c} is OMe, R^{4d} is OMe and R^{4e} is H;
a compound of Formula (50) wherein R³ is N(Me)Bu, R^{4a} is Cl, R^{4b} is H, R^{4c} is OMe, R^{4d} is OMe and R^{4e} is H;
a compound of Formula (50) wherein R³ is N(Me)propargyl, R^{4a} is Cl, R^{4b} is H, R^{4c} is OMe, R^{4d} is OMe and R^{4e} is H;
a compound of Formula (50) wherein R³ is NHCH(CH₃)CH(CH₃)CH₃, R^{4a} is Cl, R^{4b} is H, R^{4c} is OMe, R^{4d} is OMe and R^{4e} is H;
a compound of Formula (50) wherein R³ is N(CH₂CH₂OMe)-CH₂CH=CH₂, R^{4a} is Cl, R^{4b} is H, R^{4c} is OMe, R^{4d} is F and R^{4e} is H;
a compound of Formula (50) wherein R³ is N(CH₂CH₂OMe)Me, R^{4a} is Cl, R^{4b} is H, R^{4c} is OMe, R^{4d} is OMe and R^{4e} is H;
a compound of Formula (50) wherein R³ is N(CH₂CH₂OMe)Et, R^{4a} is Cl, R^{4b} is H, R^{4c} is OMe, R^{4d} is OMe and R^{4e} is H;
a compound of Formula (50) wherein R³ is N(CH₂CH₂OMe)Pr, R^{4a} is Cl, R^{4b} is H, R^{4c} is OMe, R^{4d} is OMe and R^{4e} is H;
a compound of Formula (50) wherein R³ is N(CH₂CH₂OMe)-CH₂cPr, R^{4a} is Cl, R^{4b} is H, R^{4c} is OMe, R^{4d} is OMe and R^{4e} is H;
a compound of Formula (50) wherein R³ is NHCH(CH₃)CH₂CH₃, R^{4a} is Cl, R^{4b} is H, R^{4c} is OMe, R^{4d} is OMe and R^{4e} is H;
a compound of Formula (50) wherein R³ is NHCH(cPr)₂, R^{4a} is Cl, R^{4b} is H, R^{4c} is OMe, R^{4d} is OMe and R^{4e} is H;
a compound of Formula (50) wherein R³ is N(CH₂CH₂OMe)₂, R^{4a} is Cl, R^{4b} is H, R^{4c} is OMe, R^{4d} is OMe and R^{4e} is H;
a compound of Formula (50) wherein R³ is NHCH(Et)₂, R^{4a} is Cl, R^{4b} is H, R^{4c} is OMe, R^{4d} is OMe and R^{4e} is H;
a compound of Formula (50) wherein R³ is N(Et)₂, R^{4a} is Cl, R^{4b} is H, R^{4c} is OMe, R^{4d} is OMe and R^{4e} is H;
a compound of Formula (50) wherein R³ is NHCH(Et)₂, R^{4a} is Br, R^{4b} is H, R^{4c} is OMe, R^{4d} is OMe and R^{4e} is H;
a compound of Formula (50) wherein R³ is 2-ethylpiperid-1-yl, R^{4a} is Br, R^{4b} is H, R^{4c} is OMe, R^{4d} is OMe and R^{4e} is H;
a compound of Formula (50) wherein R³ is cyclobutylamino, R^{4a} is Br, R^{4b} is H, R^{4c} is OMe, R^{4d} is OMe and R^{4e} is H;
a compound of Formula (50) wherein R³ is N(Me)CH₂CH=CH₂, R^{4a} is Br, R^{4b} is H, R^{4c} is OMe, R^{4d} is OMe and R^{4e} is H;
a compound of Formula (50) wherein R³ is N(Et)CH₂CH=CH₂, R^{4a} is Br, R^{4b} is H, R^{4c} is OMe, R^{4d} is OMe and R^{4e} is H;
a compound of Formula (50) wherein R³ is N(Me)CH₂cPr, R^{4a} is Br, R^{4b} is H, R^{4c} is OMe, R^{4d} is F and R^{4e} is H;
a compound of Formula (50) wherein R³ is N(Et)CH₂cPr, R^{4a} is Br, R^{4b} is H, R^{4c} is OMe, R^{4d} is OMe and R^{4e} is H;
a compound of Formula (50) wherein R³ is N(Pr)CH₂cPr, R^{4a} is Br, R^{4b} is H, R^{4c} is OMe, R^{4d} is OMe and R^{4e} is H;
a compound of Formula (50) wherein R³ is N(Me)Pr, R^{4a} is Br, R^{4b} is H, R^{4c} is OMe, R^{4d} is OMe and R^{4e} is H;
a compound of Formula (50) wherein R³ is N(Me)Et, R^{4a} is Br, R^{4b} is H, R^{4c} is OMe, R^{4d} is OMe and R^{4e} is H;
a compound of Formula (50) wherein R³ is N(Me)Bu, R^{4a} is Br, R^{4b} is H, R^{4c} is OMe, R^{4d} is OMe and R^{4e} is H;
a compound of Formula (50) wherein R³ is N(Me)propargyl, R^{4a} is Br, R^{4b} is H, R^{4c} is OMe, R^{4d} is OMe and R^{4e} is H;
a compound of Formula (50) wherein R³ is NHCH(CH₃)CH(CH₃)CH₃, R^{4a} is Br, R^{4b} is H, R^{4c} is OMe, R^{4d} is OMe and R^{4e} is H;
a compound of Formula (50) wherein R³ is N(CH₂CH₂OMe) - CH₂CH=CH₂, R^{4a} is Br, R^{4b} is H, R^{4c} is OMe, R^{4d} is F and R^{4e} is H;
a compound of Formula (50) wherein R³ is N(CH₂CH₂OMe)Me, R^{4a} is Br, R^{4b} is H, R^{4c} is OMe, R^{4d} is OMe and R^{4e} is H;
a compound of Formula (50) wherein R³ is N(CH₂CH₂OMe)Et, R^{4a} is Br, R^{4b} is H, R^{4c} is OMe, R^{4d} is OMe and R^{4e} is H;
a compound of Formula (50) wherein R³ is N(CH₂CH₂OMe)Pr, R^{4a} is Br, R^{4b} is H, R^{4c} is OMe, R^{4d} is OMe and R^{4e} is H;
a compound of Formula (50) wherein R³ is N(CH₂CH₂OMe) - CH₂cPr, R^{4a} is Br, R^{4b} is H, R^{4c} is OMe, R^{4d} is OMe and R^{4e} is H;
a compound of Formula (50) wherein R³ is NHCH(CH₃)CH₂CH₃, R^{4a} is Br, R^{4b} is H, R^{4c} is OMe, R^{4d} is OMe and R^{4e} is H;
a compound of Formula (50) wherein R³ is NHCH(cPr)₂, R^{4a} is Br, R^{4b} is H, R^{4c} is OMe, R^{4d} is OMe and R^{4e} is H;
a compound of Formula (50) wherein R³ is N(CH₂CH₂OMe)₂, R^{4a} is Br, R^{4b} is H, R^{4c} is OMe, R^{4d} is OMe and R^{4e} is H;
a compound of Formula (50) wherein R³ is NHCH(Et)₂, R^{4a} is Br, R^{4b} is H, R^{4c} is OMe, R^{4d} is OMe and R^{4e} is H;
a compound of Formula (50) wherein R³ is NHCH(Et)₂, R^{4a} is Me, R^{4b} is H, R^{4c} is OMe, R^{4d} is OMe and R^{4e} is H;
a compound of Formula (50) wherein R³ is 2-ethylpiperid-1-yl, R^{4a} is Me, R^{4b} is H, R^{4c} is OMe, R^{4d} is OMe and R^{4e} is H;
a compound of Formula (50) wherein R³ is cyclobutylamino, R^{4a} is Me, R^{4b} is H, R^{4c} is OMe, R^{4d} is OMe and R^{4e} is H;
a compound of Formula (50) wherein R³ is N(Me)CH₂CH=CH₂, R^{4a} is Me, R^{4b} is H, R^{4c} is OMe, R^{4d} is OMe and R^{4e} is H;
a compound of Formula (50) wherein R³ is N(Et)CH₂CH=CH₂, R^{4a} is Me, R^{4b} is H, R^{4c} is OMe, R^{4d} is OMe and R^{4e} is H;
a compound of Formula (50) wherein R³ is N(Me)CH₂cPr, R^{4a} is Me, R^{4b} is H, R^{4c} is OMe, R^{4d} is F and R^{4e} is H;
a compound of Formula (50) wherein R³ is N(Et)CH₂cPr, R^{4a} is Me, R^{4b} is H, R^{4c} is OMe, R^{4d} is OMe and R^{4e} is H;
a compound of Formula (50) wherein R³ is N(Pr)CH₂cPr, R^{4a} is Me, R^{4b} is H, R^{4c} is OMe, R^{4d} is OMe and R^{4e} is H;
a compound of Formula (50) wherein R³ is N(Me)Pr, R^{4a} is Me, R^{4b} is H, R^{4c} is OMe, R^{4d} is OMe and R^{4e} is H;
a compound of Formula (50) wherein R³ is N(Me)Et, R^{4a} is Me, R^{4b} is H, R^{4c} is OMe, R^{4d} is OMe and R^{4e} is H;
a compound of Formula (50) wherein R³ is N(Me)Bu, R^{4a} is Me, R^{4b} is H, R^{4c} is OMe, R^{4d} is OMe and R^{4e} is H;
a compound of Formula (50) wherein R³ is N(Me)propargyl, R^{4a} is Me, R^{4b} is H, R^{4c} is OMe, R^{4d} is OMe and R^{4e} is H;
a compound of Formula (50) wherein R³ is NHCH(CH₃)CH(CH₃)CH₃, R^{4a} is Br, R^{4b} is H, R^{4c} is OMe, R^{4d} is OMe and R^{4e} is H;
a compound of Formula (50) wherein R³ is N(CH₂CH₂OMe)-CH₂CH=CH₂, R^{4a} is Me, R^{4b} is H, R^{4c} is OMe, R^{4d} is F and R^{4e} is H;
a compound of Formula (50) wherein R³ is N(CH₂CH₂OMe)Me, R^{4a} is Me, R^{4b} is H, R^{4c} is OMe, R^{4d} is OMe and R^{4e} is H;
a compound of Formula (50) wherein R³ is N(CH₂CH₂OMe)Et, R^{4a} is Me, R^{4b} is H, R^{4c} is OMe, R^{4d} is OMe and R^{4e} is H;
a compound of Formula (50) wherein R³ is M(CH₂CH₂OMe)Pr, R^{4a} is Br, R^{4b} is H, R^{4c} is OMe, R^{4d} is OMe and R^{4e} is H;
a compound of Formula (50) wherein R³ is N(CH₂CH₂OMe)-CH₂cPr, R^{4a} is Me, R^{4b} is H, R^{4c} is OMe, R^{4d} is OMe and R^{4e} is H;
a compound of Formula (50) wherein R³ is NHCH(CH₃)CH₂CH₃, R^{4a} is Me, R^{4b} is H, R^{4c} is OMe, R^{4d} is OMe and R^{4e} is H;
a compound of Formula (50) wherein R³ is NHCH(cPr)₂ R^{4a} is Me, R^{4b} is H, R^{4c} is OMe, R^{4d} is OMe and R^{4e} is H;
a compound of Formula (50) wherein R³ is N(CH₂CH₂OMe)₂, R^{4a} is Me, R^{4b} is H, R^{4c} is OMe, R^{4d} is OMe and R^{4e} is H;
a compound of Formula (50) wherein R³ is NHCH(Et)₂, R^{4a} is Me, R^{4b} is H, R^{4c} is OMe, R^{4d} is OMe and R^{4e} is H; and
a compound of Formula (50) wherein R³ is N(Et)₂, R^{4a} is Me, R^{4b} is H, R^{4c} is OMe, R^{4d} is OMe and R^{4e} is H.

2. A compound of Formula (60) 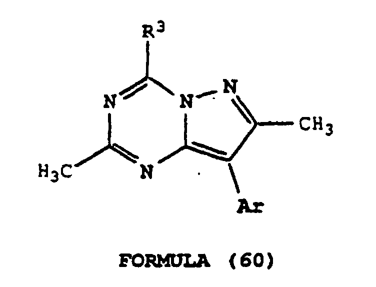 and geometric isomers thereof, stereoisomeric forms thereof, or mixtures of stereoisomeric forms thereof, and pharmaceutically acceptable salt forms thereof, selected from the group:
a compound of Formula (60) wherein R³ is NHCH(Et)₂, Ar is 6-dimethylamino-4-methylpyrid-3-yl;
a compound of Formula (60) wherein R³ is 2-ethylpiperid-1-yl, Ar is 6-dimethylamino-4-methylpyrid-3-yl;
a compound of Formula (60) wherein R³ is cyclobutylamino, Ar is 6-dimethylamino-4-methylpyrid-3-yl;
a compound of Formula (60) wherein R³ is N(Me)CH₂CH=CH₂, Ar is 6-dimethylamino-4-methylpyrid-3-yl;
a compound of Formula (60) wherein R³ is N(Et)CH₂cPr, Ar is 6-dimethylamino-4-methylpyrid-3-yl;
a compound of Formula (60) wherein R³ is N(Pr)CH₂cPr, Ar is 6-dimethylamino-4-methylpyrid-3-yl;
a compound of Formula (60) wherein R³ is N(Me)Pr, Ar is 6-dimethylamino-4-methylpyrid-3-yl;
a compound of Formula (60) wherein R³ is N(Me)Et, Ar is 6-dimethylamino-4-methylpyrid-3-yl;
a compound of Formula (60) wherein R³ is N(Me)Bu, Ar is 6-dimethylamino-4-methylpyrid-3-yl;
a compound of Formula (60) wherein R³ is N(Me)propargyl, Ar is 6-dimethylamino-4-methylpyrid-3-yl;
a compound of Formula (60) wherein R³ is N(Et)propargyl, Ar is 6-dimethylamino-4-methylpyrid-3-yl;
a compound of Formula (60) wherein R³ is NHCH(CH₃)CH(CH₃)CH₃, Ar is 6-dimethylamino-4-methylpyrid-3-yl;
a compound of Formula (60) wherein R³ is N(CH₂CH₂OMe)-CH₂CH=CH₂, Ar is 6-dimethylamino-4-methylpyrid-3-yl;
a compound of Formula (60) wherein R³ is N-(CH₂CH₂OMe)Me, Ar is 6-dimethylamino-4-methylpyrid-3-yl;
a compound of Formula (60) wherein R³ is N(CH₂CH₂OMe)Et, Ar is 6-dimethylamino-4-methylpyrid-3-yl;
a compound of Formula (60) wherein R³ is N(CH₂CH₂OMe)Pr, Ar is 6-dimethylamino-4-methylpyrid-3-yl;
a compound of Formula (60) wherein R³ is N(CH₂CH₂OMe)-CH₂cPr, Ar is 6-dimethylamino-4-methylpyrid-3-yl;
a compound of Formula (60) wherein R³ is NHCH(CH₃)CH₂CH₃, Ar is 6-dimethylamino-4-methylpyrid-3-yl;
a compound of Formula (60) wherein R³ is NHCH(cPr)₂, Ar is 6-dimethylamino-4-methylpyrid-3-yl;
a compound of Formula (60) wherein R³ is N(CH₂CH₂OMe)₂, Ar is 6-dimethylamino-4-methylpyrid-3-yl;
a compound of Formula (60) wherein R³ is NHCH(Et)₂, Ar is 6-dimethylamino-4-methylpyrid-3-yl;
a compound of Formula (60) wherein R³ is N(Et)₂, Ar is 6-dimethylamino-4-methylpyrid-3-yl;
a compound of Formula (60) wherein R³ is 2-ethylpiperid-1-yl, Ar is 6-dimethylamino-4-methylpyrid-3-yl;
a compound of Formula (60) wherein R³ is cyclobutylamino, Ar is 6-dimechylamino-4-methylpyrid-3-yl;
a compound of Formula (60) wherein R³ is N(Me)CH₂CH=CH₂, Ar is 6-dimethylamino-4-methylpyrid-3-yl;
a compound of Formula (60) wherein R³ is N(Et)CH₂cPr, Ar is 6-dimethylamino-4-methylpyrid-3-yl;
a compound of Formula (60) wherein R³ is N(Pr)CH₂cPr, Ar is 6-dimethylamino-4-methylpyrid-3-yl;
a compound of Formula (60) wherein R³ is N(Me)Pr, Ar is 6-dimethylamino-4-methylpyrid-3-yl;
a compound of Formula (60) wherein R³ is N(Me)Et, Ar is 6-dimethylamino-4-methylpyrid-3-yl;
a compound of Formula (60) wherein R³ is N(Me)Bu, Ar is 6-dimethy2amino-4-methylpyrid-3-yl;
a compound of Formula (60) wherein R³ is N(Me)propargyl, Ar is 6-dimethylamino-4-methylpyrid-3-yl;
a compound of Formula (60) wherein R³ is NHCH(CH₃)CH(CH₃)CH₃, Ar is 6-dimethylamino-4-methylpyrid-3-yl;
a compound of Formula (60) wherein R³ is N(CH₂CH₂OMe)-CH₂CH=CH₂, Ar is 6-dimethylamino-4-methylpyrid-3-yl;
a compound of Formula (60) wherein R³ is N(CH₂CH₂OMe)Me, Ar is 6-dimethylamino-4-methylpyrid-3-yl;
a compound of Formula (60) wherein R³ is N(CH₂CH₂OMe)Et, Ar is 6-dimethylamino-4-methylpyrid-3-yl;
a compound of Formula (60) wherein R³ is N(CH₂CH₂OMe)Pr, Ar is 6-dimethylamino-4-methylpyrid-3-yl;
a compound of Formula (60) wherein R³ is N(CH₂CH₂OMe)-CH₂cPr, Ar is 6-dimethylamino-4-methylpyrid-3-yl;
a compound of Formula (60) wherein R³ is NHCH(CH₃)CH₂CH₃, Ar is 6-dimethylamino-4-methylpyrid-3-yl;
a compound of Formula (60) wherein R³ is NHCH(cPr)₂, Ar is 6-dimethylamino-4-methylpyrid-3-yl;
a compound of Formula (60) wherein R³ is N(CH₂CH₂OMe)₂, Ar is 6-dimethylamino-4-methylpyrid-3-yl;
a compound of Formula (60) wherein R³ is NHCH(Et)₂, Ar is 6-dimethylamino-4-mechylpyrid-3-yl;
a compound of Formula (60) wherein R³ is N(Et)₂, Ar is 6-dimethylamino-4-methylpyrid-3-yl.
a compound of Formula (60) wherein R³ is 2-ethylpiperid-1-yl, Ar is 6- methoxy -4-methylpyrid-3-yl;
a compound of Formula (60) wherein R³ is cyclobutylamino, Ar is 6- methoxy -4-methylpyrid-3-yl;
a compound of Formula (60) wherein R³ is N(Me)CH₂CH=CH₂, Ar is 6- methoxy -4-methylpyrid-3-yl;
a compound of Formula (60) wherein R³ is N(Et)CH₂cPr, Ar is 6- methoxy -4-methylpyrid-3-yl;
a compound of Formula (60) wherein R³ is N(Pr)CH₂cPr, Ar is 6- methoxy -4-methylpyrid-3-yl;
a compound of Formula (60) wherein R³ is N(Me)Pr, Ar is 6- methoxy -4-methylpyrid-3-yl;
a compound of Formula (60) wherein R³ is N(Me)Et, Ar is 6- methoxy -4-methylpyrid-3-yl;
a compound of Formula (60) wherein R³ is N(Me)Bu, Ar is 6- methoxy -4-methylpyrid-3-yl;
a compound of Formula (60) wherein R³ is N(Me)propargyl, Ar is 6- methoxy -4-methylpyrid-3-yl;
a compound of Formula (60) wherein R³ is N(Et)propargyl, Ar is 6- methoxy -4-methylpyrid-3-yl;
a compound of Formula (60) wherein R³ is NHCH(CH₃)CH(CH₃)CH₃, Ar is 6- methoxy -4-methylpyrid-3-yl;
a compound of Formula (60) wherein R³ is N(CH₂CH₂OMe)-CH₂CH=CH₂, Ar is 6- methoxy -4-methylpyrid-3-yl;
a compound of Formula (60) wherein R³ is N(CH₂CH₂OMe)Me, Ar is 6- methoxy -4-methylpyrid-3-yl;
a compound of Formula (60) wherein R³ is N(CH₂CH₂OMe)Et, Ar is 6- methoxy -4-methylpyrid-3-yl;
a compound of Formula (60) wherein R³ is N(CH₂CH₂OMe)Pr, Ar is 6- methoxy -4-methylpyrid-3-yl;
a compound of Formula (60) wherein R³ is N(CH₂CH₂OMe)-CH₂cPr, Ar is 6- methoxy -4-methylpyrid-3-yl;
a compound of Formula (60) wherein R³ is NHCH(CH₃)CH₂CH₃, Ar is 6- methoxy -4-methylpyrid-3-yl;
a compound of Formula (60) wherein R³ is NHCH(cPr)₂, Ar is 6- methoxy -4-methylpyrid-3-yl;
a compound of Formula (60) wherein R³ is N(CH₂CH₂OMe)₂, Ar is 6- methoxy -4-methylpyrid-3-yl;
a compound of Formula (60) wherein R³ is NHCH(Et)₂ Ar is 6- methoxy -4-methylpyrid-3-yl;
a compound of Formula (60) wherein R³ is N(Et)₂, Ar is 6- methoxy -4-methylpyrid-3-yl;
a compound of Formula (60) wherein R³ is 2-ethylpiperid-1-yl, Ar is 4-methoxy-6-methylpyrid-3-yl;
a compound of Formula (60) wherein R³ is cyclobutylamino, Ar is 4-methoxy-6-methylpyrid-3-yl;
a compound of Formula (60) wherein R³ is N(Me)CH₂CH=CH₂, Ar is 4-methoxy-6-methylpyrid-3-yl;
a compound of Formula (60) wherein R³ is N(Et)CH₂cPr, Ar is 4-methoxy-6-methylpyrid-3-yl;
a compound of Formula (60) wherein R³ is N(Pr)CH₂cPr, Ar is 4-methoxy-6-methylpyrid-3-yl;
a compound of Formula (60) wherein R³ is N(Me)Pr, Ar is 4-methoxy-6-methylpyrid-3-yl;
a compound of Formula (60) wherein R³ is N(Me)Et, Ar is 4-methoxy-6-methylpyrid-3-yl;
a compound of Formula (60) wherein R³ is N(Me)Bu, Ar is 4-methoxy-6-methylpyrid-3-yl;
a compound of Formula (60) wherein R³ is N(Me)propargyl, Ar is 4-methoxy-6-methylpyrid-3-yl;
a compound of Formula (60) wherein R³ is NHCH(CH₃)CH(CH₃)CH₃, Ar is 4-methoxy-6-methylpyrid-3-yl;
a compound of Formula (60) wherein R³ is N(CH₂CH₂OMe)-CH₂CH=CH₂, Ar is 4-methoxy-6-methylpyrid-3-yl;
a compound of Formula (60) wherein R³ is N(CH₂CH₂OMe)Me, Ar is 4-methoxy-6-methylpyrid-3-yl;
a compound of Formula (60) wherein R³ is N(CH₂CH₂OMe)Et, Ar is 4-methoxy-6-methylpyrid-3-yl;
a compound of Formula (60) wherein R³ is N(CH₂CH₂OMe)Pr, Ar is 4-methoxy-6-methylpyrid-3-yl;
a compound of Formula (60) wherein R³ is N(CH₂CH₂OMe)-CH₂cPr, Ar is 4-methoxy-6-methylpyrid-3-yl;
a compound of Formula (60) wherein R³ is NHCH(CH₃)CH₂CH₃, Ar is 4-methoxy-6-methylpyrid-3-yl;
a compound of Formula (60) wherein R³ is NHCH(cPr)₂, Ar is 4-methoxy-6-methylpyrid-3-yl;
a compound of Formula (60) wherein R³ is N(CH₂CH₂OMe)₂, Ar is 4-methoxy-6-methylpyrid-3-yl;
a compound of Formula (60) wherein R³ is NHCH(Et)₂, Ar is 6- methoxy -4-methylpyrid-3-yl;
a compound of Formula (60) wherein R³ is N(Et)₂, Ar is 4-methoxy-6-methylpyrid-3-yl;
a compound of Formula (60) wherein R³ is 2-ethylpiperid-1-yl, Ar is 4,6-dimethylpyrid-3-yl;
a compound of Formula (60) wherein R³ is cyclobutylamino, Ar is 4,6-dimethylpyrid-3-yl;
a compound of Formula (60) wherein R³ is N(Me)CH₂CH=CH₂, Ar is 4,6-dimethylpyrid-3-yl;
a compound of Formula (60) wherein R³ is N(Et)CH₂cPr, Ar is 4,6-dimethylpyrid-3-yl;
a compound of Formula (60) wherein R³ is N(Pr)CH₂cPr, Ar is 4,6-dimethylpyrid-3-yl;
a compound of Formula (60) wherein R³ is N(Me)Pr, Ar is 4,6-dimethylpyrid-3-yl;
a compound of Formula (60) wherein R³ is N(Me)Et Ar is 4,6-dimethylpyrid-3-yl;
a compound of Formula (60) wherein R³ is N(Me)Bu, Ar is 4,6-dimethylpyrid-3-yl;
a compound of Formula (60) wherein R³ is N(Me)propargyl, Ar is 4,6-dimethylpyrid-3-yl;
a compound of Formula (60) wherein R³ is N(Et)propargyl, Ar is 4,6-dimethylpyrid-3-yl;
a compound of Formula (60) wherein R³ is NHCH(CH₃)CH(CH₃)CH₃, Ar is 4,6-dimethylpyrid-3-yl;
a compound of Formula (60) wherein R³ is N(CH₂CH₂OMe)-CH₂CH=CH₂, Ar is 4,6-dimethylpyrid-3-yl;
a compound of Formula (60) wherein R³ is N(CH₂CH₂OMe)Me, Ar is 4,6-dimethylpyrid-3-yl;
a compound of Formula (60) wherein R³ is N(CH₂CH₂OMe)Et, Ar is 4,6-dimethylpyrid-3-yl;
a compound of Formula (60) wherein R³ is N(CH₂CH₂OMe)Pr, Ar is 4,6-dimethylpyrid-3-yl;
a compound of Formula (60) wherein R³ is N(CH₂CH₂OMe)-CH₂cPr, Ar is 4,6-dimethylpyrid-3-yl;
a compound of Formula (60) wherein R³ is NHCH(CH₃)CH₂CH₃, Ar is 4,6-dimethylpyrid-3-yl;
a compound of Formula (60) wherein R³ is NHCH(cPr)₂, Ar is 4,6-dimethylpyrid-3-yl;
a compound of Formula (60) wherein R³ is N(CH₂CH₂OMe)₂, Ar is 4,6-dimethylpyrid-3-yl;
a compound of Formula (60) wherein R³ is NHCH(Et)₂, Ar is 4,6-dimethylpyrid-3-yl;
a compound of Formula (60) wherein R³ is N(Et)₂, Ar is 4,6-dimethylpyrid-3-yl;
a compound of Formula (60) wherein R³ is 2-ethylpiperid-1-yl, Ar is 2,6-dimethylpyrid-3-yl;
a compound of Formula (60) wherein R³ is cyclobutylamino, Ar is 2,6-dimethylpyrid-3-yl;
a compound of Formula (60) wherein R³ is N(Me)CH₂CH=CH₂, Ar is 2,6-dimethylpyrid-3-yl;
a compound of Formula (60) wherein R³ is N(Et)CH₂cPr, Ar is 2,6-dimethylpyrid-3-yl;
a compound of Formula (60) wherein R³ is N(Pr)CH₂cPr, Ar is 2,6-dimethylpyrid-3-yl;
a compound of Formula (60) wherein R³ is N(Me)Pr, Ar is 2,6-dimethylpyrid-3-yl;
a compound of Formula (60) wherein R³ is N(Me)Et, Ar is 2,6-dimethylpyrid-3-yl;
a compound of Formula (60) wherein R³ is N(Me)Bu, Ar is 2,6-dimethylpyrid-3-yl;
a compound of Formula (60) wherein R³ is N(Me)propargyl, Ar is 2,6-dimethylpyrid-3-yl;
a compound of Formula (60) wherein R³ is NHCH(CH₃)CH(CH₃)CH₃, Ar is 2,6-dimethylpyrid-3-yl;
a compound of Formula (60) wherein R³ is N(CH₂CH₂OMe)-CH₂CH=CH₂, Ar is 2,6-dimethylpyrid-3-yl;
a compound of Formula (60) wherein R³ is N(CH₂CH₂OMe)Me, Ar is 2,6-dimethylpyrid-3-yl;
a compound of Formula (60) wherein R³ is N(CH₂CH₂OMe)Et, Ar is 2,6-dimethylpyrid-3-yl;
a compound of Formula (60) wherein R³ is N(CH₂CH₂OMe)Pr, Ar is 2, 6-dimethylpyrid-3-yl;
a compound of Formula (60) wherein R³ is N(CH₂CH₂OMe)-CH₂cPr, Ar is 2,6-dimethylpyrid-3-yl;
a compound of Formula (60) wherein R³ is NHCH(CH₃)CH₂CH₃, Ar is 2, 6-dimethyl pyrid-3-yl; and
a compound of Formula (60) wherein R³ is NHCH(cPr)₂, Ar is 2,6-dimethylpyrid-3-yl.

3. A compound according to claim 1 and geometric isomers thereof, stereoisomeric forms thereof, or mixtures of stereoisomeric forms thereof, and pharmaceutically acceptable salt forms thereof, wherein said compound is selected from the group:
4-((2-butyl)amino)-2,7-dimethyl-8-(2-methyl-4-methoxyphenyl)-[1,5-a]-pyrazolo-1,3,5-triazine;
4-((2-butyl)amino)-2,7-dimethyl-8-(2,5-dimethyl-4-methoxyphenyl)-[1,5-a]-pyrazolo-1,3,5-triazine;
4-((3-pentyl)amino)-2,7-dimethyl-8-(2-methyl-4-methoxyphenyl)-[1,5-a]-pyrazolo-1,3,5-triazine;
4-(N-cyclopropylmethyl-N-propylamino)-2,7-dimethyl-8- (2-methyl-4-methoxyphenyl)-[1,5-a]-pyrazolo-1,3,5-triazine;
4-(N-cyclopropylmethyl-N-propylamino)-2,7-dimethyl-8-(2,5-dimethyl-4-methoxyphenyl)-[1,5-a]-pyrazolo-1,3,5-triazine;
4-(N-allyl-N-(2-methoxyethyl)amino)-2,7-dimethyl-8-(2-methyl-4-methoxyphenyl)-[1,5-a]-pyrazolo-1,3,5-triazine;
4-(N-allyl-N-(2-methoxyethyl)amino)-2,7-dimethyl-8-(2,5-dimethyl-4-methoxyphenyl)-[1,5-a]-pyrazolo-1,3,5-triazine;
4-(N-ethyl-N-(2-methoxyethyl)amino)-2,7-dimethyl-8-(2-methyl-4-methoxyphenyl)-[1,5-a]-pyrazolo-1,3,5-triazine; and
4-( N-ethyl-N-(2-methoxyethyl)amino)-2,7-dimethyl-8-(2,5-dimethyl-4-methoxyphenyl)-[1,5-a]-pyrazolo-1,3,5-triazine.

4. A pharmaceutical composition comprising a pharmaceutically acceptable carrier and a therapeutically effective amount of a compound of any one of claims 1, 2 and 3.

5. Use of a compound according to claim 1, 2 or 3 in the manufacture of a medicament for treating affective disorder, anxiety,

6. A compound according to claim 1 wherein R³ is NHCH(Et)₂ , R^{4a} is CL , R^{4b} is H, R^{4c} is OMe, R^{4d} is F and R^{4e} is H,

7. A pharmaceutical composition according to claim 4 wherein the compound is a compound of formula (50) wherein R³ is NHCH(Et)₂ , R^{4a} is CL , R^{4b} is H, R^{4c} is OMe, R^{4d} is F and R^{4e} is H.

8. Use of a compound according to claim 5 wherein the compound is a compound of formula (50) wherein R³ is NHCH(Et)₂ , R^{4a} is CL , R^{4b} is H, R^{4c} is OMe, R^{4d} is F and R^{4e} is H.

## Patentansprüche

1. Verbindung der Formel (50) 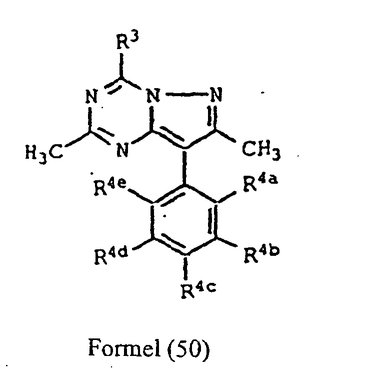 und geometrische Isomere davon, stereoisomere Formen davon, oder Gemische stereoisomerer Formen davon, und pharmazeutisch verträgliche Salzformen davon, ausgewählt aus:
einer Verbindung der Formel (50), wobei R³ - NHCH(CH₂CH₂OMe)(CH₂OMe) darstellt, R^{4a} Me ist, R^{4b} H ist, R^{4c} Me ist, R^{4d} H ist und R^{4e} Me ist;
einer Verbindung der Formel (50), wobei R³ 2-Ethylpiperid-1-yl darstellt, R^{4a} Me ist, R^{4b} H ist, R^{4c} OMe ist; R^{4d} H ist und R^{4e} H ist;
einer Verbindung der Formel (50), wobei R³ Cyclobutylamino darstellt, R^{4a} Me ist, R^{4b} H ist, R^{4c} OMe ist, R^{4d} H ist und R^{4e} H ist;
einer Verbindung der Formel (50), wobei R³ N(Me)CH₂CH=CH₂ darstellt, R^{4a} Me ist, R^{4b} H ist, R^{4c} OMe ist, R^{4d} H ist und R^{4e} H ist;
einer Verbindung der Formel (50), wobei R³ N(Et)CH₂CH=CH₂ darstellt, R^{4a} Me ist, R^{4b} H ist, R^{4c} OMe ist, R^{4d} H ist und R^{4e} H ist;
einer Verbindung der Formel (50), wobei R³ N(Me)CH₂cPr darstellt, R^{4a} Me ist, R^{4b} H ist, R^{4c} OMe ist, R^{4d} H ist und R^{4e} H ist;
einer Verbindung der Formel (50), wobei R³ N(Et)CH₂cPr darstellt, R^{4a} Me ist, R^{4b} H ist, R^{4c} OMe ist, R^{4d} H ist und R^{4e} H ist;
einer Verbindung der Formel (50), wobei R³ N(Pr)CH₂cPr darstellt, R^{4a} Me ist, R^{4b} H ist, R^{4c} OMe ist, R^{4d} H ist und R^{4e} H ist;
einer Verbindung der Formel (50), wobei R³ N(Me)Pr darstellt, R^{4a} Me ist, R^{4b} H ist, R^{4c} OMe ist, R^{4d} H ist und R^{4e} H ist;
einer Verbindung der Formel (50), wobei R³ N(Me)Et darstellt, R^{4a} Me ist, R^{4b} H ist, R^{4c} OMe ist, R^{4d} H ist und R^{4e} H ist;
einer Verbindung der Formel (50), wobei R³ N(Me)Bu darstellt, R^{4a} Me ist, R^{4b} H ist, R^{4c} OMe ist, R^{4d} H ist und R^{4e} H ist;
einer Verbindung der Formel (50), wobei R³ N(Me)propargyl darstellt, R^{4a} Me ist, R^{4b} H ist, R^{4c} OMe ist, R^{4d} H ist und R^{4e} H ist;
einer Verbindung der Formel (50), wobei R³ N(Et)propargyl darstellt, R^{4a} Me ist, R^{4b} H ist, R^{4c} OMe ist, R^{4d} H ist und R^{4e} H ist;
einer Verbindung der Formel (50), wobei R³ NHCH(CH₃)CH(CH₃)CH₃ darstellt, R^{4a} Me ist, R^{4b} H ist, R^{4c} OMe ist, R^{4d} H ist und R^{4e} H ist;
einer Verbindung der Formel (50), wobei R³ N(CH₂CH₂OMe)CH₂CH=CH₂ darstellt, R^{4a} Me ist, R^{4b} H ist, R^{4c} OMe ist, R^{4d} H ist und R^{4e} H ist;
einer Verbindung der Formel (50), wobei R³ N(CH₂CH₂OMe)Me darstellt, R^{4a} Me ist, R^{4b} H ist, R^{4c} OMe ist, R^{4d} H ist und R^{4e} H ist;
einer Verbindung der Formel (50), wobei R³ N(CH₂CH₂OMe)Et darstellt, R^{4a} Me ist, R^{4b} H ist, R^{4c} OMe ist, R^{4d} H ist und R^{4e} H ist;
einer Verbindung der Formel (50), wobei R³ N(CH₂CH₂OMe)Pr darstellt, R^{4a} Me ist, R^{4b} H ist, R^{4c} OMe ist, R^{4d} H ist und R^{4e} H ist;
einer Verbindung der Formel (50), wobei R³ N(CH₂CH₂OMe)CH₂cPr darstellt, R^{4a} Me ist, R^{4b} H ist, R^{4c} OMe ist, R^{4d} H ist und R^{4e} H ist;
einer Verbindung der Formel (50), wobei R³ NHCH(CH₃)CH₂CH₃ darstellt, R^{4a} Me ist, R^{4b} H ist, R^{4c} OMe ist, R^{4d} H ist und R^{4e} H ist;
einer Verbindung der Formel (50), wobei R³ NHCH(cPr)₂ darstellt, R^{4a} Me ist, R^{4b} H ist, R^{4c} OMe ist, R^{4d} H ist und R^{4e} H ist;
einer Verbindung der Formel (50), wobei R³ 2-Ethylpiperid-1-yl darstellt, R^{4a} Me ist, R^{4b} H ist, R^{4c} OMe ist, R^{4d} Me ist und R^{4e} H ist;
einer Verbindung der Formel (50), wobei R³ Cyclobutylamino darstellt, R^{4a} Me ist, R^{4b} H ist, R^{4c} OMe ist, R^{4d} Me ist und R^{4e} H ist;
einer Verbindung der Formel (50), wobei R³ N(Me)CH₂CH=CH₂ darstellt, R^{4a} Me ist, R^{4b} H ist, R^{4c} OMe ist, R^{4d} Me ist und R^{4e} H ist;
einer Verbindung der Formel (50), wobei R³ N(Et)CH₂CH=CH₂ darstellt, R^{4a} Me ist, R^{4b} H ist, R^{4c} OMe ist, R^{4d} Me ist und R^{4e} H ist;
einer Verbindung der Formel (50), wobei R³ N(Me)CH₂cPr darstellt, R^{4a} Me ist, R^{4b} H ist, R^{4c} OMe ist, R^{4d} Me ist und R^{4c} H ist;
einer Verbindung der Formel (50), wobei R³ N(Et)CH₂cPr darstellt, R^{4a} Me ist, R^{4b} H ist, R^{4c} OMe ist, R^{4d} Me ist und R^{4e} H ist;
einer Verbindung der Formel (50), wobei R³ N(Pr)CH₂cPr darstellt, R^{4a} Me ist, R^{4b} H ist, R^{4c} OMe ist, R^{4d} Me ist und R^{4e} H ist;
einer Verbindung der Formel (50), wobei R³ N(Me)Pr darstellt, R^{4a} Me ist, R^{4b} H ist, R^{4c} OMe ist, R^{4d} Me ist und R^{4e} H ist;
einer Verbindung der Formel (50), wobei R³ N(Me)Et darstellt, R^{4a} Me ist, R^{4b} H ist, R^{4c} OMe ist, R^{4d} Me ist und R^{4e} H ist;
einer Verbindung der Formel (50), wobei R³ N(Me)Bu darstellt, R^{4a} Me ist, R^{4b} H ist, R^{4c} OMe ist, R^{4d} Me ist und R^{4e} H ist;
einer Verbindung der Formel (50), wobei R³ N(Me)propargyl darstellt, R^{4a} Me ist, R^{4b} H ist, R^{4c} OMe ist, R^{4d} Me ist und R^{4e} H ist;
einer Verbindung der Formel (50), wobei R³ N(Et)propargyl darstellt, R^{4a} Me ist, R^{4b} H ist, R^{4c} OMe ist, R^{4d} Me ist und R^{4e} H ist;
einer Verbindung der Formel (50), wobei R³ NHCH(CH₃)CH(CH₃)CH₃ darstellt, R^{4a} Me ist, R^{4b} H ist, R^{4c} OMe ist, R^{4d} Me ist und R^{4e} H ist;
einer Verbindung der Formel (50), wobei R³ N(CH₂CH₂OMe)CH₂CH=CH₂ darstellt, R^{4a} Me ist, R^{4b} H ist, R^{4c} OMe ist, R^{4d} Me ist und R^{4e} H ist;
einer Verbindung der Formel (50), wobei R³ N(CH₂CH₂OMe)Me darstellt, R^{4a} Me ist, R^{4b} H ist, R^{4c} OMe ist, R^{4d} Me ist und R^{4e} H ist;
einer Verbindung der Formel (50), wobei R³ N(CH₂CH₂OMe)Et darstellt, R^{4a} Me ist, R^{4b} H ist, R^{4c} OMe ist, R^{4d} Me ist und R^{4e} H ist;
einer Verbindung der Formel (50), wobei R³ N(CH₂CH₂OMe)Pr darstellt, R^{4a} Me ist, R^{4b} H ist, R^{4c} OMe ist, R^{4d} Me ist und R^{4e} H ist;
einer Verbindung der Formel (50), wobei R³ N(CH₂CH₂OMe)CH₂cPr darstellt, R^{4a} Me ist, R^{4b} H ist, R^{4c} OMe ist, R^{4d} Me ist und R^{4e} H ist;
einer Verbindung der Formel (50), wobei R³ NHCH(CH₃)CH₂CH₃ darstellt, R^{4a} Me ist, R^{4b} H ist, R^{4c} OMe ist, R^{4d} Me ist und R^{4e} H ist;
einer Verbindung der Formel (50), wobei R³ NHCH(cPr)₂ darstellt, R^{4a} Me ist, R^{4b} H ist, R^{4c} OMe ist, R^{4d} Me ist und R^{4e} H ist;
einer Verbindung der Formel (50), wobei R³ -NHCH(Et)₂ darstellt, R^{4a} OMe ist, R^{4b} H ist, R^{4c} OMe ist, R^{4d} H ist und R^{4e} H ist;
einer Verbindung der Formel (50), wobei R³ 2-Ethylpiperid-1-yl darstellt, R^{4a} OMe ist, R^{4b} H ist, R^{4c} OMe ist, R^{4d} H ist und R^{4e} H ist;
einer Verbindung der Formel (50), wobei R³ Cyclobutylamino darstellt, R^{4a} OMe ist, R^{4b} H ist, R^{4c} OMe ist, R^{4d} H ist und R^{4e} H ist;
einer Verbindung der Formel (50), wobei R³ N(Me)CH₂CH=CH₂ darstellt, R^{4a} OMe ist, R^{4b} H ist, R^{4c} OMe ist, R^{4d} H ist und R^{4e} H ist;
einer Verbindung der Formel (50), wobei R³ N(Et)CH₂CH=CH₂ darstellt, R^{4a} OMe ist, R^{4b} H ist, R^{4c} OMe ist, R^{4d} H ist und R^{4e} H ist;
einer Verbindung der Formel (50), wobei R³ N(Me)CH₂cPr darstellt, R^{4a} OMe ist, R^{4b} H ist, R^{4c} OMe ist, R^{4d} H ist und R^{4e} H ist;
einer Verbindung der Formel (50), wobei R³ N(Et)CH₂cPr darstellt, R^{4a} OMe ist, R^{4b} H ist, R^{4c} OMe ist, R^{4d} H ist und R^{4e} H ist;
einer Verbindung der Formel (50), wobei R³ N(Pr)CH₂cPr darstellt, R^{4a} OMe ist, R^{4b} H ist, R^{4c} OMe ist, R^{4d} H ist und R^{4e} H ist;
einer Verbindung der Formel (50), wobei R³ N(Me)Pr darstellt, R^{4a} Ome ist, R^{4b} H ist, R^{4c} OMe ist, R^{4d} H ist und R^{4e} H ist;
einer Verbindung der Formel (50), wobei R³ N(Me)Et darstellt, R^{4a} OMe ist, R^{4b} H ist, R^{4c} OMe ist, R^{4d} H ist und R^{4e} H ist;
einer Verbindung der Formel (50), wobei R³ N(Me)Bu darstellt, R^{4a} OMe ist, R^{4b} H ist, R^{4c} OMe ist, R^{4d} H ist und R^{4e} H ist;
einer Verbindung der Formel (50), wobei R³ N(Me)propargyl darstellt, R^{4a} OMe ist, R^{4b} H ist, R^{4c} OMe ist, R^{4d} H ist und R^{4e} H ist;
einer Verbindung der Formel (50), wobei R³ N(Et)propargyl darstellt, R^{4a} OMe ist, R^{4b} H ist, R^{4c} OMe ist, R^{4d} H ist und R^{4e} H ist;
einer Verbindung der Formel (50), wobei R³ NHCH(CH₃)CH(CH₃)CH₃ darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe)CH₂CH=CH₂ darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe) Me darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe)Et darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe)Pr darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe)CH₂cPr darstellt, R^{4a} OMe ist, R^{4b} H ist, R^{4c} OMe ist, R^{4d} H ist und R^{4e} H ist;
einer Verbindung der Formel (50), wobei R³ NHCH(CH₃)CH₂CH₃ darstellt, R^{4a} OMe ist, R^{4b} H ist, R^{4c} OMe ist, R^{4d} H ist und R^{4e} H ist;
einer Verbindung der Formel (50), wobei R³ NHCH(cPr)₂ darstellt, R^{4a} OMe ist, R^{4b} H ist, R^{4c} OMe ist, R^{4d} H ist und R^{4e} H ist;
einer Verbindung der Formel (50), wobei R³ N(CH₂CH₂OMe)₂ darstellt, R^{4a} OMe ist, R^{4b} H ist, R^{4c} OMe ist, R^{4d} H ist und R^{4e} H ist;
einer Verbindung der Formel (50), wobei R³ NHCH(Et)2 darstellt, R^{4a} OMe ist, R^{4b} H ist, R^{4c} OMe ist, R^{4d} Me ist und R^{4e} H ist;
einer Verbindung der Formel (50), wobei R³ N(Et)₂ darstellt, R^{4a} OMe ist, R^{4b} H ist, R^{4c} OMe ist, R^{4d} Me ist und R^{4e} H ist;
einer Verbindung der Formel (50), wobei R³ 2-Ethylpiperid-1-yl darstellt, R^{4a} OMe ist, R^{4b} H ist, R^{4c} OMe ist, R^{4d} Me ist und R^{4e} H ist;
einer Verbindung der Formel (50), wobei R³ Cyclobutylamino darstellt, R^{4a} OMe ist, R^{4b} H ist, R^{4c} OMe ist, R^{4d} Me ist und R^{4e} H ist;
einer Verbindung der Formel (50), wobei R³ N(Me)CH₂CH=CH₂ darstellt, R^{4a} OMe ist, R^{4b} H ist, R^{4c} OMe ist, R^{4d} Me ist und R^{4e} H ist;
einer Verbindung der Formel (50), wobei R³ N(Et)CH₂CH=CH₂ darstellt, R^{4a} OMe ist, R^{4b} H ist, R^{4c} OMe ist, R^{4d} Me ist und R^{4e} H ist;
einer Verbindung der Formel (50), wobei R³ N(Me)CH₂cPr darstellt, R^{4a} OMe ist, R^{4b} H ist, R^{4c} OMe ist, R^{4d} Me ist und R^{4e} H ist;
einer Verbindung der Formel (50), wobei R³ N(Et)CH₂cPr darstellt, R^{4a} OMe ist, R^{4b} H ist, R^{4c} OMe ist, R^{4d} Me ist und R^{4e} H ist;
einer Verbindung der Formel (50), wobei R³ N(Pr)CH₂cPr darstellt, R^{4a} OMe ist, R^{4b} H ist, R^{4c} OMe ist, R^{4d} Me ist und R^{4e} H ist;
einer Verbindung der Formel (50), wobei R³ N(Me)Pr darstellt, R^{4a} OMe ist, R^{4b} H ist, R^{4c} OMe ist, R^{4d} Me ist und R^{4e} H ist;
einer Verbindung der Formel (50), wobei R³ N(Me)Et darstellt, R^{4a} OMe ist, R^{4b} H ist, R^{4c} OMe ist, R^{4d} Me ist und R^{4e} H ist;
einer Verbindung der Formel (50), wobei R³ N(Me)Bu darstellt, R^{4a} OMe ist, R^{4b} H ist, R^{4c} OMe ist, R^{4d} Me ist und R^{4e} H ist;
einer Verbindung der Formel (50), wobei R³ N(Me)propargyl darstellt, R^{4a} OMe ist, R^{4b} H ist, R^{4c} OMe ist, R^{4d} Me ist und R^{4e} H ist;
einer Verbindung der Formel (50), wobei R³ N(Et)propargyl darstellt, R^{4a} OMe ist, R^{4b} H ist, R^{4c} OMe ist, R^{4d} Me ist und R^{4e} H ist;
einer Verbindung der Formel (50), wobei R³ NHCH(CH₃)CH(CH₃)CH₃ darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)CH₂CH=CH₂ darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)Me darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)Et darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)Pr darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)CH₂cPr darstellt, R^{4a} OMe ist, R^{4b} H ist, R^{4c} OMe ist, R^{4d} Me ist und R^{4e} H ist;
einer Verbindung der Formel (50), wobei R³ NHCH(CH₃)CH₂CH₃ darstellt, R^{4a} OMe ist, R^{4b} H ist, R^{4c} OMe ist, R^{4d} Me ist und R^{4e} H ist;
einer Verbindung der Formel (50), wobei R³ NHCH(cPr)₂ darstellt, R^{4a} OMe ist, R^{4b} H ist, R^{4c} OMe ist, R^{4d} Me ist und R^{4e} H ist;
einer Verbindung der Formel (50), wobei R³ N(CH₂CH₂OMe)₂ darstellt, R^{4a} OMe ist, R^{4b} H ist, R^{4c} OMe ist, R^{4d} Me ist und R^{4e} H ist;
einer Verbindung der Formel (50), wobei R³ NHCH(Et)₂ darstellt, R^{4a} OMe ist, R^{4b} H ist, R^{4c} OMe ist, R^{4d} Me ist und R^{4e} H ist;
einer Verbindung der Formel (50), wobei R³ N(Et)₂ darstellt, R^{4a} OMe ist, R^{4b} H ist, R^{4c} OMe ist, R^{4d} Me ist und R^{4e} H ist;
einer Verbindung der Formel (50), wobei R³ 2-Ethylpiperid-1-yl darstellt, R^{4a} Me ist, R^{4b} H ist, R^{4c} OMe ist, R^{4d} H ist und R^{4e} Me ist;
einer Verbindung der Formel (50), wobei R³ Cyclobutylamino darstellt, R^{4a} Me ist, R^{4b} H ist, R^{4c} OMe ist, R^{4d} H ist und R^{4e} Me ist;
einer Verbindung der Formel (50), wobei R³ N(Me)CH₂CH=CH₂ darstellt, R^{4a} Me ist, R^{4b} H ist, R^{4c} OMe ist, R^{4d} H ist und R^{4e} Me ist;
einer Verbindung der Formel (50), wobei R³ N(Et)CH₂CH=CH₂ darstellt, R^{4a} Me ist, R^{4b} H ist, R^{4c} OMe ist, R^{4d} H ist und R^{4e} Me ist;
einer Verbindung der Formel (50), wobei R³ N(Me)CH₂cPr darstellt, R^{4a} Me ist, R^{4b} H ist, R^{4c} OMe ist, R^{4d} H ist und R^{4e} Me ist;
einer Verbindung der Formel (50), wobei R³ N(Et)CH₂cPr darstellt, R^{4a} Me ist, R^{4b} H ist, R^{4c} OMe ist, R^{4d} H ist und R^{4e} Me ist;
einer Verbindung der Formel (50), wobei R³ N(Pr)CH₂cPr darstellt, R^{4a} Me ist, R^{4b} H ist, R^{4c} OMe ist, R^{4d} H ist und R^{4e} Me ist;
einer Verbindung der Formel (50), wobei R³ N(Me)Pr darstellt, R^{4a} Me ist, R^{4b} H ist, R^{4c} OMe ist, R^{4d} H ist und R^{4e} Me ist;
einer Verbindung der Formel (50), wobei R³ N(Me)Et darstellt, R^{4a} Me ist, R^{4b} H ist, R^{4c} OMe ist, R^{4d} H ist und R^{4e} Me ist;
einer Verbindung der Formel (50), wobei R³ N(Me)Bu darstellt, R^{4a} Me ist, R^{4b} H ist, R^{4c} OMe ist, R^{4d} H ist und R^{4e} Me ist;
einer Verbindung der Formel (50), wobei R³ N(Me)propargyl darstellt, R^{4a} Me ist, R^{4b} H ist, R^{4c} OMe ist, R^{4d} H ist und R^{4e} Me ist;
einer Verbindung der Formel (50), wobei R³ N(Et)propargyl darstellt, R^{4a} Me ist, R^{4b} H ist, R^{4c} OMe ist, R^{4a} H ist und R^{4e} Me ist;
einer Verbindung der Formel (50), wobei R³ NHCH(CH₃)CH(CH₃)CH₃ darstellt, R^{4a} Me ist, R^{4b} H ist, R^{4c} OMe ist, R^{4d} H ist und R^{4e} Me ist;
einer Verbindung der Formel (50), wobei R³ N(CH₂CH₂OMe)CH₂CH=CH₂ darstellt, R^{4a} Me ist, R^{4b} H ist, R^{4c} OMe ist, R^{4d} H ist und R^{4e} Me ist;
einer Verbindung der Formel (50), wobei R³ N(CH₂CH₂OMe)Me darstellt, R^{4a} Me ist, R^{4b} H ist, R^{4c} OMe ist, R^{4d} H ist und R^{4e} Me ist;
einer Verbindung der Formel (50), wobei R³ N(CH₂CH₂OMe)Et, R^{4a} Me ist, R^{4b} H ist, R^{4c} OMe ist, R^{4d} H ist und R^{4e} Me ist;
einer Verbindung der Formel (50), wobei R³ N(CH₂CH₂OMe)Pr darstellt, R^{4a} Me ist, R^{4b} H ist, R^{4c} OMe ist, R^{4d} H ist und R^{4e} Me ist;
einer Verbindung der Formel (50), wobei R³ N(CH₂CH₂OMe)CH₂cPr darstellt, R^{4a} Me ist, R^{4b} H ist, R^{4c} OMe ist, R^{4d} H ist und R^{4e} Me ist;
einer Verbindung der Formel (50), wobei R³ NHCH(CH₃)CH₂CH₃ darstellt, R^{4a} Me ist, R^{4b} H ist, R^{4c} OMe ist, R^{4d} H ist und R^{4e} Me ist;
einer Verbindung der Formel (50), wobei R³ NHCH(Et)₂ darstellt, R^{4a} Me ist, R^{4b} H ist, R^{4c} OMe ist, R^{4d} H ist und R^{4e} Me ist;
einer Verbindung der Formel (50), wobei R³ NHCH(cPr)₂ darstellt, R^{4a} Me ist, R^{4b} H ist, R^{4c} OMe ist, R^{4d} H ist und R^{4e} Me ist;
einer Verbindung der Formel (50), wobei R³ NHCH(Et)₂ darstellt, R^{4a} Me ist, R^{4b} H ist, R^{4c} OMe ist, R^{4d} H ist und R^{4e} OMe ist;
einer Verbindung der Formel (50), wobei R³ 2-Ethylpiperid-1-yl darstellt, R^{4a} Me ist, R^{4b} H ist, R^{4c} OMe ist, R^{4d} H ist und R^{4e} OMe ist;
einer Verbindung der Formel (50), wobei R³ Cyclobutylamino darstellt, R^{4a} Me ist, R^{4b} H ist, R^{4c} OMe ist, R^{4d} H ist und R^{4e} OMe ist;
einer Verbindung der Formel (50), wobei R³ N(Me)CH₂CH=CH₂ darstellt, R^{4a} Me ist, R^{4b} H ist, R^{4c} OMe ist, R^{4d} H ist und R^{4e} OMe ist;
einer Verbindung der Formel (50), wobei R³ N(Et)CH₂CH=CH₂ darstellt, R^{4a} Me ist, R^{4b} H ist, R^{4c} OMe ist, R^{4d} H ist und R^{4e} OMe ist;
einer Verbindung der Formel (50), wobei R³ N(Me)CH₂cPr darstellt, R^{4a} Me ist, R^{4b} H ist, R^{4c} OMe ist, R^{4d} H ist und R^{4e} OMe ist;
einer Verbindung der Formel (50), wobei R³ N(Et)CH₂cPr darstellt, R^{4a} Me ist, R^{4b} H ist, R^{4c} OMe ist, R^{4d} H ist und R^{4e} OMe ist;
einer Verbindung der Formel (50), wobei R³ N(Pr)CH₂cPr darstellt, R^{4a} Me ist, R^{4b} H ist, R^{4c} OMe ist, R^{4d} H ist und R^{4e} OMe ist;
einer Verbindung der Formel (50), wobei R³ N(Me)Pr darstellt, R^{4a} Me ist, R^{4b} H ist, R^{4c} OMe ist, R^{4d} H ist und R^{4e} OMe ist;
einer Verbindung der Formel (50), wobei R³ N(Me)Et darstellt, R^{4a} Me ist, R^{4b} H ist, R^{4c} OMe ist, R^{4d} H ist und R^{4e} OMe ist;
einer Verbindung der Formel (50), wobei R³ N(Me)Bu darstellt, R^{4a} Me ist, R^{4b} H ist, R^{4c} OMe ist, R^{4d} H ist und R^{4e} OMe ist;
einer Verbindung der Formel (50), wobei R³ N(Me)propargyl darstellt, R^{4a} Me ist, R^{4b} H ist, R^{4c} OMe ist, R^{4d} H ist und R^{4e} OMe ist;
einer Verbindung der Formel (50), wobei R³ N(Et)propargyl darstellt, R^{4a} Me ist, R^{4b} H ist, R^{4c} OMe ist, R^{4d} H ist und R^{4e} OMe ist;
einer Verbindung der Formel (50), wobei R³ NHCH(CH₃)CH(CH₃)CH₃ darstellt, R^{4a} Me ist, R^{4b} H ist, R^{4c} OMe ist, R^{4d} H ist und R^{4e} OMe ist;
einer Verbindung der Formel (50), wobei R³ N(CH₂CH₂OMe)CH₂CH=CH₂ darstellt, R^{4a} Me ist, R^{4b} H ist, R^{4c} OMe ist, R^{4d} H ist und R^{4e} OMe ist;
einer Verbindung der Formel (50), wobei R³ N(CH₂CH₂OMe)Me darstellt, R^{4a} Me ist, R^{4b} H ist, R^{4c} OMe ist, R^{4d} H ist und R^{4e} OMe ist;
einer Verbindung der Formel (50), wobei R³ N(CH₂CH₂OMe)Et darstellt, R^{4a} Me ist, R^{4b} H ist, R^{4c} OMe ist, R^{4d} H ist und R^{4e} OMe ist;
einer Verbindung der Formel (50), wobei R³ N(CH₂CH₂OMe)Pr darstellt, R^{4a} Me ist, R^{4b} H ist, R^{4c} OMe ist, R^{4d} H ist und R^{4e} OMe ist;
einer Verbindung der Formel (50), wobei R³ N(CH₂CH₂OMe)CH₂cPr darstellt, R^{4a} Me ist, R^{4b} H ist, R^{4c} OMe ist, R^{4d} H ist und R^{4e} OMe ist;
einer Verbindung der Formel (50), wobei R³ NHCH(CH₃)CH₂CH₃ darstellt, R^{4a} Me ist, R^{4b} H ist, R^{4c} OMe ist, R^{4d} H ist und R^{4e} OMe ist;
einer Verbindung der Formel (50), wobei R³ NHCH(cPr)₂ darstellt, R^{4a} Me ist, R^{4b} H ist, R^{4c} OMe ist, R^{4d} H ist und R^{4e} OMe ist;
einer Verbindung der Formel (50), wobei R³ NHCH(Et)₂ darstellt, R^{4a} Cl ist, R^{4b} H ist, R^{4c} OMe ist, R^{4d} H ist und R^{4e} OMe ist;
einer Verbindung der Formel (50), wobei R³ 2-Ethylpiperid-1-yl darstellt, R^{4a} Cl ist, R^{4b} H ist, R^{4c} OMe ist, R^{4d} H ist und R^{4e} OMe ist;
einer Verbindung der Formel (50), wobei R³ Cyclobutylamino darstellt, R^{4a} Cl ist, R^{4b} H ist, R^{4c} OMe ist, R^{4d} H ist und R^{4e} OMe ist;
einer Verbindung der Formel (50), wobei R³ N(Me)CH₂CH=CH₂ darstellt, R^{4a} Cl ist, R^{4b} H ist, R^{4c} OMe ist, R^{4d} H ist und R^{4e} OMe ist;
einer Verbindung der Formel (50), wobei R³ N(Et)CH₂CH=CH₂ darstellt, R^{4a} Cl ist, R^{4b} H ist, R^{4c} OMe ist, R^{4d} H ist und R^{4e} OMe ist;
einer Verbindung der Formel (50), wobei R³ N(Me)CH₂cPr darstellt, R^{4a} Cl ist, R^{4b} H ist, R^{4c} OMe ist, R^{4d} H ist und R^{4e} OMe ist;
einer Verbindung der Formel (50), wobei R³ N(Et)CH₂cPr darstellt, R^{4a} Cl ist, R^{4b} H ist, R^{4c} OMe ist, R^{4d} H ist und R^{4e} OMe ist;
einer Verbindung der Formel (50), wobei R³ N(Pr)CH₂cPr darstellt, R^{4a} Cl ist, R^{4b} H ist, R^{4c} OMe ist, R^{4d} H ist und R^{4e} OMe ist;
einer Verbindung der Formel (50), wobei R³ N(Me)Pr darstellt, R^{4a} Cl ist, R^{4b} H ist, R^{4c} OMe ist, R^{4d} H ist und R^{4e} OMe ist;
einer Verbindung der Formel (50), wobei R³ N(Me)Et darstellt, R^{4a} Cl ist, R^{4b} H ist, R^{4c} OMe ist, R^{4d} H ist und R^{4e} OMe ist;
einer Verbindung der Formel (50), wobei R³ N(Me)Bu darstellt, R^{4a} Cl ist, R^{4b} H ist, R^{4c} OMe ist, R^{4d} H ist und R^{4e} OMe ist;
einer Verbindung der Formel (50), wobei R³ N(Me)propargyl darstellt, R^{4a} Cl ist, R^{4b} H ist, R^{4c} OMe ist, R^{4d} H ist und R^{4e} OMe ist;
einer Verbindung der Formel (50), wobei R³ N(Et)propargyl darstellt, R^{4a} Cl ist, R^{4b} H ist, R^{4c} OMe ist, R^{4d} H ist und R^{4e} OMe ist;
einer Verbindung der Formel (50), wobei R³ NHCH(CH₃)CH(CH₃)CH₃ darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)CH₂CH=CH₂ darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)Me darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)Et darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)Pr darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)CH₂cPr darstellt, R^{4a} Cl ist, R^{4b} H ist, R^{4c} OMe ist, R^{4d} H ist und R^{4e} OMe ist;
einer Verbindung der Formel (50), wobei R³ NHCH(CH₃)CH₂CH₃ darstellt, R^{4a} Cl ist, R^{4b} H ist, R^{4c} OMe ist, R^{4d} H ist und R^{4e} OMe ist;
einer Verbindung der Formel (50), wobei R³ NHCH(cPr)₂ darstellt, R^{4a} Cl ist, R^{4b} H ist, R^{4c} OMe ist, R^{4d} H ist und R^{4e} OMe ist;
einer Verbindung der Formel (50), wobei R³ N(CH₂CH₂OMe)₂ darstellt, R^{4a} Cl ist, R^{4b} H ist, R^{4c} OMe ist, R^{4d} H ist und R^{4e} OMe ist;
einer Verbindung der Formel (50), wobei R³ NHCH(Et)₂ darstellt, R^{4a} Cl ist, R^{4b} H ist, R^{4c} OMe ist, R^{4d} H ist und R^{4e} OMe ist;
einer Verbindung der Formel (50), wobei R³ N(Et)₂ darstellt, R^{4a} Cl ist, R^{4b} H ist, R^{4c} OMe ist, R^{4d} H ist und R^{4e} OMe ist;
einer Verbindung der Formel (50), wobei R³ NHCH(Et)₂ darstellt, R^{4a} Cl ist, R^{4b} H ist, R^{4c} OMe ist, R^{4d} H ist und R^{4e} H ist;
einer Verbindung der Formel (50), wobei R³ 2-Ethylpiperid-1-yl darstellt, R^{4a} Cl ist, R^{4b} H ist, R^{4c} OMe ist, R^{4d} H ist und R^{4e} H ist;
einer Verbindung der Formel (50), wobei R³ Cyclobutylamino darstellt, R^{4a} Cl ist, R^{4b} H ist, R^{4c} OMe ist, R^{4d} H ist und R^{4e} H ist;
einer Verbindung der Formel (50), wobei R³ N(Me)CH₂CH=CH₂ darstellt, R^{4a} Cl ist, R^{4b} H ist, R^{4c} OMe ist, R^{4d} H ist und R^{4e} H ist;
einer Verbindung der Formel (50), wobei R³ N(Et)CH₂CH=CH₂ darstellt, R^{4a} Cl ist, R^{4b} H ist, R^{4c} OMe ist, R^{4d} H ist und R^{4e} H ist;
einer Verbindung der Formel (50), wobei R³ N(Me)CH₂cPr darstellt, R^{4a} Cl ist, R^{4b} H ist, R^{4c} OMe ist, R^{4d} H ist und R^{4e} H ist;
einer Verbindung der Formel (50), wobei R³ N(Et)CH₂cPr darstellt, R^{4a} Cl ist, R^{4b} H ist, R^{4c} OMe ist, R^{4d} H ist und R^{4e} H ist;
einer Verbindung der Formel (50), wobei R³ N(Pr)CH₂cPr darstellt, R^{4a} Cl ist, R^{4b} H ist, R^{4c} OMe ist, R^{4d} H ist und R^{4e} H ist;
einer Verbindung der Formel (50), wobei R³ N(Me)Pr darstellt, R^{4a} Cl ist, R^{4b} H ist, R^{4c} OMe ist, R^{4d} H ist und R^{4e} H ist;
einer Verbindung der Formel (50), wobei R³ N(Me)Et darstellt, R^{4a} Cl ist, R^{4b} H ist, R^{4c} OMe ist, R^{4d} H ist und R^{4e} H ist;
einer Verbindung der Formel (50), wobei R³ N(Me)Bu darstellt, R^{4a} Cl ist, R^{4b} H ist, R^{4c} OMe ist, R^{4d} H ist und R^{4e} H ist;
einer Verbindung der Formel (50), wobei R³ N(Me)propargyl darstellt, R^{4a} Cl ist, R^{4b} H ist, R^{4c} OMe ist, R^{4d} H ist und R^{4e} H ist;
einer Verbindung der Formel (50), wobei R³ N(Et)propargyl darstellt, R^{4a} Cl ist, R^{4b} H ist, R^{4c} OMe ist, R^{4d} H ist und R^{4e} H ist;
einer Verbindung der Formel (50), wobei R³ NHCH(CH₃)CH(CH₃)CH₃ darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)CH₂CH=CH₂ darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)Me darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)Et darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)Pr darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)CH₂cPr darstellt, R^{4a} Cl ist, R^{4b} H ist, R^{4c} OMe ist, R^{4d} H ist und R^{4e} H ist;
einer Verbindung der Formel (50), wobei R³ NHCH(CH₃)CH₂CH₃ darstellt, R^{4a} Cl ist, R^{4b} H ist, R^{4c} OMe ist, R^{4d} H ist und R^{4e} H ist;
einer Verbindung der Formel (50), wobei R³ NHCH(cPr)₂ darstellt, R^{4a} Cl ist, R^{4b} H ist, R^{4c} OMe ist, R^{4d} H ist und R^{4e} H ist;
einer Verbindung der Formel (50), wobei R³ N(CH₂CH₂OMe)₂ darstellt, R^{4a} Cl ist, R^{4b} H ist, R^{4c} OMe ist, R^{4d} H ist und R^{4e} H ist;
einer Verbindung der Formel (50), wobei R³ NHCH(Et)₂ darstellt, R^{4a} Cl ist, R^{4b} H ist, R^{4c} OMe ist, R^{4d} H ist und R^{4e} H ist;
einer Verbindung der Formel (50), wobei R³ NHCH(Et)₂ darstellt, R^{4a} Cl ist, R^{4b} H ist, R^{4c} OMe ist, R^{4d} F ist und R^{4e} H ist;
einer Verbindung der Formel (50), wobei R³ 2-Ethylpiperid-1-yl darstellt, R^{4a} Cl ist, R^{4b} H ist, R^{4c} OMe ist, R^{4d} F ist und R^{4e} H ist;
einer Verbindung der Formel (50), wobei R³ Cyclobutylamino darstellt, R^{4a} Cl ist, R^{4b} H ist, R^{4c} OMe ist, R^{4d} F ist und R^{4e} H ist;
einer Verbindung der Formel (50), wobei R³ N(Me)CH₂CH=CH₂ darstellt, R^{4a} Cl ist, R^{4b} H ist, R^{4c} OMe ist, R^{4d} F ist und R^{4e} H ist;
einer Verbindung der Formel (50), wobei R³ N(Et)CH₂CH=CH₂ darstellt, R^{4a} Cl ist, R^{4b} H ist, R^{4c} OMe ist, R^{4d} F ist und R^{4e} H ist;
einer Verbindung der Formel (50), wobei R³ N(Me)CH₂cPr darstellt, R^{4a} Cl ist, R^{4b} H ist, R^{4c} OMe ist, R^{4d} F ist und R^{4e} H ist;
einer Verbindung der Formel (50), wobei R³ N(Et)CH₂cPr darstellt, R^{4a} Cl ist, R^{4b} H ist, R^{4c} OMe ist, R^{4d} F ist und R^{4e} H ist;
einer Verbindung der Formel (50), wobei R³ N(Pr)CH₂cPr darstellt, R^{4a} Cl ist, R^{4b} H ist, R^{4c} OMe ist, R^{4d} F ist und R^{4e} H ist;
einer Verbindung der Formel (50), wobei R³ N(Me)Pr darstellt, R^{4a} Cl ist, R^{4b} H ist, R^{4c} OMe ist, R^{4d} F ist und R^{4e} H ist;
einer Verbindung der Formel (50), wobei R³ N(Me)Et darstellt, R^{4a} Cl ist, R^{4b} H ist, R^{4c} OMe ist, R^{4d} F ist und R^{4e} H ist;
einer Verbindung der Formel (50), wobei R³ N(Me)Bu darstellt, R^{4a} Cl ist, R^{4b} H ist, R^{4c} OMe ist, R^{4d} F ist und R^{4e} H ist;
einer Verbindung der Formel (50), wobei R³ N(Me)propargyl darstellt, R^{4a} Cl ist, R^{4b} H ist, R^{4c} OMe ist, R^{4d} F ist und R^{4e} H ist;
einer Verbindung der Formel (50), wobei R³ NHCH(CH₃)CH(CH₃)CH₃ darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)CH₂CH=CH₂ darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)Me darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)Et darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)Pr darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)CH₂cPr darstellt, R^{4a} Cl ist, R^{4b} H ist, R^{4c} OMe ist, R^{4d} F ist und R^{4e} H ist;
einer Verbindung der Formel (50), wobei R³ NHCH(CH₃)CH₂CH₃ darstellt, R^{4a} Cl ist, R^{4b} F ist, R^{4c} OMe ist, R^{4d} H ist und R^{4e} H ist;
einer Verbindung der Formel (50), wobei R³ NHCH(cPr)₂ darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)₂ darstellt, R^{4a} Cl ist, R^{4b} H ist, R^{4c} OMe ist, R^{4d} F ist und R^{4e} H ist;
einer Verbindung der Formel (50), wobei R³ NHCH(Et)₂ darstellt, R^{4a} Cl ist, R^{4b} H ist, R^{4c} OMe ist, R^{4d} F ist und R^{4e} H ist;
einer Verbindung der Formel (50), wobei R³ N(Et)₂ darstellt, R^{4a} Cl ist, R^{4b} H ist, R^{4c} OMe ist, R^{4d} F ist und R^{4e} H ist;
einer Verbindung der Formel (50), wobei R³ NHCH(Et)₂ darstellt, R^{4a} Cl ist, R^{4b} H ist, R^{4c} OMe ist, R^{4d} OMe ist und R^{4e} H ist;
einer Verbindung der Formel (50), wobei R³ 2-Ethylpiperid-1-yl darstellt, R^{4a} Cl ist, R^{4b} H ist, R^{4c} OMe ist, R^{4d} OMe ist und R^{4e} H ist;
einer Verbindung der Formel (50), wobei R³ Cyclobutylamino darstellt, R^{4a} Cl ist, R^{4b} H ist, R^{4c} OMe ist, R^{4d} OMe ist und R^{4e} H ist;
einer Verbindung der Formel (50), wobei R³ N(Me)CH₂CH=CH₂ darstellt, R^{4a} Cl ist, R^{4b} H ist, R^{4c} OMe ist, R^{4d} OMe ist und R^{4e} H ist;
einer Verbindung der Formel (50), wobei R³ N(Et)CH₂CH=CH₂ darstellt, R^{4a} Cl ist, R^{4b} H ist, R^{4c} OMe ist, R^{4d} OMe ist und R^{4e} H ist;
einer Verbindung der Formel (50), wobei R³ N(Me)CH₂cPr darstellt, R^{4a} Cl ist, R^{4b} H ist, R^{4c} OMe ist, R^{4d} F ist und R^{4e} H ist;
einer Verbindung der Formel (50), wobei R³ N(Et)CH₂cPr darstellt, R^{4a} Cl ist, R^{4b} H ist, R^{4c} OMe ist, R^{4d} OMe ist und R^{4e} H ist;
einer Verbindung der Formel (50), wobei R³ N(Pr)CH₂cPr darstellt, R^{4a} Cl ist, R^{4b} H ist, R^{4c} OMe ist, R^{4d} OMe ist und R^{4e} H ist;
einer Verbindung der Formel (50), wobei R³ N(Me)Pr darstellt, R^{4a} Cl ist, R^{4b} H ist, R^{4c} OMe ist, R^{4d} OMe ist und R^{4e} H ist;
einer Verbindung der Formel (50), wobei R³ N(Me)Et darstellt, R^{4a} Cl ist, R^{4b} H ist, R^{4c} OMe ist, R^{4d} OMe ist und R^{4e} H ist;
einer Verbindung der Formel (50), wobei R³ N(Me)Bu darstellt, R^{4a} Cl ist, R^{4b} H ist, R^{4c} OMe ist, R^{4d} OMe ist und R^{4e} H ist;
einer Verbindung der Formel (50), wobei R³ N(Me)propargyl darstellt, R^{4a} Cl ist, R^{4b} H ist, R^{4c} OMe ist, R^{4d} OMe ist und R^{4e} H ist;
einer Verbindung der Formel (50), wobei R³ NHCH(CH₃)CH(CH₃)CH₃ darstellt, R^{4a} Cl ist, R^{4b} H ist, R^{4c} OMe ist, R^{4d} OMe ist und R^{4e} H ist;
einer Verbindung der Formel (50), wobei R³ N(CH₂CH₂OMe)CH₂CH=CH₂ darstellt, R^{4a} Cl ist, R^{4b} H ist, R^{4c} OMe ist, R^{4d} F ist und R^{4e} H ist;
einer Verbindung der Formel (50), wobei R³ N(CH₂CH₂OMe)Me darstellt, R^{4a} Cl ist, R^{4b} H ist, R^{4c} OMe ist, R^{4d} OMe ist und R^{4e} H ist;
einer Verbindung der Formel (50), wobei R³ N(CH₂CH₂OMe)Et darstellt, R^{4a} Cl ist, R^{4b} H ist, R^{4c} OMe ist, R^{4d} OMe ist und R^{4e} H ist;
einer Verbindung der Formel (50), wobei R³ N(CH₂CH₂OMe)Pr darstellt, R^{4a} Cl ist, R^{4b} H ist, R^{4c} OMe ist, R^{4d} OMe ist und R^{4e} H ist;
einer Verbindung der Formel (50), wobei R³ N(CH₂CH₂OMe)CH₂cPr darstellt, R^{4a} Cl ist, R^{4b} H ist, R^{4c} OMe ist, R^{4d} OMe ist und R^{4e} H ist;
einer Verbindung der Formel (50), wobei R³ NHCH(CH₃)CH₂CH₃ darstellt, R^{4a} Cl ist, R^{4b} H ist, R^{4c} OMe ist, R^{4d} OMe ist und R^{4e} H ist;
einer Verbindung der Formel (50), wobei R³ NHCH(cPr)₂ darstellt, R^{4a} Cl ist, R^{4b} H ist, R^{4c} OMe ist, R^{4d} OMe ist und R^{4e} H ist;
einer Verbindung der Formel (50), wobei R³ N(CH₂CH₂OMe)₂ darstellt, R^{4a} Cl ist, R^{4b} H ist, R^{4c} OMe ist, R^{4d} OMe ist und R^{4e} H ist;
einer Verbindung der Formel (50), wobei R³ NHCH(Et)₂ darstellt, R^{4a} Cl ist, R^{4b} H ist, R^{4c} OMe ist, R^{4d} OMe ist und R^{4e} H ist;
einer Verbindung der Formel (50), wobei R³ N(Et)₂ darstellt, R^{4a} Cl ist, R^{4b} H ist, R^{4c} OMe ist, R^{4d} OMe ist und R^{4e} H ist;
einer Verbindung der Formel (50), wobei R³ NHCH(Et)₂ darstellt, R^{4a} Br ist, R^{4b} H ist, R^{4c} OMe ist, R^{4d} OMe ist und R^{4e} H ist;
einer Verbindung der Formel (50), wobei R³ 2-Ethylpiperid-1-yl darstellt, R^{4a} Br ist, R^{4b} H ist, R^{4c} OMe ist, R^{4d} OMe ist und R^{4e} H ist;
einer Verbindung der Formel (50), wobei R³ Cyclobutylamino darstellt, R^{4a} Br ist, R^{4b} H ist, R^{4c} OMe ist, R^{4d} OMe ist und R^{4e} H ist;
einer Verbindung der Formel (50), wobei R³ N(Me)CH₂CH=CH₂ darstellt, R^{4a} Br ist, R^{4b} H ist, R^{4c} OMe ist, R^{4d} OMe ist und R^{4e} H ist;
einer Verbindung der Formel (50), wobei R³ N(Et)CH₂CH=CH₂ darstellt, R^{4a} Br ist, R^{4b} H ist, R^{4c} OMe ist, R^{4d} OMe ist und R^{4e} H ist;
einer Verbindung der Formel (50), wobei R³ N(Me)CH₂cPr darstellt, R^{4a} Br ist, R^{4b} H ist, R^{4c} OMe ist, R^{4d} F ist und R^{4e} H ist;
einer Verbindung der Formel (50), wobei R³ N(Et)CH₂cPr darstellt, R^{4a} Br ist, R^{4b} H ist, R^{4c} OMe ist, R^{4d} OMe ist und R^{4e} H ist;
einer Verbindung der Formel (50), wobei R³ N(Pr)CH₂cPr darstellt, R^{4a} Br ist, R^{4b} H ist, R^{4c} OMe ist, R^{4d} OMe ist und R^{4e} H ist;
einer Verbindung der Formel (50), wobei R³ N(Me)Pr darstellt, R^{4a} Br ist, R^{4b} H ist, R^{4c} OMe ist, R^{4d} OMe ist und R^{4e} H ist;
einer Verbindung der Formel (50), wobei R³ N(Me)Et darstellt, R^{4a} Br ist, R^{4b} H ist, R^{4c} OMe ist, R^{4d} OMe ist und R^{4e} H ist;
einer Verbindung der Formel (50), wobei R³ N(Me)Bu darstellt, R^{4a} Br ist, R^{4b} H ist, R^{4c} OMe ist, R^{4d} OMe ist und R^{4e} H ist;
einer Verbindung der Formel (50), wobei R³ N(Me)propargyl darstellt, R^{4a} Br ist, R^{4b} H ist, R^{4c} OMe ist, R^{4d} OMe ist und R^{4e} H ist;
einer Verbindung der Formel (50), wobei R³ NHCH(CH₃)CH(CH₃)CH₃ darstellt, R^{4a} Br ist, R^{4b} H ist, R^{4c} OMe ist, R^{4d} OMe ist und R^{4e} H ist;'
einer Verbindung der Formel (50), wobei R³ N(CH₂CH₂OMe)CH₂CH=CH₂ darstellt, R^{4a} Br ist, R^{4b} H ist, R^{4c} OMe ist, R^{4d} F ist und R^{4e} H ist;
einer Verbindung der Formel (50), wobei R³ N(CH₂CH₂OMe)Me darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)Et darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)Pr darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)CH₂cPr darstellt, R^{4a} Br ist, R^{4b} H ist, R^{4c} OMe ist, R^{4d} OMe ist und R^{4e} H ist;
einer Verbindung der Formel (50), wobei R³ NHCH(CH₃)CH₂CH₃ darstellt, R^{4a} Br ist, R^{4b} H ist, R^{4c} OMe ist, R^{4d} OMe ist und R^{4e} H ist;
einer Verbindung der Formel (50), wobei R³ NHCH(cPr)₂ darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)₂ darstellt, R^{4a} Br ist, R^{4b} H ist, R^{4c} OMe ist, R^{4d} OMe ist und R^{4e} H ist;
einer Verbindung der Formel (50), wobei R³ NHCH(Et)₂ darstellt, R^{4a} Br ist, R^{4b} H ist, R^{4c} OMe ist, R^{4d} OMe ist und R^{4e} H ist;
einer Verbindung der Formel (50), wobei R³ NHCH(Et)₂, R^{4a} Me ist, R^{4b} H ist, R^{4c} OMe ist, R^{4d} OMe ist und R^{4e} H ist;
einer Verbindung der Formel (50), wobei R³ 2-Ethylpiperid-1-yl darstellt, R^{4a} Me ist, R^{4b} H ist, R^{4c} OMe ist, R^{4d} OMe ist und R^{4e} H ist;
einer Verbindung der Formel (50), wobei R³ Cyclobutylamino darstellt, R^{4a} Me ist, R^{4b} H ist, R^{4c} OMe ist, R^{4d} OMe ist und R^{4e} H ist;
einer Verbindung der Formel (50), wobei R³ N(Me)CH₂CH=CH₂ darstellt, R^{4a} Me ist, R^{4b} H ist, R^{4c} OMe ist, R^{4d} OMe ist und R^{4e} H ist;
einer Verbindung der Formel (50), wobei R³ N(Et)CH₂CH=CH₂ darstellt, R^{4a} Me ist, R^{4b} H ist, R^{4c} OMe ist, R^{4d} OMe ist und R^{4e} H ist;
einer Verbindung der Formel (50), wobei R³ N(Me)CH₂cPr darstellt, R^{4a} Me ist, R^{4b} H ist, R^{4c} OMe ist, R^{4d} F ist und R^{4e} H ist;
einer Verbindung der Formel (50), wobei R³ N(Et)CH₂cPr darstellt, R^{4a} Me ist, R^{4b} H ist, R^{4c} OMe ist, R^{4d} OMe ist und R^{4e} H ist;
einer Verbindung der Formel (50), wobei R³ N(Pr)CH₂cPr darstellt, R^{4a} Me ist, R^{4b} H ist, R^{4c} OMe ist, R^{4d} OMe ist und R^{4e} H ist;
einer Verbindung der Formel (50), wobei R³ N(Me)Pr darstellt, R^{4a} Me ist, R^{4b} H ist, R^{4c} OMe ist, R^{4d} OMe ist und R^{4e} H ist;
einer Verbindung der Formel (50), wobei R³ N(Me)Et darstellt, R^{4a} Me ist, R^{4b} H ist, R^{4c} OMe ist, R^{4d} OMe ist und R^{4e} H ist;
einer Verbindung der Formel (50), wobei R³ N(Me)Bu darstellt, R^{4a} Me ist, R^{4b} H ist, R^{4c} OMe ist, R^{4d} OMe ist und R^{4e} H ist;
einer Verbindung der Formel (50), wobei R³ N(Me)propargyl darstellt, R^{4a} Me ist, R^{4b} H ist, R^{4c} OMe ist, R^{4d} OMe ist und R^{4e} H ist;
einer Verbindung der Formel (50), wobei R³ NHCH(CH₃)CH(CH₃)CH₃ darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)CH₂CH=CH₂ darstellt, R^{4a} Me ist, R^{4b} H ist, R^{4c} OMe ist, R^{4d} F ist und R^{4e} H ist;
einer Verbindung der Formel (50), wobei R³ N(CH₂CH₂OMe)Me darstellt, R^{4a} Me ist, R^{4b} H ist, R^{4c} OMe ist, R^{4d} OMe ist und R^{4e} H ist;
einer Verbindung der Formel (50), wobei R³ N(CH₂CH₂OMe)Et darstellt, R^{4a} Me ist, R^{4b} H ist, R^{4c} OMe ist, R^{4d} OMe ist und R^{4e} H ist;
einer Verbindung der Formel (50), wobei R³ N(CH₂CH₂OMe)Pr darstellt, R^{4a} Br ist, R^{4b} H ist, R^{4c} OMe ist, R^{4d} OMe ist und R^{4e} H ist;
einer Verbindung der Formel (50), wobei R³ N(CH₂CH₂OMe)CH₂cPr darstellt, R^{4a} Me ist, R^{4b} H ist, R^{4c} OMe ist, R^{4d} OMe ist und R^{4e} H ist;
einer Verbindung der Formel (50), wobei R³ NHCH(CH₃)CH₂CH₃ darstellt, R^{4a} Me ist, R^{4b} H ist, R^{4c} OMe ist, R^{4d} OMe ist und R^{4e} H ist;
einer Verbindung der Formel (50), wobei R³ NHCH(cPr)₂ darstellt, R^{4a} Me ist, R^{4b} H ist, R^{4c} OMe ist, R^{4d} OMe ist und R^{4e} H ist;
einer Verbindung der Formel (50), wobei R³ N(CH₂CH₂OMe)₂ darstellt, R^{4a} Me ist, R^{4b} H ist, R^{4c} OMe ist, R^{4d} OMe ist und R^{4e} H ist;
einer Verbindung der Formel (50), wobei R³ NHCH(Et)₂ darstellt, R^{4a} Me ist, R^{4b} H ist, R^{4c} OMe ist, R^{4d} OMe ist und R^{4e} H ist; und
einer Verbindung der Formel (50), wobei R³ N(Et)₂ darstellt, R^{4a} Me ist, R^{4b} H ist, R^{4c} OMe ist, R^{4d} OMe ist und R^{4e} H ist.

2. Verbindung der Formel (60) 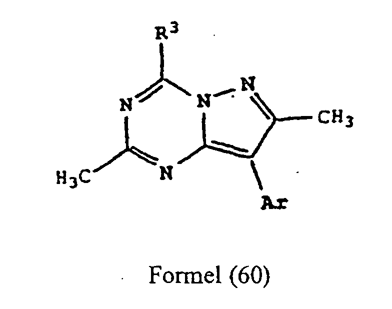 und geometrische Isomere davon, stereoisomere Formen davon, oder Gemische stereoisomerer Formen davon, und pharmazeutisch verträgliche Salzformen davon, ausgewählt aus:
einer Verbindung der Formel (60), wobei R³ NHCH(Et)₂ darstellt, Ar 6-Dimethylamino-4-methylpyrid-3-yl ist;
einer Verbindung der Formel (60), wobei R³ 2-Ethylpiperid-1-yl darstellt, Ar 6-Dimethylamino-4-methylpyrid-3-yl ist;
einer Verbindung der Formel (60), wobei R³ Cyclobutylamino darstellt, Ar 6-Dimethylamino-4-methylpyrid-3-yl ist;
einer Verbindung der Formel (60), wobei R³ N(Me)CH₂CH=CH₂ darstellt, Ar 6-Dimethylamino-4-methylpyrid-3-yl ist;
einer Verbindung der Formel (60), wobei R³ N(Et)CH₂cPr darstellt, Ar 6-Dimethylamino-4-methylpyrid-3-yl ist;
einer Verbindung der Formel (60), wobei R³ N(Pr)CH₂cPr darstellt, Ar 6-Dimethylamino-4-methylpyrid-3-yl ist;
einer Verbindung der Formel (60), wobei R³ N(Me)Pr darstellt, Ar 6-Dimethylamino-4-methylpyrid-3-yl ist;
einer Verbindung der Formel (60), wobei R³ N(Me)Et darstellt, Ar 6-Dimethylamino-4-methylpyrid-3-yl ist;
einer Verbindung der Formel (60), wobei R³ N(Me)Bu darstellt, Ar 6-Dimethylamino-4-methylpyrid-3-yl ist;
einer Verbindung der Formel (60), wobei R³ N(Me)propargyl darstellt, Ar 6-Dimethylamino-4-methylpyrid-3-yl ist;
einer Verbindung der Formel (60), wobei R³ N(Et)propargyl darstellt, Ar 6-Dimethylamino-4-methylpyrid-3-yl ist;
einer Verbindung der Formel (60), wobei R³ NHCH(CH₃)CH(CH₃)CH₃ darstellt, Ar 6-Diruethylamino-4-methylpyrid-3-yl ist;
einer Verbindung der Formel (60), wobei R³ N(CH₂CH₂OMe)CH₂CH=CH₂ darstellt, Ar 6-Dimethylamino-4-methylpyrid-3-yl ist;
einer Verbindung der Formel (60), wobei R³ N(CH₂CH₂OMe)Me darstellt, Ar 6-Dimethylamino-4-methylpyrid-3-yl ist;
einer Verbindung der Formel (60), wobei R³ N(CH₂CH₂OMe)Et darstellt, Ar 6-Dimethylamino-4-methylpyrid-3-yl ist;
einer Verbindung der Formel (60), wobei R³ N(CH₂CH₂OMe)Pr darstellt, Ar 6-Dimethylamino-4-methylpyrid-3-yl ist;
einer Verbindung der Formel (60), wobei R³ N(CH₂CH₂OMe)CH₂cPr darstellt, Ar 6-Dimethylamino-4-methylpyrid-3-yl ist;
einer Verbindung der Formel (60), wobei R³ NHCH(CH₃)CH₂CH₃ darstellt, Ar 6-Dimethylamino-4-methylpyrid-3-yl ist;
einer Verbindung der Formel (60), wobei R³ NHCH(cPr)₂ darstellt, Ar 6-Dimethylamino-4-methylpyrid-3-yl ist;
einer Verbindung der Formel (60), wobei R³ N(CH₂CH₂OMe)₂ darstellt, Ar 6-Dimethylamino-4-methylpyrid-3-yl ist;
einer Verbindung der Formel (60), wobei R³ NHCH(Et)₂ darstellt, Ar 6-Dimethylamino-4-methylpyrid-3-yl ist;
einer Verbindung der Formel (60), wobei R³ N(Et)₂ darstellt, Ar 6-Dimethylamino-4-methylpyrid-3-yl ist;
einer Verbindung der Formel (60), wobei R³ 2-Ethylpiperid-1-yl darstellt, Ar 6-Dimethylamino-4-methylpyrid-3-yl ist;
einer Verbindung der Formel (60), wobei R³ Cyclobutylamino darstellt, Ar 6-Dimethylamino-4-methylpyrid-3-yl ist;
einer Verbindung der Formel (60), wobei R³ N(Me)CH₂CH=CH₂ darstellt, Ar 6-Dimethylamino-4-methylpyrid-3-yl ist;
einer Verbindung der Formel (60), wobei R³ N(Et)CH₂cPr darstellt, Ar 6-Dimethylamino-4-methylpyrid-3-yl ist;
einer Verbindung der Formel (60), wobei R³ N(Pr)CH₂cPr darstellt, Ar 6-Dimethylamino-4-methylpyrid-3-yl ist;
einer Verbindung der Formel (60), wobei R³ N(Me)Pr darstellt, Ar 6-Dimethylamino-4-methylpyrid-3-yl ist;
einer Verbindung der Formel (60), wobei R³ N(Me)Et darstellt, Ar 6-Dimethylamino-4-methylpyrid-3-yl ist;
einer Verbindung der Formel (60), wobei R³ N(Me)Bu darstellt, Ar 6-Dimethylamino-4-methylpyrid-3-yl ist;
einer Verbindung der Formel (60), wobei R³ N(Me)propargyl darstellt, Ar 6-Dimethylamino-4-methylpyrid-3-yl ist;
einer Verbindung der Formel (60), wobei R³ NHCH(CH₃)CH(CH₃)CH₃ darstellt, Ar 6-Dimethylamino-4-methylpyrid-3-yl ist;
einer Verbindung der Formel (60), wobei R³ N(CH₂CH₂OMe)CH₂CH=CH₂ darstellt, Ar 6-Dimethylamino-4-methylpyrid-3-yl ist;
einer Verbindung der Formel (60), wobei R³ N(CH₂CH₂OMe)Me darstellt, Ar 6-Dimethylamino-4-methylpyrid-3-yl ist;
einer Verbindung der Formel (60), wobei R³ N(CH₂CH₂OMe)Et darstellt, Ar 6-Dimethylamino-4-methylpyrid-3-yl ist;
einer Verbindung der Formel (60), wobei R³ N(CH₂CH₂OMe)Pr darstellt, Ar 6-Dimethylamino-4-methylpyrid-3-yl ist;
einer Verbindung der Formel (60), wobei R³ N(CH₂CH₂OMe)CH₂cPr darstellt, Ar 6-Dimethylamino-4-methylpyrid-3-yl ist;
einer Verbindung der Formel (60), wobei R³ NHCH(CH₃)CH₂CH₃ darstellt, Ar 6-Dimethylamino-4-methylpyrid-3-yl ist;
einer Verbindung der Formel (60), wobei R³ NHCH(cPr)₂ darstellt, Ar 6-Dimethylamino-4-methylpyrid-3-yl ist;
einer Verbindung der Formel (60), wobei R³ N(CH₂CH₂OMe)₂ darstellt, Ar 6-Dimethylamino-4-methylpyrid-3-yl ist;
einer Verbindung der Formel (60), wobei R³ NHCH(Et)₂ darstellt, Ar 6-Dimethylamino-4-methylpyrid-3-yl ist;
einer Verbindung der Formel (60), wobei R³ N(Et)₂ darstellt, Ar 6-Dimethylamino-4-methylpyrid-3-yl ist;
einer Verbindung der Formel (60), wobei R³ 2-Ethylpiperid-1-yl darstellt, Ar 6-Methoxy-4-methylpyrid-3-yl ist;
einer Verbindung der Formel (60), wobei R³ Cyclobutylamino darstellt, Ar 6-Methoxy-4-methylpyrid-3-yl ist;
einer Verbindung der Formel (60), wobei R³ N(Me)CH₂CH=CH₂ darstellt, Ar 6-Methoxy-4-methylpyrid-3-yl ist;
einer Verbindung der Formel (60), wobei R³ N(Et)CH₂cPr darstellt, Ar 6-Methoxy-4-methylpyrid-3-yl ist;
einer Verbindung der Formel (60), wobei R³ N(Pr)CH₂cPr darstellt, Ar 6-Methoxy-4-methylpyrid-3-yl ist;
einer Verbindung der Formel (60), wobei R³ N(Me)Pr darstellt, Ar 6-Methoxy-4-methylpyrid-3-yl ist;
einer Verbindung der Formel (60), wobei R³ N(Me)Et darstellt, Ar 6-Methoxy-4-methylpyrid-3-yl ist;
einer Verbindung der Formel (60), wobei R³ N(Me)Bu darstellt, Ar 6-Methoxy-4-methylpyrid-3-yl ist;
einer Verbindung der Formel (60), wobei R³ N(Me)propargyl darstellt, Ar 6-Methoxy-4-methylpyrid-3-yl ist;
einer Verbindung der Formel (60), wobei R³ N(Et)propargyl darstellt, Ar 6-Methoxy-4-methylpyrid-3-yl ist;
einer Verbindung der Formel (60), wobei R³ NHCH(CH₃)CH(CH₃)CH₃ darstellt, Ar 6-Methoxy-4-methylpyrid-3-yl ist;
einer Verbindung der Formel (60), wobei R³ N(CH₂CH₂OMe)CH₂CH=CH₂ darstellt, Ar 6-Methoxy-4-methylpyrid-3-yl ist;
einer Verbindung der Formel (60), wobei R³ N(CH₂CH₂OMe)Me darstellt, Ar 6-Methoxy-4-methylpyrid-3-yl ist;
einer Verbindung der Formel (60), wobei R³ N(CH₂CH₂OMe)Et darstellt, Ar 6-Methoxy-4-methylpyrid-3-yl ist;
einer Verbindung der Formel (60), wobei R³ N(CH₂CH₂OMe)Pr darstellt, Ar 6-Methoxy-4-methylpyrid-3-yl ist;
einer Verbindung der Formel (60), wobei R³ N(CH₂CH₂OMe)CH₂cPr darstellt, Ar 6-Methoxy-4-methylpyrid-3-yl ist;
einer Verbindung der Formel (60), wobei R³ NHCH(CH₃)CH₂CH₃ darstellt, Ar 6-Methoxy-4-methylpyrid-3-yl ist;
einer Verbindung der Formel (60), wobei R³ NHCH(cPr)₂ darstellt, Ar 6-Methoxy-4-methylpyrid-3-yl ist;
einer Verbindung der Formel (60), wobei R³ N(CH₂CH₂OMe)₂ darstellt, Ar 6-Methoxy-4-methylpyrid-3-yl ist;
einer Verbindung der Formel (60), wobei R³ NHCH(Et)₂ darstellt, Ar 6-Methoxy-4-methylpyrid-3-yl ist;
einer Verbindung der Formel (60), wobei R³ N(Et)₂ darstellt, Ar 6-Methoxy-4-methylpyrid-3-yl ist;
einer Verbindung der Formel (60), wobei R³ 2-Ethylpiperid-1-yl darstellt, Ar 4-Methoxy-6-methylpyrid-3-yl ist;
einer Verbindung der Formel (60), wobei R³ Cyclobutylamino darstellt, Ar 4-Methoxy-6-methylpyrid-3-yl ist;
einer Verbindung der Formel (60), wobei R³ N(Me)CH₂CH=CH₂ darstellt, Ar 4-Methoxy-6-methylpyrid-3-yl ist;
einer Verbindung der Formel (60), wobei R³ N(Et)CH₂cPr darstellt, Ar 4-Methoxy-6-methylpyrid-3-yl ist;
einer Verbindung der Formel (60), wobei R³ N(Pr)CH₂cPr darstellt, Ar 4-Methoxy-6-methylpyrid-3-yl ist;
einer Verbindung der Formel (60), wobei R³ N(Me)Pr darstellt, Ar 4-Methoxy-6-methylpyrid-3-yl ist;
einer Verbindung der Formel (60), wobei R³ N(Me)Et darstellt, Ar 4-Methoxy-6-methylpyrid-3-yl ist;
einer Verbindung der Formel (60), wobei R³ N(Me)Bu darstellt, Ar 4-Methoxy-6-methylpyrid-3-yl ist;
einer Verbindung der Formel (60), wobei R³ N(Me)propargyl darstellt, Ar 4-Methoxy-6-methylpyrid-3-yl ist;
einer Verbindung der Formel (60), wobei R³ NHCH(CH₃)CH(CH₃)CH₃ darstellt, Ar 4-Methoxy-6-methylpyrid-3-yl ist;
einer Verbindung der Formel (60), wobei R³ N(CH₂CH₂OMe)CH₂CH=CH₂ darstellt, Ar 4-Methoxy-6-methylpyrid-3-yl ist;
einer Verbindung der Formel (60), wobei R³ N(CH₂CH₂OMe)Me darstellt, Ar 4-Methoxy-6-methylpyrid-3-yl ist;
einer Verbindung der Formel (60), wobei R³ N(CH₂CH₂OMe)Et darstellt, Ar 4-Methoxy-6-methylpyrid-3-yl ist;
einer Verbindung der Formel (60), wobei R³ N(CH₂CH₂OMe)Pr darstellt, Ar 4-Methoxy-6-methylpyrid-3-yl ist;
einer Verbindung der Formel (60), wobei R³ N(CH₂CH₂OMe)CH₂cPr darstellt, Ar 4-Methoxy-6-methylpyrid-3-yl ist;
einer Verbindung der Formel (60), wobei R³ NHCH(CH₃)CH₂CH₃ darstellt, Ar 4-Methoxy-6-methylpyrid-3-yl ist;
einer Verbindung der Formel (60), wobei R³ NHCH(cPr)₂ darstellt, Ar 4-Methoxy-6-methylpyrid-3-yl ist;
einer Verbindung der Formel (60), wobei R³ N(CH₂CH₂OMe)₂ darstellt, Ar 4-Methoxy-6-methylpyrid-3-yl ist;
einer Verbindung der Formel (60), wobei R³ NHCH(Et)₂ darstellt, Ar 6-Methoxy-4-methylpyrid-3-yl ist;
einer Verbindung der Formel (60), wobei R³ N(Et)₂ darstellt, Ar 4-Methoxy-6-methylpyrid-3-yl ist;
einer Verbindung der Formel (60), wobei R³ 2-Ethylpiperid-1-yl darstellt, Ar 4,6-Dimethylpyrid-3-yl ist;
einer Verbindung der Formel (60), wobei R³ Cyclobutylamino darstellt, Ar 4,6-Dimethylpyrid-3-yl ist;
einer Verbindung der Formel (60), wobei R³ N(Me)CH₂CH=CH₂ darstellt, Ar 4,6-Dimethylpyrid-3-yl ist;
einer Verbindung der Formel (60), wobei R³ N(Et)CH₂cPr darstellt, Ar 4,6-Dimethylpyrid-3-yl ist;
einer Verbindung der Formel (60), wobei R³ N(Pr)CH₂cPr darstellt, Ar 4,6-Dimethylpyrid-3-yl ist;
einer Verbindung der Formel (60), wobei R³ N(Me)Pr darstellt, Ar 4,6-Dimethylpyrid-3-yl ist;
einer Verbindung der Formel (60), wobei R³ N(Me)Et darstellt, Ar 4,6-Dimethylpyrid-3-yl ist;
einer Verbindung der Formel (60), wobei R³ N(Me)Bu darstellt, Ar 4,6-Dimethylpyrid-3-yl ist;
einer Verbindung der Formel (60), wobei R³ N(Me)propargyl darstellt, Ar 4,6-Dimethylpyrid-3-yl ist;
einer Verbindung der Formel (60), wobei R³ N(Et)propargyl darstellt, Ar 4,6-Dimethylpyrid-3-yl ist;
einer Verbindung der Formel (60), wobei R³ NHCH(CH₃)CH(CH₃)CH₃ darstellt, Ar 4,6-Dimethylpyrid-3-yl ist;
einer Verbindung der Formel (60), wobei R³ N(CH₂CH₂OMe)CH₂CH=CH₂ darstellt, Ar 4,6-Dimethylpyrid-3-yl ist;
einer Verbindung der Formel (60), wobei R³ N(CH₂CH₂OMe)Me darstellt, Ar 4,6-Dimethylpyrid-3-yl ist;
einer Verbindung der Formel (60), wobei R³ N(CH₂CH₂OMe)Et darstellt, Ar 4,6-Dimethylpyrid-3-yl ist;
einer Verbindung der Formel (60), wobei R³ N(CH₂CH₂OMe)Pr darstellt, Ar 4,6-Dimethylpyrid-3-yl ist;
einer Verbindung der Formel (60), wobei R³ N(CH₂CH₂OMe)H₂cPr darstellt, Ar 4,6-Dimethylpyrid-3-yl ist;
einer Verbindung der Formel (60), wobei R³ NHCH(CH₃)CH₂CH₃ darstellt, Ar 4,6-Dimethylpyrid-3-yl ist;
einer Verbindung der Formel (60), wobei R³ NHCH(cPr)₂ darstellt, Ar 4,6-Dimethylpyrid-3-yl ist;
einer Verbindung der Formel (60), wobei R³ N(CH₂CH₂OMe)₂ darstellt, Ar 4,6-Dimethylpyrid-3-yl ist;
einer Verbindung der Formel (60), wobei R³ NHCH(Et)₂ darstellt, Ar 4,6-Dimethylpyrid-3-yl ist;
einer Verbindung der Formel (60), wobei R³ N(Et)₂ darstellt, Ar 4,6-Dimethylpyrid-3-yl ist;
einer Verbindung der Formel (60), wobei R³ 2-Ethylpiperid-1-yl darstellt, Ar 2,6-Dimethylpyrid-3-yl ist;
einer Verbindung der Formel (60), wobei R³ Cyclobutylamino darstellt, Ar 2,6-Dimethylpyrid-3-yl ist;
einer Verbindung der Formel (60), wobei R³ N(Me)CH₂CH=CH₂ darstellt, Ar 2,6-Dimethylpyrid-3-yl ist;
einer Verbindung der Formel (60), wobei R³ N(Et)CH₂cPr darstellt, Ar 2,6-Dimethylpyrid-3-yl ist;
einer Verbindung der Formel (60), wobei R³ N(Pr)CH₂cPr darstellt, Ar 2,6-Dimethylpyrid-3-yl ist;
einer Verbindung der Formel (60), wobei R³ N(Me)Pr darstellt, Ar 2,6-Dimethylpyrid-3-yl ist;
einer Verbindung der Formel (60), wobei R³ N(Me)Et darstellt, Ar 2,6-Dimethylpyrid-3-yl ist;
einer Verbindung der Formel (60), wobei R³ N(Me)Bu darstellt, Ar 2,6-Dimethylpyrid-3-yl ist;
einer Verbindung der Formel (60), wobei R³ N(Me)propargyl darstellt, Ar 2,6-Dimethylpyrid-3-yl ist;
einer Verbindung der Formel (60), wobei R³ NHCH(CH₃)CH(CH₃)CH₃ darstellt, Ar 2,6-Dimethylpyrid-3-yl ist;
einer Verbindung der Formel (60), wobei R³ N(CH₂CH₂OMe)CH₂CH=CH₂ darstellt, Ar 2,6-Dimethylpyrid-3-yl ist;
einer Verbindung der Formel (60), wobei R³ N(CH₂CH₂OMe)Me darstellt, Ar 2,6-Dimethylpyrid-3-yl ist;
einer Verbindung der Formel (60), wobei R³ N(CH₂CH₂OMe)Et darstellt, Ar 2,6-Dimethylpyrid-3-yl ist;
einer Verbindung der Formel (60), wobei R³ N(CH₂CH₂OMe)Pr darstellt, Ar 2,6-Dimethylpyrid-3-yl ist;
einer Verbindung der Formel (60), wobei R³ N(CH₂CH₂OMe)CH₂cPr darstellt, Ar 2,6-Dimethylpyrid-3-yl ist;
einer Verbindung der Formel (60), wobei R³ NHCH(CH₃)CH₂CH₃ darstellt, Ar 2,6-Dimethylpyrid-3-yl ist; und
einer Verbindung der Formel (60), wobei R³ NHCH(cPr)₂ darstellt, Ar 2,6-Dimethylpyrid-3-yl ist.

3. Verbindung nach Anspruch 1 und geometrische Isomere davon, stereoisomere Formen davon oder Gemische stereoisomerer Formen davon, und pharmazeutisch verträgliche Salzformen davon, wobei die Verbindung ausgewählt ist aus:
4-((2-Butyl)amino)-2,7-dimethyl-8-(2-methyl-4-methoxyphenyl)-[1,5-a]-pyrazolo-1,3,5-triazin;
4-((2-Butyl)amino)-2,7-dimethyl-8-(2,5-dimethyl-4-methoxyphenyl)-[1,5-a]-pyrazolo-1,3,5-triazin;
4-((3-Pentyl)amino)-2,7-dimethyl-8-(2-methyl-4-methoxyphenyl)-[1,5-a]-pyrazolo-1,3,5-triazin;
4-(N-Cyclopropylmethyl-N-propylamino)-2,7-dimethyl-8-(2-methyl-4-methoxyphenyl)-[1,5-a]-pyrazolo-1,3,5-triazin;
4-(N-Cyclopropylmethyl-N-propylamino)-2,7-dimethyl-8-(2,5-dimethyl-4-methoxyphenyl)-[1,5-a]-pyrazolo-1,3,5-triazin;
4-(N-Allyl-N-(2-methoxyethyl)amino)-2,7-dimethyl-8-(2-methyl-4-methoxyphenyl)-[1,5-a]-pyrazolo-1,3,5-triazin;
4-(N-Allyl-N-(2-methoxyethyl)amino)-2,7-dimethyl-8-(2,5-dimethyl-4-methoxyphenyl)-[1,5-a]-pyrazolo-1,3,5-triazin;
4-(N-Ethyl-N-(2-methoxyethyl)amino)-2,7-dimethyl-8-(2-methyl-4-methoxyphenyl)-[1,5-a]-pyrazolo-1,3,5-triazin; und
4-(N-Ethyl-N-(2-methoxyethyl)amino)-2,7-dimethyl-8-(2,5-dimethyl-4-methoxyphenyl)-[1,5-a]-pyrazolo-1,3,5-triazin.

4. Arzneimittel, umfassend einen pharmazeutisch verträglichen Träger und eine pharmazeutisch wirksame Menge einer Verbindung nach einem der Ansprüche 1, 2 und 3.

5. Verwendung einer Verbindung nach Anspruch 1, 2 oder 3 bei der Herstellung eines Medikaments zur Behandlung von affektiver Störung, Angstzuständen, Depression, Kopfschmerzen, irritabile Darmsyndrom, posttraumatischem Streßsyndrom, supranukleärer Lähmung, Immunsuppression, Alzheimer-Krankheit, gastrointestinalen Erkrankungen, Anorexia nervosa oder anderen Eßstörungen, Drogenabhängigkeit, Symptomen in Zusammenhang mit Drogen- oder Alkoholentzug, Entzündungskrankheiten, kardiovaskulärer oder das Herz betreffenden Krankheiten, Problemen in bezug auf Fruchtbarkeit, HIV(human immunodeficiency virus)-Infektionen, hämorrhagischem Streß, Fettleibigkeit, Unfruchtbarkeit, Kopf- und Rückenmarkstraumen, Epilepsie, Schlaganfall, Geschwüren, amyotrophischer lateraler Sklerose, Hypoglykämie oder einer Erkrankung, deren Behandlung durch Antagonisierung von CRF bewirkt oder vereinfacht werden kann, einschließlich, aber nicht beschränkt von Störungen bei Säugetern, die durch CRF verursacht oder unterstützt werden.

6. Verbindung nach Anspruch 1, wobei R³ NHCH(Et)₂ darstellt, R^{4a} Cl ist, R^{4b} H ist, R^{4c} OMe ist, R^{4d} F ist und R^{4e} H ist.

7. Arzneimittel nach Anspruch 4, wobei die Verbindung eine Verbindung der Formel (50) ist, wobei R³ NHCH(Et)₂ darstellt, R^{4a} Cl ist, R^{4b} H ist, R^{4c} OMe ist, R^{4d} F ist und R^{4e} H ist.

8. Verwendung einer Verbindung nach Anspruch 5, wobei die Verbindung eine Verbindung der Formel (50) ist, wobei R³ NHCH(Et)₂ darstellt, R^{4a} Cl ist, R^{4b} H ist, R^{4c} OMe ist, R^{4d} F ist und R^{4e} H ist.

## Revendications

1. Composé de formule (50) : 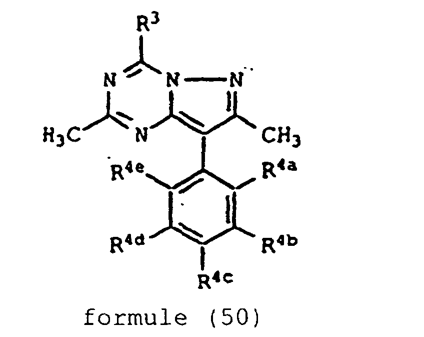 et ses isomères géométriques, ses formes stéréoisomères, ou des mélanges de ses formes stéréoisomères, et ses formes sels acceptables en pharmacie, choisis dans l'ensemble suivant :
un composé de formule (50) dans laquelle R³ est -NHCH(CH₂CH₂OMe)(CH₂OMe), R^{4a} est Me, R^{4b} est H, R^{4c} est Me, R^{4d} est H et R^{4e} est Me ;
un composé de formule (50) dans laquelle R³ est 2-éthylpipérid-1-yle, R^{4a} est Me, R^{4b} est H, R^{4c} est OMe, R^{4d} est H et R^{4e} est H ;
un composé de formule (50) dans laquelle R³ est cyclobutylamino, R^{4a} est Me, R^{4b} est H, R^{4c} est OMe, R^{4d} est H et R^{4e} est H ;
un composé de formule (50) dans lequel R³ est N(Me)CH₂CH=CH₂, R^{4a} est Me, R^{4b} est H, R^{4c} est OMe, R^{4d} est H et R^{4e} est H ;
un composé de formule (50) dans lequel R³ est N(Et)CH₂CH=CH₂, R^{4a} est Me, R^{4b} est H, R^{4c} est OMe, R^{4d} est H et R^{4e} est H ;
un composé de formule (50) dans laquelle R³ est N(Me)CH₂cPr, R^{4a} est Me, R^{4b} est H, R^{4c} est OMe, R^{4d} est H et R^{4e} est H ;
un composé de formule (50) dans laquelle R³ est N(Et)CH₂cPr, R^{4a} est Me, R^{4b} est H, R^{4c} est OMe, R^{4d} est H et R^{4e} est H ;
un composé de formule (50) dans laquelle R³ est N(Pr)CH₂cPr, R^{4a} est Me, R^{4b} est H, R^{4c} est OMe, R^{4d} est H et R^{4e} est H ;
un composé de formule (50) dans laquelle R³ est N(Me)Pr, R^{4a} est Me, R^{4b} est H, R^{4c} est OMe, R^{4d} est H et R^{4e} est H ;
un composé de formule (50) dans laquelle R³ est N(Me)Et, R^{4a} est Me, R^{4b} est H, R^{4c} est OMe, R^{4d} est H et R^{4e} est H ;
un composé de formule (50) dans laquelle R³ est N(Me)Bu, R^{4a} est Me, R^{4b} est H, R^{4c} est OMe, R^{4d} est H et R^{4e} est H ;
un composé de formule (50) dans laquelle R³ est N (Me) propargyle, R^{4a} est Me, R^{4b} est H, R^{4c} est OMe, R^{4d} est H et R^{4e} est H ;
un composé de formule (50) dans laquelle R³ est N (Et) propargyle, R^{4a} est Me, R^{4b} est H, R^{4c} est OMe, R^{4d} est H et R^{4e} est H ;
un composé de formule (50) dans laquelle R³ est NHCH(CH₃)CH(CH₃)CH₃, R^{4a} est Me, R^{4b} est H, R^{4c} est OMe, R^{4d} est H et R^{4e} est H ;
un composé de formule (50) dans laquelle R³ est N (CH₂CH₂OMe)-CH₂CH=CH₂, R^{4a} est Me, R^{4b} est H, R^{4c} est OMe, R^{4d} est H et R^{4e} est H ;
un composé de formule (50) dans laquelle R³ est N(CH₂CH₂OMe)Me, R^{4a} est Me, R^{4b} est H, R^{4c} est OMe, R^{4d} est H et R^{4e} est H ;
un composé de formule (50) dans laquelle R³ est N(CH₂CH₂OMe)Et, R^{4a} est Me, R^{4b} est H, R^{4c} est OMe, R^{4d} est H et R^{4e} est H ;
un composé de formule (50) dans laquelle R³ est N(CH₂CH₂OMe)Pr, R^{4a} est Me, R^{4b} est H, R^{4c} est OMe, R^{4d} est H et R^{4e} est H ;
un composé de formule (50) dans laquelle R³ est N(CH₂CH₂OMe)-CH₂cPr, R^{4a} est Me, R^{4b} est H, R^{4c} est OMe, R^{4d} est H et R^{4e} est H ;
un composé de formule (50) dans laquelle R³ est NHCH(CH₃)CH₂CH₃, R^{4a} est Me, R^{4b} est H, R^{4c} est OMe, R^{4d} est H et R^{4e} est H ;
un composé de formule (50) dans laquelle R³ est NHCH(cPr)₂, R^{4a} est Me, R^{4b} est H, R^{4c} est OMe, R^{4d} est H et R^{4e} est H ;
un composé de formule (50) dans laquelle R³ est 2-5-1-yle, R^{4a} est Me, R^{4b} est H, R^{4c} est OMe, R^{4d} est Me et R^{4e} est H ;
un composé de formule (50) dans laquelle R³ est cyclobutylamino, R^{4a} est Me, R^{4b} est H, R^{4c} est OMe, R^{4d} est Me et R^{4e} est H ;
un composé de formule (50) dans lequel R³ est N(Me)CH₂CH=CH₂, R^{4a} est Me, R^{4b} est H, R^{4c} est OMe, R^{4d} est Me et R^{4e} est H ;
un composé de formule (50) dans lequel R³ est N (Et)CH₂CH=CH₂, R^{4a} est Me, R^{4b} est H, R^{4c} est OMe, R^{4d} est Me et R^{4e} est H ;
un composé de formule (50) dans laquelle R³ est N(Me)CH₂cPr, R^{4a} est Me, R^{4b} est H, R^{4c} est OMe, R^{4d} est Me et R^{4e} est H ;
un composé de formule (50) dans laquelle R³ est N(Et)CH₂cPr, R^{4a} est Me, R^{4b} est H, R^{4c} est OMe, R^{4d} est Me et R^{4e} est H ;
un composé de formule (50) dans laquelle R³ est N(Pr)CH₂cPr, R^{4a} est Me, R^{4b} est H, R^{4c} est OMe, R^{4d} est Me et R^{4e} est H ;
un composé de formule (50) dans laquelle R³ est N(Me)Pr, R^{4a} est Me, R^{4b} est H, R^{4c} est OMe, R^{4d} est Me et R^{4e} est H ;
un composé de formule (50) dans laquelle R³ est N(Me)Et, R^{4a} est Me, R^{4b} est H, R^{4c} est OMe, R^{4d} est Me et R^{4e} est H ;
un composé de formule (50) dans laquelle R³ est N(Me)Bu, R^{4a} est Me, R^{4b} est H, R^{4c} est OMe, R^{4d} est Me et R^{4e} est H ;
un composé de formule (50) dans laquelle R³ est N(Me)propargyle, R^{4a} est Me, R^{4b} est H, R^{4c} est OMe, R^{4d} est Me et R^{4e} est H ;
un composé de formule (50) dans laquelle R³ est N(Et)propargyle, R^{4a} est Me, R^{4b} est H, R^{4c} est OMe, R^{4d} est Me et R^{4e} est H ;
un composé de formule (50) dans laquelle R³ est NHCH(CH₃)CH(CH₃)CH₃, R^{4a} est Me, R^{4b} est H, R^{4c} est OMe, R^{4d} est Me et R^{4e} est H ;
un composé de formule (50) dans laquelle R³ est N(CH₂CH₂OMe)-CH₂CH=CH₂, R^{4a} est Me, R^{4b} est H, R^{4c} est OMe, R^{4d} est Me et R^{4e} est H ;
un composé de formule (50) dans laquelle R³ est N(CH₂CH₂OMe)Me, R^{4a} est Me, R^{4b} est H, R^{4c} est OMe, R^{4d} est Me et R^{4e} est H ;
un composé de formule (50) dans laquelle R³ est N(CH₂CH₂OMe)Et, R^{4a} est Me, R^{4b} est H, R^{4c} est OMe, R^{4d} est Me et R^{4e} est H ;
un composé de formule (50) dans laquelle R³ est N(CH₂CH₂OMe)Pr, R^{4a} est Me, R^{4b} est H, R^{4c} est OMe, R^{4d} est Me et R^{4e} est H ;
un composé de formule (50) dans laquelle R³ est N(CH₂CH₂OMe)-CH₂cPr, R^{4a} est Me, R^{4b} est H, R^{4c} est OMe, R^{4d} est Me et R^{4e} est H ;
un composé de formule (50) dans laquelle R³ est NHCH(CH₃)CH₂CH₃, R^{4a} est Me, R^{4b} est H, R^{4c} est OMe, R^{4d} est Me et R^{4e} est H ;
un composé de formule (50) dans laquelle R³ est NHCH(cPr)₂, R^{4a} est Me, R^{4b} est H, R^{4c} est OMe, R^{4d} est Me et R^{4e} est H ;
un composé de formule (50) dans laquelle R³ est NHCH(Et)₂, R^{4a} est OMe, R^{4b} est H, R^{4c} est OMe, R^{4d} est H et R^{4e} est H ;
un composé de formule (50) dans laquelle R³ est 2-éthylpipérid-1-yle, R^{4a} est OMe, R^{4b} est H, R^{4c} est OMe, R^{4d} est H et R^{4e} est H ;
un composé de formule (50) dans laquelle R³ est cyclobutylamino, R^{4a} est OMe, R^{4b} est H, R^{4c} est OMe, R^{4d} est H et R^{4e} est H ;
un composé de formule (50) dans lequel R³ est N(Me)CH₂CH=CH₂, R^{4a} est OMe, R^{4b} est H, R^{4c} est OMe, R^{4d} est H et R^{4e} est H ;
un composé de formule (50) dans lequel R³ est N (Et)CH₂CH=CH₂, R^{4a} est OMe, R^{4b} est H, R^{4c} est OMe, R^{4d} est H et R^{4e} est H ;
un composé de formule (50) dans laquelle R³ est N(Me)CH₂cPr, R^{4a} est OMe, R^{4b} est H, R^{4c} est OMe, R^{4d} est H et R^{4e} est H ;
un composé de formule (50) dans laquelle R³ est N(Et)CH₂cPr, R^{4a} est OMe, R^{4b} est H, R^{4c} est OMe, R^{4d} est H et R^{4e} est H ;
un composé de formule (50) dans laquelle R³ est N(Pr)CH₂cPr, R^{4a} est OMe, R^{4b} est H, R^{4c} est OMe, R^{4d} est H et R^{4e} est H ;
un composé de formule (50) dans laquelle R³ est N(Me)Pr, R^{4a} est OMe, R^{4b} est H, R^{4c} est OMe, R^{4d} est H et R^{4e} est H ;
un composé de formule (50) dans laquelle R³ est N(Me)Et, R^{4a} est OMe, R^{4b} est H, R^{4c} est OMe, R^{4d} est H et R^{4e} est H ;
un composé de formule (50) dans laquelle R³ est N(Me)Bu, R^{4a} est OMe, R^{4b} est H, R^{4c} est OMe, R^{4d} est H et R^{4e} est H ;
un composé de formule (50) dans laquelle R³ est N(Me)propargyle, R^{4a} est OMe, R^{4b} est H, R^{4c} est OMe, R^{4d} est H et R^{4e} est H ;
un composé de formule (50) dans laquelle R³ est N(Et)propargyle, R^{4a} est OMe, R^{4b} est H, R^{4c} est OMe, R^{4d} est H et R^{4e} est H ;
un composé de formule (50) dans laquelle R³ est NHCH(CH₃)CH(CH₃)CH₃, R^{4a} est OMe, R^{4b} est H, R^{4c} est OMe, R^{4d} est H et R^{4e} est H ;
un composé de formule (50) dans laquelle R³ est N(CH₂CH₂OMe)-CH₂CH=CH₂, R^{4a} est OMe, R^{4b} est H, R^{4c} est OMe, R^{4d} est H et R^{4e} est H ;
un composé de formule (50) dans laquelle R³ est N(CH₂CH₂OMe)Me, R^{4a} est OMe, R^{4b} est H, R^{4c} est OMe, R^{4d} est H et R^{4e} est H ;
un composé de formule (50) dans laquelle R³ est N(CH₂CH₂OMe)Et, R^{4a} est OMe, R^{4b} est H, R^{4c} est OMe, R^{4d} est H et R^{4e} est H ;
un composé de formule (50) dans laquelle R³ est N(CH₂CH₂OMe)Pr, R^{4a} est OMe, R^{4b} est H, R^{4c} est OMe, R^{4d} est H et R^{4e} est H ;
un composé de formule (50) dans laquelle R³ est N(CH₂CH₂OMe)-CH₂cPr, R^{4a} est OMe, R^{4b} est H, R^{4c} est OMe, R^{4d} est H et R^{4e} est H ;
un composé de formule (50) dans laquelle R³ est NHCH(CH₃)CH₂CH₃, R^{4a} est OMe, R^{4b} est H, R^{4c} est OMe, R^{4d} est H et R^{4e} est H ;
un composé de formule (50) dans laquelle R³ est NHCH(cPr)₂, R^{4a} est OMe, R^{4b} est H, R^{4c} est OMe, R^{4d} est H et R^{4e} est H ;
un composé de formule (50) dans laquelle R³ est N(CH₂CH₂OMe)₂, R^{4a} est OMe, R^{4b} est H, R^{4c} est OMe, R^{4d} est H et R^{4e} est H ;
un composé de formule (50) dans laquelle R³ est NHCH(Et)₂, R^{4a} est OMe, R^{4b} est H, R^{4c} est OMe, R^{4d} est Me et R^{4e} est H ;
un composé de formule (50) dans laquelle R³ est N(Et)₂, R^{4a} est OMe, R^{4b} est H, R^{4c} est OMe, R^{4d} est Me et R^{4e} est H ;
un composé de formule (50) dans laquelle R³ est 2-éthylpipérid-1-yle, R^{4a} est OMe, R^{4b} est H, R^{4c} est OMe, R^{4d} est Me et R^{4e} est H ;
un composé de formule (50) dans laquelle R³ est cyclobutylamino, R^{4a} est OMe, R^{4b} est H, R^{4c} est OMe, R^{4d} est Me et R^{4e} est H ;
un composé de formule (50) dans lequel R³ est N(Me)CH₂CH=CH₂, R^{4a} est OMe, R^{4b} est H, R^{4c} est OMe, R^{4d} est Me et R^{4e} est H ;
un composé de formule (50) dans lequel R³ est N (Et)CH₂CH=CH₂, R^{4a} est OMe, R^{4b} est H, R^{4c} est OMe, R^{4d} est Me et R^{4e} est H ;
un composé de formule (50) dans laquelle R³ est N(Me)CH₂cPr, R^{4a} est OMe, R^{4b} est H, R^{4c} est OMe, R^{4d} est Me et R^{4e} est H ;
un composé de formule (50) dans laquelle R³ est N(Et)CH₂cPr, R^{4a} est OMe, R^{4b} est H, R^{4c} est OMe, R^{4d} est Me et R^{4e} est H ;
un composé de formule (50) dans laquelle R³ est N(Pr)CH₂cPr, R^{4a} est OMe, R^{4b} est H, R^{4c} est OMe, R^{4d} est Me et R^{4e} est H ;
un composé de formule (50) dans laquelle R³ est N(Me)Pr, R^{4a} est OMe, R^{4b} est H, R^{4c} est OMe, R^{4d} est Me et R^{4e} est H ;
un composé de formule (50) dans laquelle R³ est N(Me)Et, R^{4a} est OMe, R^{4b} est H, R^{4c} est OMe, R^{4d} est Me et R^{4e} est H ;
un composé de formule (50) dans laquelle R³ est N(Me)Bu, R^{4a} est OMe, R^{4b} est H, R^{4c} est OMe, R^{4d} est Me et R^{4e} est H ;
un composé de formule (50) dans laquelle R³ est N(Me)propargyle, R^{4a} est OMe, R^{4b} est H, R^{4c} est OMe, R^{4d} est Me et R^{4e} est H ;
un composé de formule (50) dans laquelle R³ est N(Et)propargyle, R^{4a} est OMe, R^{4b} est H, R^{4c} est OMe, R^{4d} est Me et R^{4e} est H ;
un composé de formule (50) dans laquelle R³ est NHCH(CH₃)CH(CH₃)CH₃, R^{4a} est OMe, R^{4b} est H, R^{4c} est OMe, R^{4d} est Me et R^{4e} est H ;
un composé de formule (50) dans laquelle R³ est N(CH₂CH₂OMe)-CH₂CH=CH₂, R^{4a} est OMe, R^{4b} est H, R^{4c} est OMe, R^{4d} est Me et R^{4e} est H ;
un composé de formule (50) dans laquelle R³ est N(CH₂CH₂OMe)Me, R^{4a} est OMe, R^{4b} est H, R^{4c} est OMe, R^{4d} est Me et R^{4e} est H ;
un composé de formule (50) dans laquelle R³ est N (CH₂CH₂OMe) Et, R^{4a} est OMe, R^{4b} est H, R^{4c} est OMe, R^{4d} est Me et R^{4e} est H ;
un composé de formule (50) dans laquelle R³ est N(CH₂CH₂OMe)Pr, R^{4a} est OMe, R^{4b} est H, R^{4c} est OMe, R^{4d} est Me et R^{4e} est H ;
un composé de formule (50) dans laquelle R³ est N(CH₂CH₂OMe)-CH₂cPr, R^{4a} est OMe, R^{4b} est H, R^{4c} est OMe, R^{4d} est Me et R^{4e} est H ;
un composé de formule (50) dans laquelle R³ est NHCH(CH₃)CH₂CH₃, R^{4a} est OMe, R^{4b} est H, R^{4c} est OMe, R^{4d} est Me et R^{4e} est H ;
un composé de formule (50) dans laquelle R³ est NHCH(cPr)₂, R^{4a} est OMe, R^{4b} est H, R^{4c} est OMe, R^{4d} est Me et R^{4e} est H ;
un composé de formule (50) dans laquelle R³ est N(CH₂CH₂OMe)₂, R^{4a} est OMe, R^{4b} est H, R^{4c} est OMe, R^{4d} est Me et R^{4e} est H ;
un composé de formule (50) dans laquelle R³ est NHCH(Et)₂, R^{4a} est OMe, R^{4b} est H, R^{4c} est OMe, R^{4d} est Me et R^{4e} est H ;
un composé de formule (50) dans laquelle R³ est N(Et)₂, R^{4a} est OMe, R^{4b} est H, R^{4c} est OMe, R^{4d} est Me et R^{4e} est H ;
un composé de formule (50) dans laquelle R³ est 2-éthylpipérid-1-yle, R^{4a} est Me, R^{4b} est H, R^{4c} est OMe, R^{4d} est H et R^{4e} est Me ;
un composé de formule (50) dans laquelle R³ est cyclobutylamino, R^{4a} est Me, R^{4b} est H, R^{4c} est OMe, R^{4d} est H et R^{4e} est Me ;
un composé de formule (50) dans lequel R³ est N(Me)CH₂CH=CH₂, R^{4a} est Me, R^{4b} est H, R^{4c} est OMe, R^{4d} est H et R^{4e} est Me ;
un composé de formule (50) dans lequel R³ est N(Et)CH₂CH=CH₂, R^{4a} est Me, R^{4b} est H, R^{4c} est OMe, R^{4d} est H et R^{4e} est Me ;
un composé de formule (50) dans laquelle R³ est N(Me)CH₂cPr, R^{4a} est Me, R^{4b} est H, R^{4c} est OMe, R^{4d} est H et R^{4e} est Me ;
un composé de formule (50) dans laquelle R³ est N(Et)CH₂cPr, R^{4a} est Me, R^{4b} est H, R^{4c} est OMe, R^{4d} est H et R^{4e} est Me ;
un composé de formule (50) dans laquelle R³ est N(Pr)CH₂cPr, R^{4a} est Me, R^{4b} est H, R^{4c} est OMe, R^{4d} est H et R^{4e} est Me ;
un composé de formule (50) dans laquelle R³ est N(Me)Pr, R^{4a} est Me, R^{4b} est H, R^{4c} est OMe, R^{4d} est H et R^{4e} est Me ;
un composé de formule (50) dans laquelle R³ est N(Me)Et, R^{4a} est Me, R^{4b} est H, R^{4c} est OMe, R^{4d} est H et R^{4e} est Me ;
un composé de formule (50) dans laquelle R³ est N(Me)Bu, R^{4a} est Me, R^{4b} est H, R^{4c} est OMe, R^{4d} est H et R^{4e} est Me ;
un composé de formule (50) dans laquelle R³ est N(Me)propargyle, R^{4a} est Me, R^{4b} est H, R^{4c} est OMe, R^{4d} est H et R^{4e} est Me ;
un composé de formule (50) dans laquelle R³ est N(Et)propargyle, R^{4a} est Me, R^{4b} est H, R^{4c} est OMe, R^{4d} est H et R^{4e} est Me ;
un composé de formule (50) dans laquelle R³ est NHCH (CH₃) CH (CH₃) CH₃, R^{4a} est Me, R^{4b} est H, R^{4c} est OMe, R^{4d} est H et R^{4e} est Me ;
un composé de formule (50) dans laquelle R³ est N (CH₂CH₂OMe) -CH₂CH=CH₂, R^{4a} est Me, R^{4b} est H, R^{4c} est OMe, R^{4d} est H et R^{4e} est Me ;
un composé de formule (50) dans laquelle R³ est N(CH₂CH₂OMe)Me, R^{4a} est Me, R^{4b} est H, R^{4c} est OMe, R^{4d} est H et R^{4e} est Me ;
un composé de formule (50) dans laquelle R³ est N(CH₂CH₂OMe)Et, R^{4a} est Me, R^{4b} est H, R^{4c} est OMe, R^{4d} est H et R^{4e} est Me ;
un composé de formule (50) dans laquelle R³ est N(CH₂CH₂OMe)Pr, R^{4a} est Me, R^{4b} est H, R^{4c} est OMe, R^{4d} est H et R^{4e} est Me ;
un composé de formule (50) dans laquelle R³ est N(CH₂CH₂OMe)-CH₂cPr, R^{4a} est Me, R^{4b} est H, R^{4c} est OMe, R^{4a} est H et R^{4e} est Me ;
un composé de formule (50) dans laquelle R³ est NHCH(CH₃)CH₂CH₃, R^{4a} est Me, R^{4b} est H, R^{4c} est OMe, R^{4d} est H et R^{4e} est Me ;
un composé de formule (50) dans laquelle R³ est NHCH(Et)₂, R^{4a} est Me, R^{4b} est H, R^{4c} est OMe, R^{4d} est H et R^{4e} est Me ;
un composé de formule (50) dans laquelle R³ est NHCH(cPr)₂, R^{4a} est Me, R^{4b} est H, R^{4c} est OMe, R^{4d} est H et R^{4e} est Me ;
un composé de formule (50) dans laquelle R³ est NHCH(Et)₂, R^{4a} est Me, R^{4b} est H, R^{4c} est OMe, R^{4d} est H et R^{4e} est OMe ;
un composé de formule (50) dans laquelle R³ est 2-éthylpipérid-1-yle, R^{4a} est Me, R^{4b} est H, R^{4c} est OMe, R^{4d} est H et R^{4e} est OMe ;
un composé de formule (50) dans laquelle R³ est cyclobutylamino, R^{4a} est Me, R^{4b} est H, R^{4c} est OMe, R^{4d} est H et R^{4e} est OMe ;
un composé de formule (50) dans lequel R³ est N(Me)CH₂CH=CH₂, R^{4a} est Me, R^{4b} est H, R^{4c} est OMe, R^{4d} est H et R^{4e} est OMe ;
un composé de formule (50) dans lequel R³ est N(Et)CH₂CH=CH₂, R^{4a} est Me, R^{4b} est H, R^{4c} est OMe, R^{4d} est H et R^{4e} est OMe ;
un composé de formule (50) dans laquelle R³ est N(Me)CH₂cPr, R^{4a} est Me, R^{4b} est H, R^{4c} est OMe, R^{4d} est H et R^{4e} est OMe ;
un composé de formule (50) dans laquelle R³ est N(Et)CH₂cPr, R^{4a} est Me, R^{4b} est H, R^{4c} est OMe, R^{4d} est H et R^{4e} est OMe ;
un composé de formule (50) dans laquelle R³ est N(Pr)CH₂cPr, R^{4a} est Me, R^{4b} est H, R^{4c} est OMe, R^{4d} est H et R^{4e} est OMe ;
un composé de formule (50) dans laquelle R³ est N(Me)Pr, R^{4a} est Me, R^{4b} est H, R^{4c} est OMe, R^{4d} est H et R^{4e} est OMe ;
un composé de formule (50) dans laquelle R³ est N(Me)Et, R^{4a} est Me, R^{4b} est H, R^{4c} est OMe, R^{4d} est H et R^{4e} est OMe ;
un composé de formule (50) dans laquelle R³ est N(Me)Bu, R^{4a} est Me, R^{4b} est H, R^{4c} est OMe, R^{4d} est H et R^{4e} est OMe ;
un composé de formule (50) dans laquelle R³ est N(Me)propargyle, R^{4a} est Me, R^{4b} est H, R^{4c} est OMe, R^{4d} est H et R^{4e} est OMe ;
un composé de formule (50) dans laquelle R³ est N (Et) propargyle, R^{4a} est Me, R^{4b} est H, R^{4c} est OMe, R^{4d} est H et R^{4e} est OMe ;
un composé de formule (50) dans laquelle R³ est NHCH(CH₃)CH(CH₃)CH₃, R^{4a} est Me, R^{4b} est H, R^{4c} est OMe, R^{4d} est H et R^{4e} est OMe ;
un composé de formule (50) dans laquelle R³ est N(CH₂CH₂OMe)-CH₂CH=CH₂, R^{4a} est Me, R^{4b} est H, R^{4c} est OMe, R^{4d} est H et R^{4e} est OMe ;
un composé de formule (50) dans laquelle R³ est N(CH₂CH₂OMe)Me, R^{4a} est Me, R^{4b} est H, R^{4c} est OMe, R^{4d} est H et R^{4e} est OMe ;
un composé de formule (50) dans laquelle R³ est N(CH₂CH₂OMe)Et, R^{4a} est Me, R^{4b} est H, R^{4c} est OMe, R^{4d} est H et R^{4e} est OMe ;
un composé de formule (50) dans laquelle R³ est N(CH₂CH₂OMe)Pr, R^{4a} est Me, R^{4b} est H, R^{4c} est OMe, R^{4d} est H et R^{4e} est OMe ;
un composé de formule (50) dans laquelle R³ est N(CH₂CH₂OMe)-CH₂cPr, R^{4a} est Me, R^{4b} est H, R^{4c} est OMe, R^{4d} est H et R^{4e} est OMe ;
un composé de formule (50) dans laquelle R³ est NHCH(CH₃)CH₂CH₃, R^{4a} est Me, R^{4b} est H, R^{4c} est OMe, R^{4d} est H et R^{4e} est OMe ;
un composé de formule (50) dans laquelle R³ est NHCH(cPr)₂, R^{4a} est Me, R^{4b} est H, R^{4c} est OMe, R^{4d} est H et R^{4e} est OMe ;
un composé de formule (50) dans laquelle R³ est NHCH(Et)₂, R^{4a} est Cl, R^{4b} est H, R^{4c} est OMe, R^{4d} est H et R^{4e} est OMe ;
un composé de formule (50) dans laquelle R³ est 2-éthylpipérid-1-yle, R^{4a} est Cl, R^{4b} est H, R^{4c} est OMe, R^{4d} est H et R^{4e} est OMe ;
un composé de formule (50) dans laquelle R³ est cyclobutylamino, R^{4a} est Cl, R^{4b} est H, R^{4c} est OMe, R^{4d} est H et R^{4e} est OMe ;
un composé de formule (50) dans lequel R³ est N(Me)CH₂CH=CH₂, R^{4a} est Cl, R^{4b} est H, R^{4c} est OMe, R^{4d} est H et R^{4e} est OMe ;
un composé de formule (50) dans lequel R³ est N(Et)CH₂CH=CH₂, R^{4a} est Cl, R^{4b} est H, R^{4c} est OMe, R^{4d} est H et R^{4e} est OMe ;
un composé de formule (50) dans laquelle R³ est N(Me)CH₂cPr, R^{4a} est Cl, R^{4b} est H, R^{4c} est OMe, R^{4d} est H et R^{4e} est OMe ;
un composé de formule (50) dans laquelle R³ est N(Et)CH₂cPr, R^{4a} est Cl, R^{4b} est H, R^{4c} est OMe, R^{4d} est H et R^{4e} est OMe ;
un composé de formule (50) dans laquelle R³ est N(Pr)CH₂CPr, R^{4a} est Cl, R^{4b} est H, R^{4c} est OMe, R^{4d} est H et R^{4e} est OMe ;
un composé de formule (50) dans laquelle R³ est N(Me)Pr, R^{4a} est Cl, R^{4b} est H, R^{4c} est OMe, R^{4d} est H et R^{4e} est OMe ;
un composé de formule (50) dans laquelle R³ est N(Me)Et, R^{4a} est Cl, R^{4b} est H, R^{4c} est OMe, R^{4d} est H et R^{4e} est OMe ;
un composé de formule (50) dans laquelle R³ est N(Me)Bu, R^{4a} est Cl, R^{4b} est H, R^{4c} est OMe, R^{4d} est H et R^{4e} est OMe ;
un composé de formule (50) dans laquelle R³ est N(Me)propargyle, R^{4a} est Cl, R^{4b} est H, R^{4c} est OMe, R^{4d} est H et R^{4e} est OMe ;
un composé de formule (50) dans laquelle R³ est N (Et)propargyle, R^{4a} est Cl, R^{4b} est H, R^{4c} est OMe, R^{4d} est H et R^{4e} est OMe ;
un composé de formule (50) dans laquelle R³ est NHCH(CH₃)CH(CH₃)CH₃, R^{4a} est Cl, R^{4b} est H, R^{4c} est OMe, R^{4d} est H et R^{4e} est OMe ;
un composé de formule (50) dans laquelle R³ est N (CH₂CH₂OMe)-CH₂CH=CH₂, R^{4a} est Cl, R^{4b} est H, R^{4c} est OMe, R^{4a} est H et R^{4e} est OMe ;
un composé de formule (50) dans laquelle R³ est N(CH₂CH₂OMe)Me, R^{4a} est Cl, R^{4b} est H, R^{4c} est OMe, R^{4d} est H et R^{4e} est OMe ;
un composé de formule (50) dans laquelle R³ est N (CH₂CH₂OMe)Et, R^{4a} est Cl, R^{4b} est H, R^{4c} est OMe, R^{4d} est H et R^{4e} est OMe ;
un composé de formule (50) dans laquelle R³ est N (CH₂CH₂OMe) Pr, R^{4a} est Cl, R^{4b} est H, R^{4c} est OMe, R^{4d} est H et R^{4e} est OMe ;
un composé de formule (50) dans laquelle R³ est N(CH₂CH₂OMe)-CH₂cPr, R^{4a} est Cl, R^{4b} est H, R^{4c} est OMe, R^{4d} est H et R^{4e} est OMe ;
un composé de formule (50) dans laquelle R³ est NHCH (CH₃) CH₂CH₃, R^{4d} est Cl, R^{4b} est H, R^{4c} est OMe, R^{4d} est H et R^{4e} est OMe ;
un composé de formule (50) dans laquelle R³ est NHCH(cPr)₂, R^{4a} est Cl, R^{4b} est H, R^{4c} est OMe, R^{4d} est H et R^{4e} est OMe ;
un composé de formule (50) dans laquelle R³ est N (CH₂CH₂OMe)₂, R^{4a} est Cl, R^{4b} est H, R^{4c} est OMe, R^{4d} est H et R^{4e} est OMe ;
un composé de formule (50) dans laquelle R³ est NHCH(Et)₂, R^{4a} est Cl, R^{4b} est H, R^{4c} est OMe, R^{4d} est H et R^{4e} est OMe ;
un composé de formule (50) dans laquelle R³ est N(Et)₂, R^{4a} est Cl, R^{4b} est H, R^{4c} est OMe, R^{4d} est H et R^{4e} est OMe ;
un composé de formule (50) dans lequel R³ est NHCH(Et)₂, R^{4a} est Cl, R^{4b} est H, R^{4c} est OMe, R^{4d} est H et R^{4e} est H ;
un composé de formule (50) dans laquelle R³ est éthylpipérid-1-yle, R^{4a} est Cl, R^{4b} est H, R^{4c} est OMe, R^{4d} est H et R^{4e} est H ;
un composé de formule (50) dans laquelle R³ est cyclobutylamino, R^{4a} est Cl, R^{4b} est H, R^{4c} est OMe, R^{4d} est H et R^{4e} est H ;
un composé de formule (50) dans lequel R³ est N(Me)CH₂CH=CH₂, R^{4a} est Cl, R^{4b} est H, R^{4c} est OMe, R^{4d} est H et R^{4e} est H ;
un composé de formule (50) dans lequel R³ est N(Et)CH₂CH=CH₂, R^{4a} est Cl, R^{4b} est H, R^{4c} est OMe, R^{4d} est H et R^{4e} est H ;
un composé de formule (50) dans laquelle R³ est N(Me)CH₂cPr, R^{4a} est Cl, R^{4b} est H, R^{4c} est OMe, R^{4d} est H et R^{4e} est H ;
un composé de formule (50) dans laquelle R³ est N(Et)CH₂cPr, R^{4a} est Cl, R^{4b} est H, R^{4c} est OMe, R^{4d} est H et R^{4e} est H ;
un composé de formule (50) dans laquelle R³ est N(Pr)CH₂cPr, R^{4a} est Cl, R^{4b} est H, R^{4c} est OMe, R^{4d} est H et R^{4e} est H ;
un composé de formule (50) dans laquelle R³ est N(Me)Pr, R^{4a} est Cl, R^{4b} est H, R^{4c} est OMe, R^{4d} est H et R^{4e} est H ;
un composé de formule (50) dans laquelle R³ est N(Me)Et, R^{4a} est Cl, R^{4b} est H, R^{4c} est OMe, R^{4d} est H et R^{4e} est H ;
un composé de formule (50) dans laquelle R³ est N(Me)Bu, R^{4a} est Cl, R^{4b} est H, R^{4c} est OMe, R^{4d} est H et R^{4e} est H ;
un composé de formule (50) dans laquelle R³ est N(Me)propargyle, R^{4a} est Cl, R^{4b} est H, R^{4c} est OMe, R^{4d} est H et R^{4e} est H ;
un composé de formule (50) dans laquelle R³ est N (Et) propargyle, R^{4a} est Cl, R^{4b} est H, R^{4c} est OMe, R^{4d} est H et R^{4e} est H ;
un composé de formule (50) dans laquelle R³ est NHCH(CH₃)CH(CH₃)CH₃, R^{4a} est Cl, R^{4b} est H, R^{4c} est OMe, R^{4d} est H et R^{4e} est H ;
un composé de formule (50) dans laquelle R³ est N(CH₂CH₂OMe)-CH₂CH=CH₂, R^{4a} est Cl, R^{4b} est H, R^{4c} est OMe, R^{4d} est H et R^{4e} est H ;
un composé de formule (50) dans laquelle R³ est N(CH₂CH₂OMe)Me, R^{4a} est Cl, R^{4b} est H, R^{4c} est OMe, R^{4d} est H et R^{4e} est H ;
un composé de formule (50) dans laquelle R³ est N(CH₂CH₂OMe)Et, R^{4a} est Cl, R^{4b} est H, R^{4c} est OMe, R^{4d} est H et R^{4e} est H ;
un composé de formule (50) dans laquelle R³ est N(CH₂CH₂OMe)Pr, R^{4a} est Cl, R^{4b} est H, R^{4c} est OMe, R^{4d} est H et R^{4e} est H ;
un composé de formule (50) dans laquelle R³ est N(CH₂CH₂OMe)-CH₂cPr, R^{4a} est Cl, R^{4b} est H, R^{4c} est OMe, R^{4d} est H et R^{4e} est H ;
un composé de formule (50) dans laquelle R³ est NHCH(CH₃)CH₂CH₃, R^{4a} est Cl, R^{4b} est H, R^{4c} est OMe, R^{4d} est H et R^{4e} est H ;
un composé de formule (50) dans laquelle R³ est NHCH(cPr)₂, R^{4a} est Cl, R^{4b} est H, R^{4c} est OMe, R^{4d} est H et R^{4e} est H ;
un composé de formule (50) dans laquelle R³ est N(CH₂CH₂OMe)₂, R^{4a} est Cl, R^{4b} est H, R^{4c} est OMe, R^{4d} est H et R^{4e} est H ;
un composé de formule (50) dans laquelle R³ est NHCH(Et)₂, R^{4a} est Cl, R^{4b} est H, R^{4c} est OMe, R^{4d} est H et R^{4e} est H ;
un composé de formule (50) dans lequel R³ est NHCH(Et)₂, R^{4a} est Cl, R^{4b} est H, R^{4c} est OMe, R^{4d} est F et R^{4e} est H ;
un composé de formule (50) dans laquelle R³ est éthylpipérid-1-yle, R^{4a} est Cl, R^{4b} est H, R^{4c} est OMe, R^{4d} est F et R^{4e} est H ;
un composé de formule (50) dans laquelle R³ est cyclobutylamino, R^{4a} est Cl, R^{4b} est H, R^{4c} est OMe, R^{4d} est F et R^{4e} est H ;
un composé de formule (50) dans lequel R³ est N(Me)CH₂CH=CH₂, R^{4a} est Cl, R^{4b} est H, R^{4c} est OMe, R^{4d} est F et R^{4e} est H ;
un composé de formule (50) dans lequel R³ est N(Et)CH₂CH=CH₂, R^{4a} est Cl, R^{4b} est H, R^{4c} est OMe, R^{4d} est F et R^{4e} est H ;
un composé de formule (50) dans laquelle R³ est N(Me)CH₂cPr, R^{4a} est Cl, R^{4b} est H, R^{4c} est OMe, R^{4d} est F et R^{4e} est H ;
un composé de formule (50) dans laquelle R³ est N(Et)CH₂cPr, R^{4a} est Cl, R^{4b} est H, R^{4c} est OMe, R^{4d} est F et R^{4e} est H ;
un composé de formule (50) dans laquelle R³ est N(Pr)CH₂cPr, R^{4a} est Cl, R^{4b} est H, R^{4c} est OMe, R^{4d} est F et R^{4e} est H ;
un composé de formule (50) dans laquelle R³ est N(Me)Pr, R^{4a} est Cl, R^{4b} est H, R^{4c} est OMe, R^{4d} est F et R^{4e} est H ;
un composé de formule (50) dans laquelle R³ est N(Me)Et, R^{4a} est Cl, R^{4b} est H, R^{4c} est OMe, R^{4d} est F et R^{4e} est H ;
un composé de formule (50) dans laquelle R³ est N(Me)Bu, R^{4a} est Cl, R^{4b} est H, R^{4c} est OMe, R^{4d} est F et R^{4e} est H ;
un composé de formule (50) dans laquelle R³ est N (Me) propargyle, R^{4a} est Cl, R^{4b} est H, R^{4c} est OMe, R^{4d} est F et R^{4e} est H ;
un composé de formule (50) dans laquelle R³ est NHCH(CH₃)CH(CH₃)CH₃, R^{4a} est Cl, R^{4b} est H, R^{4c} est OMe, R^{4d} est F et R^{4e} est H ;
un composé de formule (50) dans laquelle R³ est N (CH₂CH₂OMe) -CH₂CH=CH₂, R^{4a} est Cl, R^{4b} est H, R^{4c} est OMe, R^{4d} est F et R^{4e} est H ;
un composé de formule (50) dans laquelle R³ est N(CH₂CH₂OMe)Me, R^{4a} est Cl, R^{4b} est H, R^{4c} est OMe, R^{4d} est F et R^{4e} est H ;
un composé de formule (50) dans laquelle R³ est N(CH₂CH₂OMe)Et, R^{4a} est Cl, R^{4b} est H, R^{4c} est OMe, R^{4d} est F et R^{4e} est H ;
un composé de formule (50) dans laquelle R³ est N(CH₂CH₂OMe)Pr, R^{4a} est Cl, R^{4b} est H, R^{4c} est OMe, R^{4d} est F et R^{4e} est H ;
un composé de formule (50) dans laquelle R³ est N(CH₂CH₂OMe)-CH₂cPr, R^{4a} est Cl, R^{4b} est H, R^{4c} est OMe, R^{4d} est F et R^{4e} est H ;
un composé de formule (50) dans laquelle R³ est NHCH(CH₃)CH₂CH₃, R^{4a} est Cl, R^{4b} est F, R^{4c} est OMe, R^{4d} est H et R^{4e} est H ;
un composé de formule (50) dans laquelle R³ est NHCH(cPr)₂, R^{4a} est Cl, R^{4b} est H, R^{4c} est OMe, R^{4d} est F et R^{4e} est H ;
un composé de formule (50) dans laquelle R³ est N(CH₂CH₂OMe)₂, R^{4a} est Cl, R^{4b} est H, R^{4c} est OMe, R^{4d} est F et R^{4e} est H ;
un composé de formule (50) dans laquelle R³ est NHCH(Et)₂, R^{4a} est Cl, R^{4b} est H, R^{4c} est OMe, R^{4d} est F et R^{4e} est H ;
un composé de formule (50) dans laquelle R³ est N(Et)₂, R^{4a} est Cl, R^{4b} est H, R^{4c} est OMe, R^{4d} est F et R^{4e} est H ;
un composé de formule (50) dans laquelle R³ est NHCH(Et)₂, R^{4a} est Cl, R^{4b} est H, R^{4c} est OMe, R^{4d} est OMe et R^{4e} est H ;
un composé de formule (50) dans laquelle R³ est éthylpipérid-1-yle, R^{4a} est Cl, R^{4b} est H, R^{4c} est OMe, R^{4d} est OMe et R^{4e} est H ;
un composé de formule (50) dans laquelle R³ est cyclobutylamino, R^{4a} est Cl, R^{4b} est H, R^{4c} est OMe, R^{4d} est OMe et R^{4e} est H ;
un composé de formule (50) dans lequel R³ est N(Me)CH₂CH=CH₂, R^{4a} est Cl, R^{4b} est H, R^{4c} est OMe, R^{4d} est OMe et R^{4e} est H ;
un composé de formule (50) dans lequel R³ est N(Et)CH₂CH=CH₂, R^{4a} est Cl, R^{9b} est H, R^{4c} est OMe, R^{4d} est OMe et R^{4e} est H ;
un composé de formule (50) dans laquelle R³ est N(Me)CH₂cPr, R^{4a} est Cl, R^{4b} est H, R^{4c} est OMe, R^{4d} est OMe et R^{4e} est H ;
un composé de formule (50) dans laquelle R³ est N(Et)CH₂cPr, R^{4a} est Cl, R^{4b} est H, R^{4c} est OMe, R^{4d} est OMe et R^{4e} est H ;
un composé de formule (50) dans laquelle R³ est N(Pr)CH₂CPr, R^{4a} est Cl, R^{4b} est H, R^{4c} est OMe, R^{4d} est OMe et R^{4e} est H ;
un composé de formule (50) dans laquelle R³ est N(Me)Pr, R^{4a} est Cl, R^{4b} est H, R^{4c} est OMe, R^{4d} est OMe et R^{4e} est H ;
un composé de formule (50) dans laquelle R³ est N(Me)Et, R^{4a} est Cl, R^{4b} est H, R^{4c} est OMe, R^{4d} est OMe et R^{4e} est H ;
un composé de formule (50) dans laquelle R³ est N(Me)Bu, R^{4a} est Cl, R^{4b} est H, R^{4c} est OMe, R^{4d} est OMe et R^{4e} est H ;
un composé de formule (50) dans laquelle R³ est N(Me)propargyle, R^{4a} est Cl, R^{4b} est H, R^{4c} est OMe, R^{4d} est OMe et R^{4e} est H ;
un composé de formule (50) dans laquelle R³ est NHCH(CH₃)CH(CH₃)CH₃, R^{4a} est Cl, R^{4b} est H, R^{4c} est OMe, R^{4d} est OMe et R^{4e} est H ;
un composé de formule (50) dans laquelle R³ est N (CH₂CH₂OMe) -CH₂CH=CH₂, R^{4a} est Cl, R^{4b} est H, R^{4c} est OMe, R^{4d} est OMe et R^{4e} est H ;
un composé de formule (50) dans laquelle R³ est N(CH₂CH₂OMe)Me, R^{4a} est Cl, R^{4b} est H, R^{4c} est OMe, R^{4d} est OMe et R^{4e} est H ;
un composé de formule (50) dans laquelle R³ est N(CH₂CH₂OMe)Et, R^{4a} est Cl, R^{4b} est H, R^{4c} est OMe, R^{4d} est OMe et R^{4e} est H ;
un composé de formule (50) dans laquelle R³ est N(CH₂CH₂OMe)Pr, R^{4a} est Cl, R^{4b} est H, R^{4c} est OMe, R^{4d} est OMe et R^{4e} est H ;
un composé de formule (50) dans laquelle R³ est N(CH₂CH₂OMe)-CH₂cPr, R^{4a} est Cl, R^{4b} est H, R^{4c} est OMe, R^{4d} est OMe et R^{4e} est H ;
un composé de formule (50) dans laquelle R³ est NHCH(CH₃)CH₂CH₃, R^{4a} est Cl, R^{4b} est H, R^{4c} est OMe, R^{4d} est OMe et R^{4e} est H ;
un composé de formule (50) dans laquelle R³ est NHCH(cPr)₂, R^{4a} est Cl, R^{4b} est H, R^{4c} est OMe, R^{4d} est OMe et R^{4e} est H ;
un composé de formule (50) dans laquelle R³ est N(CH₂CH₂OMe)₂, R^{4a} est Cl, R^{4b} est H, R^{4c} est OMe, R^{4d} est OMe et R^{4e} est H ;
un composé de formule (50) dans laquelle R³ est NHCH(Et)₂, R^{4a} est Cl, R^{4b} est H, R^{4c} est OMe, R^{4d} est OMe et R^{4e} est H ;
un composé de formule (50) dans laquelle R³ est N(Et)₂, R^{4a} est Cl, R^{4b} est H, R^{4c} est OMe, R^{4d} est OMe et R^{4e} est H ;
un composé de formule (50) dans laquelle R³ est NHCH(Et)₂, R^{4a} est Br, R^{4b} est H, R^{4c} est OMe, R^{4d} est OMe et R^{4e} est H ;
un composé de formule (50) dans laquelle R³ est éthylpipérid-1-yle, R^{4a} est Br, R^{4b} est H, R^{4c} est OMe, R^{4d} est OMe et R^{4e} est H ;
un composé de formule (50) dans laquelle R³ est cyclobutylamino, R^{4a} est Br, R^{4b} est H, R^{4c} est OMe, R^{4d} est OMe et R^{4e} est H ;
un composé de formule (50) dans lequel R³ est N(Me)CH₂CH=CH₂, R^{4a} est Br, R^{4b} est H, R^{4c} est OMe, R^{4d} est OMe et R^{4e} est H ;
un composé de formule (50) dans lequel R³ est N(Et)CH₂CH=CH₂, R^{4a} est Br, R^{4b} est H, R^{4c} est OMe, R^{4d} est OMe et R^{4e} est H ;
un composé de formule (50) dans laquelle R³ est N(Me)CH₂cPr, R^{4a} est Br, R^{4b} est H, R^{4c} est OMe, R^{4d} est OMe et R^{4e} est H ;
un composé de formule (50) dans laquelle R³ est N(Et)CH₂cPr, R^{4a} est Br, R^{4b} est H, R^{4c} est OMe, R^{4d} est OMe et R^{4e} est H ;
un composé de formule (50) dans laquelle R³ est N(Pr)CH₂cPr, R^{4a} est Br, R^{4b} est H, R^{4c} est OMe, R^{4d} est OMe et R^{4e} est H ;
un composé de formule (50) dans laquelle R³ est N(Me)Pr, R^{4a} est Br, R^{4b} est H, R^{4c} est OMe, R^{4d} est OMe et R^{4e} est H ;
un composé de formule (50) dans laquelle R³ est N(Me)Et, R^{4a} est Br, R^{4b} est H, R^{4c} est OMe, R^{4d} est OMe et R^{4e} est H ;
un composé de formule (50) dans laquelle R³ est N(Me)Bu, R^{4a} est Br, R^{4b} est H, R^{4c} est OMe, R^{4d} est OMe et R^{4e} est H ;
un composé de formule (50) dans laquelle R³ est N(Me)propargyle, R^{4a} est Br, R^{4b} est H, R^{4c} est OMe, R^{4d} est OMe et R^{4e} est H ;
un composé de formule (50) dans laquelle R³ est NHCH(CH₃)CH(CH₃)CH₃, R^{4a} est Br, R^{4b} est H, R^{4c} est OMe, R^{4d} est OMe et R^{4e} est H ;
un composé de formule (50) dans laquelle R³ est N (CH₂CH₂OMe) -CH₂CH=CH₂, R^{4a} est Br, R^{4b} est H, R^{4c} est OMe, R^{4d} est OMe et R^{4e} est H ;
un composé de formule (50) dans laquelle R³ est N(CH₂CH₂OMe)Me, R^{4a} est Br, R^{4b} est H, R^{4c} est OMe, R^{4d} est OMe et R^{4e} est H ;
un composé de formule (50) dans laquelle R³ est N (CH₂CH₂OMe) Et, R^{4a} est Br, R^{4b} est H, R^{4c} est OMe, R^{4d} est OMe et R^{4e} est H ;
un composé de formule (50) dans laquelle R³ est N(CH₂CH₂OMe)Pr, R^{4a} est Br, R^{4b} est H, R^{4c} est OMe, R^{4d} est OMe et R^{4e} est H ;
un composé de formule (50) dans laquelle R³ est N(CH₂CH₂OMe)-CH₂cPr, R^{4a} est Br, R^{4b} est H, R^{4c} est OMe, R^{4d} est OMe et R^{4e} est H ;
un composé de formule (50) dans laquelle R³ est NHCH(CH₃)CH₂CH₃, R^{4a} est Br, R^{4b} est H, R^{4c} est OMe, R^{4d} est OMe et R^{4e} est H ;
un composé de formule (50) dans laquelle R³ est NHCH(cPr)₂, R^{4a} est Br, R^{4b} est H, R^{4c} est OMe, R^{4d} est OMe et R^{4e} est H ;
un composé de formule (50) dans laquelle R³ est N(CH₂CH₂OMe)₂, R^{4a} est Br, R^{4b} est H, R^{4c} est OMe, R^{4d} est OMe et R^{4e} est H ;
un composé de formule (50) dans laquelle R³ est NHCH(Et)₂, R^{4a} est Br, R^{4b} est H, R^{4c} est OMe, R^{4d} est OMe et R^{4e} est H ;
un composé de formule (50) dans laquelle R³ est NHCH(Et)₂, R^{4a} est Me, R^{4b} est H, R^{4c} est OMe, R^{4d} est OMe et R^{4e} est H ;
un composé de formule (50) dans laquelle R³ est éthylpipérid-1-yle, R^{4a} est Me, R^{4b} est H, R^{4c} est OMe, R^{4d} est OMe et R^{4e} est H ;
un composé de formule (50) dans laquelle R³ est cyclobutylamino, R^{4a} est Me, R^{4b} est H, R^{4c} est OMe, R^{4d} est OMe et R^{4e} est H ;
un composé de formule (50) dans lequel R³ est N(Me)CH₂CH=CH₂, R^{4a} est Me, R^{4b} est H, R^{4c} est OMe, R^{4d} est OMe et R^{4e} est H ;
un composé de formule (50) dans lequel R³ est N(Et)CH₂CH=CH₂, R^{4a} est Me, R^{4b} est H, R^{4c} est OMe, R^{4d} est OMe et R^{4e} est H ;
un composé de formule (50) dans laquelle R³ est N(Me)CH₂cPr, R^{4a} est Me, R^{4b} est H, R^{4c} est OMe, R^{4d} est OMe et R^{4e} est H ;
un composé de formule (50) dans laquelle R³ est N(Et)CH₂cPr, R^{4a} est Me, R^{4b} est H, R^{4c} est OMe, R^{4d} est OMe et R^{4e} est H ;
un composé de formule (50) dans laquelle R³ est N(Pr)CH₂cPr, R^{4a} est Me, R^{4b} est H, R^{4c} est OMe, R^{4d} est OMe et R^{4e} est H ;
un composé de formule (50) dans laquelle R³ est N(Me)Pr, R^{4a} est Me, R^{4b} est H, R^{4c} est OMe, R^{4d} est OMe et R^{4e} est H ;
un composé de formule (50) dans laquelle R³ est N(Me)Et, R^{4a} est Me, R^{4b} est H, R^{4c} est OMe, R^{4d} est OMe et R^{4e} est H ;
un composé de formule (50) dans laquelle R³ est N(Me)Bu, R^{4a} est Me, R^{4b} est H, R^{4c} est OMe, R^{4d} est OMe et R^{4e} est H ;
un composé de formule (50) dans laquelle R³ est N(Me)propargyle, R^{4a} est Me, R^{4b} est H, R^{4c} est OMe, R^{4d} est OMe et R^{4e} est H ;
un composé de formule (50) dans laquelle R³ est NHCH(CH₃)CH(CH₃)CH₃, R^{4a} est Me, R^{4b} est H, R^{4c} est OMe, R^{4d} est OMe et R^{4e} est H ;
un composé de formule (50) dans laquelle R³ est N (CH₂CH₂OMe) -CH₂CH=CH₂, R^{4a} est Me, R^{4b} est H, R^{4c} est OMe, R^{4d} est OMe et R^{4e} est H ;
un composé de formule (50) dans laquelle R³ est N(CH₂CH₂OMe)Me, R^{4a} est Me, R^{4b} est H, R^{4c} est OMe, R^{4d} est OMe et R^{4e} est H ;
un composé de formule (50) dans laquelle R³ est N(CH₂CH₂OMe)Et, R^{4a} est Me, R^{4b} est H, R^{4c} est OMe, R^{4d} est OMe et R^{4e} est H ;
un composé de formule (50) dans laquelle R³ est N(CH₂CH₂OMe)Pr, R^{4a} est Me, R^{4b} est H, R^{4c} est OMe, R^{4d} est OMe et R^{4e} est H ;
un composé de formule (50) dans laquelle R³ est N(CH₂CH₂OMe)-CH₂cPr, R^{4a} est Me, R^{4b} est H, R^{4c} est OMe, R^{4d} est OMe et R^{4e} est H ;
un composé de formule (50) dans laquelle R³ est NHCH(CH₃)CH₂CH₃, R^{4a} est Me, R^{4b} est H, R^{4c} est OMe, R^{4d} est OMe et R^{4e} est H ;
un composé de formule (50) dans laquelle R³ est NHCH(cPr)₂, R^{4a} est Me, R^{4b} est H, R^{4c} est OMe, R^{4d} est OMe et R^{4e} est H ;
un composé de formule (50) dans laquelle R³ est N(CH₂CH₂OMe)₂, R^{4a} est Me, R^{4b} est H, R^{4c} est OMe, R^{4d} est OMe et R^{4e} est H ;
un composé de formule (50) dans laquelle R³ est NHCH(Et)₂, R^{4a} est Me, R^{4b} est H, R^{4c} est OMe, R^{4d} est OMe et R^{4e} est H ;
un composé de formule (50) dans laquelle R³ est NHCH(Et)₂, R^{4a} est Me, R^{4b} est H, R^{4c} est OMe, R^{4d} est OMe et R^{4e} est H ;
un composé de formule (50) dans laquelle R³ est N(Et)₂, R^{4a} est Me, R^{4b} est H, R^{4c} est OMe, R^{4d} est OMe et R^{4e} est H.

2. Composé de formule (60) : 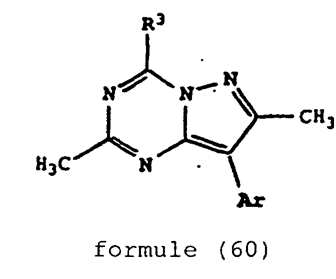 et ses isomères géométriques, ses formes stéréoisomères, ou des mélanges de ses formes stéréoisomères, et ses formes sels acceptables en pharmacie, choisis dans l'ensemble suivant :
un composé de formule (60) dans laquelle R³ est NHCH(Et)₂, Ar est 6-diméthylamino-4-méthylpyrid-3-yle ;
un composé de formule (60) dans laquelle R³ est 2-éthylpipérid-1-yle, Ar est 6-diméthylamino-4-méthylpyrid-3-yle ;
un composé de formule (60) dans laquelle R³ est cyclobutylamino, Ar est 6-diméthylamino-4-méthylpyrid-3-yle ;
un composé de formule (60) dans laquelle R³ est N(Me)CH₂CH=CH₂, Ar est 6-diméthylamino-4-méthylpyrid-3-yle ;
un composé de formule (60) dans laquelle R³ est N(Et)CH₂cPr, Ar est 6-diméthylamino-4-méthylpyrid-3-yle ;
un composé de formule (60) dans laquelle R³ est N(Pr)CH₂cPr, Ar est 6-diméthylamino-4-méthylpyrid-3-yle ;
un composé de formule (60) dans laquelle R³ est N(Me)Pr, Ar est 6-diméthylamino-4-méthylpyrid-3-yle ;
un composé de formule (60) dans laquelle R³ est N(Me)Et, Ar est 6-diméthylamino-4-méthylpyrid-3-yle ;
un composé de formule (60) dans laquelle R³ est N(Me)Bu, Ar est 6-diméthylamino-4-méthylpyrid-3-yle ;
un composé de formule (60) dans laquelle R³ est N(Me)propargyle, Ar est 6-diméthylamino-4-méthylpyrid-3-yle ;
un composé de formule (60) dans laquelle R³ est N(Et)propargyle, Ar est 6-diméthylamino-4-méthylpyrid-3-yle ;
un composé de formule (60) dans laquelle R³ est NHCH(CH₃)CH(CH₃)CH₃, Ar est 6-diméthylamino-4-méthylpyrid-3-yle ;
un composé de formule (60) dans laquelle R³ est N(CH₂CH₂OMe)-CH₂CH=CH₂, Ar est 6-diméthylamino-4-méthylpyrid-3-yle ;
un composé de formule (60) dans laquelle R³ est N(CH₂CH₂OMe)Me, Ar est 6-diméthylamino-4-méthylpyrid-3-yle ;
un composé de formule (60) dans laquelle R³ est N(CH₂CH₂OMe)Et, Ar est 6-diméthylamino-4-méthylpyrid-3-yle ;
un composé de formule (60) dans laquelle R³ est N(CH₂CH₂OMe)Pr, Ar est 6-diméthylamino-4-méthylpyrid-3-yle ;
un composé de formule (60) dans laquelle R³ est N(CH₂CH₂OMe)-CH₂cPr, Ar est 6-diméthylamino-4-méthylpyrid-3-yle ;
un composé de formule (60) dans laquelle R³ est NHCH(CH₃)CH₂CH₃, Ar est 6-diméthylamino-4-méthylpyrid-3-yle ;
un composé de formule (60) dans laquelle R³ est NHCH(cPr)₂, Ar est 6-diméthylamino-4-méthylpyrid-3-yle ;
un composé de formule (60) dans laquelle R³ est N(CH₂CH₂OMe)₂, Ar est 6-diméthylamino-4-méthylpyrid-3-yle ;
un composé de formule (60) dans laquelle R³ est NHCH(Et)₂, Ar est 6-diméthylamino-4-méthylpyrid-3-yle ;
un composé de formule (60) dans laquelle R³ est N(Et)₂, Ar est 6-diméthylamino-4-méthylpyrid-3-yle ;
un composé de formule (60) dans laquelle R³ est 2-éthylpipérid-1-yle, Ar est 6-diméthylamino-4-méthylpyrid-3-yle ;
un composé de formule (60) dans laquelle R³ est cyclobutylamino, Ar est 6-diméthylamino-4-méthylpyrid-3-yle ;
un composé de formule (60) dans laquelle R³ est N(Me)CH₂CH=CH₂, Ar est 6-diméthylamino-4-méthylpyrid-3-yle ;
un composé de formule (60) dans laquelle R³ est N(Et)CH₂cPr, Ar est 6-diméthylamino-4-méthylpyrid-3-yle ;
un composé de formule (60) dans laquelle R³ est N(Pr)CH₂cPr, Ar est 6-diméthylamino-4-méthylpyrid-3-yle ;
un composé de formule (60) dans laquelle R³ est N(Me)Pr, Ar est 6-diméthylamino-4-méthylpyrid-3-yle ;
un composé de formule (60) dans laquelle R³ est N(Me)Et, Ar est 6-diméthylamino-4-méthylpyrid-3-yle ;
un composé de formule (60) dans laquelle R³ est N(Me)Bu, Ar est 6-diméthylamino-4-méthylpyrid-3-yle ;
un composé de formule (60) dans laquelle R³ est N(Me)propargyle, Ar est 6-diméthylamino-4-méthylpyrid-3-yle ;
un composé de formule (60) dans laquelle R³ est NHCH(CH₃)CH(CH₃)CH₃, Ar est 6-diméthylamino-4-méthylpyrid-3-yle ;
un composé de formule (60) dans laquelle R³ est N(CH₂CH₂OMe)-CH₂CH=CH₂, Ar est 6-diméthylamino-4-méthylpyrid-3-yle ;
un composé de formule (60) dans laquelle R³ est N(CH₂CH₂OMe)Me, Ar est 6-diméthylamino-4-méthylpyrid-3-yle ;
un composé de formule (60) dans laquelle R³ est N(CH₂CH₂OMe)Et, Ar est 6-diméthylamino-4-méthylpyrid-3-yle ;
un composé de formule (60) dans laquelle R³ est N(CH₂CH₂OMe)Pr, Ar est 6-diméthylamino-4-méthylpyrid-3-yle ;
un composé de formule (60) dans laquelle R³ est N(CH₂CH₂OMe)-CH₂cPr, Ar est 6-diméthylamino-4-méthylpyrid-3-yle ;
un composé de formule (60) dans laquelle R³ est NHCH(CH₃)CH₂CH₃, Ar est 6-diméthylamino-4-méthylpyrid-3-yle ;
un composé de formule (60) dans laquelle R³ est NHCH(cPr)₂, Ar est 6-diméthylamino-4-méthylpyrid-3-yle ;
un composé de formule (60) dans laquelle R³ est N(CH₂CH₂OMe)₂, Ar est 6-diméthylamino-4-méthylpyrid-3-yle ;
un composé de formule (60) dans laquelle R³ est NHCH(Et)₂, Ar est 6-diméthylamino-4-méthylpyrid-3-yle ;
un composé de formule (60) dans laquelle R³ est N(Et)₂, Ar est 6-diméthylamino-4-méthylpyrid-3-yle ;
un composé de formule (60) dans laquelle R³ est 2-éthylpipérid-1-yle, Ar est 6-méthoxy-4-méthylpyrid-3-yle ;
un composé de formule (60) dans laquelle R³ est cyclobutylamino, Ar est 6-méthoxy-4-méthylpyrid-3-yle ;
un composé de formule (60) dans laquelle R³ est N(Me)CH₂CH=CH₂, Ar est 6-méthoxy-4-méthylpyrid-3-yle ;
un composé de formule (60) dans laquelle R³ est N(Et)CH₂cPr, Ar est 6-méthoxy-4-méthylpyrid-3-yle ;
un composé de formule (60) dans laquelle R³ est N(Pr)CH₂cPr, Ar est 6-méthoxy-4-méthylpyrid-3-yle ;
un composé de formule (60) dans laquelle R³ est N(Me)Pr, Ar est 6-méthoxy-4-méthylpyrid-3-yle ;
un composé de formule (60) dans laquelle R³ est N(Me)Et, Ar est 6-méthoxy-4-méthylpyrid-3-yle ;
un composé de formule (60) dans laquelle R³ est N(Me)Bu, Ar est 6-méthoxy-4-méthylpyrid-3-yle ;
un composé de formule (60) dans laquelle R³ est N(Me)propargyle, Ar est 6-méthoxy-4-méthylpyrid-3-yle ;
un composé de formule (60) dans laquelle R³ est N(Et)propargyle, Ar est 6-méthoxy-4-méthylpyrid-3-yle ;
un composé de formule (60) dans laquelle R³ est NH(CH(CH₃)CH(CH₃)CH₃, Ar est 6-méthoxy-4-méthylpyrid-3-yle ;
un composé de formule (60) dans laquelle R³ est N (CH₂CH₂OMe)-CH₂CH=CH₂, Ar est 6-méthoxy-4-méthylpyrid-3-yle ;
un composé de formule (60) dans laquelle R³ est N(CH₂CH₂OMe)Me, Ar est 6-méthoxy-4-méthylpyrid-3-yle ;
un composé de formule (60) dans laquelle R³ est N(CH₂CH₂OMe)Et, Ar est 6-méthoxy-4-méthylpyrid-3-yle ;
un composé de formule (60) dans laquelle R³ est N(CH₂CH₂OMe)Pr, Ar est 6-méthoxy-4-méthylpyrid-3-yle ;
un composé de formule (60) dans laquelle R³ est N(CH₂CH₂OMe)-CH₂cPr, Ar est 6-méthoxy-4-méthylpyrid-3-yle ;
un composé de formule (60) dans laquelle R³ est NH(CH(CH₃)CH₂CH₃,, Ar est 6-méthoxy-4-méthylpyrid-3-yle ;
un composé de formule (60) dans laquelle R³ est NHCH(cPr)₂, Ar est 6-méthoxy-4-méthylpyrid-3-yle ;
un composé de formule (60) dans laquelle R³ est N(CH₂CH₂OMe)₂, Ar est 6-méthoxy-4-méthylpyrid-3-yle ;
un composé de formule (60) dans laquelle R³ est NHCH(Et)₂, Ar est 6-méthoxy-4-méthylpyrid-3-yle ;
un composé de formule (60) dans laquelle R³ est N(Et)₂, Ar est 6-méthoxy-4-méthylpyrid-3-yle ;
un composé de formule (60) dans laquelle R³ est 2-éthylpipérid-1-yle, Ar est 4-méthoxy-6-méthylpyrid-3-yle ;
un composé de formule (60) dans laquelle R³ est cyclobutylamino, Ar est 4-méthoxy-6-méthylpyrid-3-yle ;
un composé de formule (60) dans laquelle R³ est N(Me)CH₂CH=CH₂, Ar est 4-méthoxy-6-méthylpyrid-3-yle ;
un composé de formule (60) dans laquelle R³ est N(Et)CH₂cPr, Ar est 4-méthoxy-6-méthylpyrid-3-yle ;
un composé de formule (60) dans laquelle R³ est N(Pr)CH₂cPr, Ar est 4-méthoxy-6-méthylpyrid-3-yle ;
un composé de formule (60) dans laquelle R³ est N(Me)Pr, Ar est 4-méthoxy-6-méthylpyrid-3-yle ;
un composé de formule (60) dans laquelle R³ est N(Me)Et, Ar est 4-méthoxy-6-méthylpyrid-3-yle ;
un composé de formule (60) dans laquelle R³ est N(Me)Bu, Ar est 4-méthoxy-6-méthylpyrid-3-yle ;
un composé de formule (60) dans laquelle R³ est N(Me)propargyle, Ar est 4-méthoxy-6-méthylpyrid-3-yle ;
un composé de formule (60) dans laquelle R³ est NH(CH(CH₃)CH(CH₃)CH₃, Ar est 4-méthoxy-6-méthylpyrid-3-yle ;
un composé de formule (60) dans laquelle R³ est N(CH₂CH₂OMe)-CH₂CH=CH₂, Ar est 4-méthoxy-6-méthylpyrid-3-yle ;
un composé de formule (60) dans laquelle R³ est N(CH₂CH₂OMe)Me, Ar est 4-méthoxy-6-méthylpyrid-3-yle ;
un composé de formule (60) dans laquelle R³ est N(CH₂CH₂OMe)Et, Ar est 4-méthoxy-6-méthylpyrid-3-yle ;
un composé de formule (60) dans laquelle R³ est N(CH₂CH₂OMe)Pr, Ar est 4-méthoxy-6-méthylpyrid-3-yle ;
un composé de formule (60) dans laquelle R³ est N(CH₂CH₂OMe)-CH₂cPr, Ar est 4-méthoxy-6-méthylpyrid-3-yle ;
un composé de formule (60) dans laquelle R³ est NHCH(CH₃)CH₂CH₃, Ar est 4-méthoxy-6-méthylpyrid-3-yle ;
un composé de formule (60) dans laquelle R³ est NHCH(cPr)₂, Ar est 4-méthoxy-6-méthylpyrid-3-yle ;
un composé de formule (60) dans laquelle R³ est N(CH₂CH₂OMe)₂, Ar est 4-méthoxy-6-méthylpyrid-3-yle ;
un composé de formule (60) dans laquelle R³ est NHCH(Et)₂, Ar est 6-méthoxy-4-méthylpyrid-3-yle ;
un composé de formule (60) dans laquelle R³ est N(Et)₂, Ar est 4-méthoxy-6-méthylpyrid-3-yle ;
un composé de formule (60) dans laquelle R³ est 2-éthylpipérid-1-yle, Ar est 4,6-diméthylpyrid-3-yle ;
un composé de formule (60) dans laquelle R³ est cyclobutylamino, Ar est 4,6-diméthylpyrid-3-yle ;
un composé de formule (60) dans laquelle R³ est N(Me)CH₂CH=CH₂, Ar est 4,6-diméthylpyrid-3-yle ;
un composé de formule (60) dans laquelle R³ est N(Et)CH₂cPr, Ar est 4,6-diméthylpyrid-3-yle ;
un composé de formule (60) dans laquelle R³ est N(Pr)CH₂cPr, Ar est 4,6-diméthylpyrid-3-yle ;
un composé de formule (60) dans laquelle R³ est N(Me)Pr, Ar est 4,6-diméthylpyrid-3-yle ;
un composé de formule (60) dans laquelle R³ est N(Me)Et, Ar est 4,6-diméthylpyrid-3-yle ;
un composé de formule (60) dans laquelle R³ est N(Me)Bu, Ar est 4,6-diméthylpyrid-3-yle ;
un composé de formule (60) dans laquelle R³ est N(Me)propargyle, Ar est 4,6-diméthylpyrid-3-yle ;
un composé de formule (60) dans laquelle R³ est N(Et)propargyle, Ar est 4,6-diméthylpyrid-3-yle ;
un composé de formule (60) dans laquelle R³ est NHCH(CH₃)CH(CH₃)CH₃, Ar est 4,6-diméthylpyrid-3-yle ;
un composé de formule (60) dans laquelle R³ est N(CH₂CH₂OMe)-CH₂CH=CH₂, Ar est 4,6-diméthylpyrid-3-yle ;
un composé de formule (60) dans laquelle R³ est N(CH₂CH₂OMe)Me, Ar est 4,6-diméthylpyrid-3-yle ;
un composé de formule (60) dans laquelle R³ est N(CH₂CH₂OMe)Et, Ar est 4,6-diméthylpyrid-3-yle ;
un composé de formule (60) dans laquelle R³ est N(CH₂CH₂OMe)Pr, Ar est 4,6-diméthylpyrid-3-yle ;
un composé de formule (60) dans laquelle R³ est N(CH₂CH₂OMe)-CH₂cPr, Ar est 4,6-diméthylpyrid-3-yle ;
un composé de formule (60) dans laquelle R³ est NHCH(CH₃)CH₂CH₃, Ar est 4,6-diméthylpyrid-3-yle ;
un composé de formule (60) dans laquelle R³ est NHCH(cPr)₂, Ar est 4,6-diméthylpyrid-3-yle ;
un composé de formule (60) dans laquelle R³ est N(CH₂CH₂OMe)₂, Ar est 4,6-diméthylpyrid-3-yle ;
un composé de formule (60) dans laquelle R³ est NHCH(Et)₂, Ar est 4,6-diméthylpyrid-3-yle ;
un composé de formule (60) dans laquelle R³ est N(Et)₂, Ar est 4,6-diméthylpyrid-3-yle ;
un composé de formule (60) dans laquelle R³ est 2-éthylpipérid-1-yle, Ar est 2,6-diméthylpyrid-3-yle ;
un composé de formule (60) dans laquelle R³ est cyclobutylamino, Ar est 2,6-diméthylpyrid-3-yle ;
un composé de formule (60) dans laquelle R³ est N(Me)CH₂CH=CH₂, Ar est 2,6-diméthylpyrid-3-yle ;
un composé de formule (60) dans laquelle R³ est N(Et)CH₂cPr, Ar est 2,6-diméthylpyrid-3-yle ;
un composé de formule (60) dans laquelle R³ est N(Pr)CH₂cPr, Ar est 2,6-diméthylpyrid-3-yle ;
un composé de formule (60) dans laquelle R³ est N(Me)Pr, Ar est 2,6-diméthylpyrid-3-yle ;
un composé de formule (60) dans laquelle R³ est N(Me)Et, Ar est 2,6-diméthylpyrid-3-yle ;
un composé de formule (60) dans laquelle R³ est N(Me)Bu, Ar est 2,6-diméthylpyrid-3-yle ;
un composé de formule (60) dans laquelle R³ est N(Me)propargyle, Ar est 2,6-diméthylpyrid-3-yle ;
un composé de formule (60) dans laquelle R³ est NHCH(CH₃)CH(CH₃)CH₃, Ar est 2,6-diméthylpyrid-3-yle ;
un composé de formule (60) dans laquelle R³ est N(CH₂CH₂OMe)-CH₂CH=CH₂, Ar est 2,6-diméthylpyrid-3-yle ;
un composé de formule (60) dans laquelle R³ est N(CH₂CH₂OMe)Me, Ar est 2,6-diméthylpyrid-3-yle ;
un composé de formule (60) dans laquelle R³ est N(CH₂CH₂OMe)Et, Ar est 2,6-diméthylpyrid-3-yle ;
un composé de formule (60) dans laquelle R³ est N(CH₂CH₂OMe)Pr, Ar est 2,6-diméthylpyrid-3-yle ;
un composé de formule (60) dans laquelle R³ est N(CH₂CH₂OMe)-CH₂cPr, Ar est 2,6-diméthylpyrid-3-yle ;
un composé de formule (60) dans laquelle R³ est NH(CH(CH₃)CH₂CH₃,, Ar est 2,6-diméthylpyrid-3-yle ; et
un composé de formule (60) dans laquelle R³ est NHCH(cPr)₂, Ar est 2,6-diméthylpyrid-3-yle.

3. Composé selon la revendication 1 et ses isomères géométriques, ses formes stéréoisomères, ou des mélanges de ses formes stéréoisomères, et ses formes sels acceptables en pharmacie, ledit composé étant choisi dans l'ensemble suivant :
4-((2-butylamino)-2,7-diméthyl-8-(2-méthyl-4-méthoxyphényl)-[1,5-a]pyrazolo-1,3,5-triazine ;
4-((2-butylamino)-2,7-diméthyl-8-(2,5-diméthyl-4-méthoxyphényl)-[1,5-a]pyrazolo-1,3,5-triazine ;
4-((3-pentylamino)-2,7-diméthyl-8-(2-méthyl-4-méthoxyphényl)-[1,5-a]pyrazolo-1,3,5-triazine ;
4-(N-cyclopropylméthyl-N-propylamino)-2,7-diméthyl-8-(2-méthyl-4-méthoxyphényl)-[1,5-a]pyrazolo-1,3,5-triazine ;
4-(N-cyclopropylméthyl-N-propylamino)-2,7-diméthyl-8-(2,5-diméthyl-4-méthoxyphényl)-[1,5-a]pyrazolo-1,3,5-triazine ;
4-(N-allyl-N-(2-méthoxyéthyl)amino)-2,7-diméthyl-8-(2-méthyl-4-méthoxyphényl)-[1,5-a]pyrazolo-1,3,5-triazine ;
4-(N-allyl-N-(2-méthoxyéthyl)amino)-2,7-diméthyl-8-(2,5-diméthyl-4-méthoxyphényl)-[1,5-a]pyrazolo-1,3,5-triazine ;
4-(N-éthyl-N-(2-méthoxyéthyl)amino)-2,7-diméthyl-8-(2-méthyl-4-méthoxyphényl)-[1,5-a]pyrazolo-1,3,5-triazine ;
4-(N-éthyl-N-(2-méthoxyéthyl)amino)-2,7-diméthyl-8-(2,5-diméthyl-4-méthoxyphényl)-[1,5-a]pyrazolo-1,3,5-triazine ;

4. Composition pharmaceutique comprenant un véhicule acceptable en pharmacie et une quantité efficace, du point de vue thérapeutique, d'un composé de l'une quelconque des revendications 1, 2 et 3.

5. Utilisation d'un composé selon la revendication 1, 2 ou 3 dans la fabrication d'un médicament destiné au traitement d'un trouble affectif, de l'anxiété, de la dépression, des céphalées, du syndrome du côlon irritable, d'un trouble de stress post-traumatique, de la paralysie pseudo-bulbaire, d'une dépression immunitaire, de la maladie d'Alzheimer, de troubles gastro-intestinaux, de l'anorexie mentale ou d'autres troubles de l'alimentation, de la toxicomanie, de symptômes de sevrage médicamenteux ou alcoolique, de maladies inflammatoires, de troubles cardiovasculaires ou cardiaques, de problèmes de fertilité, d'infections par le virus de l'immunodéficience humaine, d'un stress hémorragique, de l'obésité, de l'infertilité, de traumatismes crâniens et de la moelle épinière, de l'épilepsie, de l'ictus, d'ulcères, de la sclérose latérale amyotrophique, de l'hypoglycémie ou d'un trouble dont le traitement peut être effectué ou facilité par l'antagonisme du CRF, y compris, mais sans s'y limiter, des troubles induits ou facilités par le CRF, chez les mammifères.

6. Composé selon la revendication 1, dans lequel R³ est NHCH(Et)₂, R^{4a} est Cl, R^{4b} est H, R^{4c} est OMe, R^{4d} est F et R^{4e} est H.

7. Composition pharmaceutique selon la revendication 4, dans laquelle le composé est un composé de formule (50) dans laquelle R³ est NHCH(Et)₂, R^{4a} est Cl, R^{4b} est H, R^{4c} est OMe, R^{4d} est F et R^{4e} est H.

8. Utilisation d'un composé selon la revendication 5, dans laquelle le composé est un composé de formule (50) dans laquelle R³ est NHCH(Et)₂, R^{4a} est Cl, R^{4b} est H, R^{4c} est OMe, R^{4d} est F et R^{4e} est H.
